# EUROPÄISCHE PATENTSCHRIFT

(11) **EP 1 240 163 B1**
(45) Veröffentlichungstag und Bekanntmachung des Hinweises auf die Patenterteilung: **31.03.2004**
(21) Anmeldenummer: 00991216.3
(22) Anmeldetag: 19.12.2000
(51) Int. Cl.: C07D 413/00, C07D 413/14, A01N 43/80, C07D 413/10

(54) **3-(4,5-DIHYDROISOXAZOL-5-YL)BENZOYLPYRAZOLE**
3-(4,5-DIHYDROISOXAZOLE-5-YL)BENZOYLPYRAZOLE
3-(4,5-DIHYDROISOXAZOL-5-YL)BENZOYLPYRAZOLES

(30) Priorität: 22.12.1999 DE 19962249; 07.03.2000 DE 10010551
(43) Veröffentlichungstag der Anmeldung: 18.09.2002
(73) Patentinhaber: BASF AKTIENGESELLSCHAFT, 67056 Ludwigshafen (DE)
(72) Erfinder: BAUMANN, Ernst, 67373 Dudenhofen (DE); VON DEYN, Wolfgang, 67435 Neustadt (DE); KUDIS, Steffen, 68167 Mannheim (DE); LANGEMANN, Klaus, 34270 Schauenburg (DE); MAYER, Guido, 67161 Gönnheim (DE); MI LITZ, Ulf, 67433 Neustadt (DE); NEIDLEIN, Ulf, 68165 Mannheim (DE); WALTER, Helmut, 67283 Obrigheim (DE); WESTPHALEN, Karl-Otto, 67346 Speyer (DE); WITSCHEL, Matthias, 67098 Bad Dürkheim (DE)
(86) Internationale Anmeldenummer: PCT/EP2000/012950
(87) Internationale Veröffentlichungsnummer: WO 2001/046182

(56) Entgegenhaltungen:
- EP-A- 0 509 433
- EP-A- 0 900 795
- WO-A-96/26206
- WO-A-98/21187
- WO-A-98/31681
- WO-A-98/31682
- WO-A-99/21852

## Beschreibung

Die vorliegende Erfindung betrifft 3-(4,5-Dihydroisoxazol-5-yl)benzoylpyrazole der Formel I 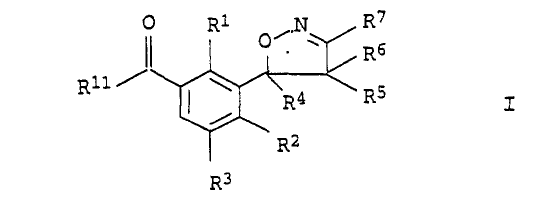 in der die Variablen folgende Bedeutungen haben:
- R¹, R²: Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
- R³: Wasserstoff, Halogen oder C₁-C₆-Alkyl;
- R⁴: Wasserstoff oder C₁-C₄-Alkyl;
- R⁵, R⁶: Wasserstoff, Halogen, Cyano, Nitro, C₁-C₄-Alkyl, C₁-C₄-Alkoxy-C₁-C₄-alkyl, Di-(C₁-C₄-alkoxy)-C₁-C₄-alkyl, Di- (C₁-C₄-alkyl) -amino-C₁-C₄-alkyl, Di- (C₁-C₄-alkyl)-amino-imino-C₁-C₄-alkyl, Hydroxyimino-C₁-C₄-alkyl, C₁-C₆-Alkoxyimino-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl)-amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy oder C₁-C₄-Halogenalkoxy;
oder
- R⁵ und R⁶: bilden gemeinsam eine C₂-C₆-Alkandiyl-Kette, die einbis vierfach durch C₁-C₄-Alkyl substituiert sein kann und/oder durch Sauerstoff oder einen gegebenenfalls C₁-C₄-Alkyl substituierten Stickstoff unterbrochen sein kann;
- R⁷: Halogen, Cyano, Hydroxy, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di-(C₁-C₆-alkyl)amino, Di-(C₁-C₄-alkoxy)methyl, Hydroxyimino-C₁-C₄-alkyl, C₁-C₆-Alkoxyimino-C₁-C₄-alkyl oder COR⁸;
- R⁸: Wasserstoff, C₁-C₄-Alkyl, C₁-C₄-Halogenalkyl, Hydroxy, C₁-C₄-Alkoxy, C₁-C₄-Alkoxy-C₂-C₄-alkoxy, C₁-C₄-Halogenalkoxy, C₃-C₆-Alkenyloxy, C₃-C₆-Alkinyloxy oder NR⁹R¹⁰;
- R⁹: Wasserstoff oder C₁-C₄-Alkyl;
- R¹⁰: C₁-C₄-Alkyl;
- R¹¹: ein in 4-Stellung verknüpftes Pyrazol der Formel II 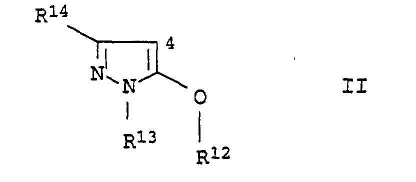 wobei
R¹² Wasserstoff, C₁-C₄-Alkyl, C₁-C₄-Halogenalkyl, C₃-C₆-Alkenyl, C₃-C₄-Alkinyl, C₁-C₄-Alkylcarbonyl, C₁-C₄-Halogenalkylcarbonyl, C₁-C₄-Alkylsulfonyl, C₁-C₄-Halogenalkylsulfonyl, Thienylmethyl, Phenyl, Benzyl, Phenylcarbonyl, Phenylsulfonyl oder Phenylcarbonylmethyl, wobei der Phenylrest der fünf letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;

R¹³ C₁-C₆-Alkyl, C₁-C₆-Halogenalkyl oder C₃-C₆-Cycloalkyl;
R¹⁴ Wasserstoff, C₁-C₆-Alkyl oder C₃-C₆-Cycloalkyl; bedeuten;
sowie deren landwirtschaftlich brauchbaren Salze.

Außerdem betrifft die Erfindung Verfahren und Zwischenprodukte zur Herstellung von Verbindungen der Formel I, Mittel welche diese enthalten sowie die Verwendung dieser Derivate oder diese enthaltende Mittel zur Schadpflanzenbekämpfung.

Aus der Literatur, beispielsweise aus WO 96/26206 sind Pyrazol-4-yl-benzoylderivate bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die 3-(4,5-Dihydroisoxazol-5-yl)benzoylpyrazol der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Ammonium, Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹⁵ oder als Reste an Phenylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cyanoalkyl-, Alkenyl-, Alkinyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, Alkylcarbonyl, Halogenalkylcarbonyl, Alkoxycarbonyl-, Halogenalkoxycarbonyl, Alkenyloxy-, Alkinyloxy-, Alkylamino, Dialkylamino-, Alkoxyalkyl-, Dialkoxymethyl, Dialkoxyalkyl-, Alkylthioalkyl-, Dialkylaminoalkyl-, Dialkylaminoiminoalkyl-, Hydroxyiminoalkyl, Alkoxyiminoalkyl-, Alkoxycarbonylalkyl- und Alkoxyalkoxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl: sowie die Alkylteile von C₁-C₄-Alkylcarbonyl, Di- (C₁-C₄-alkoxy) -C₁-C₄-alkyl, Di- (C₁-C₄-alkyl) -aminoimino-C₁-C₄-alkyl, C₁-C₆-Alkoxyimino-C₁-C₄-alkyl und Hydroxyimino-C₁-C₄-alkyl: z.B. Methyl, Ethyl, Propyl, 1-Methylethyl, Butyl, 1-Methylpropyl, 2-Methylpropyl und 1,1-Dimethylethyl;
- C₁-C₆-Alkyl: C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl sowie die Halogenalkylteile von C₁-C₄-Halogenalkylcarbonyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, Brommethyl, Iodmethyl, 1-Fluorethyl, 1-Chlorethyl, 1-Brommethyl, 1-Iodethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl: C₁-C₄-Halogenalkyl wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Cyanoalkyl: z.B. Cyanomethyl, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 1-Cyanobut-3-yl, 2-Cyanobut-3-yl, 1-Cyano-2-methyl-prop-3-yl, 2-Cyano-2-methylprop-3-yl, 3-Cyano-2-methyl-prop-3-yl und 2-Cyanomethylprop-2-yl;
- C₃-C₄-Alkenyl: z.B. Prop-1-en-1-yl, Prop-2-en-1-yl, 1-Methylethen-1-yl, Buten-1-yl, Buten-2-yl, Buten-3-yl, 1-Methylprop-1-en-1-yl, 2-Methyl-prop-1-en-1-yl, 1-Methylprop-2-en-1-yl oder 2-Methyl-prop-2-en-1-yl;
- C₃-C₄-Alkinyl: z.B. Prop-1-in-1-yl, Prop-2-in-1-yl, But-1-in-1-yl, But-1-in-3-yl, But-1-in-4-yl oder But-2-in-1-yl;
- C₁-C₄-Alkoxy: sowie die Alkoxyteile von Di-(C₁-C₄-alkoxy)methyl und Di-(C₁-C₄-alkoxy)-C₁-C₄-alkyl, z.B. Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy sowie die Alkoxyteile von C₁-C₆-Alkoxyimino-C₁-C₄-alkyl: C₁-C₄-Alkoxy wie voranstehend genannt, sowie z.B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₆-Halogenalkoxy: C₁-C₄-Halogenalkoxy wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy und Dodecafluorhexoxy;
- C₁-C₄-Alkylthio: z.B. Methylthio, Ethylthio, Propylthio, 1-Methylethylthio, Butylthio, 1-Methylpropylthio, 2-Methylpropylthio und 1,1-Dimethylethylthio;
- C₁-C₆-Alkylthio: C₁-C₄-Alkylthio wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
   C₁-C₄-Halogenalkylthio: einen C₁-C₄-Alkylthiorest wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2- chlorethylthio, 1-(Brommethyl)-2-bromethylthio, 4-Fluorbutylthio, 4-Chlorbutylthio, 4-Brombutylthio und Nonafluorbutylthio;
   C₁-C₆-Halogenalkylthio: C₁-C₄-Halogenalkylthio wie voranstehend genannt, sowie z.B. 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio und Dodecafluorhexylthio;
   C₁-C₆-Alkylsulfinyl (C₁-C₆-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
   C₁-C₆-Halogenalkylsulfinyl: C₁-C₆-Alkylsulfinylrest wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl und Dodecafluorhexylsulfinyl;
   C₁-C₄-Alkylsulfonyl (C₁-C₄-Alkyl-S(=O)₂-): z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
   C₁-C₆-Alkylsulfonyl: C₁-C₄-Alkylsulfonyl wie voranstehend genannt, sowie z.B. Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₄-Halogenalkylsulfonyl: einen C₁-C₄-Alkylsulfonylrest wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl und Nonafluorbutylsulfonyl;
- C₁-C₆-Halogenalkylsulfonyl: einen C₁-C₄-Halogenalkylsulfonylrest wie voranstehend genannt, sowie 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;
- C₁-C₄-Alkoxycarbonyl: z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl und 1,1-Dimethylethoxycarbonyl;
- C₁-C₄-Halogenalkoxycarbonyl: einen C₁-C₄-Alkoxycarbonylrest wie voranstehend genannt, der partiell oder vollständig durch Fluor, Chlor, Brom und/oder Iod substituiert ist, also z.B. Fluormethoxycarbonyl, Difluormethoxycarbonyl, Trifluormethoxycarbonyl, Chlordifluormethoxycarbonyl, Bromdifluormethoxycarbonyl, 2-Fluorethoxycarbonyl, 2-Chlorethoxycarbonyl, 2-Bromethoxycarbonyl, 2-Iodethoxycarbonyl, 2,2-Difluorethoxycarbonyl, 2,2,2-Trifluorethoxycarbonyl, 2-Chlor-2-fluorethoxycarbonyl, 2-Chlor-2,2-difluorethoxycarbonyl, 2,2-Dichlor-2-fluorethoxycarbonyl, 2,2,2-Trichlorethoxycarbonyl, Pentafluorethoxycarbonyl, 2-Fluorpropoxycarbonyl, 3-Fluorpropoxycarbonyl, 2-Chlorpropoxycarbonyl, 3-Chlorpropoxycarbonyl, 2-Brompropoxycarbonyl, 3-Brompropoxycarbonyl, 2,2-Difluorpropoxycarbonyl, 2,3-Difluorpropoxycarbonyl, 2,3-Dichlorpropoxycarbonyl, 3,3,3-Trifluorpropoxycarbonyl, 3,3,3-Trichlorpropoxycarbonyl, 2,2,3,3,3-Pentafluorpropoxycarbonyl, Heptafluorpropoxycarbonyl, 1-(Fluormethyl)-2-fluorethoxycarbonyl, 1-(Chlormethyl)-2-chlorethoxycarbonyl, 1-(Brommethyl)-2-bromethoxycarbonyl, 4-Fluorbutoxycarbonyl, 4-Chlorbutoxycarbonyl, 4-Brombutoxycarbonyl und 4-Iodbutoxycarbonyl;
   C₃-C₆-Alkenyloxy: z.B. Prop-1-en-1-yloxy, Prop-2-en-1-yloxy, 1-Methylethenyloxy, Buten-1-yloxy, Buten-2-yloxy, Buten-3-yloxy, 1-Methyl-prop-1-en-1-yloxy, 2-Methyl-prop-1-en-1-yloxy, 1-Methyl-prop-2-en-1-yloxy, 2-Methyl-prop-2-en-1-yloxy, Penten-1-yloxy, Penten-2-yloxy, Penten-3-yloxy, Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methylbut-3-en-1-yloxy, 2-Methyl-but-3-en-1-yloxy, 3-Methylbut-3-en-1-yloxy, 1,1-Dimethyl-prop-2-en-1-yloxy, 1,2-Dimethyl-prop-1-en-1-yloxy, 1,2-Dimethyl-prop-2-en-1-yloxy, 1-Ethyl-prop-1-en-2-yloxy, 1-Ethyl-prop-2-en-1-yloxy, Hex-1-en-1-yloxy, Hex-2-en-1-yloxy, Hex-3-en-1-yloxy, Hex-4-en-1-yloxy, Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloxy, 1-Methyl-pent-2-en-1-yloxy, 2-Methyl-pent-2-en-1-yloxy, 3-Methyl-pent-2-en-1-yloxy, 4-Methylpent-2-en-1-yloxy, 1-Methyl-pent-3-en-1-yloxy, 2-Methylpent-3-en-1-yloxy, 3-Methyl-pent-3-en-1-yloxy, 4-Methylpent-3-en-1-yloxy, 1-Methyl-pent-4-en-1-yloxy, 2-Methylpent-4-en-1-yloxy, 3-Methyl-pent-4-en-1-yloxy, 4-Methylpent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethyl-but-3-en-1-yloxy, 2-Ethylbut-1-en-1-yloxy, 2-Ethyl-but-2-en-1-yloxy, 2-Ethylbut-3-en-1-yloxy, 1,1,2-Trimethyl-prop-2-en-1-yloxy, 1-Ethyl-1-methyl-prop-2-en-1-yloxy, 1-Ethyl-2-methylprop-1-en-1-yloxy und 1-Ethyl-2-methyl-prop-2-en-1-yloxy;
   C₃-C₆-Alkinyloxy: z.B. Prop-1-in-1-yloxy, Prop-2-in-1-yloxy, But-1-in-1-yloxy, But-1-in-3-yloxy, But-1-in-4-yloxy, But-2-in-1-yloxy, Pent-1-in-1-yloxy, Pent-1-in-3-yloxy, Pent-1-in-4-yloxy, Pent-1-in-5-yloxy, Pent-2-in-1-yloxy, Pent-2-in-4-yloxy, Pent-2-in-5-yloxy, 3-Methyl-but-1-in-3-yloxy, 3-Methyl-but-1-in-4-yloxy, Hex-1-in-1-yloxy, Hex-1-in-3-yloxy, Hex-1-in-4-yloxy, Hex-1-in-5-yloxy, Hex-1-in-6-yloxy, Hex-2-in-1-yloxy, Hex-2-in-4-yloxy, Hex-2-in-5-yloxy, Hex-2-in-6-yloxy, Hex-3-in-1-yloxy, Hex-3-in-2-yloxy, 3-Methylpent-1-in-1-yloxy, 3-Methylpent-1-in-3-yloxy, 3-Methyl-pent-1-in-4-yloxy, 3-Methylpent-1-in-5-yloxy, 4-Methyl-pent-1-in-1-yloxy, 4-Methylpent-2-in-4-yloxy und 4-Methylpent-2-in-5-yloxy;
   C₁-C₆-Alkylamino: z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
   Di-(C₁-C₄-alkyl)-amino sowie die Dialkylamino-Teile von Di-(C₁-C₄-alkyl)aminoimino-C₁-C₄-alkyl: z.B. N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethy1-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)-amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl) amino; Di-(C₁-C₆-alkyl)amino: Di-(C₁-C₄-alkyl)amino wie voranstehend genannt, sowie N,N-Dipentylamino, N,N-Dihexylamino, N-Methyl-N-pentylamino, N-Ethyl-N-pentylamino, N-Methyl-N-hexylamino oder N-Ethyl-N-hexylamino.
   Di-(C₁-C₄-alkyl)-amino-C₁-C₄-alkyl: durch Di-(C₁-C₄-alkyl)-amino wie voranstehend genannt substituiertes C₁-C₄-Alkyl, also z.B. N,N-Dimethylaminomethyl, N,N-Diethylaminomethyl, N,N-Dipropylaminomethyl, N,N-Di-(1-methylethyl)aminomethyl, N,N-Dibutylaminomethyl, N,N-Di-(1-methylpropyl)aminomethyl, N,N-Di-(2-methylpropyl)aminomethyl, N,N-Di- (1,1-dimethylethyl)aminomethyl, N-Ethyl-N-methylaminomethyl, N-Methyl-N-propylaminomethyl, N-Methyl-N-(1-methylethyl)aminomethyl, N-Butyl-N-methylaminomethyl, N-Methyl-N- (1-methylpropyl)aminomethyl, N-Methyl-N-(2-methylpropyl)aminomethyl, N-(1,1-Dimethylethyl)-N-methylaminomethyl, N-Ethyl-N-propylaminomethyl, N-Ethyl-N-(1-methylethyl)aminomethyl, N-Butyl-N-ethylaminomethyl, N-Ethyl-N-(1-methylpropyl)aminomethyl, N-Ethyl-N-(2-methylpropyl)aminomethyl, N-Ethyl-N-(1,1-dimethylethyl)aminomethyl, N-(1-Methylethyl)-N-propylaminomethyl, N-Butyl-N-propylaminomethyl, N-(1-Methylpropyl)-N-propylaminomethyl, N-(2-Methylpropyl) -N-propylaminomethyl, N-(1,1-dimethylethyl)-N-propylaminomethyl, N-Butyl-N-(1-methylethyl)aminomethyl, N-(1-Methylethyl)-N-(1-methylpropyl)aminomethyl, N-(1-Methylethyl)-N-(2-methylpropyl)aminomethyl, N-(1,1-Dimethylethyl)-N-(1-methylethyl)aminomethyl, N-Butyl-N-(1-methylpropyl)aminomethyl, N-Butyl-N-(2-methylpropyl)aminomethyl, N-Butyl-N-(1,1-dimethylethyl)aminomethyl, N-(1-Methylpropyl) -N- (2-methylpropyl)aminomethyl, N-(1,1-Dimethylethyl)-N-(1-methylpropyl)aminomethyl, N-(1,1-Dimethylethyl)-N-(2-methylpropyl)aminomethyl, 2-(N,N-Dimethylamino)-ethyl, 2-(N,N-Diethylamino)ethyl, 2-(N,N-Dipropylamino)-ethyl, 2-[N,N-Di(1-methylethyl)amino]ethyl, 2-[N,N-Dibutylamino]ethyl, 2-[N,N-Di-(1-methylpropyl)amino]ethyl, 2-[N,N-Di-(2-methylpropyl)amino]ethyl, 2-[N,N-Di-(1,1-Dimethylethyl)amino]ethyl, 2-[N-Ethyl-N-methylamino]ethyl, 2- [N-Methyl-N-propylamino]ethyl, 2-[N-Methyl-N-(1-methylethyl)amino]ethyl, 2- [N-Butyl-N-methylamino]ethyl, 2-[N-Methyl-N-(1-methylpropyl)amino]ethyl, 2-[N-Methyl-N-(2-methylpropyl)amino]ethyl, 2-[N-(1,1-Dimethylethyl)-N-methylamino]ethyl, 2-[N-Ethyl-N-propylamino]ethyl, 2-[N-Ethyl-N-(1-methylethyl)amino]ethyl, 2-[N-Butyl-N-ethylamino]ethyl, 2-[N-Ethyl-N-(1-methylpropyl)amino]ethyl, 2-[N-Ethyl-N-(2-methylpropyl)amino]ethyl, 2-[N-Ethyl-N-(1,1-dimethylethylamino]ethyl, 2-[N-(1-Methylethyl)-N-propylamino]ethyl, 2-[N-Butyl-N-propylamino]ethyl, 2-[N-(1-Methylpropyl)-N-propylamino]ethyl, 2-[N-(2-Methylpropyl)-N-propylamino]ethyl, 2-[N-(1,1-Dimethylethyl)-N-propylamino]ethyl, 2-[N-Butyl-N-(1-methyl-ethyl)amino]ethyl, 2-[N-(1-Methylethyl)-N-(1-methylpropyl)amino]ethyl, 2-[N-(1-Methylethyl)-N-(2-methylpropyl)amino]ethyl, 2-[N-(1,1-Dimethylethyl)-N-(1-methylethyl)amino]ethyl, 2-[N-Butyl-N-(1-methylpropyl)amino]ethyl, 2- [N-Butyl-N-(2-methylpropyl)amino]ethyl, 2-[N-Butyl-N-(1,1-Dimethylethyl) amino]ethyl, 2-[N-(1-Methylpropyl)-N-(2-methylpropyl)amino]ethyl, 2-[N-(1,1-Dimethylethyl)-N- (1-methylpropyl)amino]ethyl, 2-[N-(1,1-Dimethylethyl)-N-(2-methylpropyl)amino]ethyl, 3-(N,N-Dimethylamino)propyl, 3-(N,N-Diethylamino)propyl, 4-(N,N-Dimethylamino)butyl und 4-(N,N-Diethylamino)butyl;
   C₁-C₄-Alkoxy-C₁-C₄-alkyl: durch C₁-C₄-Alkoxy wie vorstehend genannt substituiertes C₁-C₄-Alkyl, also z.B. für Methoxymethyl, Ethoxymethyl, Propoxymethyl, (1-Methylethoxy)methyl, Butoxymethyl, (1-Methylpropoxy)methyl, (2-Methylpropoxy)methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)ethyl, 2-(2-Methylpropoxy) ethyl, 2-(1,1-Dimethylethoxy)ethyl, 2-(Methoxy)-propyl, 2-(Ethoxy)propyl, 2-(Propoxy)propyl, 2-(1-Methylethoxy)propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy)propyl, 3-(1,1-Dimethylethoxy)propyl, 2-(Methoxy)-butyl, 2-(Ethoxy)butyl, 2-(Propoxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)-butyl, 3-(Propoxy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(Propoxy)butyl, 4-(1-Methylethoxy)butyl, 4-(Butoxy)butyl, 4-(1-Methylpropoxy)butyl, 4-(2-Methylpropoxy)butyl und 4-(1,1-Dimethylethoxy)butyl;
   C₁-C₄-Alkylthio-C₁-C₄-alkyl: durch C₁-C₄-Alkylthio wie vorstehend genannt substituiertes C₁-C₄-Alkyl, also z.B. für Methylthiomethyl, Ethylthiomethyl, Propylthiomethyl, (1-Methylethylthio)methyl, Butylthiomethyl, (1-Methyl-propylthio)methyl, (2-Methyipropylthio)methyl, (1,1-Dimethylethylthio)methyl, 2-Methylthioethyl, 2-Ethylthioethyl, 2-(Propylthio)ethyl, 2-(1-Methylethylthio)ethyl, 2-(Butylthio)ethyl, 2-(1-Methylpropylthio)ethyl, 2-(2-Methylpropylthio)ethyl, 2-(1,1-Dimethylethylthio)ethyl, 2-(Methylthio)propyl, 3-(Methylthio)propyl, 2-(Ethylthio)propyl, 3-(Ethylthio)propyl, 3-(Propylthio)propyl, 3-(Butylthio)propyl, 4-(Methylthio)butyl, 4-(Ethylthio)butyl, 4-(Propylthio)butyl und 4-(Butylthio)butyl;
   C₁-C₄-Alkoxycarbonyl-C₁-C₄-alkyl: durch C₁-C₄-Alkoxycarbonyl substituiertes C₁-C₄-Alkyl, also z.B. für Methoxycarbonylmethyl, Ethoxycarbonylmethyl, Propoxycarbonylmethyl, (1-Methylethoxycarbonyl)methyl, Butoxycarbonylmethyl, (1-Methylpropoxycarbonyl)methyl, (2-Methylpropoxycarbonyl)methyl, (1,1-Dimethylethoxycarbonyl)methyl, 2-(Methoxycarbonyl)ethyl, 2-(Ethoxycarbonyl)ethyl, 2-(Propoxycarbonyl)ethyl, 2-(1-Methylethoxycarbonyl)ethyl, 2-(Butoxycarbonyl) ethyl, 2-(1-Methylpropoxycarbonyl)ethyl, 2-(2-Methylpropoxycarbonyl)ethyl, 2-(1,1-Dimethylethoxycarbonyl)ethyl, 2-(Methoxycarbonyl)propyl, 2-(Ethoxycarbonyl)propyl, 2-(Propoxycarbonyl)propyl, 2-(1-Methylethoxycarbonyl)propyl, 2-(Butoxycarbonyl)propyl, 2-(1-Methylpropoxycarbonyl)propyl, 2-(2-Methylpropoxycarbonyl)propyl, 2-(1,1-Dimethylethoxycarbonyl)propyl, 3-(Methoxycarbonyl)-propyl, 3-(Ethoxycarbonyl)propyl, 3-(Propoxycarbonyl)propyl, 3-(1-Methylethoxycarbonyl)propyl, 3-(Butoxycarbonyl)propyl, 3-(1-Methylpropoxycarbonyl)propyl, 3-(2-Methylpropoxycarbonyl)propyl, 3-(1,1-Dimethylethoxycarbonyl)propyl, 2-(Methoxycarbonyl)butyl, 2-(Ethoxycarbonyl)butyl, 2-(Propoxycarbonyl)butyl, 2-(1-Methylethoxycarbonyl)butyl, 2-(Butoxycarbonyl)butyl, 2-(1-Methylpropoxycarbonyl)butyl, 2-(2-Methylpropoxycarbonyl)butyl, 2-(1,1-Dimethylethoxycarbonyl)butyl, 3-(Methoxycarbonyl)butyl, 3-(Ethoxycarbonyl)butyl, 3-(Propoxycarbonyl)butyl, 3-(1-Methylethoxycarbonyl)butyl, 3-(Butoxycarbonyl)butyl, 3-(1-Methylpropoxycarbonyl)butyl, 3-(2-Methylpropoxycarbonyl)butyl, 3-(1,1-Dimethylethoxycarbonyl)butyl, 4-(Methoxycarbonyl)-butyl, 4-(Ethoxycarbonyl)butyl, 4-(Propoxycarbonyl)butyl, 4-(1-Methylethoxycarbonyl)butyl, 4-(Butoxycarbonyl)butyl, 4-(1-Methylpropoxy)butoxy, 4-(2-Methylpropoxy)butoxy und 4-(1,1-Dimethylethoxycarbonyl)butyl;
   C₁-C₄-Alkoxy-C₂-C₄-alkoxy: durch C₁-C₄-Alkoxy wie vorstehend genannt substituiertes C₂-C₄-Alkoxy, also z.B. für 2-(Methoxy)ethoxy, 2-(Ethoxy)ethoxy, 2-(Propoxy)ethoxy, 2-(1-Methylethoxy)ethoxy, 2-(Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(Propoxy)propoxy, 2-(1-Methylethoxy)propoxy, 2-(Butoxy)propoxy, 2-(1-Methylpropoxy)propoxy, 2-(2-Methylpropoxy)propoxy, 2-(1,1-Dimethylethoxy)propoxy, 3-(Methoxy)-propoxy, 3-(Ethoxy)propoxy, 3-(Propoxy)propoxy, 3-(1-Methylethoxy)propoxy, 3-(Butoxy)propoxy, 3-(1-Methylpropoxy)propoxy, 3-(2-Methylpropoxy)propoxy, 3-(1,1-Dimethylethoxy)propoxy, 2-(Methoxy)butoxy, 2-(Ethoxy)butoxy, 2-(Propoxy)butoxy, 2-(1-Methylethoxy)butoxy, 2-(Butoxy)-butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy)-butoxy, 3-(Propoxy)butoxy, 3-(1-Methylethoxy)butoxy, 3-(Butoxy)butoxy, 3-(1-Methylpropoxy)butoxy, 3-(2-Methylpropoxy)butoxy, 3-(1,1-Dimethylethoxy)butoxy, 4-(Methoxy)-butoxy, 4-(Ethoxy)butoxy, 4-(Propoxy)butoxy, 4-(1-Methylethoxy)butoxy, 4-(Butoxy)butoxy, 4-(1-Methylpropoxy)butoxy, 4-(2-Methylpropoxy)butoxy und 4-(1,1-Dimethylethoxy)butoxy;
   C₂-C₆-Alkandiyl: z.B. Ethan-1,2-diyl, Propan-1,3-diyl, Butan-1,4-diyl, Pentan-1,5-diyl und Hexan-1,6-diyl;
   C₃-C₆-Cycloalkyl: z.B. Cyclopropyl, Cyclobutyl, Cyclopentyl oder Cyclohexyl
   C₃-C₈-Cycloalkyl: C₃-C₆-Cycloalkyl, wie voranstehend genannt, sowie Cycloheptyl oder Cyclooctyl;

Alle Phenylringe sind vorzugsweise unsubstituiert oder tragen ein bis drei Halogenatome und/oder eine Nitrogruppe, einen Cyanorest und/oder einen oder zwei Methyl-, Trifluormethyl-, Methoxy- oder Trifluormethoxysubstituenten.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹, R²: Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
besonders bevorzugt Nitro, Halogen, wie z.B. Chlor und Brom, C₁-C₆-Alkyl, wie z.B. Methyl und Ethyl, C₁-C₆-Alkoxy, wie z.B. Methoxy und Ethoxy, C₁-C₆-Halogenalkyl, wie z.B. Difluormethyl und Trifluormethyl, C₁-C₆-Alkylthio, wie z.B. Methylthio und Ethylthio, C₁-C₆-Alkylsulfinyl, wie z.B. Methylsulfinyl und Ethylsulfinyl, C₁-C₆-Alkylsulfonyl, wie z.B. Methylsulfonyl, Ethylsulfonyl und Propylsulfonyl oder C₁-C₆-Halogenalkylsulfonyl, wie z.B. Trifluormethylsulfonyl und Pentafluorethylsulfonyl;
- R³: Wasserstoff;
- R⁴: Wasserstoff;
- R⁵, R⁶: Wasserstoff, Halogen, Cyano, Nitro, C₁-C₄-Alkyl, C₁-C₄-Alkoxy-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl)-amino, COR⁸, Phenyl oder Benzyl, wobei die beiden letzgenannten Substituenten partiell oder vollständig halogeniert sein können und/oder eine bis drei der folgenden Gruppen tragen können: Nitro, Cyano, C₁-C₄-Alkyl, C₁-C₄-Halogenalkyl, C₁-C₄-Alkoxy oder C₁-C₄-Halogenalkoxy;
oder
- R⁵ und R⁶: bilden gemeinsam eine C₂-C₆-Alkandiyl-Kette, die ein bis vierfach durch C₁-C₄-Alkyl substituiert sein kann und/oder durch Sauerstoff oder einen gegebenenfalls C₁-C₄-Alkyl substituierten Stickstoff unterbrochen sein kann;
besonders bevorzugt steht R⁵ für Wasserstoff, Halogen, Cyano, Nitro, C₁-C₄-Alkyl, C₁-C₄-Alkoxy-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄alkoxy, C₁-C₄-Halogenalkoxy, C₁-C₄-Alkylthio, C₁-C₄-Halogenalkylthio, Di-(C₁-C₄-alkyl)-amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy oder C₁-C₄-Halogenalkoxy; besonders bevorzugt steht R⁶ für Wasserstoff oder C₁-C₄-Alkyl;
insbesonders bevorzugt steht R⁵ für Wasserstoff, C₁-C₄-Alkyl, C₁-C₄-Halogenalkyl, C₁-C₄-Alkoxycarbonyl oder CONR⁹R¹⁰;
insbesonders bevorzugt steht R⁶ für Wasserstoff oder C₁-C₄-Alkyl;
außerordentlich bevorzugt steht R⁵ für Wasserstoff oder C₁-C₄-Alkyl, insbesondere für Wasserstoff; außerordentlich bevorzugt steht R⁶ für Wasserstoff;
- R⁷: Halogen, C₁-C₆-Alkyl, C₁-C₆-Halogenalkyl, C₁-C₆-Alkoxy, C₁-C₆-Alkylthio, C₁-C₆-Alkylsulfinyl, C₁-C₆-Alkylsulfonyl, Amino, C₁-C₆-Alkylamino, Di-(C₁-C₆-alkyl)amino, Di-(C₁-C₄-alkoxy)methyl oder COR⁸;
besonders bevorzugt C₁-C₄-Alkyl, wie z.B. Methyl, Ethyl, 1-Methylethyl oder 1,1-Dimethylethyl, Hydroxycarbonyl oder C₁-C₄-Alkoxycarbonyl, wie z.B. Methoxycarbonyl oder Ethoxycarbonyl;
- R⁸: C₁-C₄-Alkyl, Hydroxy, C₁-C₄-Alkoxy oder NR⁹R¹⁰;
- R⁹: Wasserstoff oder C₁-C₄-Alkyl;
- R¹⁰: C₁-C₄-Alkyl;
- R¹²: Wasserstoff, C₁-C₄-Alkyl, C₁-C₄-Halogenalkyl, C₁-C₄-Alkylcarbonyl, C₁-C₄-Halogenalkylcarbonyl, C₁-C₄-Alkylsulfonyl, C₁-C₄-Halogenalkylsulfonyl, Phenyl, Benzyl, Phenylcarbonyl oder Phenylcarbonyl oder Phenylsulfonyl, wobei der Phenylrest der fünf letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;
besonders bevorzugt Wasserstoff, C₁-C₄-Alkyl, C₁-C₄-Alkylsulfonyl, C₁-C₄-Halogenalkylsulfonyl, Phenyl, Benzyl oder Phenylsulfonyl, wobei der Phenylrest der drei letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy; insbesondere bevorzugt Wasserstoff oder C₁-C₄-Alkylsulfonyl, C₁-C₄-Halogenalkylsulfonyl oder Phenylsulfonyl, wobei der Phenylrest partiell oder vollständig halogeniert sein kann und/oder eine bis drei der folgenden Gruppen tragen kann:
      Nitro, Cyano, C₁-C₄-Alkyl, C₁-C₄-Halogenalkyl, C₁-C₄-Alkoxy oder C₁-C₄-Halogenalkoxy;
      ebenso besondere bevorzugt Wasserstoff, C₁-C₄-Alkyl, C₁-C₄-Alkylsulfonyl, C₁-C₄-Halogenalkylsulfonyl, Thienylmethyl, Phenyl, Benzyl, Phenylsulfonyl oder Phenylcarbonylmethyl, wobei der Phenylrest der vier letztgenannten Substituenten partiell oder vollständig halogeniert sein kann und/oder eine bis drei der folgenden Gruppen tragen kann:
         Nitro, Cyano, C₁-C₄-Alkyl, C₁-C₄-Halogenalkyl, C₁-C₄-Alkoxy oder C₁-C₄-Halogenalkoxy;
- R¹³: C₁-C₆-Alkyl oder C₃-C₆-Cycloalkyl; besonders bevorzugt Methyl, Ethyl, 1-Methylethyl oder 1,1-Dimethylethyl oder Cyclopropyl;
- R¹⁴: Wasserstoff oder C₁-C₆-Alkyl; besonders bevorzugt Wasserstoff oder Methyl.

Folgende Ausführungsformen der 3-(4,5-Dihydroisoxazol-5-yl)-benzoylpyrazole der Formel I sind hervorzuheben:
1. In einer bevorzugten Ausführungsform der 3-(4,5-Dihydroisoxazol-5-yl)-benzoylpyrazole der Formel I stehen
   - R¹: für Nitro, Halogen, C₁-C₆-Alkyl, C₁-C₆-Halogenalkyl, C₁-C₆-Alkoxy oder C₁-C₆-Halogenalkoxy; besonders bevorzugt für Nitro, Halogen wie Chlor oder Brom, C₁-C₄-Alkyl, wie Methyl oder Ethyl, C₁-C₄-Halogenalkyl, wie Difluormethyl oder Trifluormethyl, C₁-C₄-Alkoxy, wie Methoxy oder Ethoxy, oder C₁-C₄-Halogenalkoxy wie Difluormethoxy oder Trifluormethoxy;
   insbesondere bevorzugt für Halogen, wie Chlor oder Brom, C₁-C₄-Alkyl wie Methyl oder Ethyl oder C₁-C₄-Alkoxy wie Methoxy oder Ethoxy; außerordentlich bevorzugt für Halogen, wie Chlor oder Brom, C₁-C₄-Alkyl, wie Methyl oder Ethyl;
   - R²: für Halogen, C₁-C₆-Halogenalkyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
   besonders bevorzugt für Halogen wie Fluor oder Chlor, C₁-C₄-Halogenalkyl, wie Difluormethyl oder Trifluormethyl, oder C₁-C₄-Alkylsulfonyl, wie Methylsulfonyl oder Ethylsulfonyl;
   insbesondere bevorzugt für Halogen wie Fluor oder Chlor, oder C₁-C₄-Alkylsulfonyl, wie Methylsulfonyl oder Ethylsulfonyl.
2. In einer weiteren bevorzugten Ausführungsform der 3-(4,5-Dihydroisoxazol-5-yl)-benzoylpyrazole der Formel I steht
   - R³: für Wasserstoff.
3. In einer weiteren bevorzugten Ausführungsform der 3-(4,5-Dihydroisoxazol-5-yl)-benzoylpyrazole der Formel I steht
   - R³: für Halogen oder C₁-C₄-Alkyl;
   besonders bevorzugt für Chlor oder Methyl.
4. In einer weiteren Ausführungsform der 3-(4,5-Dihydroisoxazol-5-yl)-benzoylpyrazole der Formel I stehen
   - R⁴: für Wasserstoff;
   - R⁵: für Wasserstoff, Halogen, Cyano, Nitro, C₁-C₄-Alkyl, C₁-C₄-Alkoxy-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di(C₁-C₄-alkyl)-amino, C₁-C₄-Alkylcarbonyl, C₁-C₄-Alkoxycarbonyl, CONR⁹R¹⁰, Phenyl oder Benzyl, wobei die beiden letztgenannten Substituenten partiell oder vollständig halogeniert sein können und/oder eine bis drei der folgenden Gruppen tragen können:
   Nitro, Cyano, C₁-C₄-Alkyl, C₁-C₄-Halogenalkyl, C₁-C₄-Alkoxy oder C₁-C₄-Halogenalkoxy;
   besonders bevorzugt für Wasserstoff, C₁-C₄-Alkyl,
   C₁-C₄-Halogenalkyl, C₁-C₄-Alkoxycarbonyl oder CONR⁹R¹⁰;
   insbesondere bevorzugt für Wasserstoff oder
   C₁-C₄-Alkyl, wie Methyl oder Ethyl;
   außerordentlich bevorzugt für Wasserstoff;
   - R⁶: für Wasserstoff oder C₁-C₄-Alkyl, wie Methyl oder Ethyl;
   besonders bevorzugt für Wasserstoff.
5. In einer weiteren bevorzugten Ausführungsform der 3-(4,5-Dihydroisoxazol-5-yl)-benzoylpyrazole der Formel I steht
   - R⁷: für Halogen, C₁-C₆-Alkyl, C₁-C₆-Halogenalkyl, C₁-C₆-Alkoxy, C₁-C₆-Alkylthio, C₁-C₆-Alkylsulfinyl, C₁-C₆-Alkylsulfonyl, Amino, C₁-C₆-Alkylamino, Di-(C₁-C₆-alkyl)amino, Di(C₁-C₆-alkoxy)methyl, Formyl, C₁-C₄-Alkylcarbonyl, C₁-C₄-Halogenalkylcarbonyl, Hydroxycarbonyl, C₁-C₄-Alkoxycarbonyl, C₁-C₄-Halogenalkoxycarbonyl oder CONR⁹R¹⁰;
   besonders bevorzugt Halogen, wie Chlor oder Brom, C₁-C₄-Alkyl, wie Methyl, Ethyl, 1-Methylethyl oder 1,1-Dimethylethyl, C₁-C₄-Alkoxy, wie Methoxy oder Ethoxy, C₁-C₄-Alkylthio, wie Methylthio oder Ethylthio, C₁-C₄-Alkylsulfinyl, wie Methylsulfinyl oder Ethylsulfinyl, C₁-C₄-Alkylsulfonyl, wie Methylsulfonyl oder Ethylsulfonyl, Amino, C₁-C₄-Alkylamino, wie Methylamino oder Ethylamino, Di-(C₁-C₄-alkyl)amino, wie Dimethylamino oder Diethylamino, Di(C₁-C₄-alkoxy)methyl, wie Dimethoxymethyl oder Diethoxymethyl, C₁-C₄-Alkylcarbonyl, wie Methylcarbonyl oder Ethylcarbonyl, Hydroxycarbonyl, C₁-C₄-Alkoxycarbonyl, wie Methoxycarbonyl oder Ethoxycarbonyl oder CONR⁹R¹⁰;
   insbesonders bevorzugt C₁-C₄-Alkyl, wie Methyl, Ethyl, 1-Methylethyl oder 1,1-Dimethylethyl, Hydroxycarbonyl oder C₁-C₄-Alkoxycarbonyl, wie Methoxycarbonyl oder Ethoxycarbonyl; außerordentlich bevorzugt für C₁-C₄-Alkyl, wie Methyl, Ethyl, 1-Methylethyl oder 1,1-Dimethylethyl;
6. In einer weiteren bevorzugten Ausführungsform der 3-(4,5-Dihydroisoxazol-5-yl)benzoylpyrazole der Formel I steht
   - R¹²: für C₁-C₄-Alkyl, C₁-C₄-Halogenalkyl, C₁-C₄-Alkylcarbonyl, C₁-C₄-Halogenalkylcarbonyl, C₁-C₄-Alkylsulfonyl, C₁-C₄-Halogenalkylsulfonyl, Phenyl, Benzyl, Phenylcarbonyl oder Phenylsulfonyl, wobei der Phenylrest der vier letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy; besonders bevorzugt für C₁-C₄-Alkyl, C₁-C₄-Alkylsulfonyl, C₁-C₄-Halogenalkylsulfonyl, Phenyl, Benzyl oder Phenylsulfonyl, wobei der Phenylrest der drei letztgenannten Substituenten partiell oder vollständig halogeniert sein kann und/oder eine bis drei der folgenden Gruppen tragen kann:
      Nitro, Cyano, C₁-C₄-Alkyl, C₁-C₄-Halogenalkyl, C₁-C₄-Alkoxy oder C₁-C₄-Halogenalkoxy; insbesondere bevorzugt für C₁-C₄-Alkylsulfonyl, C₁-C₄-Halogenalkylsulfonyl oder Phenylsulfonyl, wobei der Phenylrest partiell oder vollständig halogeniert sein kann und/oder eine bis drei der folgenden Gruppen tragen kann:
         Nitro, Cyano, C₁-C₄-Alkyl, C₁-C₄-Halogenalkyl, C₁-C₄-Alkoxy oder C₁-C₄-Halogenalkoxy;
         ebenso insbesondere bevorzugt für Benzyl, wobei der Phenylrest partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy.
7. In einer weiteren bevorzugten Ausführungsform der 3-(4,5-Dihydroisoxazol-5-yl)benzoylpyrazole der Formel I steht
   - R¹²: für C₁-C₄-Alkyl, C₃-C₄-Alkinyl, Thienylmethyl, Benzyl, Phenylcarbonyl, Phenylsulfonyl oder Phenylcarbonylmethyl, wobei der Phenylrest der vier letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy; besonders bevorzugt für Thienylmethyl wie 3-Thienylmethyl, Benzyl, Phenylcarbonyl, Phenylsulfonyl oder Phenylcarbonylmethyl, wobei der Phenylrest der vier letztgenannnten Substituenten partiell oder vollständig halogeniert sein kann und/oder eine bis drei der folgenden Gruppen tragen kann:
      Nitro, Cyano, C₁-C₄-Alkyl, C₁-C₄-Halogenalkyl, C₁-C₄-Alkoxy oder C₁-C₄-Halogenalkoxy.
8. In einer weiteren bevorzugten Ausführungsfom der 3-(4,5-Dihydroisoxazol-5-yl)benzoylpyrazole der Formel I steht R¹² für Hydroxy.
9. In einer weiteren bevorzugten Ausführungsfom der 3-(4,5-Dihydroisoxazol-5-yl)benzoylpyrazole der Formel I stehen
   - R¹³: für C₁-C₄-Alkyl, wie Methyl, Ethyl, Methylethyl oder 1,1-Dimethylethyl oder C₃-C₆-Cycloalkyl, wie Cyclopropyl;
   besonders bevorzugt für C₁-C₄-Alkyl, wie Methyl, Ethyl, 1-Methylethyl oder 1,1-Dimethylethyl; ebenso bevorzugt für C₃-C₆-Cycloalkyl, wie Cyclopropyl;
   - R¹⁴: für Wasserstoff oder C₁-C₄-Alkyl, wie z.B. Methyl oder Ethyl;
   besonders bevorzugt für Wasserstoff oder Methyl;
10. In einer weiteren bevorzugten Ausführungsform der 3-(4,5-Dihydroisoxazol-5-yl)benzoylpyrazole der Formel I stehen
   - R¹: für Halogen, C₁-C₄-Alkyl oder C₁-C₄-Alkoxy;
   - R²: für Halogen oder C₁-C₄-Alkylsulfonyl;
   - R³, R⁴ und R⁶: für Wasserstoff;
   - R⁵: für Wasserstoff oder C₁-C₄-Alkyl; insbesondere Wasserstoff
   - R⁷: für C₁-C₄-Alkyl, Di(C₁-C₄-Alkoxy)methyl, Hydroxycarbonyl oder C₁-C₄-Alkoxycarbonyl;
   - R¹²: für Wasserstoff, C₃-C₄-Alkinyl, Thienylmethyl, Benzyl, Phenylcarbonyl, Phenylsulfonyl oder Phenylcarbonylmethyl, wobei der Phenylrest der vier letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   - R¹³: für C₁-C₄-Alkyl, C₁-C₄-Halogenalkyl oder Cyclopropyl;
   insbesondere C₁-C₄-Alkyl oder Cyclopropyl;
   - R¹⁴: für Wasserstoff, C₁-C₄-Alkyl oder Cyclopropyl; insbesondere für Wasserstoff oder C₁-C₄-Alkyl.

Insbesonders außerordentlich bevorzugt sind die 3-(4,5-Dihydroisoxazol-5-yl)benzoylpyrazole der Formel Ia1 (≡ I mit R¹ = Cl; R² = SO₂CH₃; R³, R⁴, R¹², R¹⁴ = H; R¹³ = CH₃), besonders die Verbindungen Ia1.1 bis Ia1.64, wobei die Restedefinitionen R¹ bis R¹⁴ nicht nur in Kombination miteinander, sondern auch jeweils für sich allein betrachtet für die erfindungsgemäßen Verbindungen eine besondere Bedeutung haben.

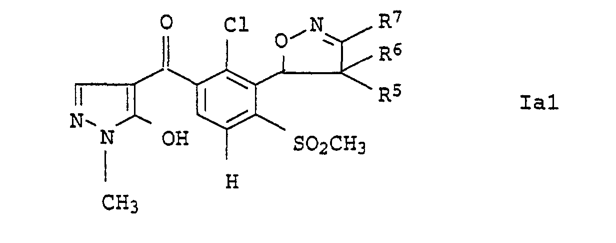

**Tabelle 1:**

| Nr. | R⁵ | R⁶ | R⁷ |
|---|---|---|---|
| Ia1.1 | H | H | CH₃ |
| Ia1.2 | Cl | H | CH₃ |
| Ia1.3 | Br | H | CH₃ |
| Ia1.4 | CN | H | CH₃ |
| Ia1.5 | NO₂ | H | CH₃ |
| Ia1.6 | CH₃ | H | CH₃ |
| Ia1.7 | CH₂CH₃ | H | CH₃ |
| Ia1.8 | CH(CH₃)₂ | H | CH₃ |
| Ia1.9 | C(CH₃)₃ | H | CH₃ |
| Ia1.10 | CHO | H | CH₃ |
| Ia1.11 | CH=NOH | H | CH₃ |
| Ia1.12 | CH=NOCH₃ | H | CH₃ |
| Ia1.13 | CH=NOCH₂CH₃ | H | CH₃ |
| Ia1.14 | COCH₃ | H | CH₃ |
| Ia1.15 | C(=NOH)CH₃ | H | CH₃ |
| Ia1.16 | C(=NOCH₃)CH₃ | H | CH₃ |
| Ia1.17 | C (=NOCH₂CH₃) CH₃ | H | CH₃ |
| Ia1.18 | CH=NN(CH₃)₂ | H | CH₃ |
| Ia1.19 | C [=NN(CH₃)₂]CH₃ | H | CH₃ |
| Ia1.20 | COOH | H | CH₃ |
| Ia1.21 | COOCH₃ | H | CH₃ |
| Ia1.22 | COOCH₂CH₃ | H | CH₃ |
| Ia1.23 | OCH₃ | H | CH₃ |
| Ia1.24 | OCH₂CH₃ | H | CH₃ |
| Ia1.25 | SCH₃ | H | CH₃ |
| Ia1.26 | CH₂F | H | CH₃ |
| Ia1.27 | CHF₂ | H | CH₃ |
| Ia1.28 | CF₃ | H | CH₃ |
| Ia1.29 | CF₂CF₃ | H | CH₃ |
| Ia1.30 | CH₂Cl | H | CH₃ |
| Ia1.31 | CH₂CN | H | CH₃ |
| Ia1.32 | CH₂-CH₂ | | CH₃ |
| Ia1.33 | CH₂-CH₂-CH₂ | | CH₃ |
| Ia1.34 | CH₂-CH₂-CH₂-CH₂ | | CH₃ |
| Ia1.35 | CH₂-CH₂-CH₂-CH₂-CH₂ | | CH₃ |
| Ia1.36 | CH₃ | CH₃ | CH₃ |
| Ia1.37 | CH₃ | CH₂CH₃ | CH₃ |
| Ia1.38 | CF₃ | COCH₃ | CH₃ |
| Ia1.39 | CF₃ | COOCH₃ | CH₃ |
| Ia1.40 | CN | COCH₃ | CH₃ |
| Ia1.41 | CN | COOCH₃ | CH₃ |
| Ia1.42 | CN | CN | CH₃ |
| Ia1.43 | COCH₃ | COCH₃ | CH₃ |
| Ia1.44 | COCH₃ | COOCH₃ | CH₃ |
| Ia1.45 | H | H | CH₂CH₃ |
| Ia1.46 | H | H | CH(CH₃)₂ |
| Ia1.47 | H | H | C(CH₃)₃ |
| Ia1.48 | H | H | Cl |
| Ia1.49 | H | H | OCH₃ |
| Ia1.50 | H | H | SCH₃ |
| Ia1.51 | H | H | SOCH₃ |
| Ial. 52 | H | H | SO₂CH₃ |
| Ia1.53 | H | H | CN |
| Ia1.54 | H | H | CHO |
| Ia1.55 | H | H | CH=NOH |
| Ia1.56 | H | H | CH=NOCH₃ |
| Ia1.57 | H | H | CH=NOCH₂CH₃ |
| Ia1.58 | H | H | COCH₃ |
| Ial.59 | H | H | C(=NOH)CH₃ |
| Ia1.60 | H | H | C(=NOCH₃)CH₃ |
| Ia1.61 | H | H | COOH |
| Ia1.62 | H | H | COOCH₃ |
| Ia1.63 | H | H | COOCH₂CH₃ |
| Ia1.64 | H | H | CH(OCH₂CH₃)₂ |

Ebenso insbesondere bevorzugt sind die Verbindungen Ia2, besonders die Verbindungen Ia2.1 - Ia2.64, die sich von den Verbindungen Ia1.1 - Ia1.64 dadurch unterscheiden, daß R¹³ für Ethyl steht.

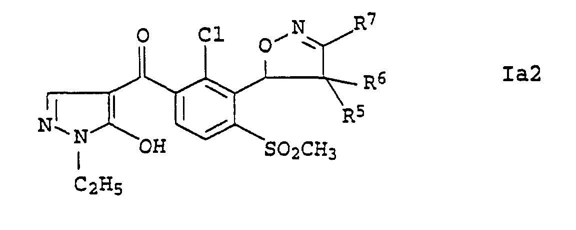

Ebenso insbesondere bevorzugt sind die Verbindungen Ia3., besonders die Verbindungen Ia3.1 - Ia3.64, die sich von den Verbindungen Ia1.1 - Ia1.64 dadurch unterscheiden, daß R¹³ für Isopropyl steht.

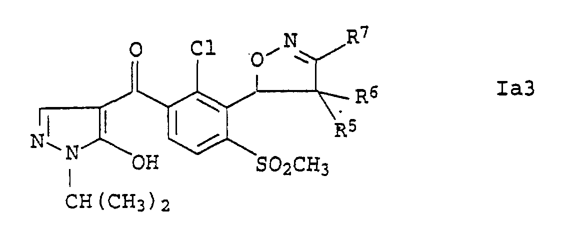

Ebenso insbesondere bevorzugt sind die Verbindungen Ia4., besonders die Verbindungen Ia4.1 - Ia4.64, die sich von den Verbindungen Ia1.1 - Ial.64 dadurch unterscheiden, daß R¹³ für tert.-Butyl steht.

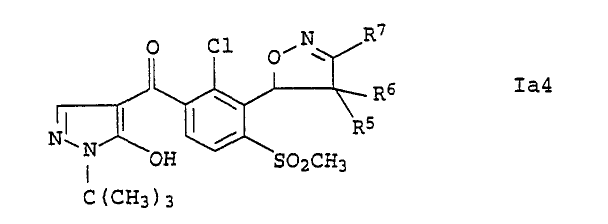

Ebenso insbesondere bevorzugt sind die Verbindungen Ia5., besonders die Verbindungen Ia5.1 - Ia5.64, die sich von den Verbindungen Ia1.1 - Ial.64 dadurch unterscheiden, daß R¹⁴ für Methyl steht.

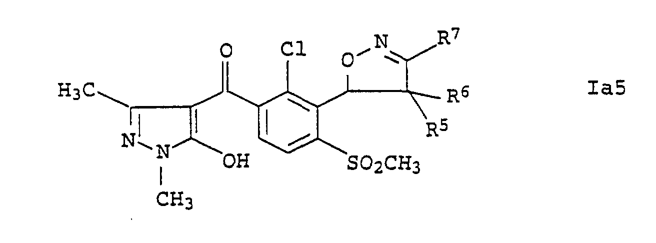

Ebenso insbesondere bevorzugt sind die Verbindungen Ia6., besonders die Verbindungen Ia6.1 - Ia6.64, die sich von den Verbindungen Ia1.1 - Ia1.64 dadurch unterscheiden, daß R¹³ für Ethyl und R¹⁴ für Methyl stehen.

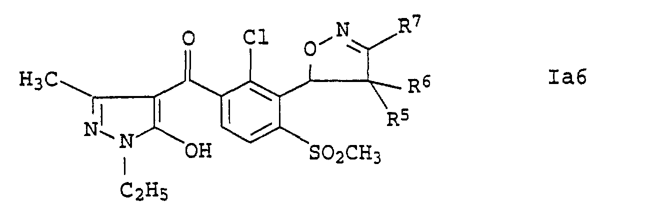

Ebenso insbesondere bevorzugt sind die Verbindungen Ia7., besonders die Verbindungen Ia7.1 - Ia7.64, die sich von den Verbindungen Ia1.1 - Ia1.64 dadurch unterscheiden, daß R¹³ für Isopropyl und R¹⁴ für Methyl stehen.

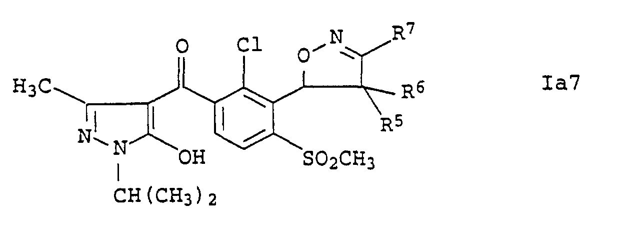

Ebenso insbesondere bevorzugt sind die Verbindungen Ia8., besonders die Verbindungen Ia8.1 - Ia8.64, die sich von den Verbindungen Ia1.1 - Ia1.64 dadurch unterscheiden, daß R¹³ für tert.-Butyl und R¹⁴ für Methyl stehen.

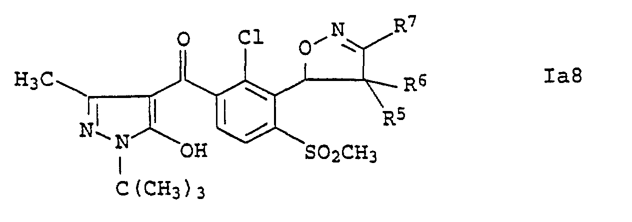

Ebenso insbesondere bevorzugt sind die Verbindungen Ia9., besonders die Verbindungen Ia9.1 - Ia9.64, die sich von den Verbindungen Ia1.1 - Ia1.64 dadurch unterscheiden, daß R¹ für Methyl steht.

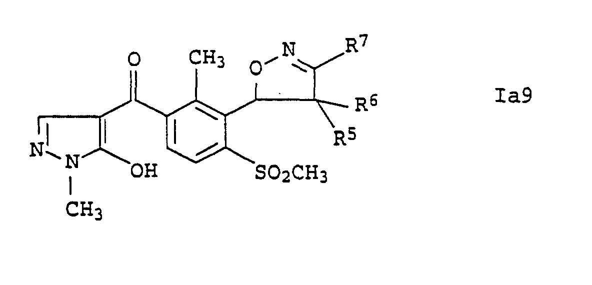

Ebenso insbesondere bevorzugt sind die Verbindungen Ia10., besonders die Verbindungen Ia10.1 - Ia10.64, die sich von den Verbindungen Ia1.1 - Ia1.64 dadurch unterscheiden, daß R¹ für Methyl und R¹³ für Ethyl stehen.

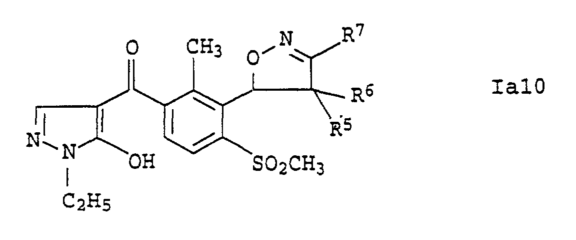

Ebenso insbesondere bevorzugt sind die Verbindungen Ia11., besonders die Verbindungen Ia11.1 - Ia11.64, die sich von den Verbindungen Ia1.1 - Ia1.64 dadurch unterscheiden, daß R¹ für Methyl und R¹³ für Isopropyl stehen.

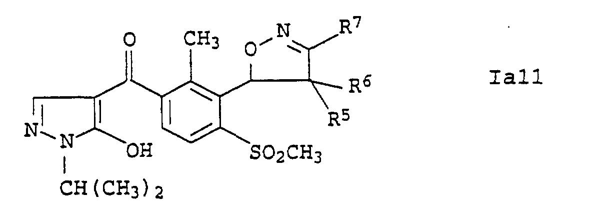

Ebenso insbesondere bevorzugt sind die Verbindungen Ia12., besonders die Verbindungen Ia12.1 - Ia12.64, die sich von den Verbindungen Ia1.1 - Ia1.64 dadurch unterscheiden, daß R¹ für Methyl und R¹³ für tert.-Butyl stehen.

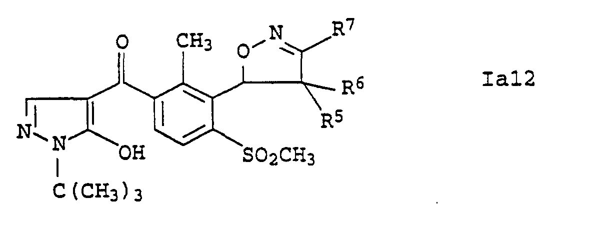

Ebenso insbesondere bevorzugt sind die Verbindungen Ia13., besonders die Verbindungen Ia13.1 - Ia13.64, die sich von den Verbindungen Ia1.1 - Ia1.64 dadurch unterscheiden, daß R¹ und R¹⁴ für Methyl stehen.

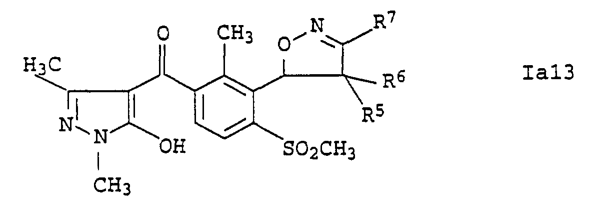

Ebenso insbesondere bevorzugt sind die Verbindungen Ia14., besonders die Verbindungen Ia14.1 - Ia14.64, die sich von den Verbindungen Ia1.1 - Ia1.64 dadurch unterscheiden, daß R¹ und R¹⁴ für Methyl und R¹³ für Ethyl stehen.

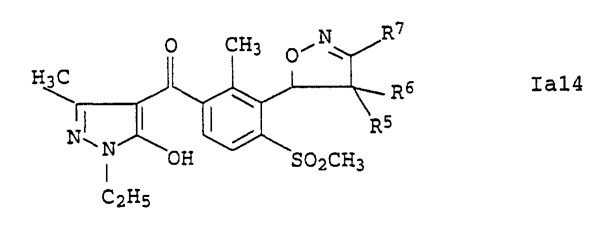

Ebenso insbesondere bevorzugt sind die Verbindungen Ia15., besonders die Verbindungen Ia15.1 - Ia15.64, die sich von den Verbindungen Ia1.1 - Ial.64 dadurch unterscheiden, daß R¹ und R¹⁴ für Methyl und R¹³ für Isopropyl stehen.

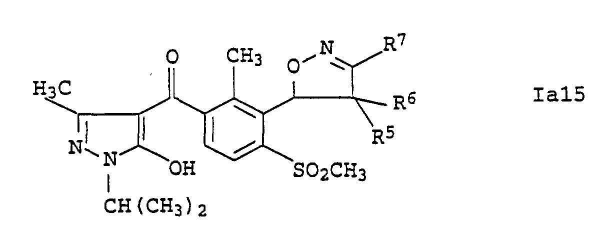

Ebenso insbesondere bevorzugt sind die Verbindungen Ia16., besonders die Verbindungen Ia16.1 - Ia16.64, die sich von den Verbindungen Ia1.1 - Ia1.64 dadurch unterscheiden, daß R¹ und R¹⁴ für Methyl und R¹³ für tert.-Butyl stehen.

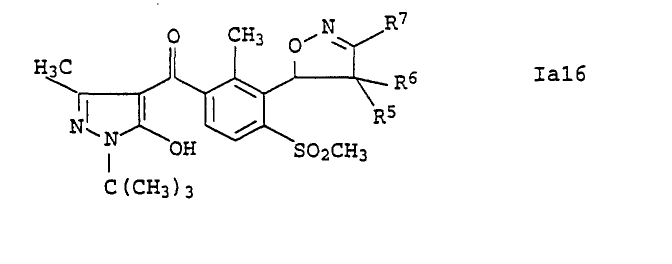

Ebenso insbesondere bevorzugt sind die Verbindungen Ia17., besonders die Verbindungen Ia17.1 - Ia17.64, die sich von den Verbindungen Ia1.1 - Ial.64 dadurch unterscheiden, daß R¹ für Trifluormethyl steht.

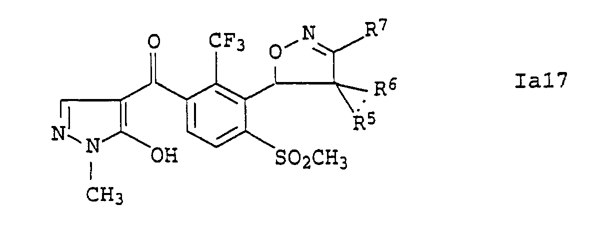

Ebenso insbesondere bevorzugt sind die Verbindungen Ia18., besonders die Verbindungen Ia18.1 - Ia18.64, die sich von den Verbindungen Ia1.1 - Ia1.64 dadurch unterscheiden, daß R¹ für Trifluormethyl und R¹³ Ethyl stehen.

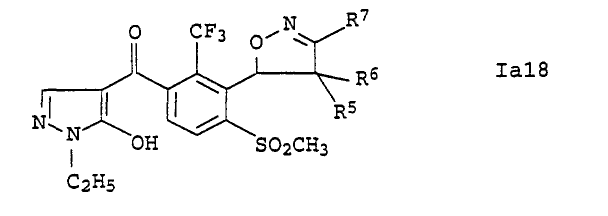

Ebenso insbesondere bevorzugt sind die Verbindungen Ia19., besonders die Verbindungen Ia19.1 - Ia19.64, die sich von den Verbindungen Ia1.1 - Ia1.64 dadurch unterscheiden, daß R¹ für Trifluormethyl und R¹³ für Isopropyl stehen.

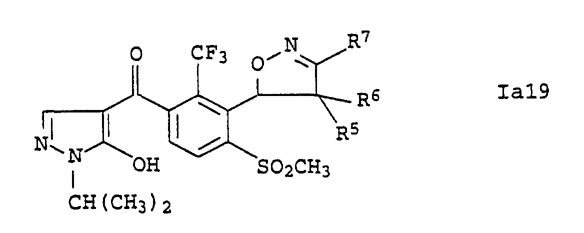

Ebenso insbesondere bevorzugt sind die Verbindungen Ia20., besonders die Verbindungen Ia20.1 - Ia20.64, die sich von den Verbindungen Ia1.1 - Ial.64 dadurch unterscheiden, daß R¹ für Trifluormethyl und R¹³ für tert.-Butyl stehen.

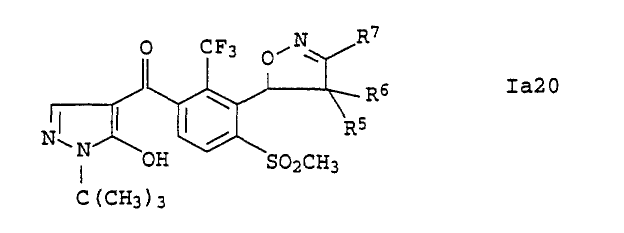

Ebenso insbesondere bevorzugt sind die Verbindungen Ia21., besonders die Verbindungen Ia21.1 - Ia21.64, die sich von den Verbindungen Ia1.1 - Ial.64 dadurch unterscheiden, daß R¹ für Trifluormethyl und R¹⁴ für Methyl stehen.

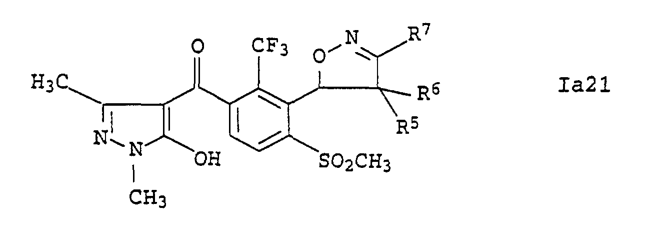

Ebenso insbesondere bevorzugt sind die Verbindungen Ia22., besonders die Verbindungen Ia22.1 - Ia22.64, die sich von den Verbindungen Ia1.1 - Ia1.64 dadurch unterscheiden, daß R¹ für Trifluormethyl, R¹³ für Ethyl und R¹⁴ für Methyl stehen.

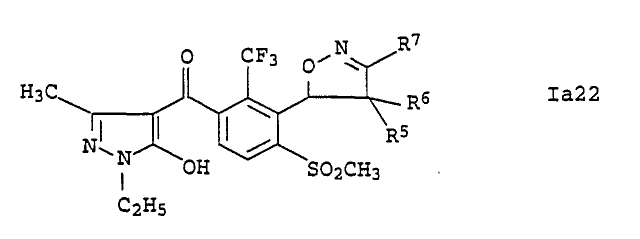

Ebenso insbesondere bevorzugt sind die Verbindungen Ia23., besonders die Verbindungen Ia23.1 - Ia23.64, die sich von den Verbindungen Ia1.1 - Ial.64 dadurch unterscheiden, daß R¹ für Trifluormethyl, R¹³ für Isopropyl und R¹⁴ für Methyl stehen.

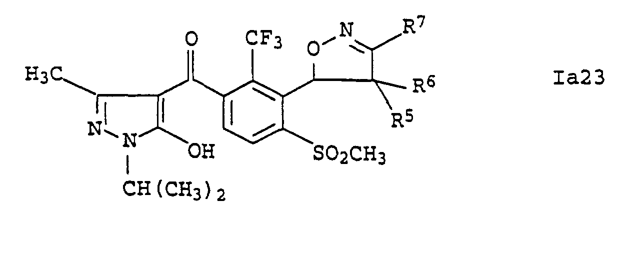

Ebenso insbesondere bevorzugt sind die Verbindungen Ia24., besonders die Verbindungen Ia24.1 - Ia24.64, die sich von den Verbindungen Ia1.1 - Ia1.64 dadurch unterscheiden, daß R¹ für Trifluormethyl, R¹³ für tert.-Butyl und R¹⁴ für Methyl stehen.

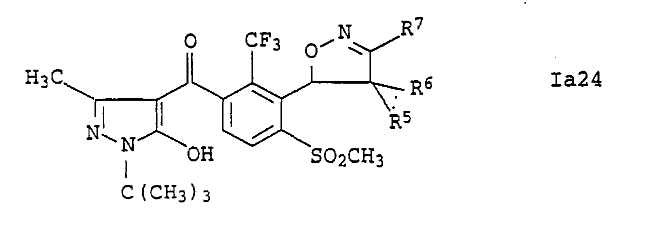

Ebenso insbesondere bevorzugt sind die Verbindungen Ia25., besonders die Verbindungen Ia25.1 - Ia25.64, die sich von den Verbindungen Ia1.1 - Ia1.64 dadurch unterscheiden, daß R¹ für Methoxy steht.

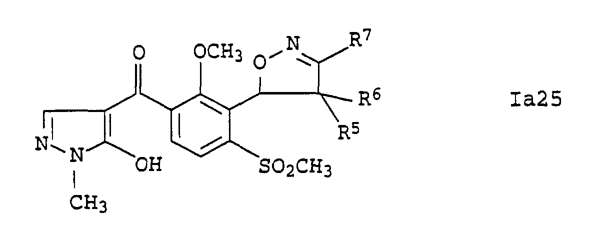

Ebenso insbesondere bevorzugt sind die Verbindungen Ia26., besonders die Verbindungen Ia26.1 - Ia26.64, die sich von den Verbindungen Ia1.1 - Ia1.64 dadurch unterscheiden, daß R¹ für Methoxy und R¹³ für Ethyl stehen.

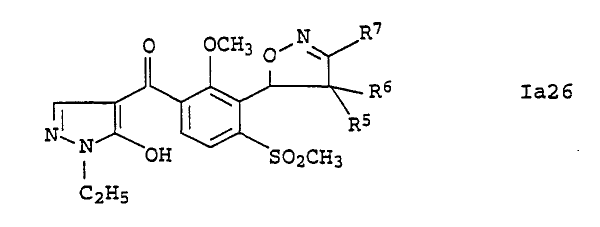

Ebenso insbesondere bevorzugt sind die Verbindungen Ia27., besonders die Verbindungen Ia27.1 - Ia27.64, die sich von den Verbindungen Ia1.1 - Ia1.64 dadurch unterscheiden, daß R¹ für Methoxy und R¹³ für Isopropyl stehen.

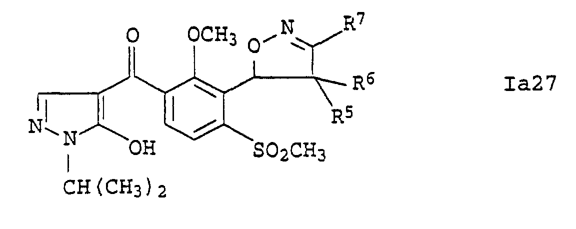

Ebenso insbesondere bevorzugt sind die Verbindungen Ia28., besonders die Verbindungen Ta28.1 - Ia28.64, die sich von den Verbindungen Ia1.1 - Ial.64 dadurch unterscheiden, daß R¹ für Methoxy und R¹³ für tert.-Butyl stehen.

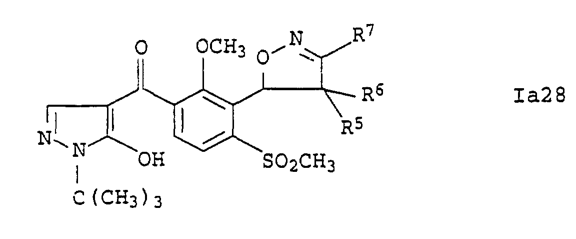

Ebenso insbesondere bevorzugt sind die Verbindungen Ia29., besonders die Verbindungen Ia29.1 - Ia29.64, die sich von den Verbindungen Ia1.1 - Ia1.64 dadurch unterscheiden, daß R¹ für Methoxy und R¹⁴ für Methyl stehen.

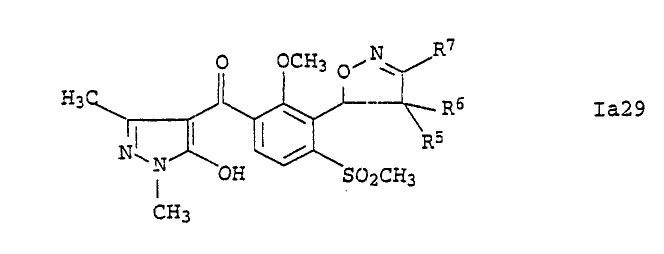

Ebenso insbesondere bevorzugt sind die Verbindungen Ia30., besonders die Verbindungen Ia30.1 - Ia30.64, die sich von den Verbindungen Ia1.1 - Ia1.64 dadurch unterscheiden, daß R¹ für Methoxy, R¹³ für Ethyl und R¹⁴ für Methyl stehen.

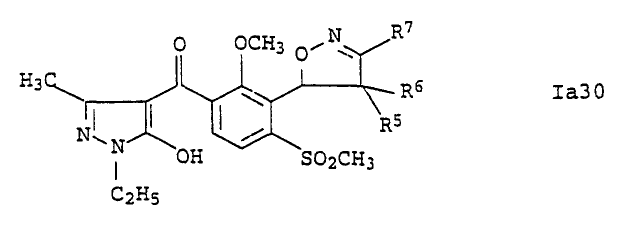

Ebenso insbesondere bevorzugt sind die Verbindungen Ia31., besonders die Verbindungen Ia31.1 - Ia31.64, die sich von den Verbindungen Ia1.1 - Ia1.64 dadurch unterscheiden, daß R¹ für Methoxy, R¹³ für Isopropyl und R¹⁴ für Methyl stehen.

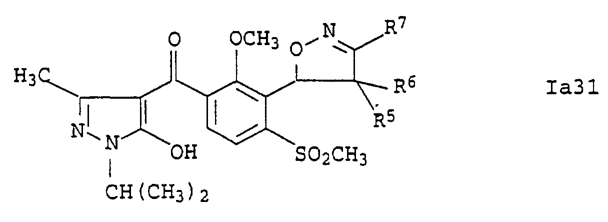

Ebenso insbesondere bevorzugt sind die Verbindungen Ia32., besonders die Verbindungen Ia32.1 - Ia32.64, die sich von den Verbindungen Ia1.1 - Ia1.64 dadurch unterscheiden, daß R¹ für Methoxy, R¹³ für tert.-Butyl und R¹⁴ für Methyl stehen.

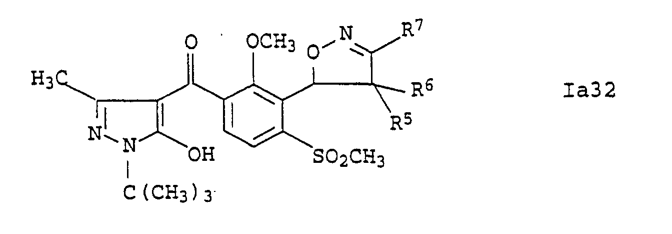

Ebenso insbesondere bevorzugt sind die Verbindungen Ia33., besonders die Verbindungen Ia33.1 - Ia33.64, die sich von den Verbindungen Ia1.1 - Ia1.64 dadurch unterscheiden, daß R² für Ethylsulfonyl steht.

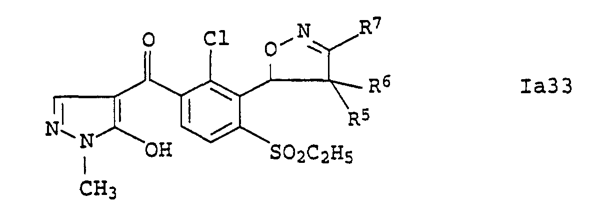

Ebenso insbesondere bevorzugt sind die Verbindungen Ia34., besonders die Verbindungen Ia34.1 - Ia34.64, die sich von den Verbindungen Ial.1 - Ial.64 dadurch unterscheiden, daß R² für Ethylsulfonyl und R¹³ für Ethyl stehen.

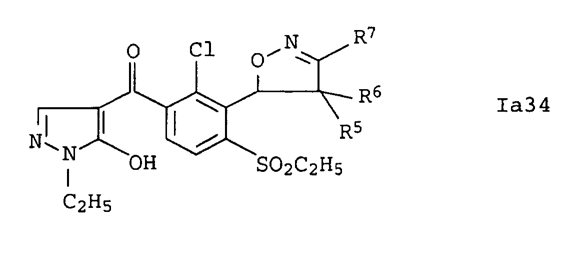

Ebenso insbesondere bevorzugt sind die Verbindungen Ia35., besonders die Verbindungen Ia35.1 - Ia35.64, die sich von den Verbindungen Ia1.1 - Ia1.64 dadurch unterscheiden, daß R² für Ethylsulfonyl und R¹³ für Isopropyl stehen.

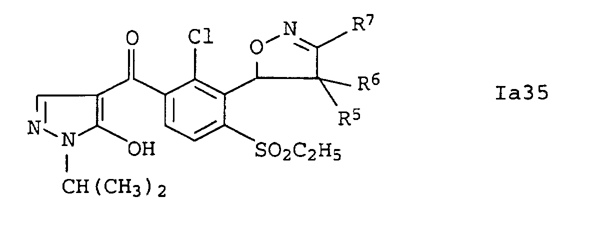

Ebenso insbesondere bevorzugt sind die Verbindungen Ia36., besonders die Verbindungen Ia36.1 - Ia36.64, die sich von den Verbindungen Ia1.1 - Ia1.64 dadurch unterscheiden, daß R² für Ethylsulfonyl und R¹³ für tert.-Butyl stehen.

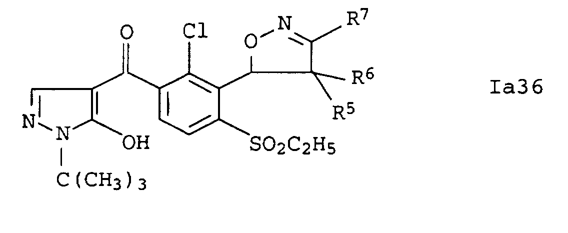

Ebenso insbesondere bevorzugt sind die Verbindungen Ia37., besonders die Verbindungen Ia37.1 - Ia37.64, die sich von den Verbindungen Ia1.1 - Ia1.64 dadurch unterscheiden, daß R² für Ethylsulfonyl und R¹⁴ für Methyl stehen.

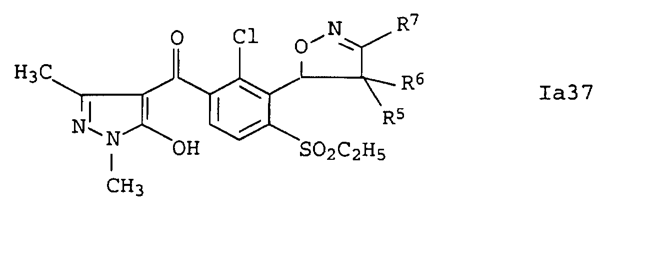

Ebenso insbesondere bevorzugt sind die Verbindungen Ia38., besonders die Verbindungen Ia38.1 - Ia38.64, die sich von den Verbindungen Ia1.1 - Ial.64 dadurch unterscheiden, daß R² für Ethylsulfonyl, R¹³ für Ethyl und R¹⁴ für Methyl stehen.

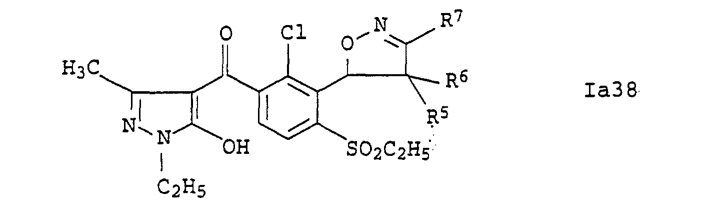

Ebenso insbesondere bevorzugt sind die Verbindungen Ia39., besonders die Verbindungen Ia39.1 - Ia39.64, die sich von den Verbindungen Ia1.1 - Ial.64 dadurch unterscheiden, daß R² für Ethylsulfonyl, R¹³ für Isopropyl und R¹⁴ für Methyl stehen.

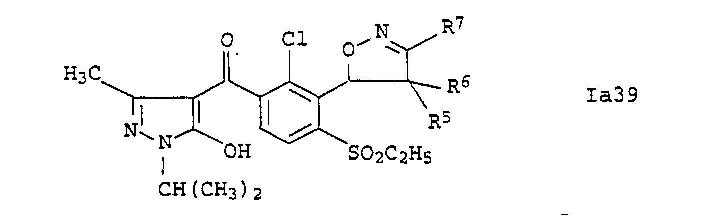

Ebenso insbesondere bevorzugt sind die Verbindungen Ia40., besonders die Verbindungen Ia40.1 - Ia40.64, die sich von den Verbindungen Ia1.1 - Ia1.64 dadurch unterscheiden, daß R² für Ethylsulfonyl, R¹³ für tert.-Butyl und R¹⁴ für Methyl stehen.

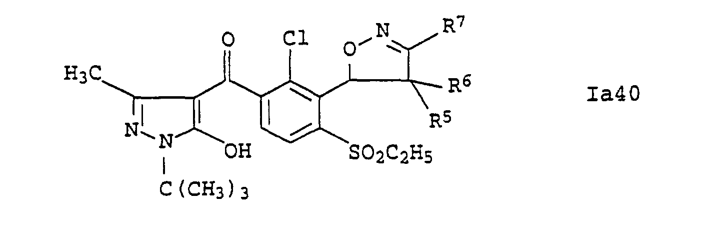

Ebenso insbesondere bevorzugt sind die Verbindungen Ia41., besonders die Verbindungen Ia41.1 - Ia41.64, die sich von den Verbindungen Ia1.1 - Ia1.64 dadurch unterscheiden, daß R¹ für Methyl und R² für Ethylsulfonyl stehen.

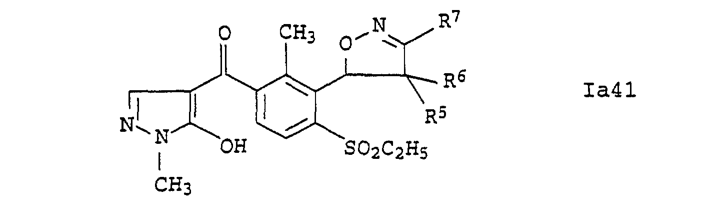

Ebenso insbesondere bevorzugt sind die Verbindungen Ia42., besonders die Verbindungen Ia42.1 - Ia42.64, die sich von den Verbindungen Ia1.1 - Ia1.64 dadurch unterscheiden, daß R¹ für Methyl, R² für Ethylsulfonyl und R¹³ für Ethyl stehen.

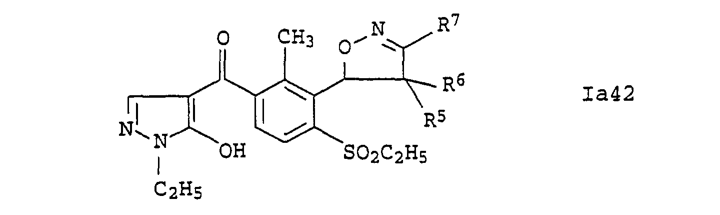

Ebenso insbesondere bevorzugt sind die Verbindungen Ia43., besonders die Verbindungen Ia43.1 - Ia43.64, die sich von den Verbindungen Ia1.1 - Ial.64 dadurch unterscheiden, daß R¹ für Methyl, R² für Ethylsulfonyl und R¹³ für Isopropyl stehen.

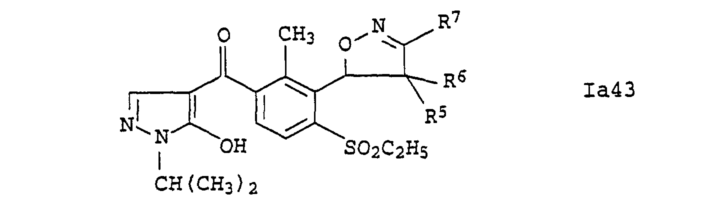

Ebenso insbesondere bevorzugt sind die Verbindungen Ia44., besonders die Verbindungen Ia44.1 - Ia44.64, die sich von den Verbindungen Ia1.1 - Ia1.64 dadurch unterscheiden, daß R¹ für Methyl, R² für Ethylsulfonyl und R¹³ für tert.-Butyl stehen.

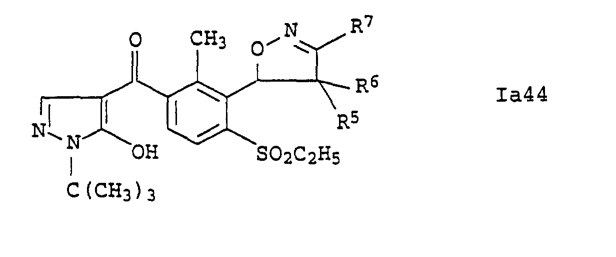

Ebenso insbesondere bevorzugt sind die Verbindungen Ia45., besonders die Verbindungen Ia45.1 - Ia45.64, die sich von den Verbindungen Ia1.1 - Ia1.64 dadurch unterscheiden, daß R¹ und R¹⁴ für Methyl und R² für Ethylsulfonyl stehen.

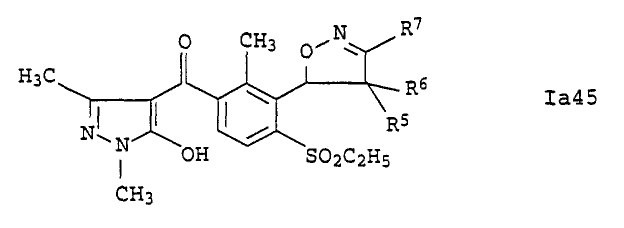

Ebenso insbesondere bevorzugt sind die Verbindungen Ia46., besonders die Verbindungen Ia46.1 - Ia46.64, die sich von den Verbindungen Ia1.1 - Ial.64 dadurch unterscheiden, daß R¹ und R¹⁴ für Methyl, R² für Ethylsulfonyl und R¹³ für Ethyl stehen.

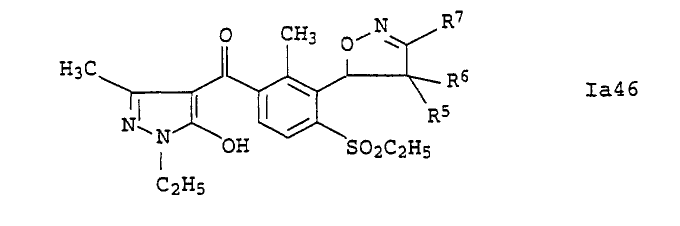

Ebenso insbesondere bevorzugt sind die Verbindungen Ia47., besonders die Verbindungen Ia47.1 - Ia47.64, die sich von den Verbindungen Ia1.1 - Ia1.64 dadurch unterscheiden, daß R¹ und R¹⁴ für Methyl, R² für Ethylsulfonyl und R¹³ für Isopropyl stehen.

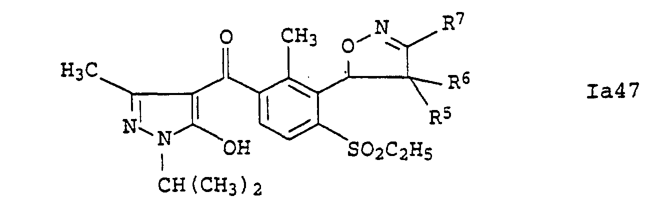

Ebenso insbesondere bevorzugt sind die Verbindungen Ia48., besonders die Verbindungen Ia48.1 - Ia48.64, die sich von den Verbindungen Ia1.1 - Ia1.64 dadurch unterscheiden, daß R¹ und R¹⁴ für Methyl, R² für Ethylsulfonyl und R¹³ für tert.-Butyl stehen.

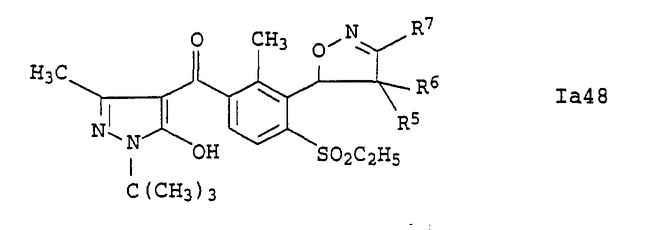

Ebenso insbesondere bevorzugt sind die Verbindungen Ia49., besonders die Verbindungen Ia49.1 - Ia49.64, die sich von den Verbindungen Ia1.1 - Ia1.64 dadurch unterscheiden, daß R¹ für Trifluormethyl und R² für Ethylsulfonyl stehen.

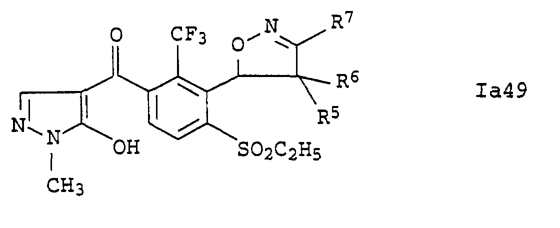

Ebenso insbesondere bevorzugt sind die Verbindungen Ia50., besonders die Verbindungen Ia50.1 - Ia50.64, die sich von den Verbindungen Ia1.1 - Ial.64 dadurch unterscheiden, daß R¹ für Trifluormethyl, R² für Ethylsulfonyl und R¹³ für Ethyl stehen.

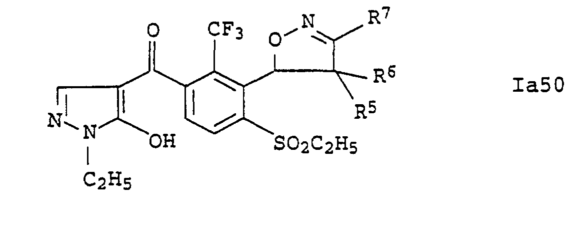

Ebenso insbesondere bevorzugt sind die Verbindungen Ia51., besonders die Verbindungen Ia51.1 - Ia51.64, die sich von den Verbindungen Ia1.1 - Ia1.64 dadurch unterscheiden, daß R¹ für Trifluormethyl, R² für Ethylsulfonyl und R¹³ für Isopropyl stehen.

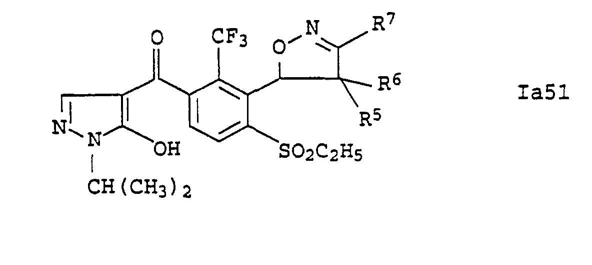

Ebenso insbesondere bevorzugt sind die Verbindungen Ia52., besonders die Verbindungen Ia52.1 - Ia52.64, die sich von den Verbindungen Ia1.1 - Ia1.64 dadurch unterscheiden, daß R¹ für Trifluormethyl, R² für Ethylsulfonyl und R¹³ für tert.-Butyl stehen.

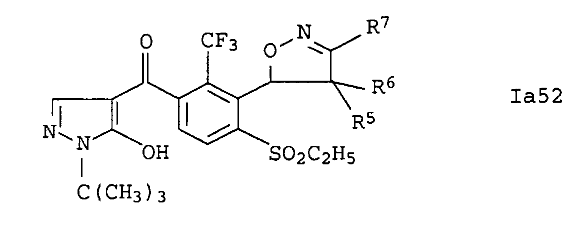

Ebenso insbesondere bevorzugt sind die Verbindungen Ia53., besonders die Verbindungen Ia53.1 - Ia53.64, die sich von den Verbindungen Ia1.1 - Ia1.64 dadurch unterscheiden, daß R¹ für Trifluormethyl, R² für Ethylsulfonyl und R¹⁴ für Methyl stehen.

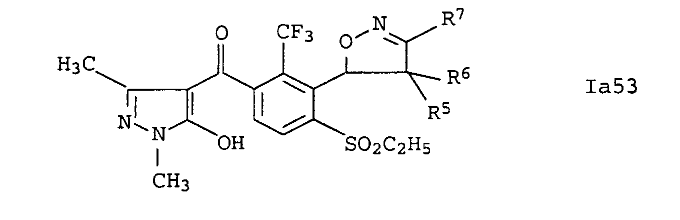

Ebenso insbesondere bevorzugt sind die Verbindungen Ia54., besonders die Verbindungen Ia54.1 - Ia54.64, die sich von den Verbindungen Ia1.1 - Ia1.64 dadurch unterscheiden, daß R¹ für Trifluormethyl, R² für Ethylsulfonyl, R¹³ für Ethyl und R¹⁴ für Methyl stehen.

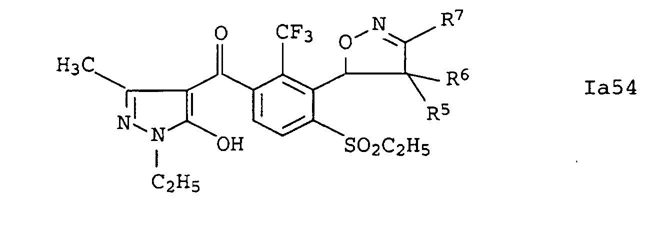

Ebenso insbesondere bevorzugt sind die Verbindungen Ia55., besonders die Verbindungen Ia55.1 - Ia55.64, die sich von den Verbindungen Ia1.1 - Ia1.64 dadurch unterscheiden, daß R¹ für Trifluormethyl, R² für Ethylsulfonyl, R¹³ für Isopropyl und R¹⁴ für Methyl stehen.

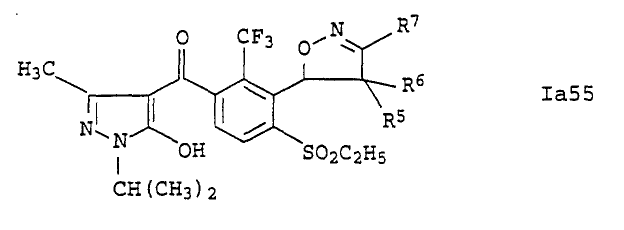

Ebenso insbesondere bevorzugt sind die Verbindungen Ia56., besonders die Verbindungen Ia56.1 - Ia56.64, die sich von den Verbindungen Ia1.1 - Ial.64 dadurch unterscheiden, daß R¹ für Trifluormethyl, R² für Ethylsulfonyl, R¹³ für tert.-Butyl und R¹⁴ für Methyl stehen.

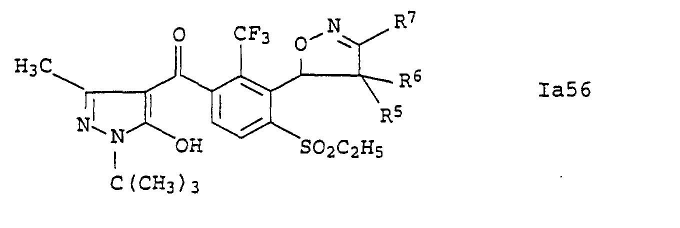

Ebenso insbesondere bevorzugt sind die Verbindungen Ia57., besonders die Verbindungen Ia57.1 - Ia57.64, die sich von den Verbindungen Ia1.1 - Ia1.64 dadurch unterscheiden, daß R¹ für Methoxy und R² für Ethylsulfonyl stehen.

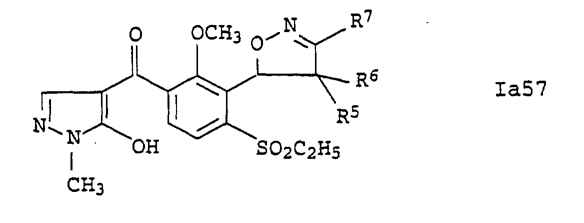

Ebenso insbesondere bevorzugt sind die Verbindungen Ia58., besonders die Verbindungen Ia58.1 - Ia58.64, die sich von den Verbindungen Ia1.1 - Ia1.64 dadurch unterscheiden, daß R¹ für Methoxy, R² für Ethylsulfonyl und R¹³ für Ethyl stehen.

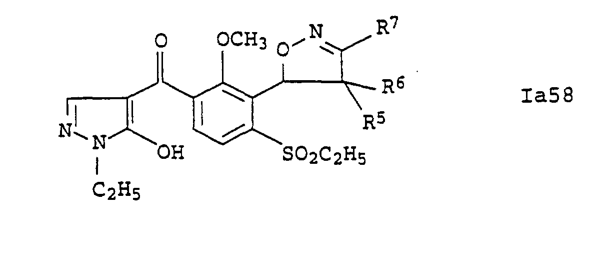

Ebenso insbesondere bevorzugt sind die Verbindungen Ia59., besonders die Verbindungen Ia59.1 - Ia59.64, die sich von den Verbindungen Ia1.1 - Ia1.64 dadurch unterscheiden, daß R¹ für Methoxy, R² für Ethylsulfonyl und R¹³ für Isopropyl stehen.

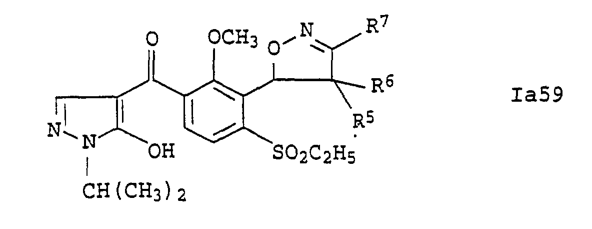

Ebenso insbesondere bevorzugt sind die Verbindungen Ia60., besonders die Verbindungen Ia60.1 - Ia60.64, die sich von den Verbindungen Ia1.1 - Ia1.64 dadurch unterscheiden, daß R¹ für Methoxy, R² für Ethylsulfonyl und R¹³ für tert.-Butyl stehen.

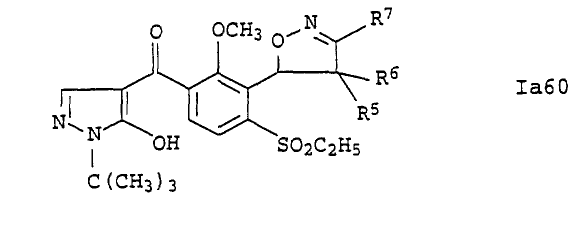

Ebenso insbesondere bevorzugt sind die Verbindungen Ia61., besonders die Verbindungen Ia61.1 - Ia61.64, die sich von den Verbindungen Ia1.1 - Ia1.64 dadurch unterscheiden, daß R¹ für Methoxy, R² für Ethylsulfonyl und R¹⁴ für Methyl stehen.

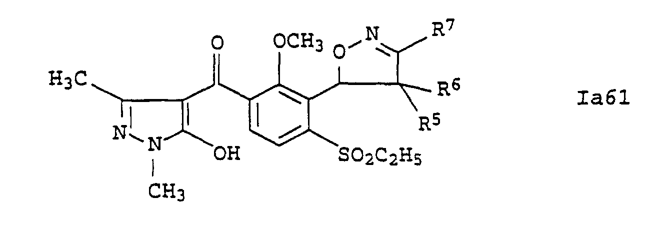

Ebenso insbesondere bevorzugt sind die Verbindungen Ia62., besonders die Verbindungen Ia62.1 - Ia62.64, die sich von den Verbindungen Ia1.1 - Ia1.64 dadurch unterscheiden, daß R¹ für Methoxy, R² für Ethylsulfonyl, R¹³ für Ethyl und R¹⁴ für Methyl stehen.

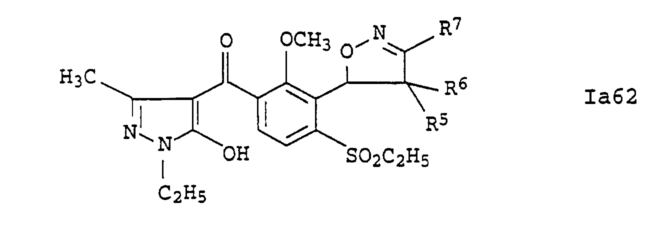

Ebenso insbesondere bevorzugt sind die Verbindungen Ia63., besonders die Verbindungen Ia63.1 - Ia63.64, die sich von den Verbindungen Ia1.1 - Ia1.64 dadurch unterscheiden, daß R¹ für Methoxy, R² für Ethylsulfonyl, R¹³ für Isopropyl und R¹⁴ für Methyl stehen.

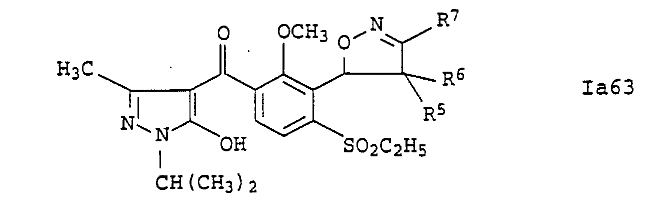

Ebenso insbesondere bevorzugt sind die Verbindungen Ia64., besonders die Verbindungen Ia64.1 - Ia64.64, die sich von den Verbindungen Ia1.1 - Ia1.64 dadurch unterscheiden, daß R¹ für Methoxy, R² für Ethylsulfonyl, R¹³ für tert.-Butyl und R¹⁴ für Methyl stehen.

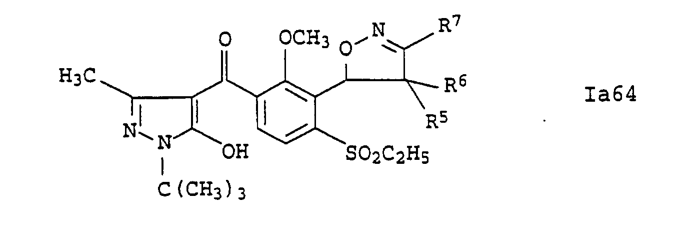

Ebenso insbesondere bevorzugt sind die Verbindungen Ia65., besonders die Verbindungen Ia65.1 - Ia65.64, die sich von den Verbindungen Ia1.1 - Ial.64 dadurch unterscheiden, daß R² für Chlor steht.

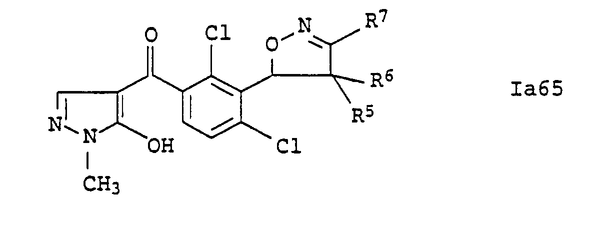

Ebenso insbesondere bevorzugt sind die Verbindungen Ia66., besonders die Verbindungen Ia66.1 - Ia66.64, die sich von den Verbindungen Ia1.1 - Ial.64 dadurch unterscheiden, daß R² für Chlor und R¹³ für Ethyl stehen.

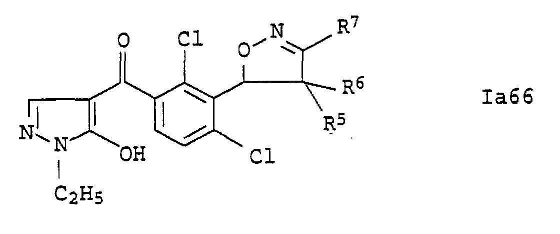

Ebenso insbesondere bevorzugt sind die Verbindungen Ia67., besonders die Verbindungen Ia67.1 - Ia67.64, die sich von den Verbindungen Ia1.1 - Ia1.64 dadurch unterscheiden, daß R² für Chlor und R¹³ für Isopropyl stehen.

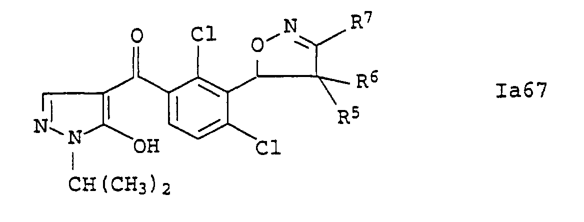

Ebenso insbesondere bevorzugt sind die Verbindungen Ia68., besonders die Verbindungen Ia68.1 - Ia68.64, die sich von den Verbindungen Ia1.1 - Ia1.64 dadurch unterscheiden, daß R² für Chlor und R¹³ für tert.-Butyl stehen.

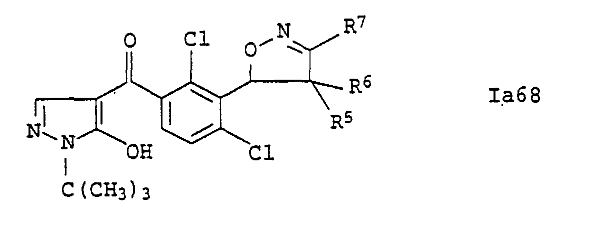

Ebenso insbesondere bevorzugt sind die Verbindungen Ia69., besonders die Verbindungen Ia69.1 - Ia69.64, die sich von den Verbindungen Ia1.1 - Ia1.64 dadurch unterscheiden, daß R² für Chlor und R¹⁴ für Methyl stehen.

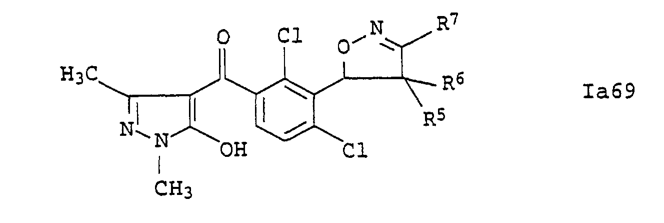

Ebenso insbesondere bevorzugt sind die Verbindungen Ia70., besonders die Verbindungen Ia70.1 - Ia70.64, die sich von den Verbindungen Ia1.1 - Ia1.64 dadurch unterscheiden, daß R² für Chlor, R¹³ für Ethyl und R¹⁴ für Methyl stehen.

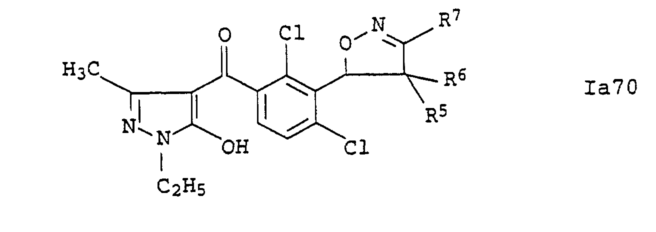

Ebenso insbesondere bevorzugt sind die Verbindungen Ia71., besonders die Verbindungen Ia71.1 - Ia71.64, die sich von den Verbindungen Ia1.1 - Ia1.64 dadurch unterscheiden, daß R² für Chlor, R¹³ für Isopropyl und R¹⁴ für Methyl stehen.

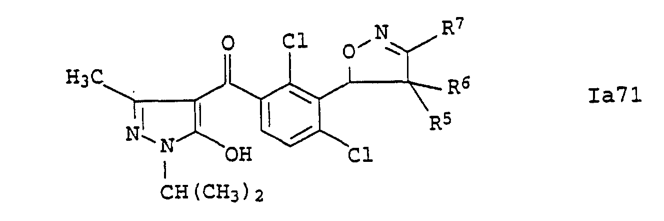

Ebenso insbesondere bevorzugt sind die Verbindungen Ia72., besonders die Verbindungen Ia72.1 - Ia72.64, die sich von den Verbindungen Ia1.1 - Ia1.64 dadurch unterscheiden, daß R² für Chlor, R¹³ für tert.-Butyl und R¹⁴ für Methyl stehen.

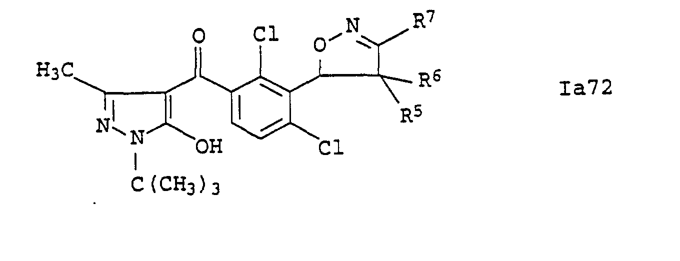

Ebenso insbesondere bevorzugt sind die Verbindungen Ia73., besonders die Verbindungen Ia73.1 - Ia73.64, die sich von den Verbindungen Ia1.1 - Ia1.64 dadurch unterscheiden, daß R¹ für Methyl und R² für Chlor stehen.

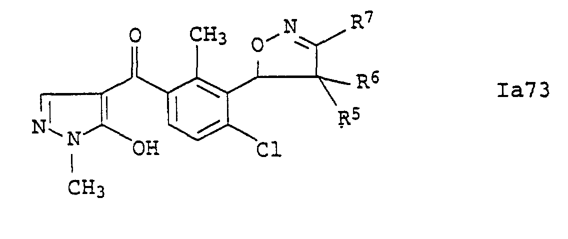

Ebenso insbesondere bevorzugt sind die Verbindungen Ia74., besonders die Verbindungen Ia74.1 - Ia74.64, die sich von den Verbindungen Ia1.1 - Ial.64 dadurch unterscheiden, daß R¹ für Methyl, R² für Chlor und R¹³ für Ethyl stehen.

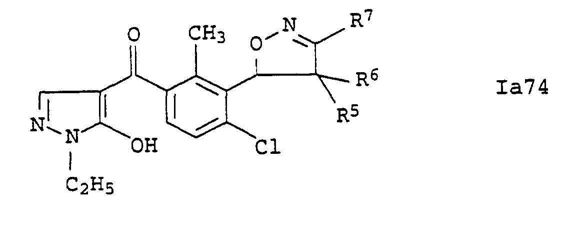

Ebenso insbesondere bevorzugt sind die Verbindungen Ia75., besonders die Verbindungen Ia75.1 - Ia75.64, die sich von den Verbindungen Ia1.1 - Ia1.64 dadurch unterscheiden, daß R¹ für Methyl, R² für Chlor und R¹³ für Isopropyl stehen.

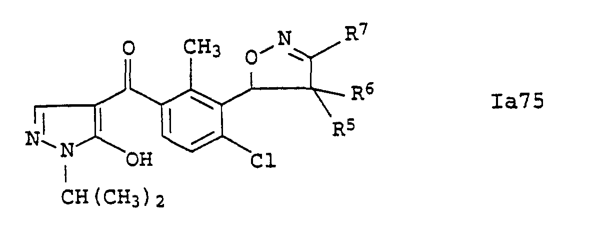

Ebenso insbesondere bevorzugt sind die Verbindungen Ia76., besonders die Verbindungen Ia76.1 - Ia76.64, die sich von den Verbindungen Ia1.1 - Ial.64 dadurch unterscheiden, daß R¹ für Methyl, R² für Chlor und R¹³ für tert.-Butyl stehen.

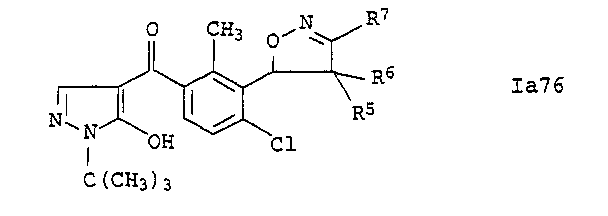

Ebenso insbesondere bevorzugt sind die Verbindungen Ia77., besonders die Verbindungen Ia77.1 - Ia77.64, die sich von den Verbindungen Ia1.1 - Ia1.64 dadurch unterscheiden, daß R¹ und R¹⁴ für Methyl und R² für Chlor stehen.

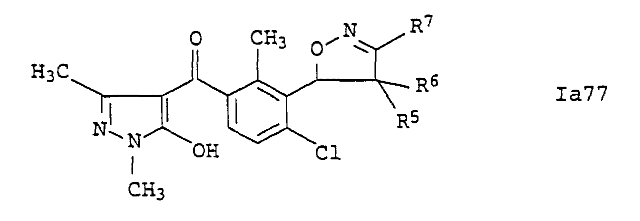

Ebenso insbesondere bevorzugt sind die Verbindungen Ia78., besonders die Verbindungen Ia78.1 - Ia78.64, die sich von den Verbindungen Ia1.1 - Ia1.64 dadurch unterscheiden, daß R¹ und R¹⁴ für Methyl, R² für Chlor und R¹³ für Ethyl stehen.

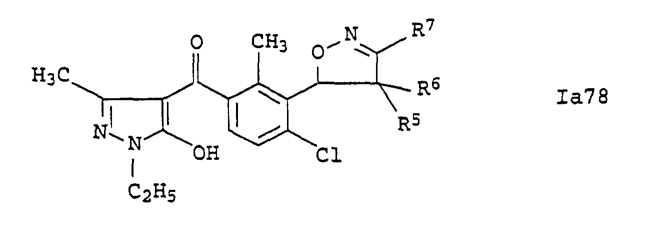

Ebenso insbesondere bevorzugt sind die Verbindungen Ia79., besonders die Verbindungen Ia79.1 - Ia79.64, die sich von den Verbindungen Ia1.1 - Ial.64 dadurch unterscheiden, daß R¹ und R¹⁴ für Methyl, R² für Chlor und R¹³ für Isopropyl stehen.

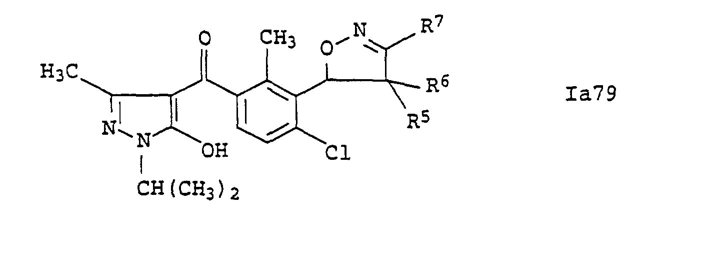

Ebenso insbesondere bevorzugt sind die Verbindungen Ia80., besonders die Verbindungen Ia80.1 - Ia80.64, die sich von den Verbindungen Ia1.1 - Ia1.64 dadurch unterscheiden, daß R¹ und R¹⁴ für Methyl, R² für Chlor und R¹³ für tert.-Butyl stehen.

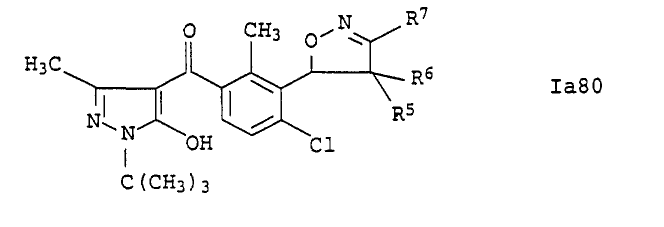

Ebenso insbesondere bevorzugt sind die Verbindungen Ia81., besonders die Verbindungen Ia81.1 - Ia81.64, die sich von den Verbindungen Ia1.1 - Ial.64 dadurch unterscheiden, daß R¹ für Trifluormethyl und R² für Chlor stehen.

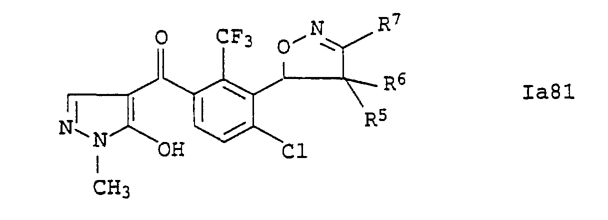

Ebenso insbesondere bevorzugt sind die Verbindungen Ia82., besonders die Verbindungen Ia82.1 - Ia82.64, die sich von den Verbindungen Ia1.1 - Ia1.64 dadurch unterscheiden, daß R¹ für Trifluormethyl, R² für Chlor und R¹³ für Ethyl stehen.

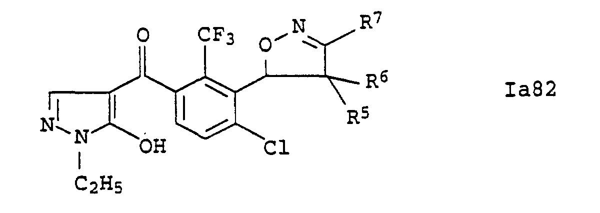

Ebenso insbesondere bevorzugt sind die Verbindungen Ia83., besonders die Verbindungen Ia83.1 - Ia83.64, die sich von den Verbindungen Ia1.1 - Ia1.64 dadurch unterscheiden, daß R¹ für Trifluormethyl, R² für Chlor und R¹³ für Isopropyl stehen.

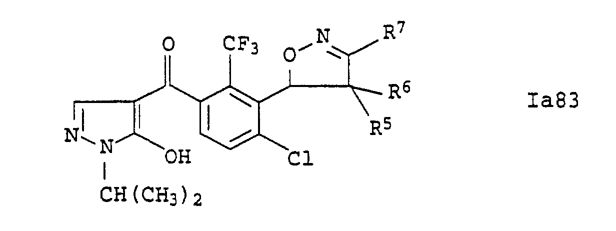

Ebenso insbesondere bevorzugt sind die Verbindungen Ia84., besonders die Verbindungen Ia84.1 - Ia84.64, die sich von den Verbindungen Ia1.1 - Ial.64 dadurch unterscheiden, daß R¹ für Trifluormethyl, R² Chlor und R¹³ für tert.-Butyl stehen.

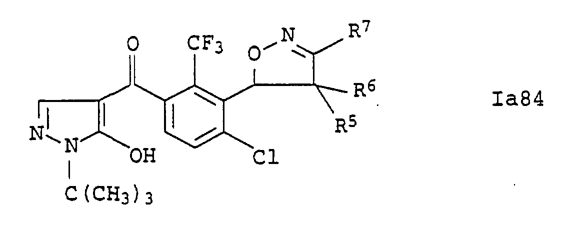

Ebenso insbesondere bevorzugt sind die Verbindungen Ia85., besonders die Verbindungen Ia85.1 - Ia85.64, die sich von den Verbindungen Ia1.1 - Ia1.64 dadurch unterscheiden, daß R¹ für Trifluormethyl, R² für Chlor und R¹⁴ für Methyl stehen.

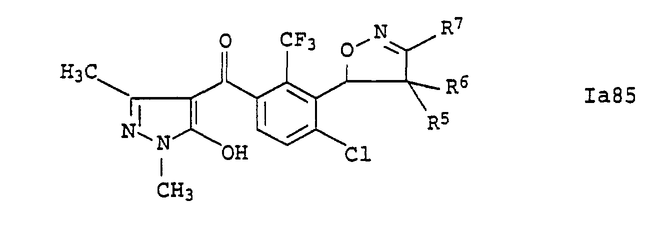

Ebenso insbesondere bevorzugt sind die Verbindungen Ia86., besonders die Verbindungen Ia86.1 - Ia86.64, die sich von den Verbindungen Ia1.1 - Ia1.64 dadurch unterscheiden, daß R¹ für Trifluormethyl, R² für Chlor, R¹³ für Ethyl und R¹⁴ für Methyl stehen.

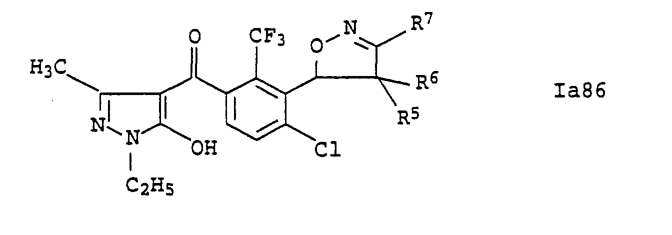

Ebenso insbesondere bevorzugt sind die Verbindungen Ia87., besonders die Verbindungen Ia87.1 - Ia87.64, die sich von den Verbindungen Ia1.1 - Ia1.64 dadurch unterscheiden, daß R¹ für Trifluormethyl, R² für Chlor, R¹³ für Isopropyl und R¹⁴ für Methyl stehen.

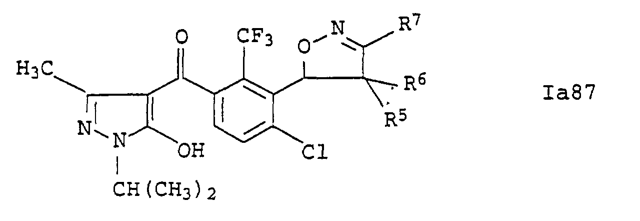

Ebenso insbesondere bevorzugt sind die Verbindungen Ia88., besonders die Verbindungen Ia88.1 - Ia88.64, die sich von den Verbindungen Ia1.1 - Ia1.64 dadurch unterscheiden, daß R¹ für Trifluormethyl, R² für Chlor, R¹³ für tert.-Butyl und R¹⁴ für Methyl stehen.

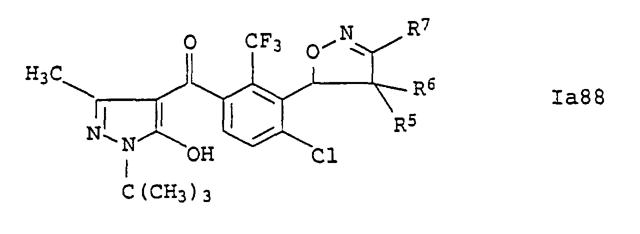

Ebenso insbesondere bevorzugt sind die Verbindungen Ia89., besonders die Verbindungen Ia89.1 - Ia89.64, die sich von den Verbindungen Ia1.1 - Ia1.64 dadurch unterscheiden, daß R¹ für Methoxy und R² für Chlor stehen.

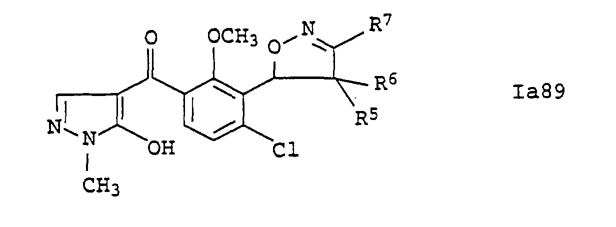

Ebenso insbesondere bevorzugt sind die Verbindungen Ia90., besonders die Verbindungen Ia90.1 - Ia90.64, die sich von den Verbindungen Ia1.1 - Ia1.64 dadurch unterscheiden, daß R¹ für Methoxy, R² für Chlor und R¹³ für Ethyl stehen.

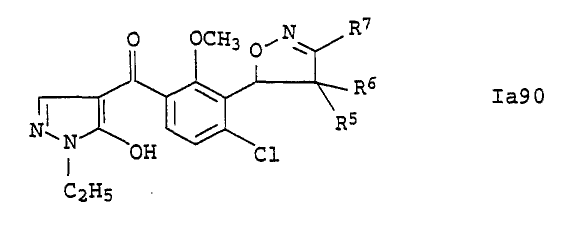

Ebenso insbesondere bevorzugt sind die Verbindungen Ia91., besonders die Verbindungen Ia91.1 - Ia91.64, die sich von den Verbindungen Ia1.1 - Ia1.64 dadurch unterscheiden, daß R¹ für Methoxy, R² für Chlor und R¹³ für Isopropyl stehen.

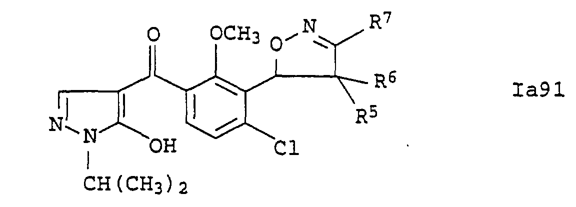

Ebenso insbesondere bevorzugt sind die Verbindungen Ia92., besonders die Verbindungen Ia92.1 - Ia92.64, die sich von den Verbindungen Ia1.1 - Ial.64 dadurch unterscheiden, daß R¹ für Methoxy, R² für Chlor und R¹³ für tert.-Butyl stehen.

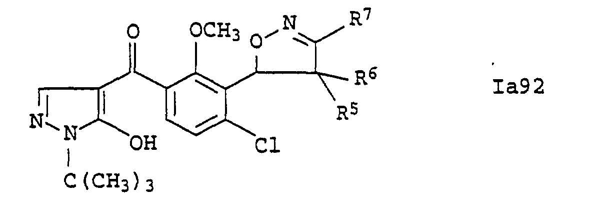

Ebenso insbesondere bevorzugt sind die Verbindungen Ia93., besonders die Verbindungen Ia93.1 - Ia93.64, die sich von den Verbindungen Ia1.1 - Ia1.64 dadurch unterscheiden, daß R¹ für Methoxy, R² für Chlor und R¹⁴ für Methyl stehen.

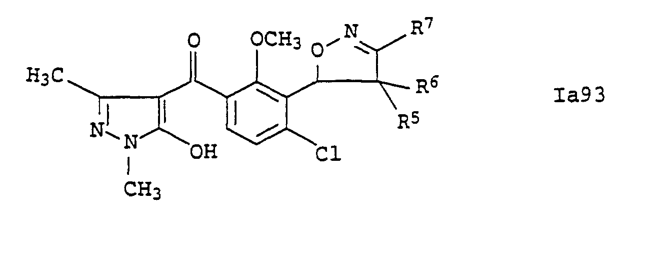

Ebenso insbesondere bevorzugt sind die Verbindungen Ia94., besonders die Verbindungen Ia94.1 - Ia94.64, die sich von den Verbindungen Ia1.1 - Ia1.64 dadurch unterscheiden, daß R¹ für Methoxy, R² für Chlor, R¹³ für Ethyl und R¹⁴ für Methyl stehen.

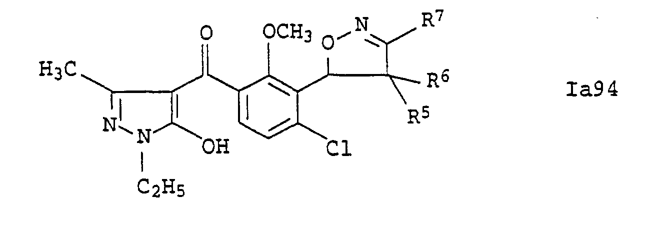

Ebenso insbesondere bevorzugt sind die Verbindungen Ia95., besonders die Verbindungen Ia95.1 - Ia95.64, die sich von den Verbindungen Ia1.1 - Ia1.64 dadurch unterscheiden, daß R¹ für Methoxy, R² für Chlor, R¹³ für Isopropyl und R¹⁴ für Methyl stehen.

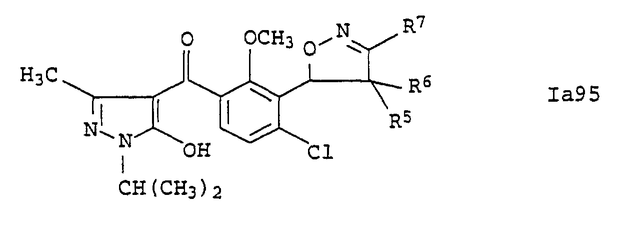

Ebenso insbesondere bevorzugt sind die Verbindungen Ia96., besonders die Verbindungen Ia96.1 - Ia96.64, die sich von den Verbindungen Ia1.1 - Ia1.64 dadurch unterscheiden, daß R¹ für Methoxy, R² für Chlor, R¹³ für tert.-Butyl und R¹⁴ für Methyl stehen.

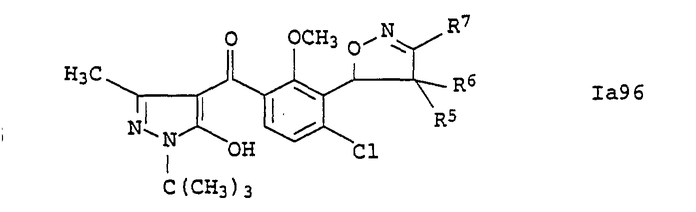

Ebenso insbesondere bevorzugt sind die Verbindungen Ia97., besonders die Verbindungen Ia97.1 - Ia97.64, die sich von den Verbindungen Ia1.1 - Ia1.64 dadurch unterscheiden, daß R² für Trifluormethyl steht.

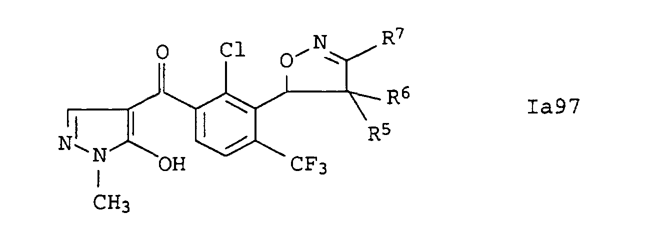

Ebenso insbesondere bevorzugt sind die Verbindungen Ia98., besonders die Verbindungen Ia98.1 - Ia98.64, die sich von den Verbindungen Ia1.1 - Ia1.64 dadurch unterscheiden, daß R² für Trifluormethyl und R¹³ für Ethyl stehen.

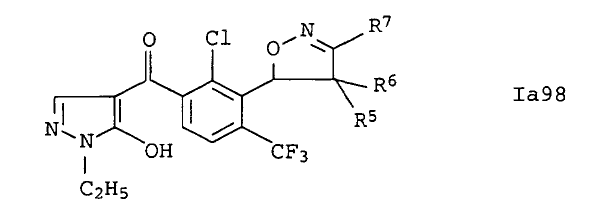

Ebenso insbesondere bevorzugt sind die Verbindungen Ia99., besonders die Verbindungen Ia99.1 - Ia99.64, die sich von den Verbindungen Ia1.1 - Ia1.64 dadurch unterscheiden, daß R² für Trifluormethyl und R¹³ für Isopropyl stehen.

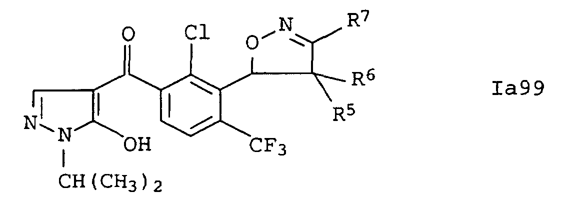

Ebenso insbesondere bevorzugt sind die Verbindungen Ia100., besonders die Verbindungen Ia100.1 - Ia100.64, die sich von den Verbindungen Ia1.1 - Ia1.64 dadurch unterscheiden, daß R² für Trifluormethyl und R¹³ für tert.-Butyl stehen.

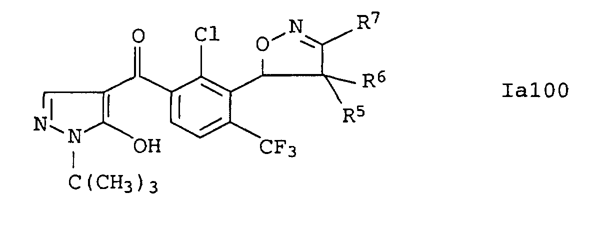

Ebenso insbesondere bevorzugt sind die Verbindungen Ia101., besonders die Verbindungen Ia101.1 - Ia101.64, die sich von den Verbindungen Ia1.1 - Ia1.64 dadurch unterscheiden, daß R² für Trifluormethyl und R¹⁴ für Methyl stehen.

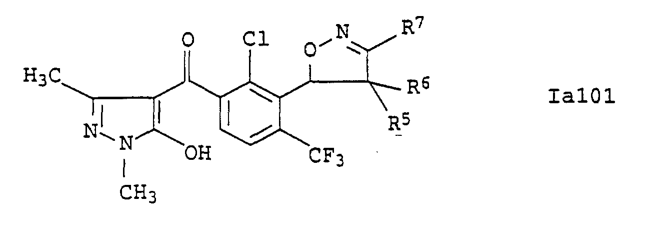

Ebenso insbesondere bevorzugt sind die Verbindungen Ia102., besonders die Verbindungen Ia102.1 - Ia102.64, die sich von den Verbindungen Ia1.1 - Ial.64 dadurch unterscheiden, daß R² für Trifluormethyl, R¹³ für Ethyl und R¹⁴ für Methyl stehen.

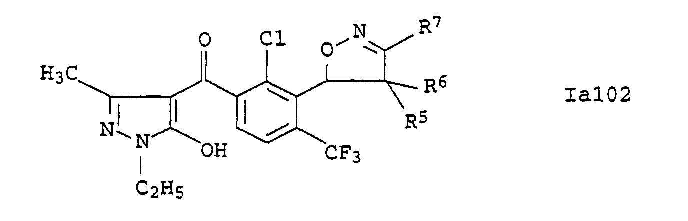

Ebenso insbesondere bevorzugt sind die Verbindungen Ia103., besonders die Verbindungen Ia103.1 - Ia103.64, die sich von den Verbindungen Ia1.1 - Ia1.64 dadurch unterscheiden, daß R² für Trifluormethyl, R¹³ für Isopropyl und R¹⁴ für Methyl stehen.

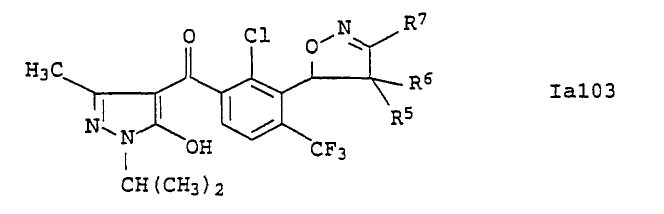

Ebenso insbesondere bevorzugt sind die Verbindungen Ia104., besonders die Verbindungen Ia104.1 - Ia104.64, die sich von den Verbindungen Ia1.1 - Ia1.64 dadurch unterscheiden, daß R² für Trifluormethyl, R¹³ für tert.-Butyl und R¹⁴ für Methyl stehen.

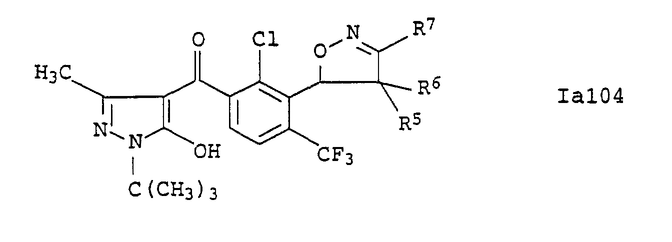

Ebenso insbesondere bevorzugt sind die Verbindungen Ia105., besonders die Verbindungen Ia105.1 - Ia105.64, die sich von den Verbindungen Ia1.1 - Ia1.64 dadurch unterscheiden, daß R¹ für Methyl und R² für Trifluormethyl stehen.

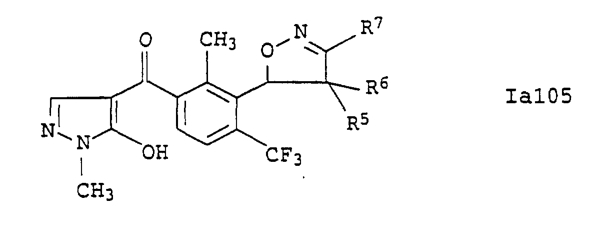

Ebenso insbesondere bevorzugt sind die Verbindungen Ia106., besonders die Verbindungen Ia106.1 - Ia106.64, die sich von den Verbindungen Ia1.1 - Ial.64 dadurch unterscheiden, daß R¹ für Methyl, R² für Trifluormethyl und R¹³ für Ethyl stehen.

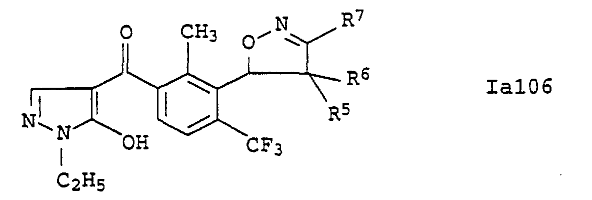

Ebenso insbesondere bevorzugt sind die Verbindungen Ia107., besonders die Verbindungen Ia107.1 - Ia107.64, die sich von den Verbindungen Ia1.1 - Ia1.64 dadurch unterscheiden, daß R¹ für Methyl, R² für Trifluormethyl und R¹³ für Isopropyl stehen.

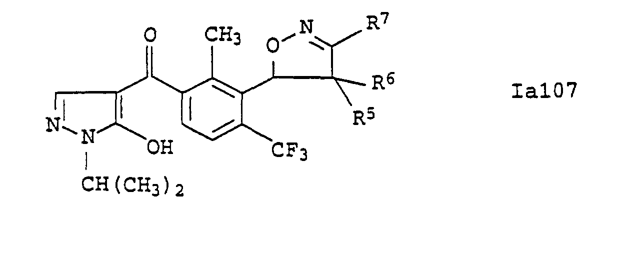

Ebenso insbesondere bevorzugt sind die Verbindungen Ia108., besonders die Verbindungen Ia108.1 - Ia108.64, die sich von den Verbindungen Ia1.1 - Ia1.64 dadurch unterscheiden, daß R¹ für Methyl, R² für Trifluormethyl und R¹³ für tert.-Butyl stehen.

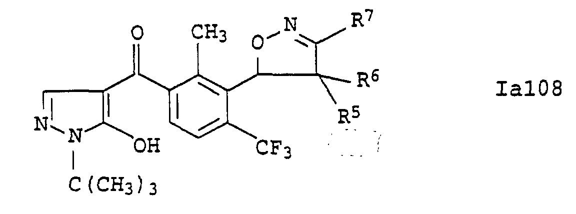

Ebenso insbesondere bevorzugt sind die Verbindungen Ia109., besonders die Verbindungen Ia109.1 - Ia109.64, die sich von den Verbindungen Ia1.1 - Ia1.64 dadurch unterscheiden, daß R¹ und R¹⁴ für Methyl und R² für Trifluormethyl stehen.

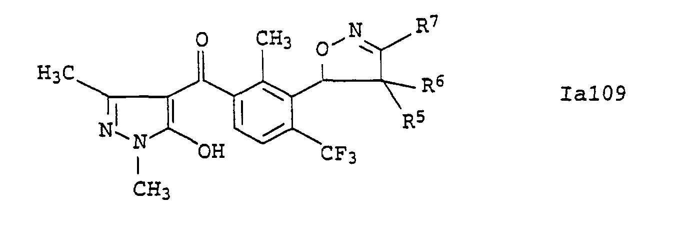

Ebenso insbesondere bevorzugt sind die Verbindungen Ia110., besonders die Verbindungen Ia110.1 - Ia110.64, die sich von den Verbindungen Ia1.1 - Ia1.64 dadurch unterscheiden, daß R¹ und R¹⁴ für Methyl, R² für Trifluormethyl und R¹³ für Ethyl stehen.

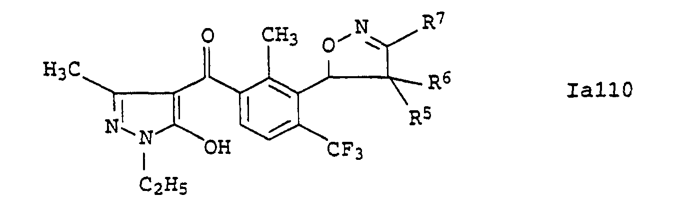

Ebenso insbesondere bevorzugt sind die Verbindungen Ia111., besonders die Verbindungen Ia111.1 - Ia111.64, die sich von den Verbindungen Ia1.1 - Ia1.64 dadurch unterscheiden, daß R¹ und R¹⁴ für Methyl, R² für Trifluormethyl und R¹³ für Isopropyl stehen.

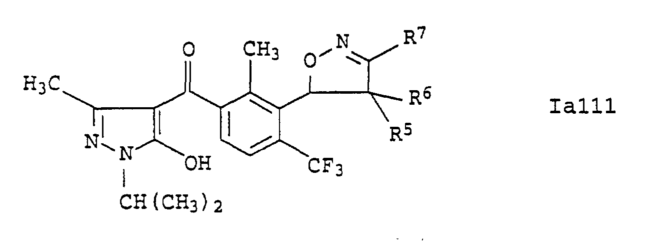

Ebenso insbesondere bevorzugt sind die Verbindungen Ia112., besonders die Verbindungen Ia112.1 - Ia112.64, die sich von den Verbindungen Ia1.1 - Ia1.64 dadurch unterscheiden, daß R¹ und R¹⁴ für Methyl, R² für Trifluormethyl und R¹³ für tert.-Butyl stehen.

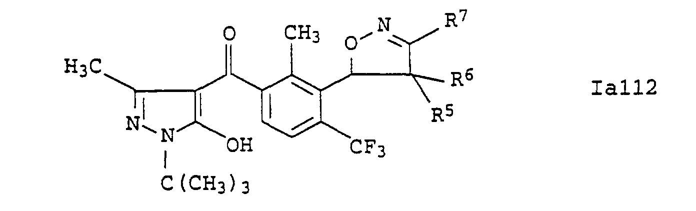

Ebenso insbesondere bevorzugt sind die Verbindungen Ia113., besonders die Verbindungen Ia113.1 - Ia113.64, die sich von den Verbindungen Ia1.1 - Ia1.64 dadurch unterscheiden, daß R¹ und R² für Trifluormethyl stehen.

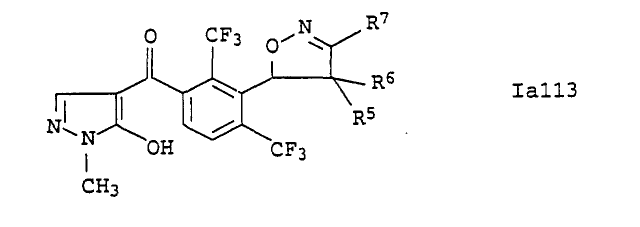

Ebenso insbesondere bevorzugt sind die Verbindungen Ia114., besonders die Verbindungen Ia114.1 - Ia114.64, die sich von den Verbindungen Ia1.1 - Ia1.64 dadurch unterscheiden, daß R¹ und R² für Trifluormethyl und R¹³ für Ethyl stehen.

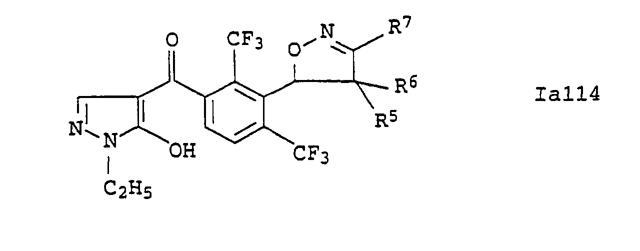

Ebenso insbesondere bevorzugt sind die Verbindungen Ia115., besonders die Verbindungen Ia115.1 - Ia115.64, die sich von den Verbindungen Ia1.1 - Ia1.64 dadurch unterscheiden, daß R¹ und R² für Trifluormethyl und R¹³ für Isopropyl stehen.

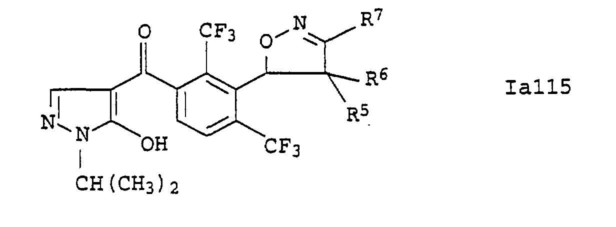

Ebenso insbesondere bevorzugt sind die Verbindungen Ia116., besonders die Verbindungen Ia116.1 - Ia116.64, die sich von den Verbindungen Ia1.1 - Ial.64 dadurch unterscheiden, daß R¹ und R² für Trifluormethyl und R¹³ für tert.-Butyl stehen.

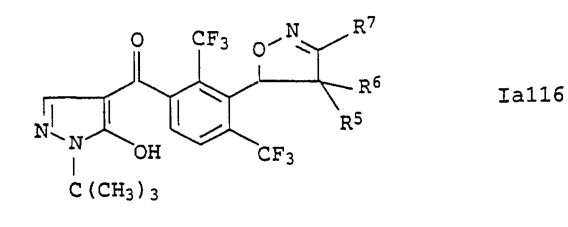

Ebenso insbesondere bevorzugt sind die Verbindungen Ia117., besonders die Verbindungen Ia117.1 - Ia117.64, die sich von den Verbindungen Ia1.1 - Ia1.64 dadurch unterscheiden, daß R¹ und R² für Trifluormethyl und R¹⁴ für Methyl stehen.

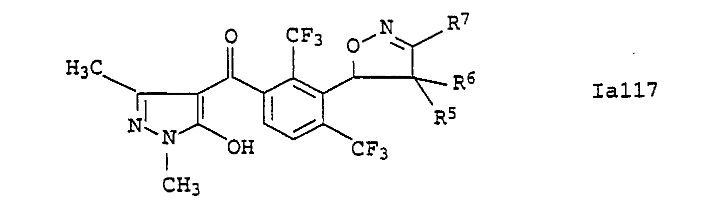

Ebenso insbesondere bevorzugt sind die Verbindungen Ia118., besonders die Verbindungen Ia118.1 - Ia118.64, die sich von den Verbindungen Ia1.1 - Ia1.64 dadurch unterscheiden, daß R¹ und R² für Trifluormethyl, R¹³ für Ethyl und R¹⁴ für Methyl stehen.

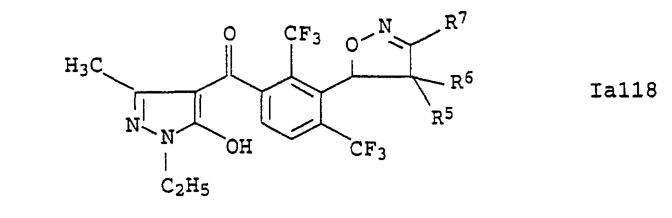

Ebenso insbesondere bevorzugt sind die Verbindungen Ia119., besonders die Verbindungen Ia119.1 - Ia119.64, die sich von den Verbindungen Ia1.1 - Ia1.64 dadurch unterscheiden, daß R¹ und R² für Trifluormethyl, R¹³ für Isopropyl und R¹⁴ für Methyl stehen.

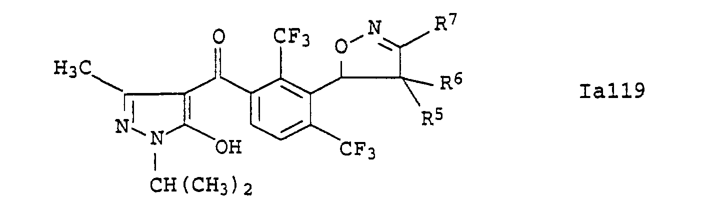

Ebenso insbesondere bevorzugt sind die Verbindungen Ia120., besonders die Verbindungen Ia120.1 - Ia120.64, die sich von den Verbindungen Ia1.1 - Ia1.64 dadurch unterscheiden, daß R¹ und R² für Trifluormethyl, R¹³ für tert.-Butyl und R¹⁴ für Methyl stehen.

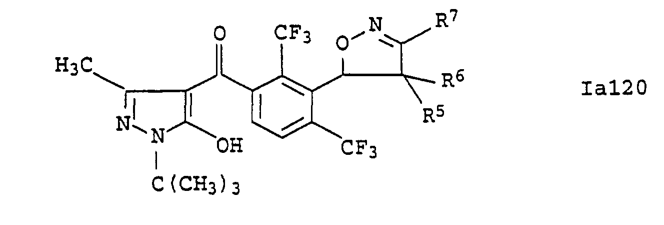

Ebenso insbesondere bevorzugt sind die Verbindungen Ia121., besonders die Verbindungen Ia121.1 - Ia121.64, die sich von den Verbindungen Ia1.1 - Ia1.64 dadurch unterscheiden, daß R¹ für Methoxy und R² für Trifluormethyl stehen.

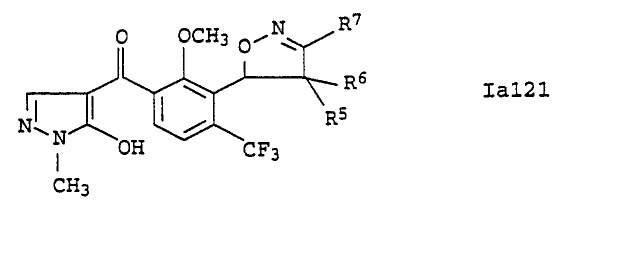

Ebenso insbesondere bevorzugt sind die Verbindungen Ia122., besonders die Verbindungen Ia122.1 - Ia122.64, die sich von den Verbindungen Ia1.1 - Ia1.64 dadurch unterscheiden, daß R¹ für Methoxy, R² für Trifluormethyl und R¹³ für Ethyl stehen.

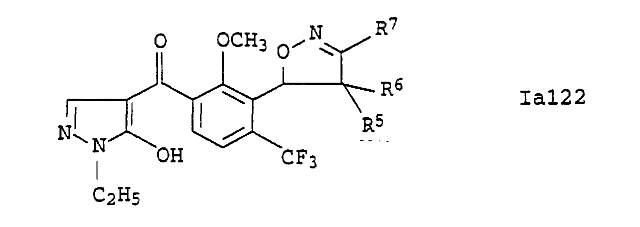

Ebenso insbesondere bevorzugt sind die Verbindungen Ia123., besonders die Verbindungen Ia123.1 - Ia123.64, die sich von den Verbindungen Ia1.1 - Ia1.64 dadurch unterscheiden, daß R¹ für Methoxy, R² für Trifluormethyl und R¹³ für Isopropyl stehen.

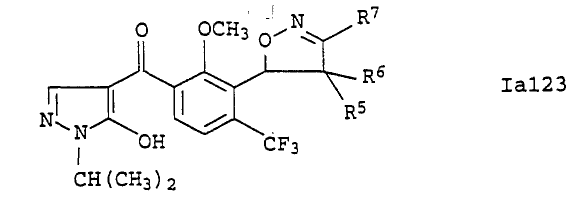

Ebenso insbesondere bevorzugt sind die Verbindungen Ia124., besonders die Verbindungen Ia124.1 - Ia124.64, die sich von den Verbindungen Ia1.1 - Ial.64 dadurch unterscheiden, daß R¹ für Methoxy, R² für Trifluormethyl und R¹³ für tert.-Butyl stehen.

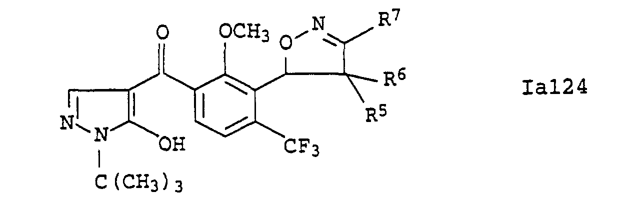

Ebenso insbesondere bevorzugt sind die Verbindungen Ia125., besonders die Verbindungen Ia125.1 - Ia125.64, die sich von den Verbindungen Ia1.1 - Ial.64 dadurch unterscheiden, daß R¹ für Methoxy, R² für Trifluormethyl und R¹⁴ für Methyl stehen.

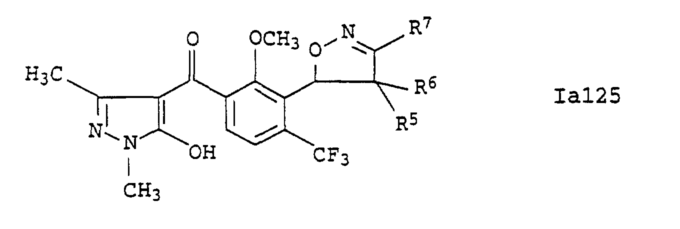

Ebenso insbesondere bevorzugt sind die Verbindungen Ia126., besonders die Verbindungen Ia126.1 - Ia126.64, die sich von den Verbindungen Ia1.1 - Ia1.64 dadurch unterscheiden, daß R¹ für Methoxy, R² für Trifluormethyl, R¹³ für Ethyl und R¹⁴ für Methyl stehen.

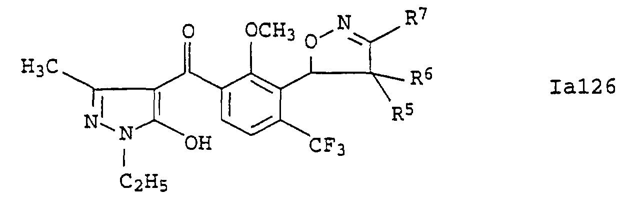

Ebenso insbesondere bevorzugt sind die Verbindungen Ia127., besonders die Verbindungen Ia127.1 - Ia127.64, die sich von den Verbindungen Ia1.1 - Ia1.64 dadurch unterscheiden, daß R¹ für Methoxy, R² für Trifluormethyl, R¹³ für Isopropyl und R¹⁴ für Methyl stehen.

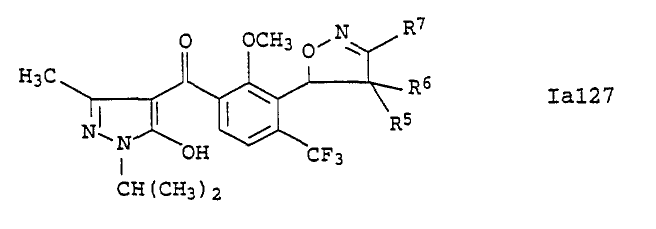

Ebenso insbesondere bevorzugt sind die Verbindungen Ia128., besonders die Verbindungen Ia128.1 - Ia128.64, die sich von den Verbindungen Ia1.1 - Ial.64 dadurch unterscheiden, daß R¹ für Methoxy, R² für Trifluormethyl, R¹³ für tert.-Butyl und R¹⁴ für Methyl stehen.

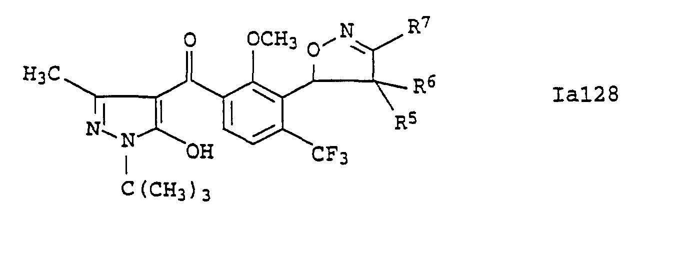

Ebenso insbesondere bevorzugt sind die Verbindungen Ia129., besonders die Verbindungen Ia129.1 - Ia129.64, die sich von den Verbindungen Ia1.1 - Ial.64 dadurch unterscheiden, daß R³ für Chlor steht.

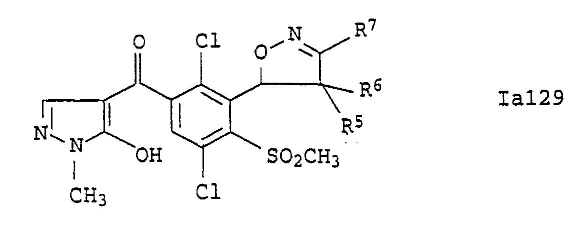

Ebenso insbesondere bevorzugt sind die Verbindungen Ia130., besonders die Verbindungen Ia130.1 - Ia130.64, die sich von den Verbindungen Ia1.1 - Ia1.64 dadurch unterscheiden, daß R³ für Chlor und R¹³ für Ethyl steht.

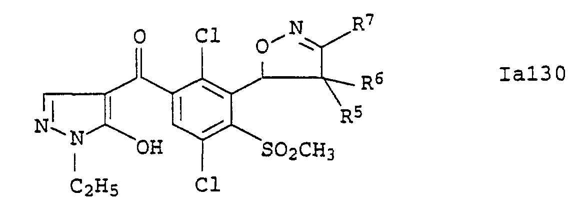

Ebenso insbesondere bevorzugt sind die Verbindungen Ia131., besonders die Verbindungen Ia131.1 - Ia131.64, die sich von den Verbindungen Ia1.1 - Ia1.64 dadurch unterscheiden, daß R³ für Chlor und R¹³ für Isopropyl steht.

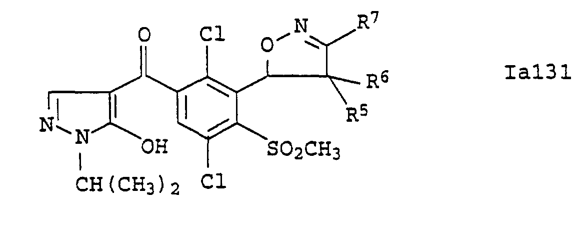

Ebenso insbesondere bevorzugt sind die Verbindungen Ia132., besonders die Verbindungen Ia132.1 - Ia132.64, die sich von den Verbindungen Ia1.1 - Ia1.64 dadurch unterscheiden, daß R³ für Chlor und R¹³ für tert.-Butyl steht.

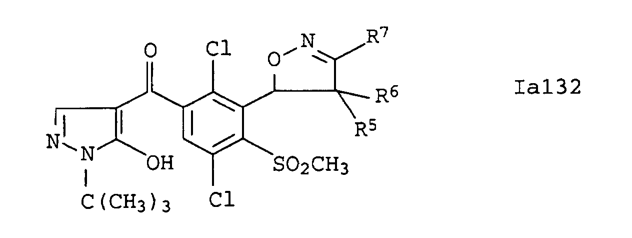

Ebenso insbesondere bevorzugt sind die Verbindungen Ia133., besonders die Verbindungen Ia133.1 - Ia133.64, die sich von den Verbindungen Ia1.1 - Ia1.64 dadurch unterscheiden, daß R³ für Chlor und R¹⁴ für Methyl steht.

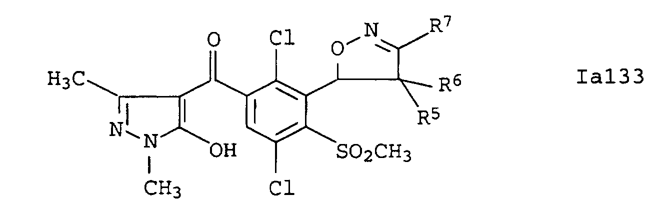

Ebenso insbesondere bevorzugt sind die Verbindungen Ia134., besonders die Verbindungen Ia134.1 - Ia134.64, die sich von den Verbindungen Ia1.1 - Ia1.64 dadurch unterscheiden, daß R³ für Chlor, R¹³ für Ethyl und R¹⁴ für Methyl stehen.

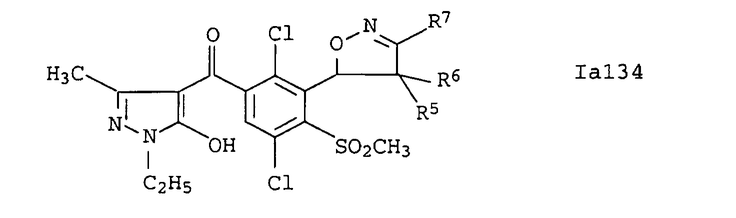

Ebenso insbesondere bevorzugt sind die Verbindungen Ia135., besonders die Verbindungen Ia135.1 - Ia135.64, die sich von den Verbindungen Ia1.1 - Ia1.64 dadurch unterscheiden, daß R³ für Chlor, R¹³ für Isopropyl und R¹⁴ für Methyl stehen.

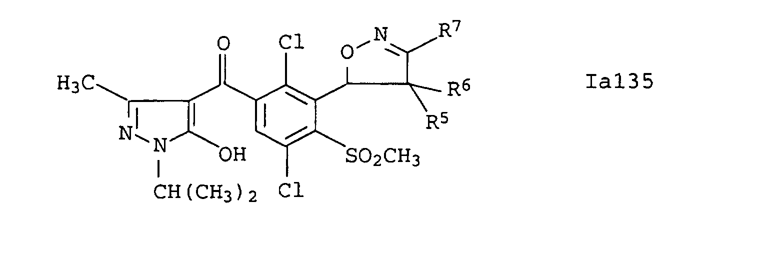

Ebenso insbesondere bevorzugt sind die Verbindungen Ia136., besonders die Verbindungen Ia136.1 - Ia136.64, die sich von den Verbindungen Ia1.1 - Ial.64 dadurch unterscheiden, daß R³ für Chlor, R¹³ für tert.-Butyl und R¹⁴ für Methyl stehen.

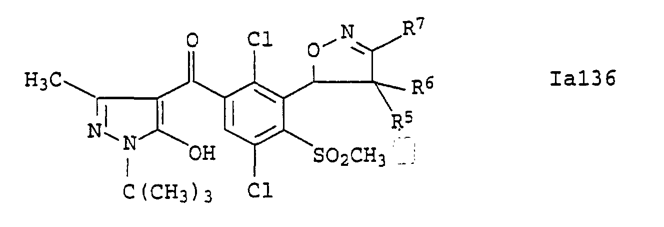

Ebenso insbesondere bevorzugt sind die Verbindungen Ia137., besonders die Verbindungen Ia137.1 - Ia137.64, die sich von den Verbindungen Ia1.1 - Ia1.64 dadurch unterscheiden, daß R¹ für Methyl und R³ für Chlor stehen.

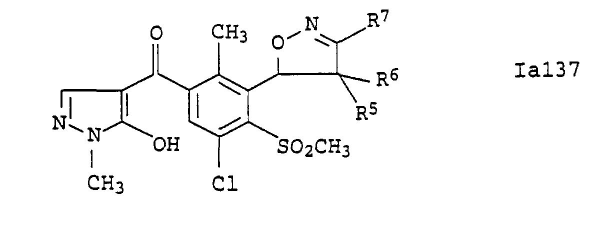

Ebenso insbesondere bevorzugt sind die Verbindungen Ia138., besonders die Verbindungen Ia238.1 - Ia138.64, die sich von den Verbindungen Ia1.1 - Ia1.64 dadurch unterscheiden, daß R¹ für Methyl, R³ für Chlor und R¹³ für Ethyl stehen.

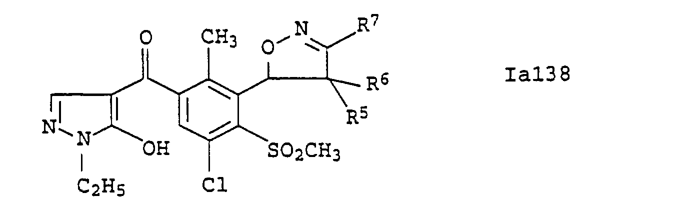

Ebenso insbesondere bevorzugt sind die Verbindungen Ia139., besonders die Verbindungen Ia139.1 - Ia139.64, die sich von den Verbindungen Ia1.1 - Ia1.64 dadurch unterscheiden, daß R¹ für Methyl, R³ für Chlor und R¹³ für Isopropyl stehen.

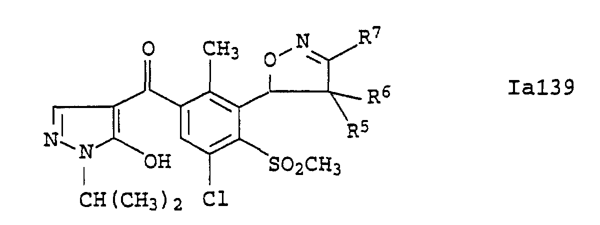

Ebenso insbesondere bevorzugt sind die Verbindungen Ia140., besonders die Verbindungen Ia140.1 - Ia140.64, die sich von den Verbindungen Ia1.1 - Ia1.64 dadurch unterscheiden, daß R¹ für Methyl, R³ für Chlor und R¹³ für tert.-Butyl stehen.

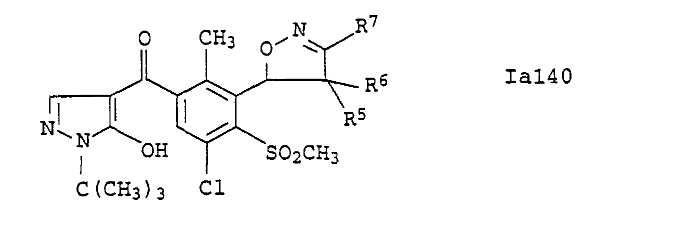

Ebenso insbesondere bevorzugt sind die Verbindungen Ia141., besonders die Verbindungen Ia141.1 - Ia141.64, die sich von den Verbindungen Ia1.1 - Ia1.64 dadurch unterscheiden, daß R¹ und R¹⁴ für Methyl und R³ für Chlor stehen.

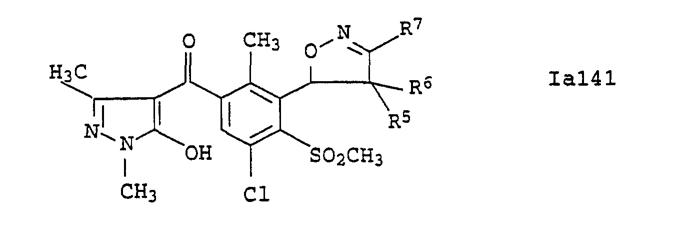

Ebenso insbesondere bevorzugt sind die Verbindungen Ia142., besonders die Verbindungen Ia142.1 - Ia142.64, die sich von den Verbindungen Ia1.1 - Ia1.64 dadurch unterscheiden, daß R¹ und R¹⁴ für Methyl, R³ für Chlor und R¹³ für Ethyl stehen.

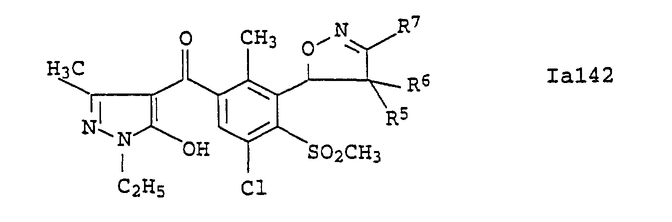

Ebenso insbesondere bevorzugt sind die Verbindungen Ia143., besonders die Verbindungen Ia143.I - Ia143.64, die sich von den Verbindungen Ia1.1 - Ia1.64 dadurch unterscheiden, daß R¹ und R¹⁴ für Methyl, R³ für Chlor und R¹³ für Isopropyl stehen.

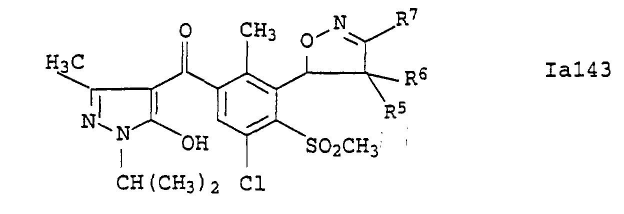

Ebenso insbesondere bevorzugt sind die Verbindungen Ia144., besonders die Verbindungen Ia144.1 - Ia144.64, die sich von den Verbindungen Ia1.1 - Ia1.64 dadurch unterscheiden, daß R¹ und R¹⁴ für Methyl, R³ für Chlor und R¹³ für tert.-Butyl stehen.

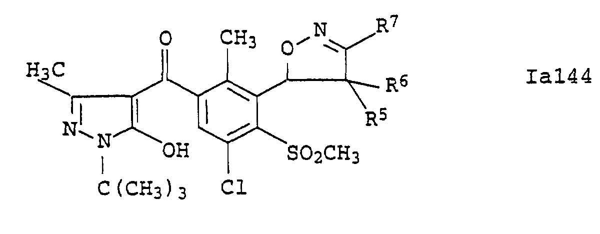

Ebenso insbesondere bevorzugt sind die Verbindungen Ia145., besonders die Verbindungen Ia145.1 - Ia145.64, die sich von den Verbindungen Ia1.1 - Ia1.64 dadurch unterscheiden, daß R² für Ethylsulfonyl und R³ für Chlor stehen.

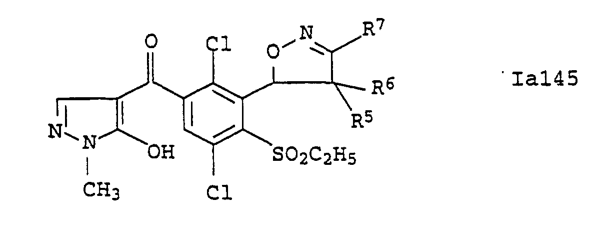

Ebenso insbesondere bevorzugt sind die Verbindungen Ia146., besonders die Verbindungen Ia146.1 - Ia146.64, die sich von den Verbindungen Ia1.1 - Ia1.64 dadurch unterscheiden, daß R² für Ethylsulfonyl, R³ für Chlor und R¹³ Ethyl stehen.

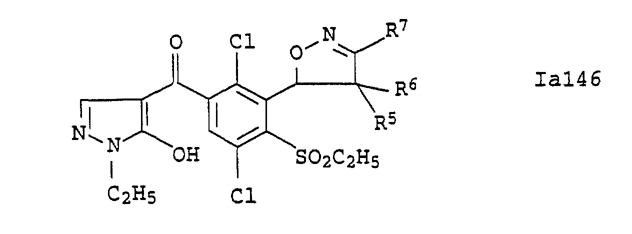

Ebenso insbesondere bevorzugt sind die Verbindungen Ia147., besonders die Verbindungen Ia147.1 - Ia147.64, die sich von den Verbindungen Ia1.1 - Ia1.64 dadurch unterscheiden, daß R² für Ethylsulfonyl, R³ für Chlor und R¹³ für Isopropyl stehen.

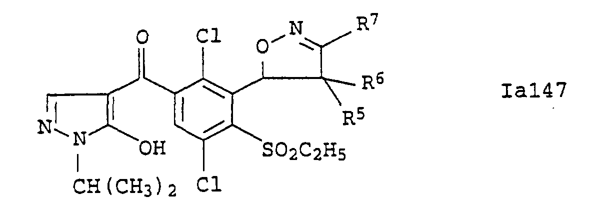

Ebenso insbesondere bevorzugt sind die Verbindungen Ia148., besonders die Verbindungen Ia148.1 - Ia148.64, die sich von den Verbindungen Ia1.1 - Ia1.64 dadurch unterscheiden, daß R² für Ethylsulfonyl, R³ für Chlor und R¹³ für tert.-Butyl stehen.

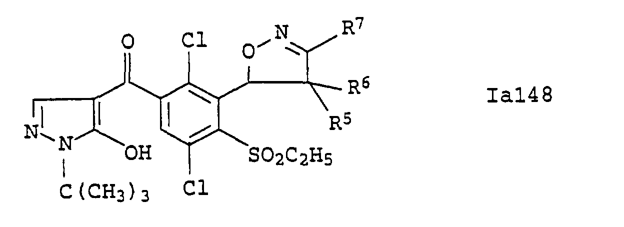

Ebenso insbesondere bevorzugt sind die Verbindungen Ia149., besonders die Verbindungen Ia149.1 - Ia149.64, die sich von den Verbindungen Ia1.1 - Ia1.64 dadurch unterscheiden, daß R² für Ethylsulfonyl, R³ für Chlor und R¹⁴ für Methyl stehen.

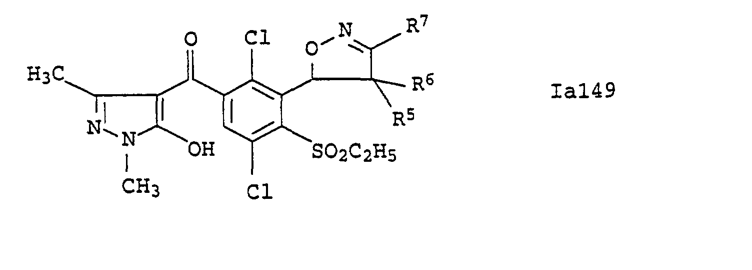

Ebenso insbesondere bevorzugt sind die Verbindungen Ia150., besonders die Verbindungen Ia150.1 - Ia150.64, die sich von den Verbindungen Ia1.1 - Ia1.64 dadurch unterscheiden, daß R² für Ethylsulfonyl, R³ für Chlor, R¹³ für Ethyl und R¹⁴ für Methyl stehen.

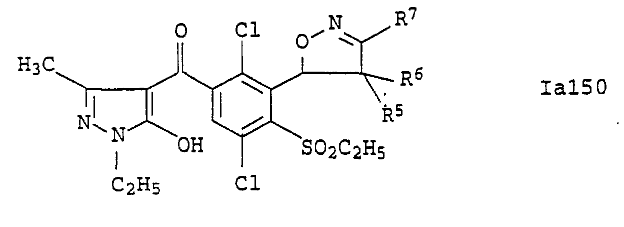

Ebenso insbesondere bevorzugt sind die Verbindungen Ia151., besonders die Verbindungen Ia151.1 - Ia151.64, die sich von den Verbindungen Ia1.1 - Ial.64 dadurch unterscheiden, daß R² für Ethylsulfonyl, R³ für Chlor, R¹³ für Isopropyl und R¹⁴ für Methyl stehen.

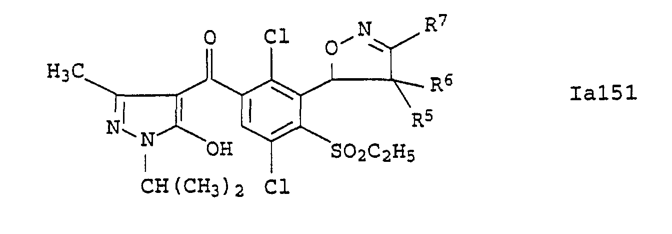

Ebenso insbesondere bevorzugt sind die Verbindungen Ia152., besonders die Verbindungen Ia152.1 - Ia152.64, die sich von den Verbindungen Ia1.1 - Ia1.64 dadurch unterscheiden, daß R² für Ethylsulfonyl, R³ für Chlor, R¹³ für tert.-Butyl und R¹⁴ für Methyl stehen.

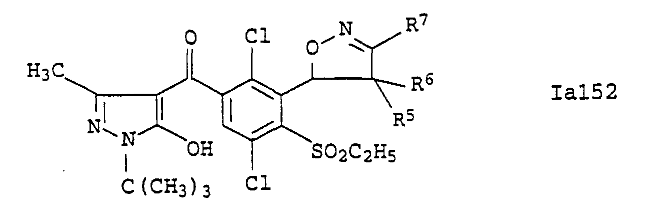

Ebenso insbesondere bevorzugt sind die Verbindungen Ia153., besonders die Verbindungen Ia153.1 - Ia153.64, die sich von den Verbindungen Ia1.1 - Ia1.64 dadurch unterscheiden, daß R¹ für Methyl und R² für Ethylsulfonyl und R³ für Chlor stehen.

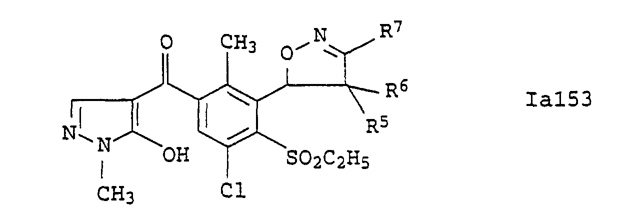

Ebenso insbesondere bevorzugt sind die Verbindungen Ia154., besonders die Verbindungen Ia154.1 - Ia154.64, die sich von den Verbindungen Ia1.1 - Ia1.64 dadurch unterscheiden, daß R¹ für Methyl, R² für Ethylsulfonyl, R³ für Chlor und R¹³ für Ethyl stehen.

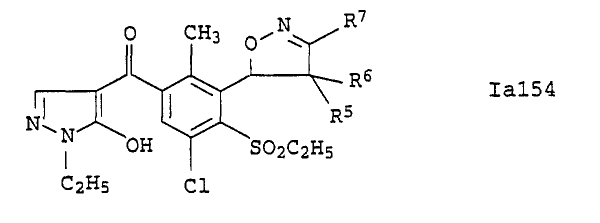

Ebenso insbesondere bevorzugt sind die Verbindungen Ia155., besonders die Verbindungen Ia155.1 - Ia155.64, die sich von den Verbindungen Ia1.1 - Ia1.64 dadurch unterscheiden, daß R¹ für Methyl, R² für Ethylsulfonyl, R³ für Chlor und R¹³ für Isopropyl stehen.

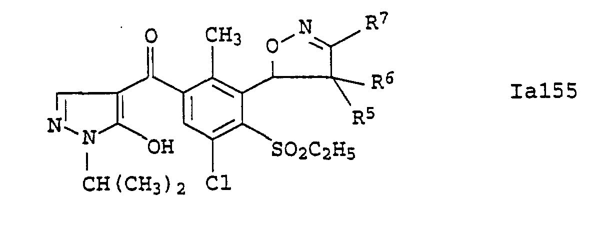

Ebenso insbesondere bevorzugt sind die Verbindungen Ia156., besonders die Verbindungen Ia156.1 - Ia156.64, die sich von den Verbindungen Ia1.1 - Ia1.64 dadurch unterscheiden, daß R¹ für Methyl, R² für Ethylsulfonyl, R³ für Chlor und R¹³ für tert.-Butyl stehen.

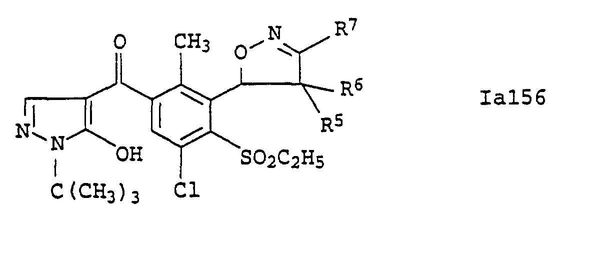

Ebenso insbesondere bevorzugt sind die Verbindungen Ia157., besonders die Verbindungen Ia157.1 - Ia157.64, die sich von den Verbindungen Ia1.1 - Ia1.64 dadurch unterscheiden, daß R¹ und R¹⁴ für Methyl, R² für Ethylsulfonyl und R³ für Chlor stehen.

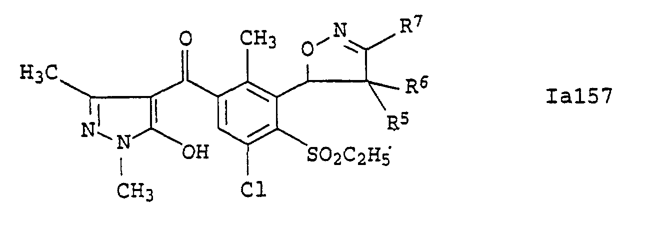

Ebenso insbesondere bevorzugt sind die Verbindungen Ia158., besonders die Verbindungen Ia158.1 - Ia158.64, die sich von den Verbindungen Ia1.1 - Ia1.64 dadurch unterscheiden, daß R¹ und R¹⁴ für Methyl, R² für Ethylsulfonyl, R³ für Chlor und R¹³ für Ethyl stehen.

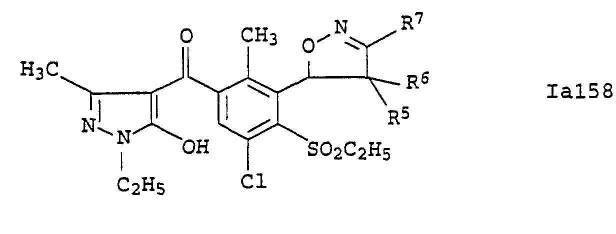

Ebenso insbesondere bevorzugt sind die Verbindungen Ia159., besonders die Verbindungen Ia159.1 - Ia159.64, die sich von den Verbindungen Ia1.1 - Ia1.64 dadurch unterscheiden, daß R¹ und R¹⁴ für Methyl, R² für Ethylsulfonyl, R³ für Chlor und R¹³ für Isopropyl stehen.

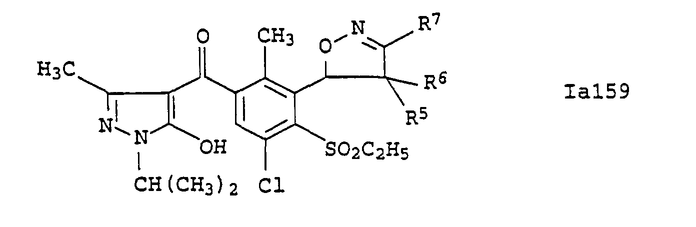

Ebenso insbesondere bevorzugt sind die Verbindungen Ia160., besonders die Verbindungen Ia160.1 - Ia160.64, die sich von den Verbindungen Ia1.1 - Ia1.64 dadurch unterscheiden, daß R¹ und R¹⁴ für Methyl, R² für Ethylsulfonyl, R³ für Chlor und R¹³ für tert.-Butyl stehen.

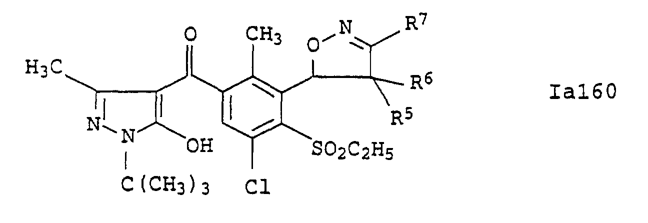

Ebenso insbesondere bevorzugt sind die Verbindungen Ia161., besonders die Verbindungen Ia161.1 - Ia161.64, die sich von den Verbindungen Ia1.1 - Ial.64 dadurch unterscheiden, daß R² und R³ für Chlor stehen.

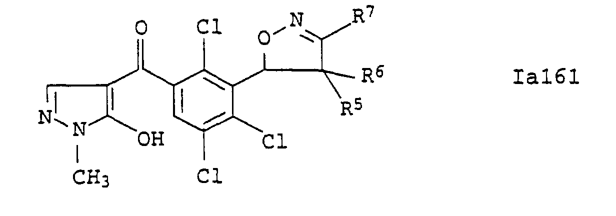

Ebenso insbesondere bevorzugt sind die Verbindungen Ia162., besonders die Verbindungen Ia162.1 - Ia162.64, die sich von den Verbindungen Ia1.1 - Ia1.64 dadurch unterscheiden, daß R² und R³ für Chlor und R¹³ für Ethyl stehen.

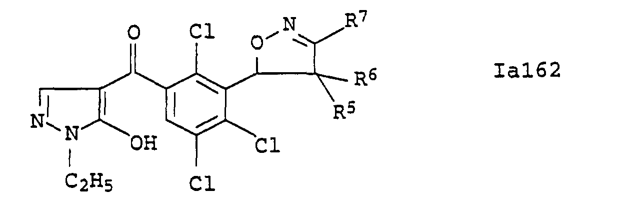

Ebenso insbesondere bevorzugt sind die Verbindungen Ia163., besonders die Verbindungen Ia163.1 - Ia163.64, die sich von den Verbindungen Ia1.1 - Ia1.64 dadurch unterscheiden, daß R² und R³ für Chlor und R¹³ für Isopropyl stehen.

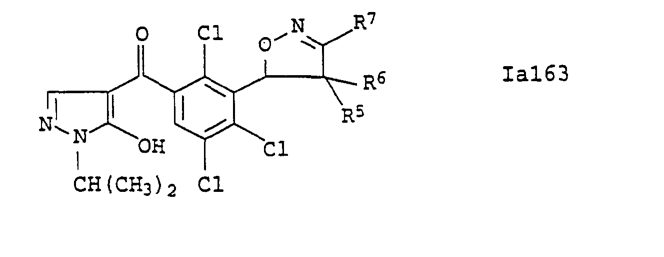

Ebenso insbesondere bevorzugt sind die Verbindungen Ia164., besonders die Verbindungen Ia164.1 - Ia164.64, die sich von den Verbindungen Ia1.1 - Ia1.64 dadurch unterscheiden, daß R² und R³ für Chlor und R¹³ für tert.-Butyl stehen.

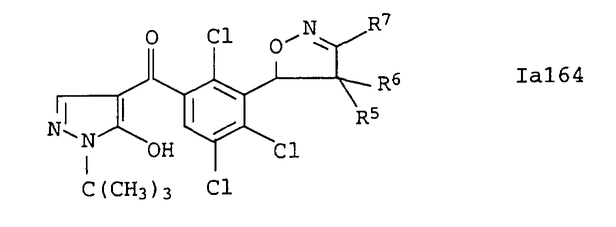

Ebenso insbesondere bevorzugt sind die Verbindungen Ia165., besonders die Verbindungen Ia165.1 - Ia165.64, die sich von den Verbindungen Ia1.1 - Ial.64 dadurch unterscheiden, daß R² und R³ für Chlor und R¹⁴ für Methyl stehen.

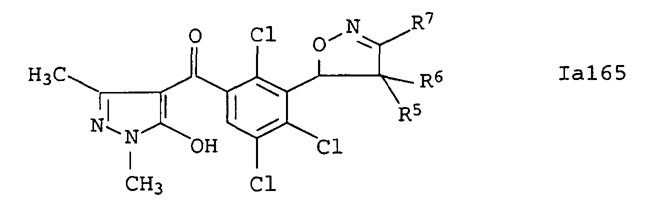

Ebenso insbesondere bevorzugt sind die Verbindungen Ia166., besonders die Verbindungen Ia166.1 - Ia166.64, die sich von den Verbindungen Ia1.1 - Ia1.64 dadurch unterscheiden, daß R² und R³ für Chlor, R¹³ für Ethyl und R¹⁴ für Methyl stehen.

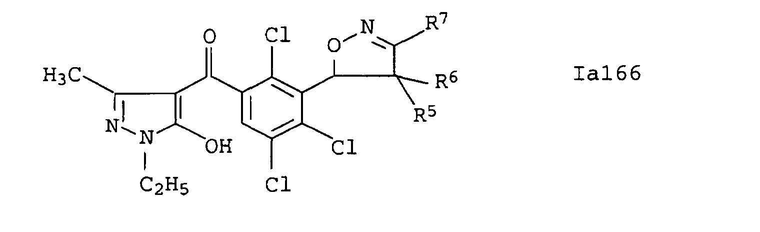

Ebenso insbesondere bevorzugt sind die Verbindungen Ia167., besonders die Verbindungen Ia167.1 - Ia167.64, die sich von den Verbindungen Ia1.1 - Ia1.64 dadurch unterscheiden, daß R² und R³ für Chlor, R¹³ für Isopropyl und R¹⁴ für Methyl stehen.

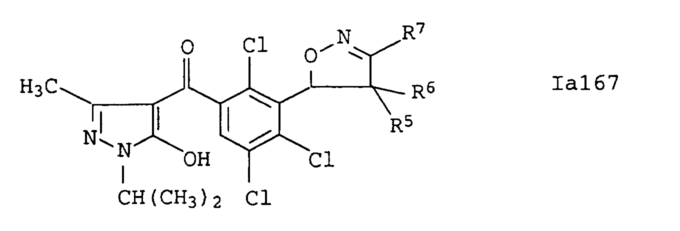

Ebenso insbesondere bevorzugt sind die Verbindungen Ia168., besonders die Verbindungen Ia168.1 - Ia168.64, die sich von den Verbindungen Ia1.1 - Ia1.64 dadurch unterscheiden, daß R² und R³ für Chlor, R¹³ für tert.-Butyl und R¹⁴ für Methyl stehen.

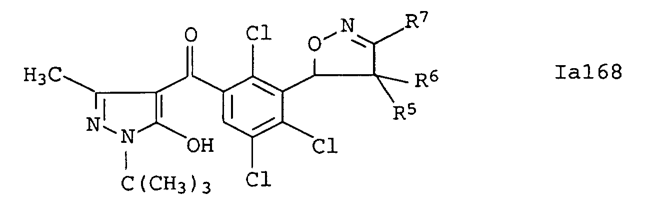

Ebenso insbesondere bevorzugt sind die Verbindungen Ia169., besonders die Verbindungen Ia169.1 - Ia169.64, die sich von den Verbindungen Ia1.1 - Ia1.64 dadurch unterscheiden, daß R¹ für Methyl und R² und R³ für Chlor stehen.

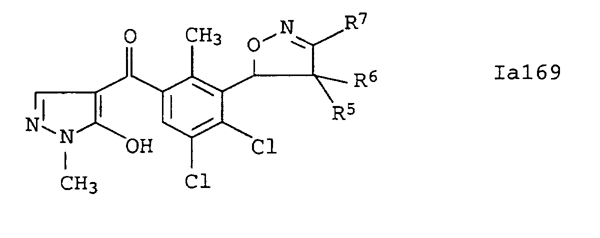

Ebenso insbesondere bevorzugt sind die Verbindungen Ia170., besonders die Verbindungen Ia170.1 - Ia170.64, die sich von den Verbindungen Ia1.1 - Ia1.64 dadurch unterscheiden, daß R¹ für Methyl, R² und R³ für Chlor und R¹³ für Ethyl stehen.

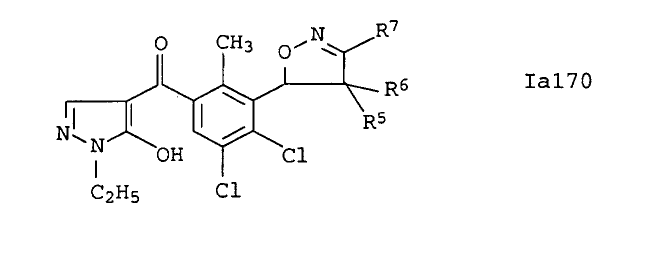

Ebenso insbesondere bevorzugt sind die Verbindungen Ia171., besonders die Verbindungen Ia171.1 - Ia171.64, die sich von den Verbindungen Ia1.1 - Ia1.64 dadurch unterscheiden, daß R¹ für Methyl, R² und R³ für Chlor und R¹³ für Isopropyl stehen.

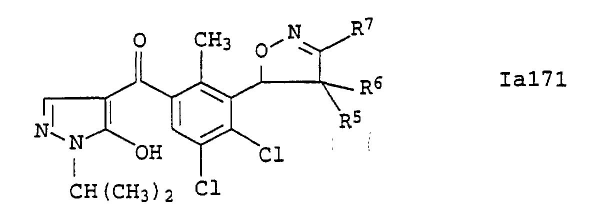

Ebenso insbesondere bevorzugt sind die Verbindungen Ia172., besonders die Verbindungen Ia172.1 - Ia172.64, die sich von den Verbindungen Ia1.1 - Ial.64 dadurch unterscheiden, daß R¹ für Methyl, R² und R³ für Chlor und R¹³ für tert.-Butyl stehen.

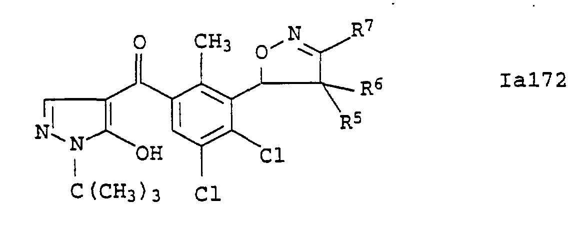

Ebenso insbesondere bevorzugt sind die Verbindungen Ia173., besonders die Verbindungen Ia173.1 - Ia173.64, die sich von den Verbindungen Ia1.1 - Ia1.64 dadurch unterscheiden, daß R¹ und R¹⁴ für Methyl und R² und R³ für Chlor stehen.

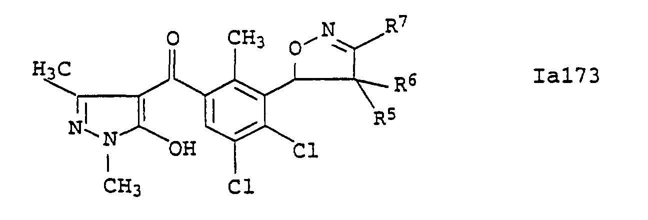

Ebenso insbesondere bevorzugt sind die Verbindungen Ia174., besonders die Verbindungen Ia174.1 - Ia174.64, die sich von den Verbindungen Ia1.1 - Ia1.64 dadurch unterscheiden, daß R¹ und R¹⁴ für Methyl, R² und R³ für Chlor und R¹³ für Ethyl stehen.

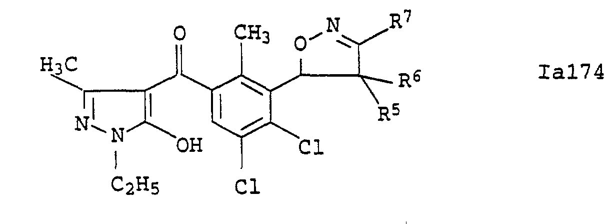

Ebenso insbesondere bevorzugt sind die Verbindungen Ia175., besonders die Verbindungen Ia175.1 - Ia175.64, die sich von den Verbindungen Ia1.1 - Ial.64 dadurch unterscheiden, daß R¹ und R¹⁴ für Methyl, R² und R³ für Chlor und R¹³ für Isopropyl stehen.

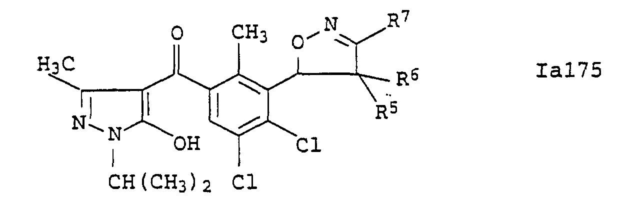

Ebenso insbesondere bevorzugt sind die Verbindungen Ia176., besonders die Verbindungen Ia176.1 - Ia176.64, die sich von den Verbindungen Ia1.1 - Ia1.64 dadurch unterscheiden, daß R¹ und R¹⁴ für Methyl, R² und R³ für Chlor und R¹³ für tert.-Butyl stehen.

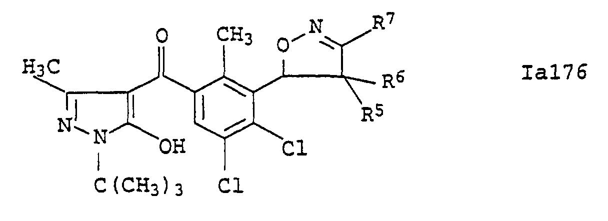

Ebenso insbesondere bevorzugt sind die Verbindungen Ia177., besonders die Verbindungen Ia177.1 - Ia177.64, die sich von den Verbindungen Ia1.1 - Ia1.64 dadurch unterscheiden, daß R¹ für Chlor und R³ für Methyl stehen.

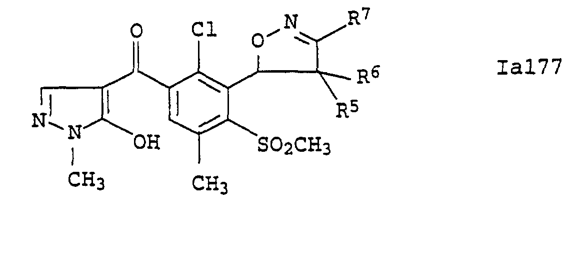

Ebenso insbesondere bevorzugt sind die Verbindungen Ia178., besonders die Verbindungen Ia178.1 - Ia178.64, die sich von den Verbindungen Ia1.1 - Ia1.64 dadurch unterscheiden, daß R³ für Methyl und R¹³ für Ethyl stehen.

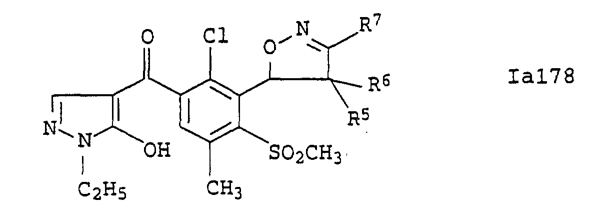

Ebenso insbesondere bevorzugt sind die Verbindungen Ia179., besonders die Verbindungen Ia179.1 - Ia178.64, die sich von den Verbindungen Ia1.1 - Ia1.64 dadurch unterscheiden, daß R³ für Methyl und R¹³ für Isopropyl stehen.

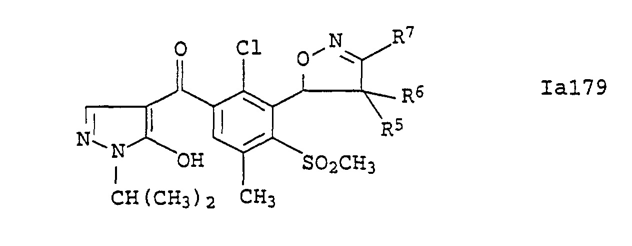

Ebenso insbesondere bevorzugt sind die Verbindungen Ia180., besonders die Verbindungen Ia180.1 - Ia180.64, die sich von den Verbindungen Ia1.1 - Ial.64 dadurch unterscheiden, daß R³ für Methyl und R¹³ für tert.-Butyl stehen.

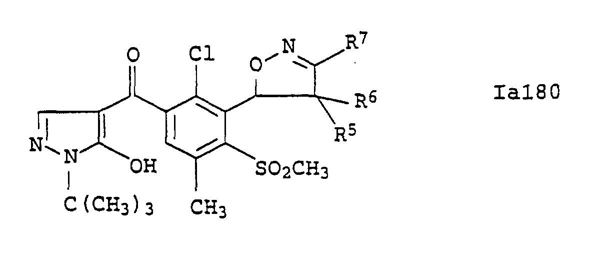

Ebenso insbesondere bevorzugt sind die Verbindungen Ia181., besonders die Verbindungen Ia181.1 - Ia181.64, die sich von den Verbindungen Ia1.1 - Ia1.64 dadurch unterscheiden, daß R³ für Methyl und R¹⁴ für Methyl stehen.

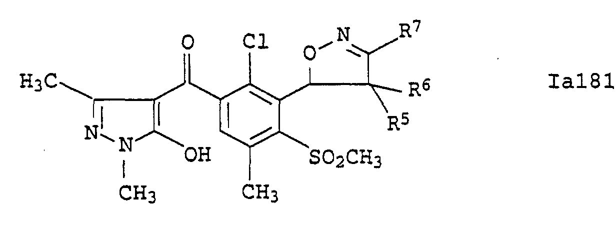

Ebenso insbesondere bevorzugt sind die Verbindungen Ia182., besonders die Verbindungen Ia182.1 - Ia182.64, die sich von den Verbindungen Ia1.1 - Ia1.64 dadurch unterscheiden, daß R³ für Methyl, R¹³ für Ethyl und R¹⁴ für Methyl stehen.

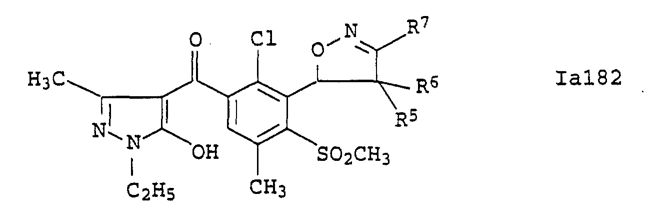

Ebenso insbesondere bevorzugt sind die Verbindungen Ia183., besonders die Verbindungen Ia183.1 - Ia183.64, die sich von den Verbindungen Ia1.1 - Ia1.64 dadurch unterscheiden, daß R³ für Methyl, R¹³ für Isopropyl und R¹⁴ für Methyl stehen.

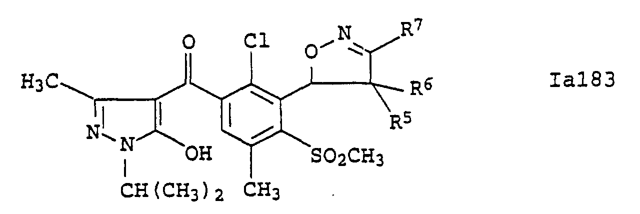

Ebenso insbesondere bevorzugt sind die Verbindungen Ia184., besonders die Verbindungen Ia184.1 - Ia184.64, die sich von den Verbindungen Ia1.1 - Ial.64 dadurch unterscheiden, daß R³ für Methyl, R¹³ für tert.-Butyl und R¹⁴ für Methyl stehen.

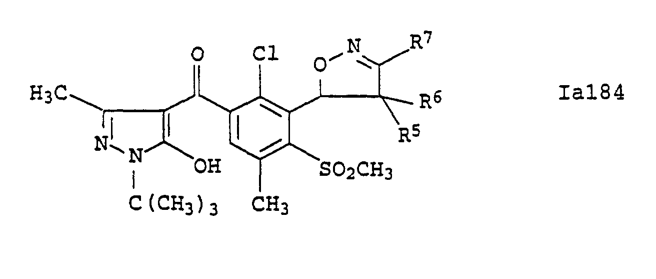

Ebenso insbesondere bevorzugt sind die Verbindungen Ia185., besonders die Verbindungen Ia185.1 - Ia185.64, die sich von den Verbindungen Ia1.1 - Ia1.64 dadurch unterscheiden, daß R¹ und R³ für Methyl stehen.

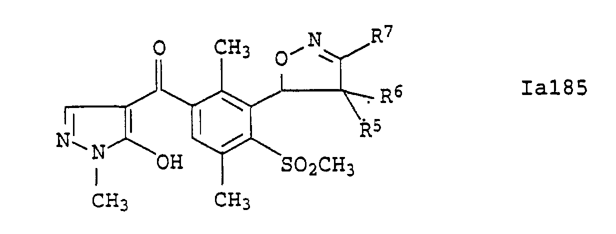

Ebenso insbesondere bevorzugt sind die Verbindungen Ia186., besonders die Verbindungen Ia186.1 - Ia186.64, die sich von den Verbindungen Ia1.2 - Ia1.64 dadurch unterscheiden, daß R¹ und R³ für Methyl und R¹³ für Ethyl stehen.

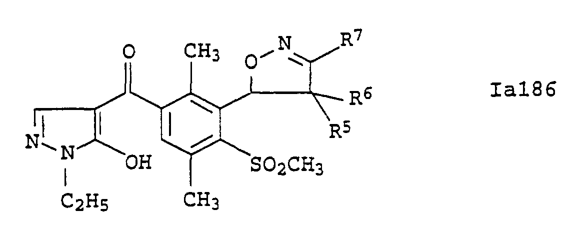

Ebenso insbesondere bevorzugt sind die Verbindungen Ia187., besonders die Verbindungen Ia187.1 - Ia187.64, die sich von den Verbindungen Ia1.1 - Ia1.64 dadurch unterscheiden, daß R¹ und R³ für Methyl und R¹³ für Isopropyl stehen.

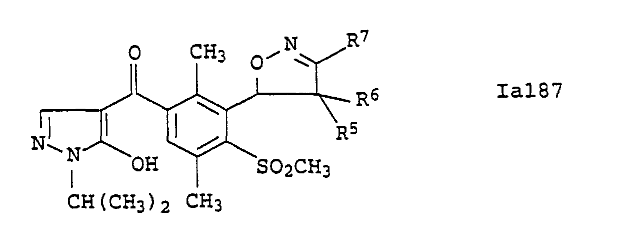

Ebenso insbesondere bevorzugt sind die Verbindungen Ia188., besonders die Verbindungen Ia188.1 - Ia188.64, die sich von den Verbindungen Ia1.1 - Ia1.64 dadurch unterscheiden, daß R¹ und R³ für Methyl und R¹³ für tert.-Butyl stehen.

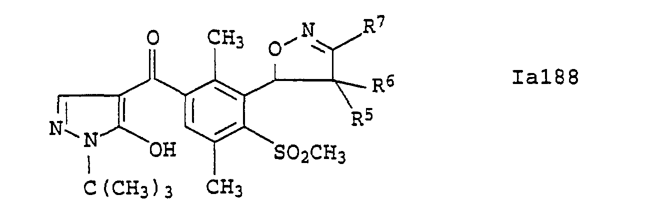

Ebenso insbesondere bevorzugt sind die Verbindungen Ia189., besonders die Verbindungen Ia189.1 - Ia189.64, die sich von den Verbindungen Ia1.1 - Ial.64 dadurch unterscheiden, daß R¹, R³ und R¹⁴ für Methyl stehen.

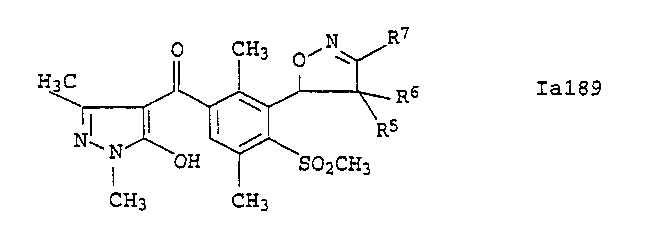

Ebenso insbesondere bevorzugt sind die Verbindungen Ia190., besonders die Verbindungen Ia190.1 - Ia190.64, die sich von den Verbindungen Ia1.1 - Ial.64 dadurch unterscheiden, daß R¹, R³ und R¹⁴ für Methyl und R¹³ für Ethyl stehen.

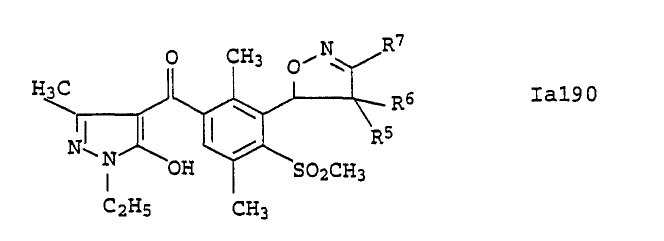

Ebenso insbesondere bevorzugt sind die Verbindungen Ia191., besonders die Verbindungen Ia191.1 - Ia191.64, die sich von den Verbindungen Ia1.1 - Ia1.64 dadurch unterscheiden, daß R¹, R³ und R¹⁴ für Methyl und R¹³ für Isopropyl stehen.

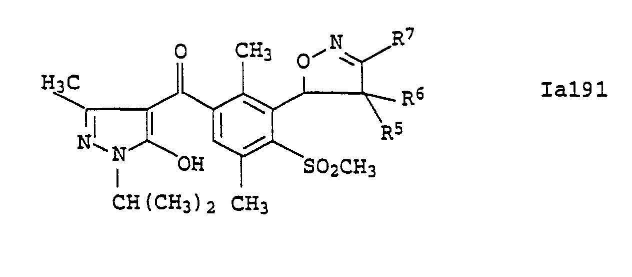

Ebenso insbesondere bevorzugt sind die Verbindungen Ia192., besonders die Verbindungen Ia192.1 - Ia192.64, die sich von den Verbindungen Ia1.1 - Ia1.64 dadurch unterscheiden, daß R¹, R³ und R¹⁴ für Methyl und R¹³ für tert.-Butyl stehen.

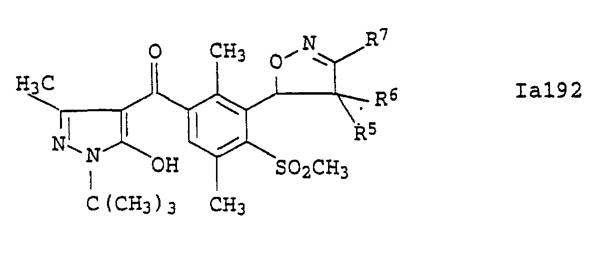

Ebenso insbesondere bevorzugt sind die Verbindungen Ia193., besonders die Verbindungen Ia193.1 - Ia193.64, die sich von den Verbindungen Ia1.1 - Ia1.64 dadurch unterscheiden, daß R² für Ethylsulfonyl und R³ für Methyl steht.

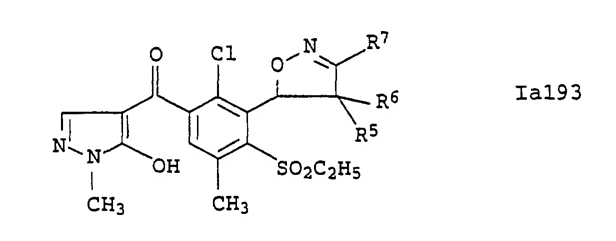

Ebenso insbesondere bevorzugt sind die Verbindungen Ia194., besonders die Verbindungen Ia194.1 - Ia194.64, die sich von den Verbindungen Ia1.1 - Ia1.64 dadurch unterscheiden, daß R² für Ethylsulfonyl, R³ für Methyl und R¹³ für Ethyl stehen.

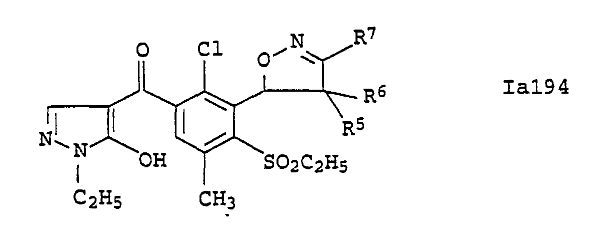

Ebenso insbesondere bevorzugt sind die Verbindungen Ia195., besonders die Verbindungen Ia195.1 - Ia195.64, die sich von den Verbindungen Ia1.1 - Ia1.64 dadurch unterscheiden, daß R² für Ethylsulfonyl, R³ für Methyl und R¹³ für Isopropyl stehen.

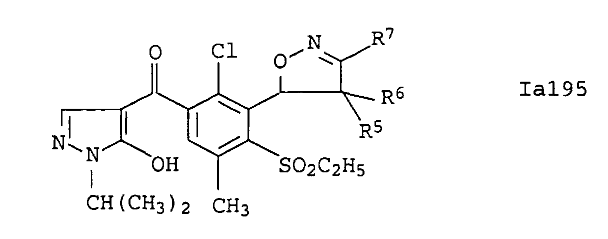

Ebenso insbesondere bevorzugt sind die Verbindungen Ia196., besonders die Verbindungen Ia196.1 - Ia196.64, die sich von den Verbindungen Ia1.1 - Ia1.64 dadurch unterscheiden, daß R² für Ethylsulfonyl, R³ für Methyl und R¹³ für tert.-Butyl stehen.

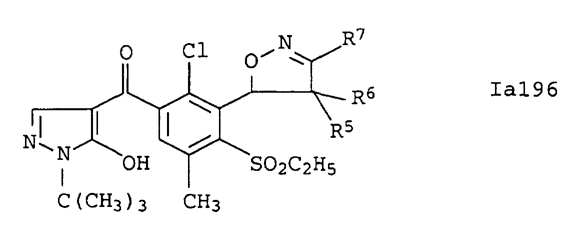

Ebenso insbesondere bevorzugt sind die Verbindungen Ia97., besonders die Verbindungen Ia197.1 - Ia197.64, die sich von den Verbindungen Ia1.1 - Ia1.64 dadurch unterscheiden, daß R² für Ethylsulfonyl, R³ für Methyl und R¹⁴ für Methyl steht.

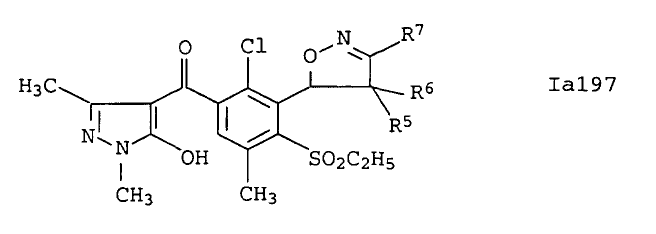

Ebenso insbesondere bevorzugt sind die Verbindungen Ia198., besonders die Verbindungen Ia198.1 - Ia198.64, die sich von den Verbindungen Ia1.1 - Ia1.64 dadurch unterscheiden, daß R² für Ethylsulfonyl, R³ für Methyl und R¹³ für Ethyl und R¹⁴ für Methyl stehen.

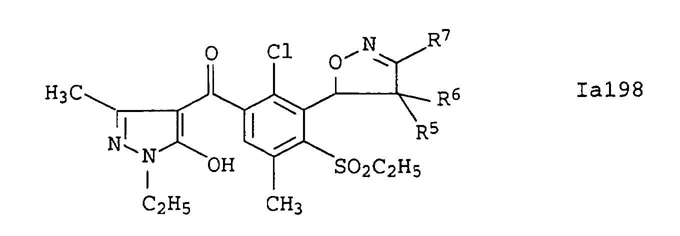

Ebenso insbesondere bevorzugt sind die Verbindungen Ia199., besonders die Verbindungen Ia199.1 - Ia199.64, die sich von den Verbindungen Ia1.1 - Ia1.64 dadurch unterscheiden, daß R² für Ethylsulfonyl, R³ für Methyl, R¹³ für Isopropyl und R¹⁴ für Methyl stehen.

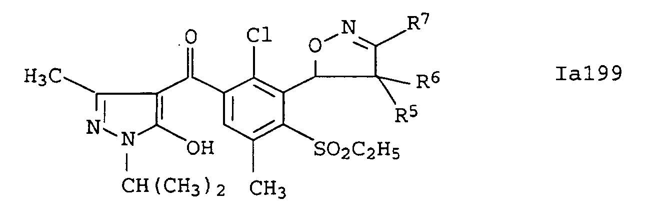

Ebenso insbesondere bevorzugt sind die Verbindungen Ia200., besonders die Verbindungen Ia200.1 - Ia200.64, die sich von den Verbindungen Ia1.1 - Ia1.64 dadurch unterscheiden, daß R² für Ethylsulfonyl, R³ für Methyl, R¹³ für tert.-Butyl und R¹⁴ für Methyl stehen.

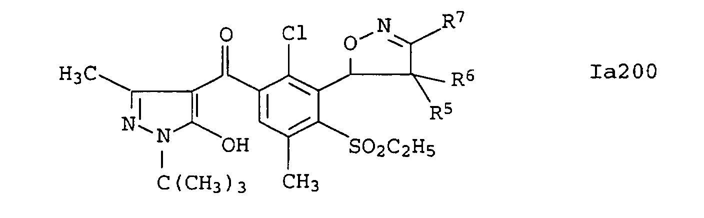

Ebenso insbesondere bevorzugt sind die Verbindungen Ia201., besonders die Verbindungen Ia201.1 - Ia201.64, die sich von den Verbindungen Ia1.1 - Ia1.64 dadurch unterscheiden, daß R¹ und R³ für Methyl und R² für Ethylsulfonyl stehen.

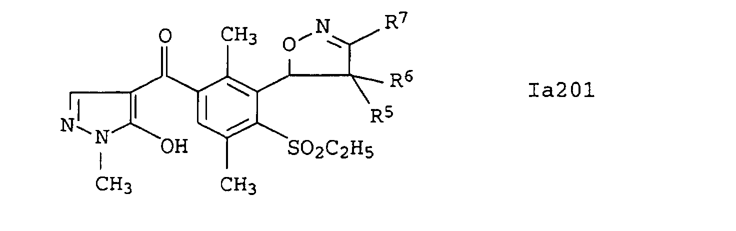

Ebenso insbesondere bevorzugt sind die Verbindungen Ia202., besonders die Verbindungen Ia202.1 - Ia202.64, die sich von den Verbindungen Ia1.1 - Ia1.64 dadurch unterscheiden, daß R¹ und R³ für Methyl, R² für Ethylsulfonyl und R¹³ für Ethyl stehen.

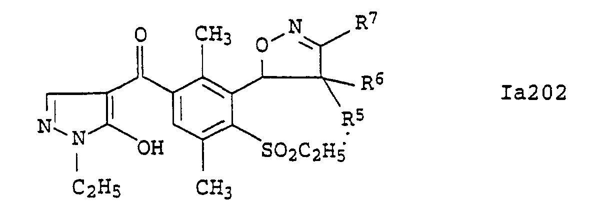

Ebenso insbesondere bevorzugt sind die Verbindungen Ia203., besonders die Verbindungen Ia203.1 - Ia203.64, die sich von den Verbindungen Ia1.1 - Ial.64 dadurch unterscheiden, daß R¹ und R³ für Methyl, R² für Ethylsulfonyl und R¹³ für Isopropyl stehen.

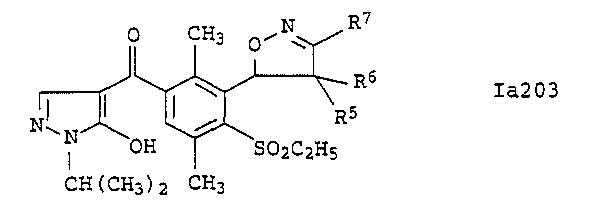

Ebenso insbesondere bevorzugt sind die Verbindungen Ia204., besonders die Verbindungen Ia204.1 - Ia204.64, die sich von den Verbindungen Ia1.1 - Ia1.64 dadurch unterscheiden, daß R¹ und R³ für Methyl, R² für Ethylsulfonyl und R¹³ für tert.-Butyl stehen.

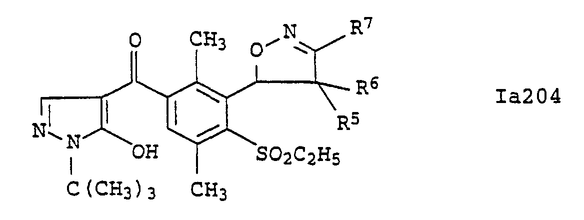

Ebenso insbesondere bevorzugt sind die Verbindungen Ia205., besonders die Verbindungen Ia205.1 - Ia205.64, die sich von den Verbindungen Ia1.1 - Ia1.64 dadurch unterscheiden, daß R¹, R³ und R¹⁴ für Methyl und R² für Ethylsulfonyl stehen.

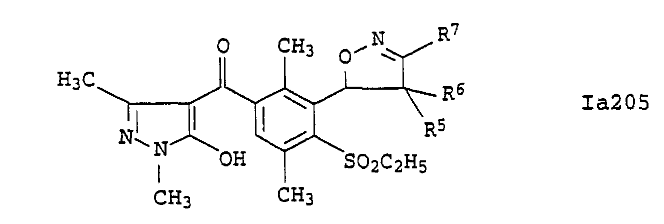

Ebenso insbesondere bevorzugt sind die Verbindungen Ia206., besonders die Verbindungen Ia206.1 - Ia206.64, die sich von den Verbindungen Ia1.1 - Ia1.64 dadurch unterscheiden, daß R¹, R³ und R¹⁴ für Methyl, R² für Ethylsulfonyl und R¹³ für Ethyl stehen.

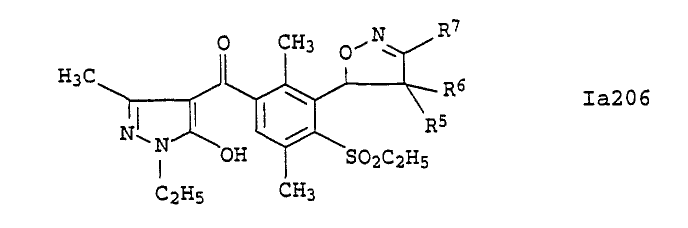

Ebenso insbesondere bevorzugt sind die Verbindungen Ia207., besonders die Verbindungen Ia207.1 - Ia207.64, die sich von den Verbindungen Ia1.1 - Ia1.64 dadurch unterscheiden, daß R¹, R³ und R¹⁴ für Methyl, R² für Ethylsulfonyl und R¹³ für Isopropyl stehen.

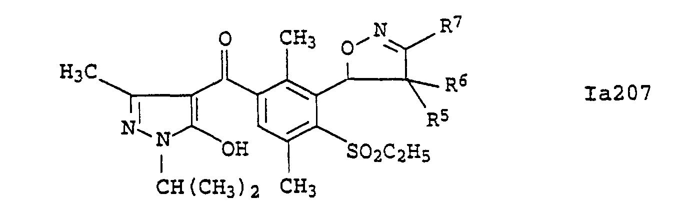

Ebenso insbesondere bevorzugt sind die Verbindungen Ia208., besonders die Verbindungen Ia208.1 - Ia208.64, die sich von den Verbindungen Ia1.1 - Ia1.64 dadurch unterscheiden, daß R¹, R³ und R¹⁴ für Methyl, R² für Ethylsulfonyl und R¹³ für tert.-Butyl stehen.

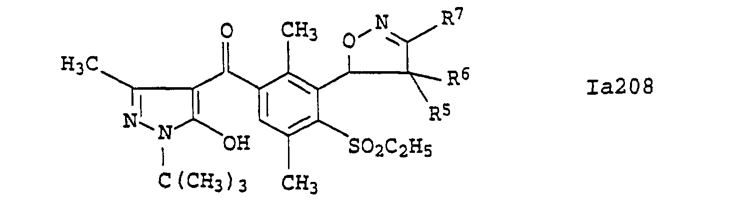

Ebenso insbesondere bevorzugt sind die Verbindungen Ia209., besonders die Verbindungen Ia209.1 - Ia209.64, die sich von den Verbindungen Ia1.1 - Ia1.64 dadurch unterscheiden, daß R² für Chlor und R³ für Methyl stehen.

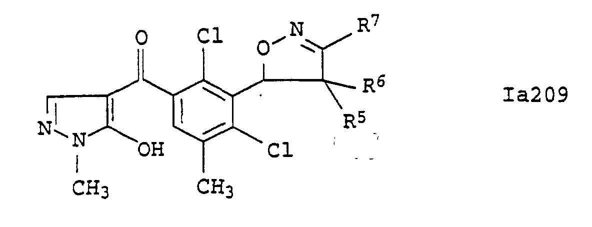

Ebenso insbesondere bevorzugt sind die Verbindungen Ia210., besonders die Verbindungen Ia210.1 - Ia210.64, die sich von den Verbindungen Ia1.1 - Ia1.64 dadurch unterscheiden, daß R² für Chlor, R³ für Methyl und R¹³ für Ethyl stehen.

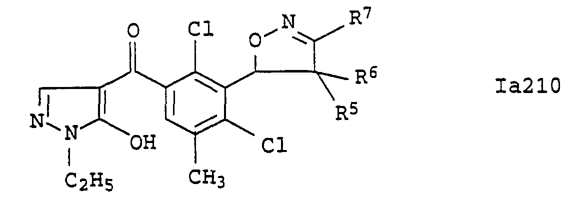

Ebenso insbesondere bevorzugt sind die Verbindungen Ia211., besonders die Verbindungen Ia211.1 - Ia211.64, die sich von den Verbindungen Ia1.1 - Ia1.64 dadurch unterscheiden, daß R² für Chlor, R³ für Methyl und R¹³ für Isopropyl stehen.

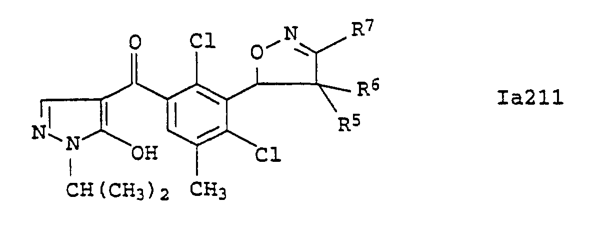

Ebenso insbesondere bevorzugt sind die Verbindungen Ia212., besonders die Verbindungen Ia212.1 - Ia212.64, die sich von den Verbindungen Ia1.1 - Ial.64 dadurch unterscheiden, daß R² für Chlor, R³ für Methyl und R¹³ für tert.-Butyl stehen.

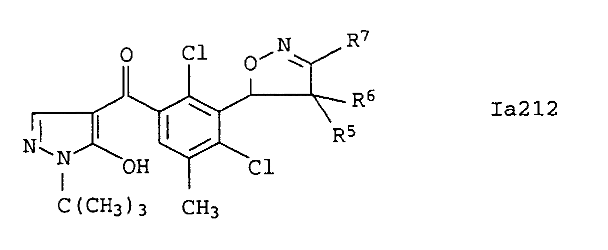

Ebenso insbesondere bevorzugt sind die Verbindungen Ia213., besonders die Verbindungen Ia213.1 - Ia213.64, die sich von den Verbindungen Ia1.1 - Ia1.64 dadurch unterscheiden, daß R² für Chlor, R³ für Methyl und R¹⁴ für Methyl stehen.

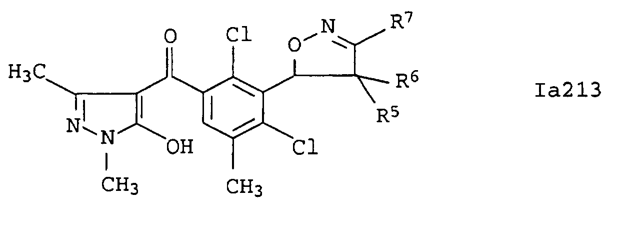

Ebenso insbesondere bevorzugt sind die Verbindungen Ia214., besonders die Verbindungen Ia214.1 - Ia214.64, die sich von den Verbindungen Ia1.1 - Ia1.64 dadurch unterscheiden, daß R² für Chlor, R³ für Methyl, R¹³ für Ethyl und R¹⁴ für Methyl stehen.

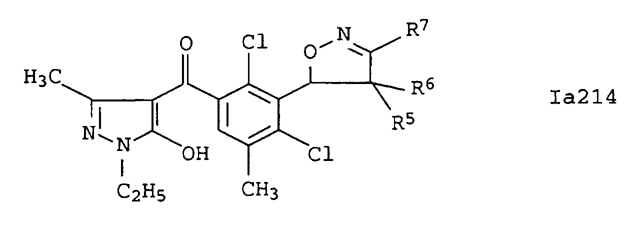

Ebenso insbesondere bevorzugt sind die Verbindungen Ia215., besonders die Verbindungen Ia215.1 - Ia215.64, die sich von den Verbindungen Ia1.1 - Ia1.64 dadurch unterscheiden, daß R² für Chlor, R³ für Methyl, R¹³ für Isopropyl und R¹⁴ für Methyl stehen.

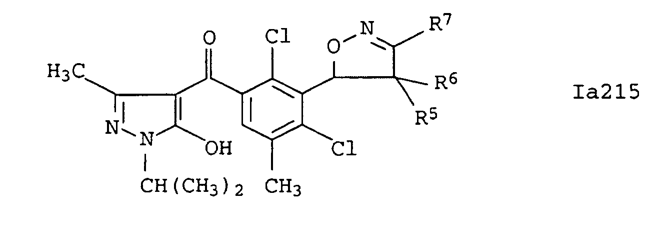

Ebenso insbesondere bevorzugt sind die Verbindungen Ia216., besonders die Verbindungen Ia216.1 - Ia216.64, die sich von den Verbindungen Ia1.1 - Ial.64 dadurch unterscheiden, daß R² für Chlor, R³ für Methyl, R¹³ für tert.-Butyl und R¹⁴ für Methyl stehen.

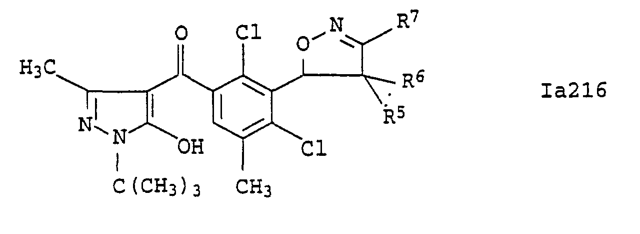

Ebenso insbesondere bevorzugt sind die Verbindungen Ia217., besonders die Verbindungen Ia217.1 - Ia217.64, die sich von den Verbindungen Ia1.1 - Ia1.64 dadurch unterscheiden, daß R¹ und R³ für Methyl und R² für Chlor stehen.

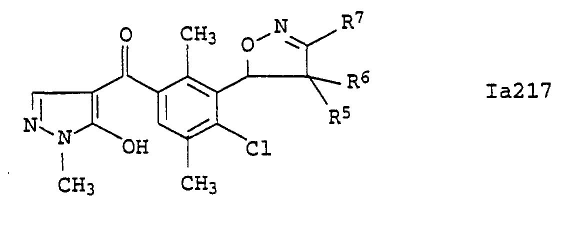

Ebenso insbesondere bevorzugt sind die Verbindungen Ia218., besonders die Verbindungen Ia218.1 - Ia218.64, die sich von den Verbindungen Ia1.1 - Ia1.64 dadurch unterscheiden, daß R¹ und R³ für Methyl, R² für Chlor und R¹³ für Ethyl stehen.

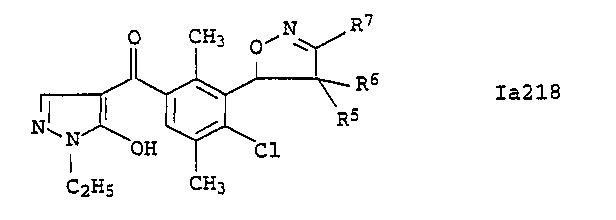

Ebenso insbesondere bevorzugt sind die Verbindungen Ia219., besonders die Verbindungen Ia219.1 - Ia219.64, die sich von den Verbindungen Ia1.1 - Ia1.64 dadurch unterscheiden, daß R¹ und R³ für Methyl, R² für Chlor und R¹³ für Isopropyl stehen.

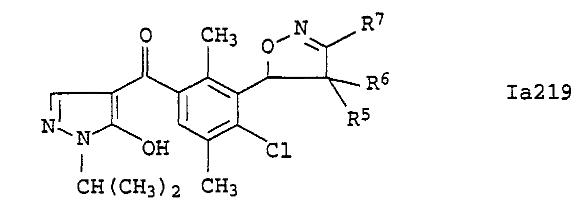

Ebenso insbesondere bevorzugt sind die Verbindungen Ia220., besonders die Verbindungen Ia220.1 - Ia220.64, die sich von den Verbindungen Ia1.1 - Ia1.64 dadurch unterscheiden, daß R¹ und R³ für Methyl, R² für Chlor und R¹³ für tert.-Butyl stehen.

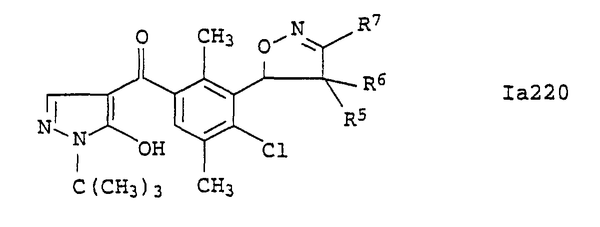

Ebenso insbesondere bevorzugt sind die Verbindungen Ia221., besonders die Verbindungen Ia221.1 - Ia221.64, die sich von den Verbindungen Ia1.1 - Ia1.64 dadurch unterscheiden, daß R¹, R³ und R¹⁴ für Methyl und R² für Chlor stehen.

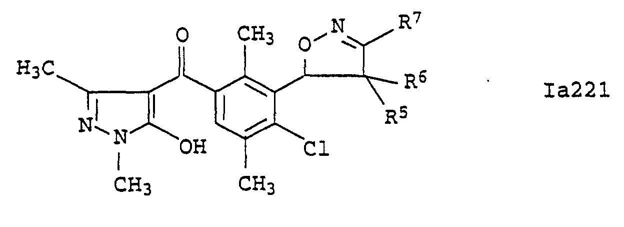

Ebenso insbesondere bevorzugt sind die Verbindungen Ia222., besonders die Verbindungen Ia222.1 - Ia222.64, die sich von den Verbindungen Ia1.1 - Ia1.64 dadurch unterscheiden, daß R¹, R³ und R¹⁴ für Methyl, R² für Chlor und R¹³ für Ethyl stehen.

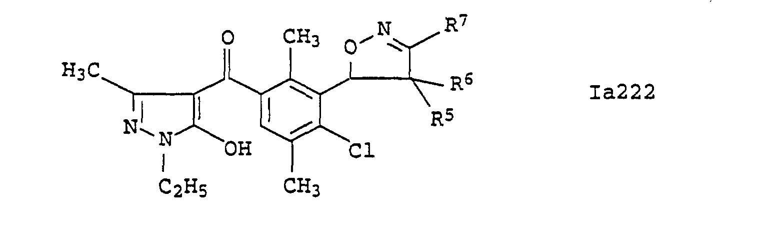

Ebenso insbesondere bevorzugt sind die Verbindungen Ia223., besonders die Verbindungen Ia223.1 - Ia223.64, die sich von den Verbindungen Ia1.1 - Ia1.64 dadurch unterscheiden, daß R¹, R³ und R¹⁴ für Methyl, R² für Chlor und R¹³ für Isopropyl stehen.

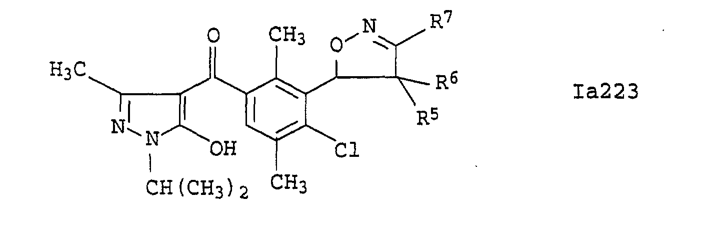

Ebenso insbesondere bevorzugt sind die Verbindungen Ia224., besonders die Verbindungen Ia224.1 - Ia224.64, die sich von den Verbindungen Ia1.1 - Ia1.64 dadurch unterscheiden, daß R¹, R³ und R¹⁴ für Methyl, R² für Chlor und R¹³ für tert.-Butyl stehen.

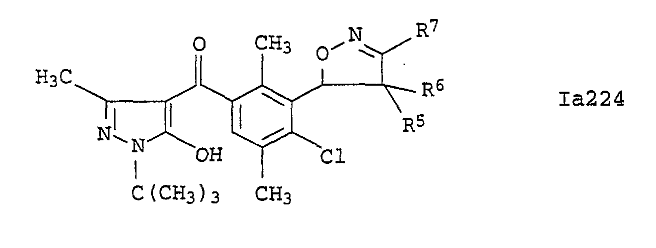

Ebenso insbesondere bevorzugt sind die Verbindungen Ia225., besonders die Verbindungen Ia225.1 - Ia225.64, die sich von den Verbindungen Ia1.1 - Ia1.64 dadurch unterscheiden, daß R¹² für Methylsulfonyl steht.

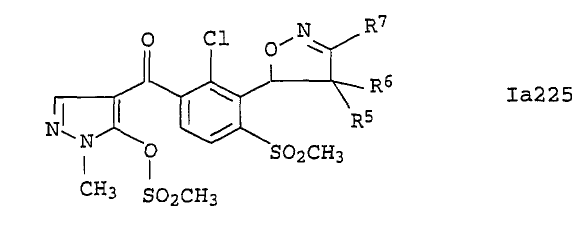

Ebenso insbesondere bevorzugt sind die Verbindungen Ia226., besonders die Verbindungen Ia226.1 - Ia226.64, die sich von den Verbindungen Ia1.1 - Ia1.64 dadurch unterscheiden, daß R¹² für Methylsulfonyl und R¹³ für Ethyl stehen.

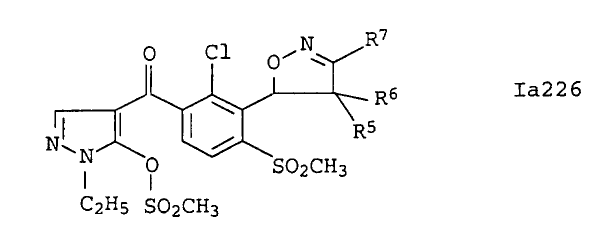

Ebenso insbesondere bevorzugt sind die Verbindungen Ia227., besonders die Verbindungen Ia227.1 - Ia227.64, die sich von den Verbindungen Ia1.1 - Ia1.64 dadurch unterscheiden, daß R¹² für Methylsulfonyl und R¹³ für Isopropyl stehen.

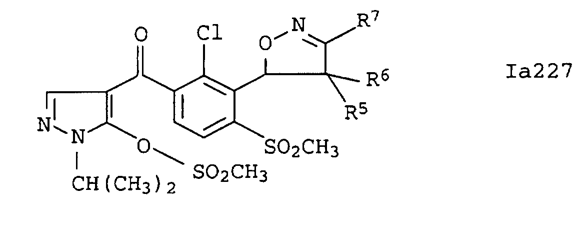

Ebenso insbesondere bevorzugt sind die Verbindungen Ia228., besonders die Verbindungen Ia228.1 - Ia228.64, die sich von den Verbindungen Ia1.1 - Ia1.64 dadurch unterscheiden, daß R¹² für Methylsulfonyl und R¹³ für tert.-Butyl stehen.

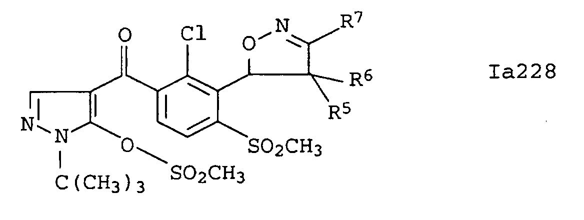

Ebenso insbesondere bevorzugt sind die Verbindungen Ia229., besonders die Verbindungen Ia229.1 - Ia229.64, die sich von den Verbindungen Ia1.1 - Ial.64 dadurch unterscheiden, daß R¹² für Methylsulfonyl und R¹⁴ für Methyl stehen.

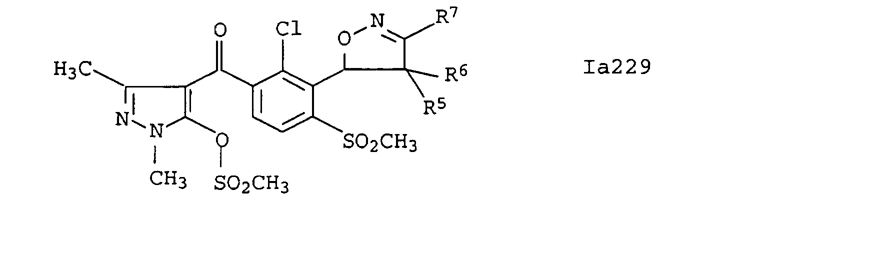

Ebenso insbesondere bevorzugt sind die Verbindungen Ia230., besonders die Verbindungen Ia230.1 - Ia230.64, die sich von den Verbindungen Ia1.1 - Ia1.64 dadurch unterscheiden, daß R¹² für Methylsulfonyl, R¹³ für Ethyl und R¹⁴ für Methyl stehen.

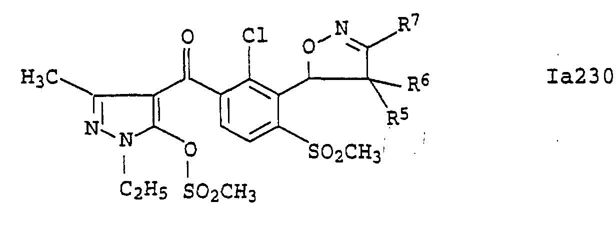

Ebenso insbesondere bevorzugt sind die Verbindungen Ia231., besonders die Verbindungen Ia231.1 - Ia231.64, die sich von den Verbindungen Ia1.1 - Ial.64 dadurch unterscheiden, daß R¹² für Methylsulfonyl, R¹³ für Isopropyl und R¹⁴ für Methyl stehen.

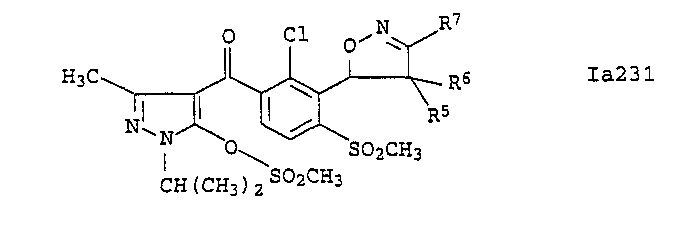

Ebenso insbesondere bevorzugt sind die Verbindungen Ia232., besonders die Verbindungen Ia232.1 - Ia232.64, die sich von den Verbindungen Ia1.1 - Ia1.64 dadurch unterscheiden, daß R¹² für Methylsulfonyl, R¹³ für tert.-Butyl und R¹⁴ für Methyl stehen.

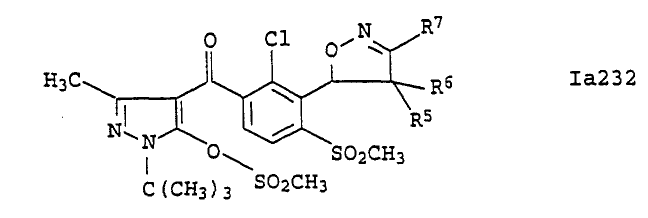

Ebenso insbesondere bevorzugt sind die Verbindungen Ia233., besonders die Verbindungen Ia233.1 - Ia233.64, die sich von den Verbindungen Ia1.1 - Ia1.64 dadurch unterscheiden, daß R¹ für Methyl und R¹² für Methylsulfonyl stehen.

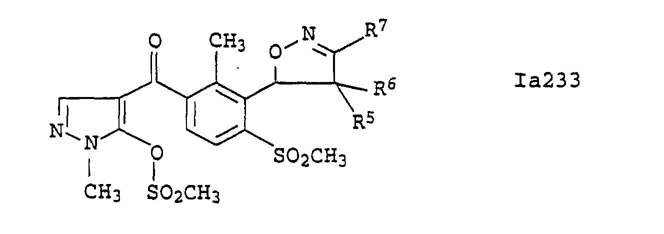

Ebenso insbesondere bevorzugt sind die Verbindungen Ia234., besonders die Verbindungen Ia234.1 - Ia234.64, die sich von den Verbindungen Ia1.1 - Ia1.64 dadurch unterscheiden, daß R¹ für Methyl, R¹² für Methylsulfonyl und R¹³ für Ethyl stehen.

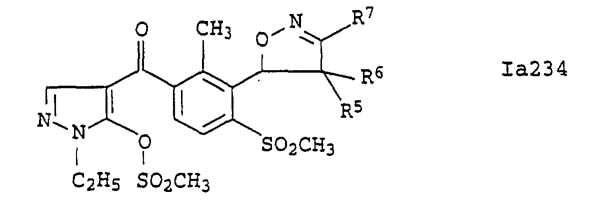

Ebenso insbesondere bevorzugt sind die Verbindungen Ia235., besonders die Verbindungen Ia235.1 - Ia235.64, die sich von den Verbindungen Ia1.1 - Ia1.64 dadurch unterscheiden, daß R¹ für Methyl, R¹² für Methylsulfonyl und R¹³ für Isopropyl stehen.

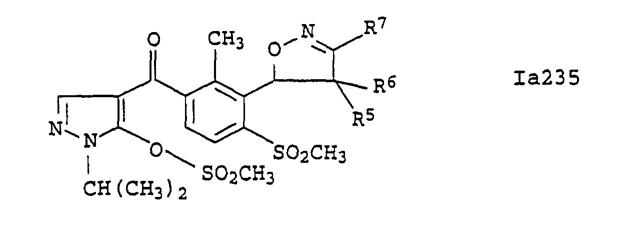

Ebenso insbesondere bevorzugt sind die Verbindungen Ia236., besonders die Verbindungen Ia236.1 - Ia236.64, die sich von den Verbindungen Ia1.1 - Ia1.64 dadurch unterscheiden, daß R¹ für Methyl, R¹² für Methylsulfonyl und R¹³ für tert.-Butyl stehen.

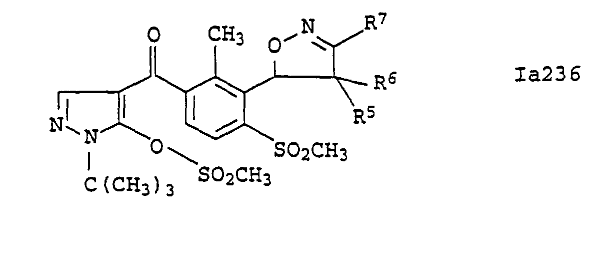

Ebenso insbesondere bevorzugt sind die Verbindungen Ia237., besonders die Verbindungen Ia237.1 - Ia273.64, die sich von den Verbindungen Ia1.1 - Ial.64 dadurch unterscheiden, daß R¹ und R¹⁴ für Methyl und R¹² für Methylsulfonyl stehen.

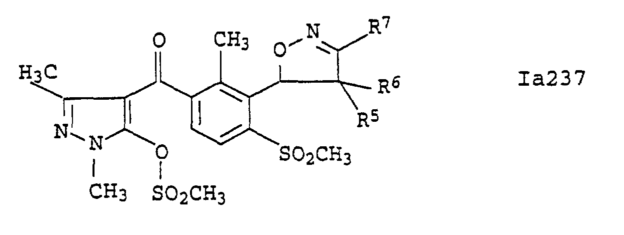

Ebenso insbesondere bevorzugt sind die Verbindungen Ia238., besonders die Verbindungen Ia238.1 - Ia238.64, die sich von den Verbindungen Ia1.1 - Ia1.64 dadurch unterscheiden, daß R¹ und R¹⁴ für Methyl, R¹² für Methylsulfonyl und R¹³ für Ethyl stehen.

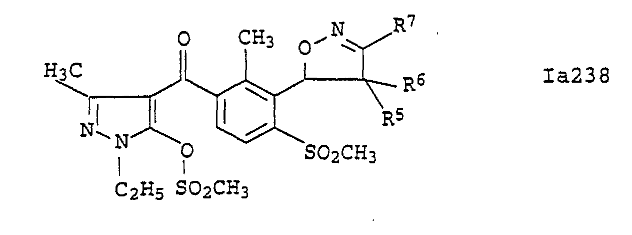

Ebenso insbesondere bevorzugt sind die Verbindungen Ia239., besonders die Verbindungen Ia239.1 - Ia239.64, die sich von den Verbindungen Ia1.1 - Ia1.64 dadurch unterscheiden, daß R¹ und R¹⁴ für Methyl, R¹² für Methylsulfonyl und R¹³ für Isopropyl stehen.

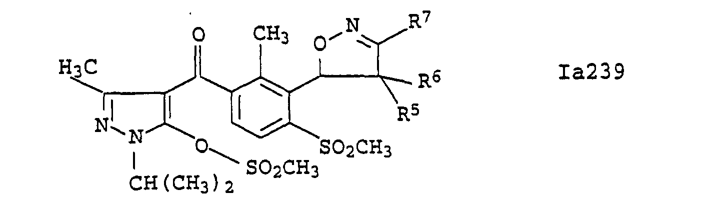

Ebenso insbesondere bevorzugt sind die Verbindungen Ia240., besonders die Verbindungen Ia240.1 - Ia240.64, die sich von den Verbindungen Ia1.1 - Ia1.64 dadurch unterscheiden, daß R¹ und R¹⁴ für Methyl, R¹² für Methylsulfonyl und R¹³ für tert.-Butyl stehen.

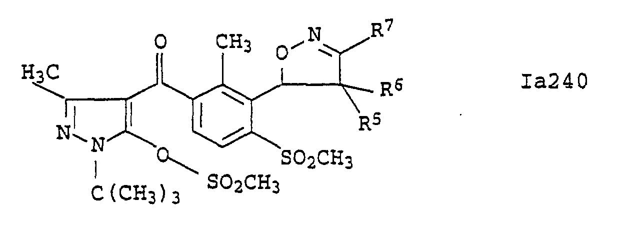

Ebenso insbesondere bevorzugt sind die Verbindungen Ia241., besonders die Verbindungen Ia241.1 - Ia241.64, die sich von den Verbindungen Ia1.1 - Ial.64 dadurch unterscheiden, daß R² für Ethylsulfonyl und R¹² für Methylsulfonyl stehen.

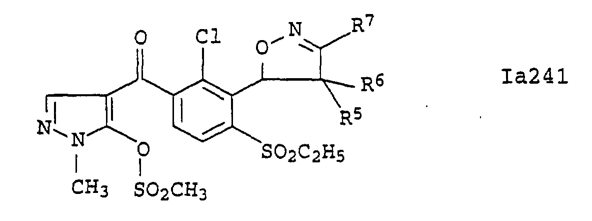

Ebenso insbesondere bevorzugt sind die Verbindungen Ia242., besonders die Verbindungen Ia242.1 - Ia242.64, die sich von den Verbindungen Ia1.1 - Ial.64 dadurch unterscheiden, daß R² für Ethylsulfonyl, R¹² für Methylsulfonyl und R¹³ für Ethyl stehen.

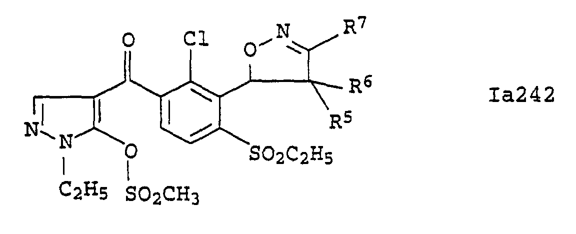

Ebenso insbesondere bevorzugt sind die Verbindungen Ia243., besonders die Verbindungen Ia243.1 - Ia243.64, die sich von den Verbindungen Ia1.1 - Ia1.64 dadurch unterscheiden, daß R² für Ethylsulfonyl, R¹² für Methylsulfonyl und R¹³ für Isopropyl stehen.

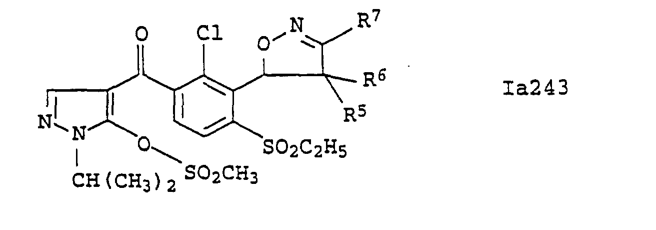

Ebenso insbesondere bevorzugt sind die Verbindungen Ia244., besonders die Verbindungen Ia244.1 - Ia244.64, die sich von den Verbindungen Ia1.1 - Ia1.64 dadurch unterscheiden, daß R² für Ethylsulfonyl, R¹² für Methylsulfonyl und R¹³ für tert.-Butyl stehen.

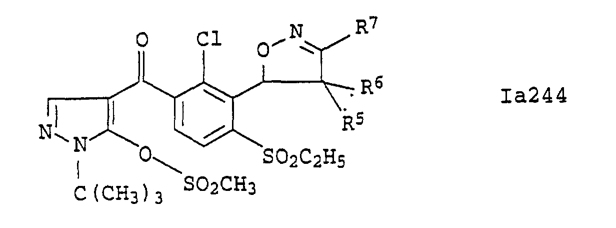

Ebenso insbesondere bevorzugt sind die Verbindungen Ia245., besonders die Verbindungen Ia245.1 - Ia245.64, die sich von den Verbindungen Ia1.1 - Ia1.64 dadurch unterscheiden, daß R² für Ethylsulfonyl, R¹² für Methylsulfonyl und R¹⁴ für Methyl stehen.

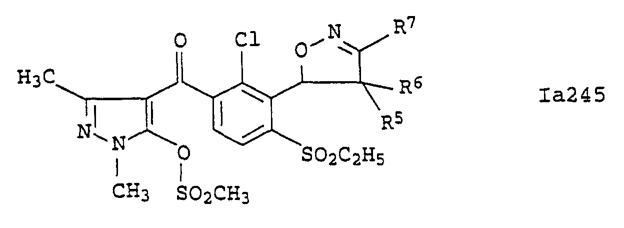

Ebenso insbesondere bevorzugt sind die Verbindungen Ia246., besonders die Verbindungen Ia246.1 - Ia246.64, die sich von den Verbindungen Ia1.1 - Ia1.64 dadurch unterscheiden, daß R¹² für Methylsulfonyl, R² für Ethylsulfonyl, R¹³ für Ethyl und R¹⁴ für Methyl stehen.

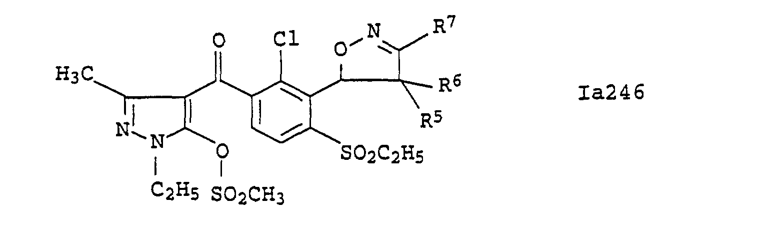

Ebenso insbesondere bevorzugt sind die Verbindungen Ia247., besonders die Verbindungen Ia247.1 - Ia247.64, die sich von den Verbindungen Ia1.1 - Ia1.64 dadurch unterscheiden, daß R² für Ethylsulfonyl, R¹² für Methylsulfonyl, R¹³ für Isopropyl und R¹⁴ für Methyl stehen.

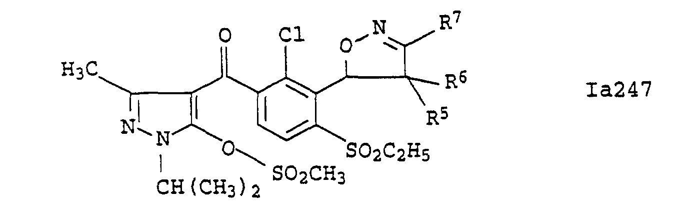

Ebenso insbesondere bevorzugt sind die Verbindungen Ia248., besonders die Verbindungen Ia248.1 - Ia248.64, die sich von den Verbindungen Ia1.1 - Ial.64 dadurch unterscheiden, daß R² für Ethylsulfonyl, R¹² für Methylsulfonyl, R¹³ für tert.-Butyl und R¹⁴ für Methyl stehen.

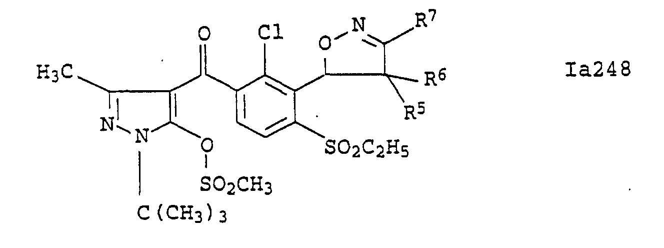

Ebenso insbesondere bevorzugt sind die Verbindungen Ia249., besonders die Verbindungen Ia249.1 - Ia249.64, die sich von den Verbindungen Ia1.1 - Ial.64 dadurch unterscheiden, daß R¹ für Methyl, R² für Ethylsulfonyl und R¹² für Methylsulfonyl stehen.

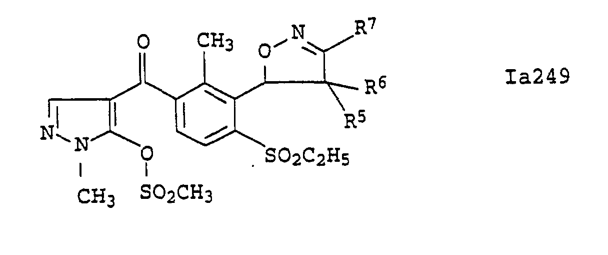

Ebenso insbesondere bevorzugt sind die Verbindungen Ia250., besonders die Verbindungen Ia250.1 - Ia250.64, die sich von den Verbindungen Ia1.1 - Ial.64 dadurch unterscheiden, daß R¹ für Methyl, R² für Ethylsulfonyl, R¹² für Methylsulfonyl und R¹³ für Ethyl stehen.

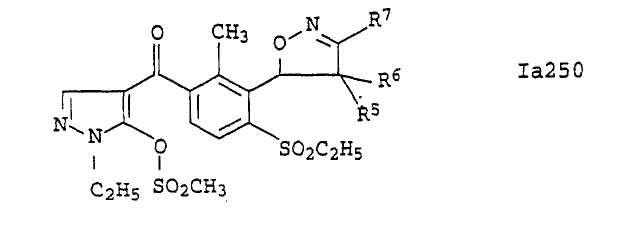

Ebenso insbesondere bevorzugt sind die Verbindungen Ia251., besonders die Verbindungen Ia251.1 - Ia251.64, die sich von den Verbindungen Ia1.1 - Ia1.64 dadurch unterscheiden, daß R¹ für Methyl, R² für Ethylsulfonyl, R¹² für Methylsulfonyl und R¹³ für Isopropyl stehen.

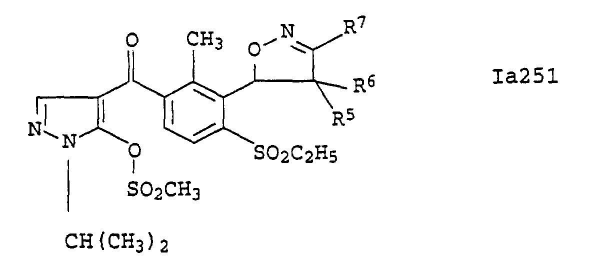

Ebenso insbesondere bevorzugt sind die Verbindungen Ia252., besonders die Verbindungen Ia252.1 - Ia252.64, die sich von den Verbindungen Ia1.1 - Ia1.64 dadurch unterscheiden, daß R¹ für Methyl, R² für Ethylsulfonyl, R¹² für Methylsulfonyl und R¹³ für tert.-Butyl stehen.

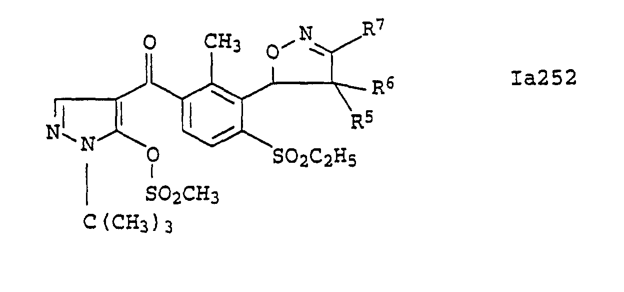

Ebenso insbesondere bevorzugt sind die Verbindungen Ia253., besonders die Verbindungen Ia253.1 - Ia253.64, die sich von den Verbindungen Ia1.1 - Ial.64 dadurch unterscheiden, daß R¹ und R¹⁴ für Methyl, R² für Ethylsulfonyl und R¹² für Methylsulfonyl stehen.

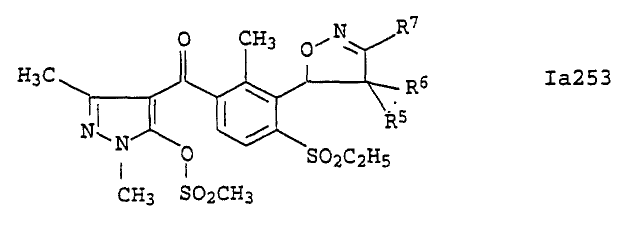

Ebenso insbesondere bevorzugt sind die Verbindungen Ia254., besonders die Verbindungen Ia254.1 - Ia254.64, die sich von den Verbindungen Ia1.1 - Ia1.64 dadurch unterscheiden, daß R¹ und R¹⁴ für Methyl, R² für Ethylsulfonyl, R¹² für Methylsulfonyl und R¹³ für Ethyl stehen.

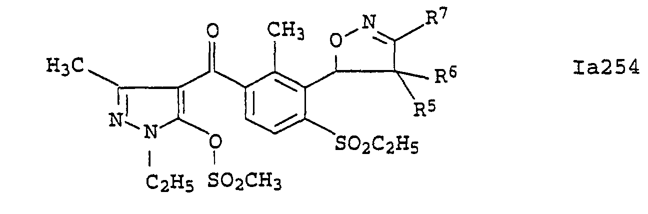

Ebenso insbesondere bevorzugt sind die Verbindungen Ia255., besonders die Verbindungen Ia255.1 - Ia255.64, die sich von den Verbindungen Ia1.1 - Ia1.64 dadurch unterscheiden, daß R¹ und R¹⁴ für Methyl, R² für Ethylsulfonyl, R¹² für Methylsulfonyl und R¹³ für Isopropyl stehen.

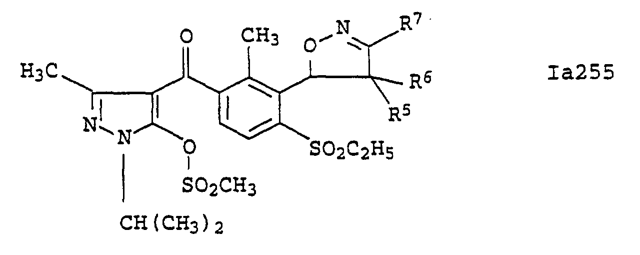

Ebenso insbesondere bevorzugt sind die Verbindungen Ia256., besonders die Verbindungen Ia256.1 -.Ia256.64, die sich von den Verbindungen Ia1.1 - Ia1.64 dadurch unterscheiden, daß R¹ und R¹⁴ für Methyl R² für Ethylsulfonyl, R¹² für Methylsulfonyl und R¹⁴ für tert.-Butyl stehen.

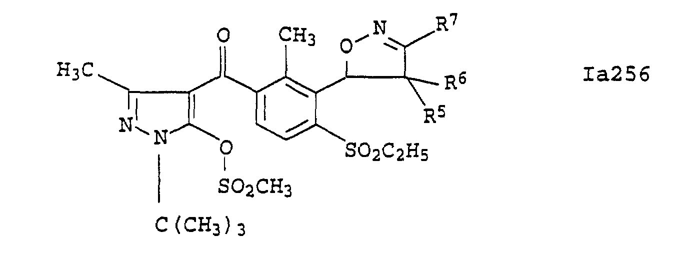

Ebenso insbesondere bevorzugt sind die Verbindungen Ia257., besonders die Verbindungen Ia257.1 - Ia257.64, die sich von den Verbindungen Ia1.1 - Ia1.64 dadurch unterscheiden, daß R² für Chlor und R¹² für Methylsulfonyl stehen.

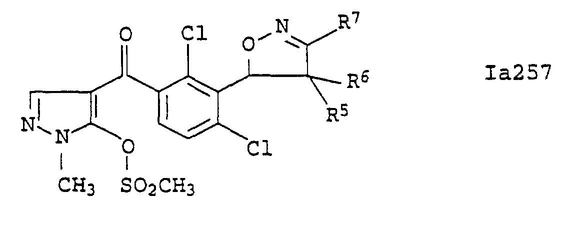

Ebenso insbesondere bevorzugt sind die Verbindungen Ia258., besonders die Verbindungen Ia258.1 - Ia258.64, die sich von den Verbindungen Ia1.1 - Ia1.64 dadurch unterscheiden, daß R² für Chlor, R¹² für Methylsulfonyl und R¹³ für Ethyl stehen.

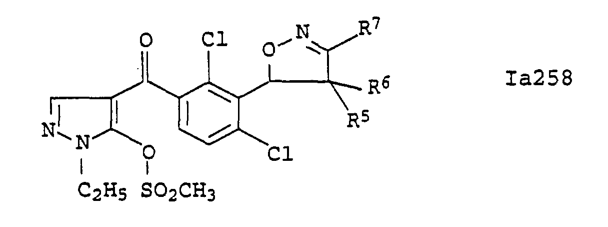

Ebenso insbesondere bevorzugt sind die Verbindungen Ia259., besonders die Verbindungen Ia259.1 - Ia259.64, die sich von den Verbindungen Ia1.1 - Ia1.64 dadurch unterscheiden, daß R² für Chlor, R¹² für Methylsulfonyl und R¹³ für Isopropyl stehen.

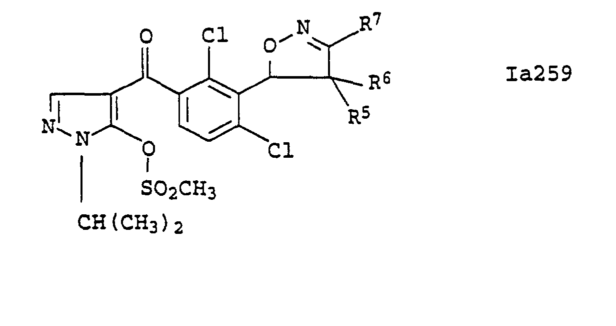

Ebenso insbesondere bevorzugt sind die Verbindungen Ia260., besonders die Verbindungen Ia260.1 - Ia260.64, die sich von den Verbindungen Ia1.1 - Ia1.64 dadurch unterscheiden, daß R² für Chlor, R¹² für Methylsulfonyl und R¹³ für tert.-Butyl stehen.

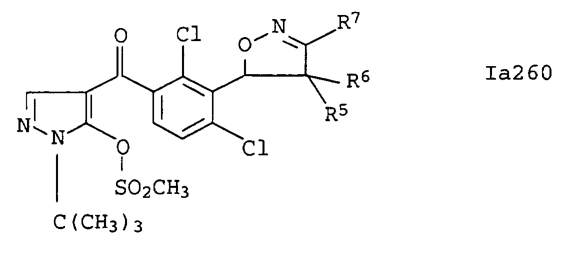

Ebenso insbesondere bevorzugt sind die Verbindungen Ia261., besonders die Verbindungen Ia261.1 - Ia261.64, die sich von den Verbindungen Ia1.1 - Ial.64 dadurch unterscheiden, daß R² für Chlor, R¹² für Methylsulfonyl und R¹⁴ für Methyl stehen.

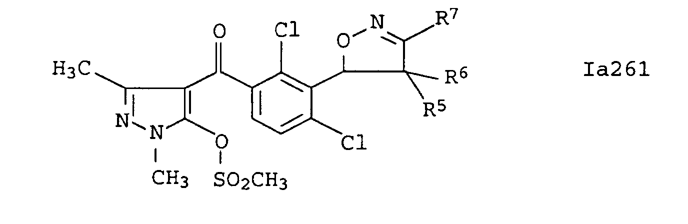

Ebenso insbesondere bevorzugt sind die Verbindungen Ia262., besonders die Verbindungen Ia262.1 - Ia262.64, die sich von den Verbindungen Ial.1 - Ia1.64 dadurch unterscheiden, daß R² für Chlor, R¹² für Methylsulfonyl, R¹³ für Ethyl und R¹⁴ für Methyl stehen.

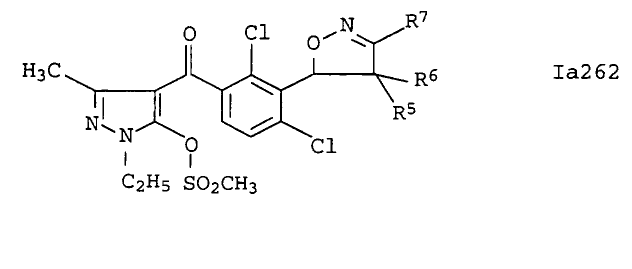

Ebenso insbesondere bevorzugt sind die Verbindungen Ia263., besonders die Verbindungen Ia263.1 - Ia263.64, die sich von den Verbindungen Ia1.1 - Ia1.64 dadurch unterscheiden, daß R² für Chlor, R¹² für Methylsulfonyl, R¹³ für Isopropyl und R¹⁴ für Methyl stehen.

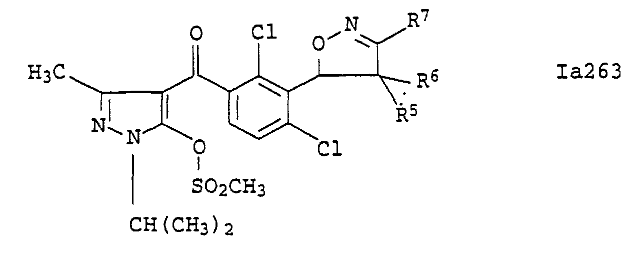

Ebenso insbesondere bevorzugt sind die Verbindungen Ia264., besonders die Verbindungen Ia264.1 - Ia264.64, die sich von den Verbindungen Ia1.1 - Ial.64 dadurch unterscheiden, daß R² für Chlor, R¹² für Methylsulfonyl, R¹³ für tert.-Butyl und R¹⁴ für Methyl stehen.

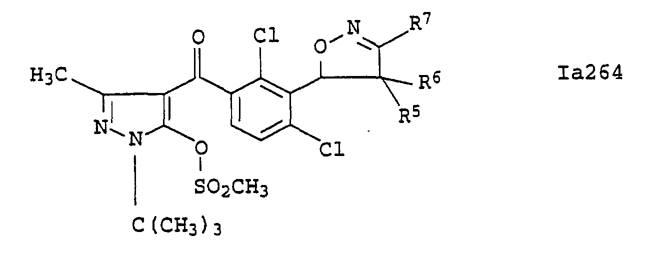

Ebenso insbesondere bevorzugt sind die Verbindungen Ia265., besonders die Verbindungen Ia265.1 - Ia265.64, die sich von den Verbindungen Ia1.1 - Ia1.64 dadurch unterscheiden, daß R¹ für Methyl, R² für Chlor und R¹² für Methylsulfonyl stehen.

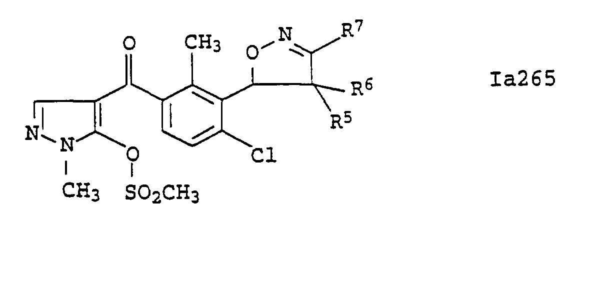

Ebenso insbesondere bevorzugt sind die Verbindungen Ia266., besonders die Verbindungen Ia266.1 - Ia266.64, die sich von den Verbindungen Ia1.1 - Ial.64 dadurch unterscheiden, daß R¹ für Methyl, R² für Chlor, R¹² für Methylsulfonyl und R¹³ für Ethyl stehen.

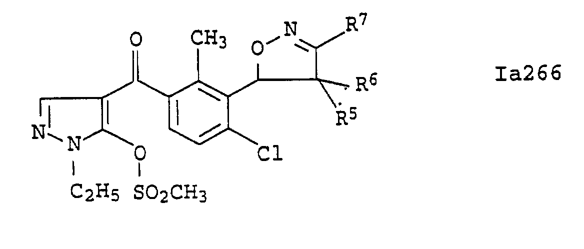

Ebenso insbesondere bevorzugt sind die Verbindungen Ia267., besonders die Verbindungen Ia267.1 - Ia267.64, die sich von den Verbindungen Ia1.1 - Ia1.64 dadurch unterscheiden, daß R¹ für Methyl, R² für Chlor, R¹² für Methylsulfonyl und R¹³ für Isopropyl stehen.

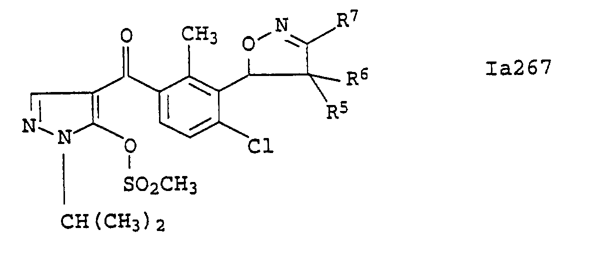

Ebenso insbesondere bevorzugt sind die Verbindungen Ia268., besonders die Verbindungen Ia268.1 - Ia268.64, die sich von den Verbindungen Ia1.1 - Ia1.64 dadurch unterscheiden, daß R¹ für Methyl, R² für Chlor, R¹² für Methylsulfonyl und R¹³ für tert.-Butyl stehen.

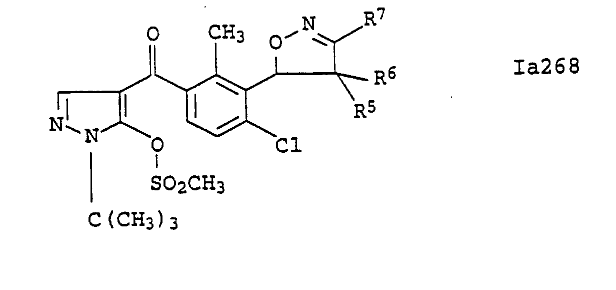

Ebenso insbesondere bevorzugt sind die Verbindungen Ia269., besonders die Verbindungen Ia269.1 - Ia269.64, die sich von den Verbindungen Ia1.1 - Ia1.64 dadurch unterscheiden, daß R¹ und R¹⁴ für Methyl, R² für Chlor und R¹² für Methylsulfonyl stehen.

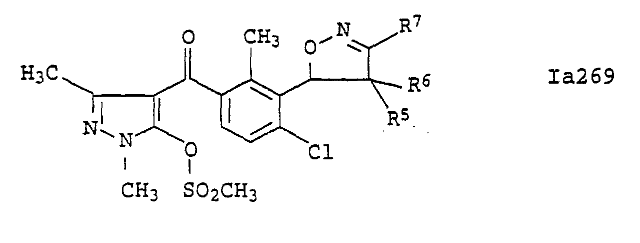

Ebenso insbesondere bevorzugt sind die Verbindungen Ia270., besonders die Verbindungen Ia270.1 - Ia270.64, die sich von den Verbindungen Ia1.1 - Ia1.64 dadurch unterscheiden, daß R¹ und R¹⁴ für Methyl, R² für Chlor, R¹² für Methylsulfonyl und R¹³ für Ethyl stehen.

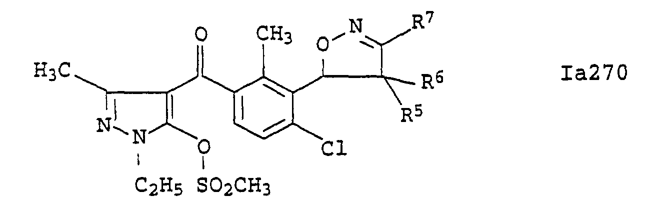

Ebenso insbesondere bevorzugt sind die Verbindungen Ia271., besonders die Verbindungen Ia271.1 - Ia271.64, die sich von den Verbindungen Ia1.1 - Ia1.64 dadurch unterscheiden, daß R¹ und R¹⁴ für Methyl, R² für Chlor, R¹² für Methylsulfonyl und R¹³ für Isopropyl stehen.

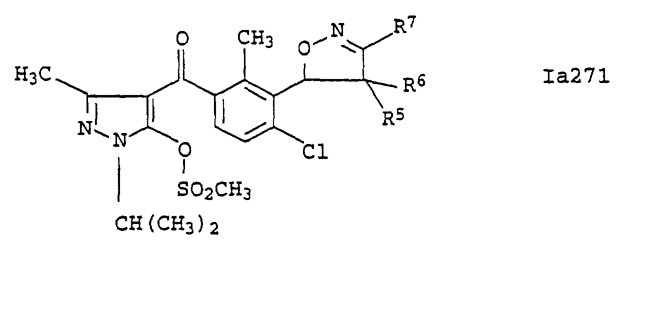

Ebenso insbesondere bevorzugt sind die Verbindungen Ia272., besonders die Verbindungen Ia272.1 - Ia272.64, die sich von den Verbindungen Ia1.1 - Ia1.64 dadurch unterscheiden, daß R¹ und R¹⁴ für Methyl, R² für Chlor, R³ für Methylsulfonyl und R¹³ für tert.-Butyl stehen.

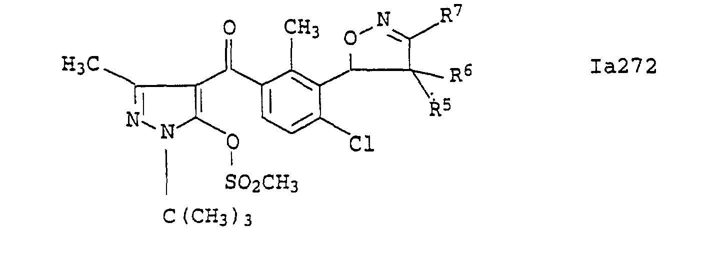

Ebenso insbesondere bevorzugt sind die Verbindungen Ia273., besonders die Verbindungen Ia273.1 - Ia273.64, die sich von den Verbindungen Ia1.1 - Ial.64 dadurch unterscheiden, daß R¹² für Phenylsulfonyl steht.

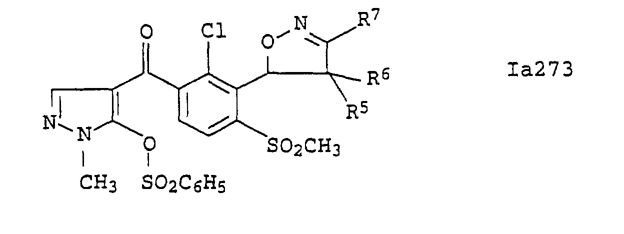

Ebenso insbesondere bevorzugt sind die Verbindungen Ia274., besonders die Verbindungen Ia274.1 - Ia274.64, die sich von den Verbindungen Ia1.1 - Ia1.64 dadurch unterscheiden, daß R¹² für Phenylsulfonyl und R¹³ für Ethyl stehen.

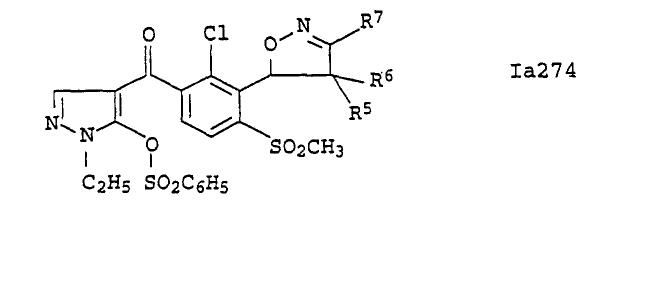

Ebenso insbesondere bevorzugt sind die Verbindungen Ia275., besonders die Verbindungen Ia275.1 - Ia275.64, die sich von den Verbindungen Ia1.1 - Ia1.64 dadurch unterscheiden, daß R¹² für Phenylsulfonyl und R¹³ für Isopropyl stehen.

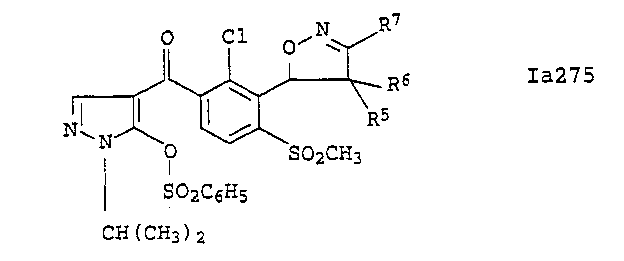

Ebenso insbesondere bevorzugt sind die Verbindungen Ia276., besonders die Verbindungen Ia276.1 - Ia276.64, die sich von den Verbindungen Ia1.1 - Ia1.64 dadurch unterscheiden, daß R¹² für Phenylsulfonyl und R¹³ für tert.-Butyl stehen.

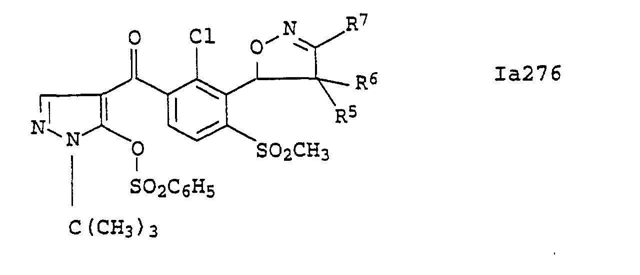

Ebenso insbesondere bevorzugt sind die Verbindungen Ia277., besonders die Verbindungen Ia277.1 - Ia277.64, die sich von den Verbindungen Ia1.1 - Ia1.64 dadurch unterscheiden, daß R¹² für Phenylsulfonyl und R¹⁴ für Methyl stehen.

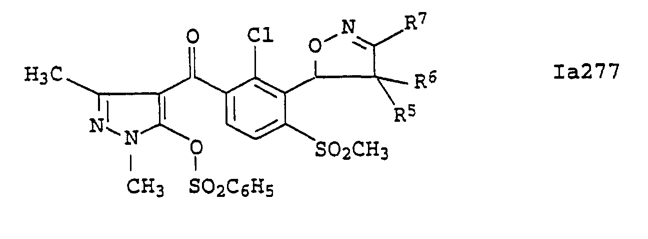

Ebenso insbesondere bevorzugt sind die Verbindungen Ia278., besonders die Verbindungen Ia278.1 - Ia278.64, die sich von den Verbindungen Ia1.1 - Ia1.64 dadurch unterscheiden, daß R¹² für Phenylsulfonyl, R¹³ für Ethyl und R¹⁴ für Methyl stehen.

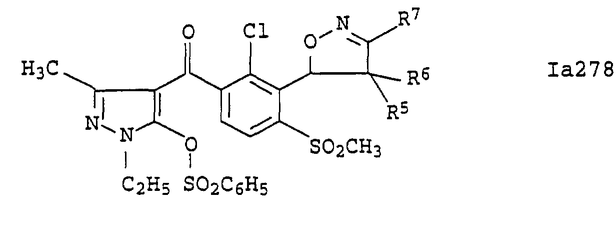

Ebenso insbesondere bevorzugt sind die Verbindungen Ia279., besonders die Verbindungen Ia279.1 - Ia279.64, die sich von den Verbindungen Ia1.1 - Ia1.64 dadurch unterscheiden, daß R¹² für Phenylsulfonyl, R¹³ für Isopropyl und R¹⁴ für Methyl stehen.

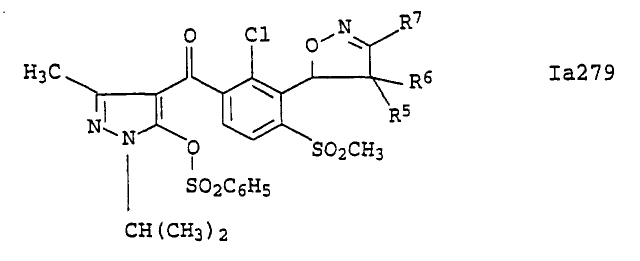

Ebenso insbesondere bevorzugt sind die Verbindungen Ia280., besonders die Verbindungen Ia280.1 - Ia280.64, die sich von den Verbindungen Ia1.1 - Ia1.64 dadurch unterscheiden, daß R¹² für Phenylsulfonyl, R¹³ für tert.-Butyl und R¹⁴ für Methyl stehen.

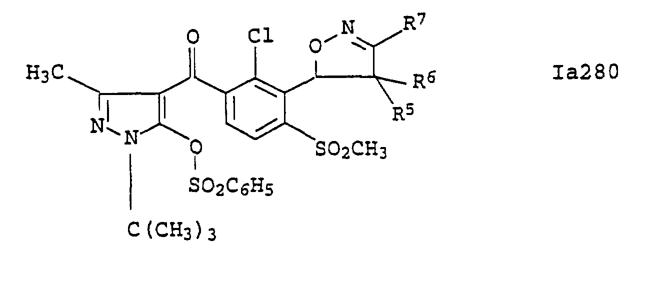

Ebenso insbesondere bevorzugt sind die Verbindungen Ia281., besonders die Verbindungen Ia281.1 - Ia281.64, die sich von den Verbindungen Ia1.1 - Ia1.64 dadurch unterscheiden, daß R¹ für Methyl und R¹² für Phenylsulfonyl stehen.

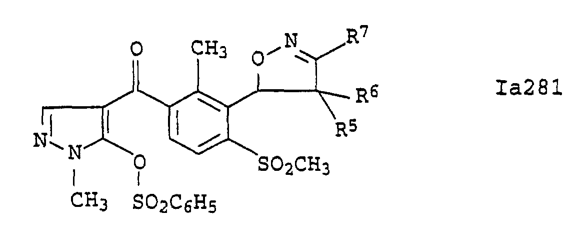

Ebenso insbesondere bevorzugt sind die Verbindungen Ia282., besonders die Verbindungen Ia282.1 - Ia282.64, die sich von den Verbindungen Ia1.1 - Ia1.64 dadurch unterscheiden, daß R¹ für Methyl, R¹² für Phenylsulfonyl und R¹³ für Ethyl stehen.

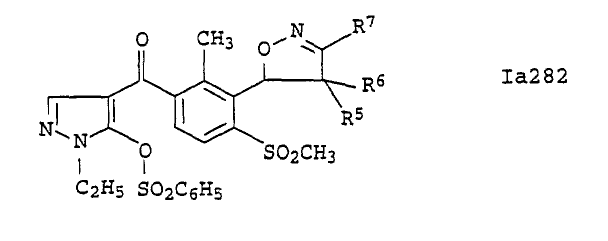

Ebenso insbesondere bevorzugt sind die Verbindungen Ia283., besonders die Verbindungen Ia283.1 - Ia283.64, die sich von den Verbindungen Ia1.1 - Ia1.64 dadurch unterscheiden, daß R¹ für Methyl, R¹² für Phenylsulfonyl und R¹³ für Isopropyl stehen.

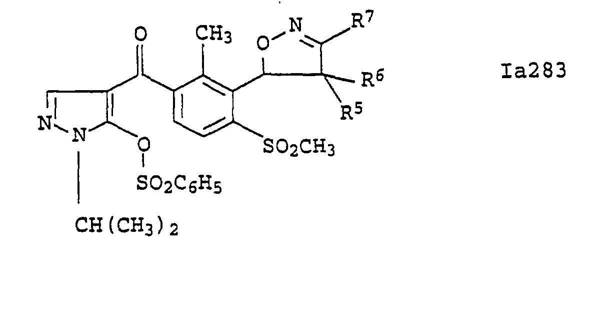

Ebenso insbesondere bevorzugt sind die Verbindungen Ia284., besonders die Verbindungen Ia284.1 - Ia284.64, die sich von den Verbindungen Ia1.1 - Ial.64 dadurch unterscheiden, daß R¹ für Methyl, R¹² für Phenylsulfonyl und R¹³ für tert.-Butyl stehen.

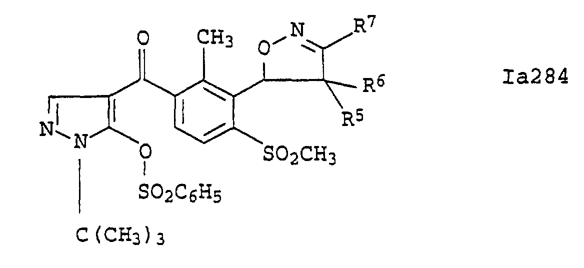

Ebenso insbesondere bevorzugt sind die Verbindungen Ia285., besonders die Verbindungen Ia285.1 - Ia285.64, die sich von den Verbindungen Ia1.1 - Ia1.64 dadurch unterscheiden, daß R¹ und R¹⁴ für Methyl und R¹² für Phenylsulfonyl stehen.

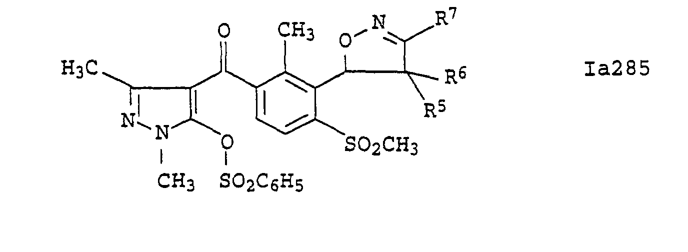

Ebenso insbesondere bevorzugt sind die Verbindungen Ia286., besonders die Verbindungen Ia286.1 - Ia286.64, die sich von den Verbindungen Ia1.1 - Ia1.64 dadurch unterscheiden, daß R¹ und R¹⁴ für Methyl und R¹² für Phenylsulfonyl und R¹³ für Ethyl stehen.

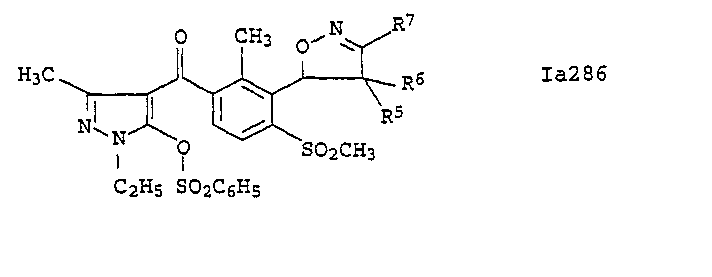

Ebenso insbesondere bevorzugt sind die Verbindungen Ia287., besonders die Verbindungen Ia287.1 - Ia287.64, die sich von den Verbindungen Ia1.1 - Ia1.64 dadurch unterscheiden, daß R¹ und R¹⁴ für Methyl, R¹² für Phenylsulfonyl und R¹³ für Isopropyl stehen.

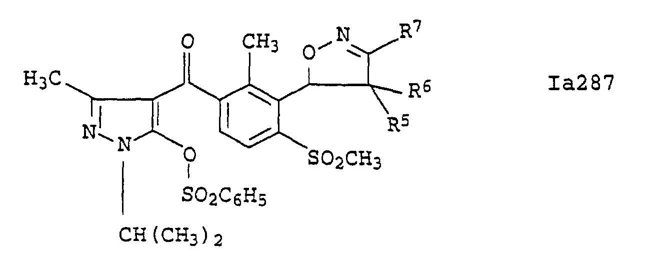

Ebenso insbesondere bevorzugt sind die Verbindungen Ia288., besonders die Verbindungen Ia288.1 - Ia288.64, die sich von den Verbindungen Ia1.1 - Ia1.64 dadurch unterscheiden, daß R¹ und R¹⁴ für Methyl, R¹² für Phenylsulfonyl und R¹³ für tert.-Butyl stehen.

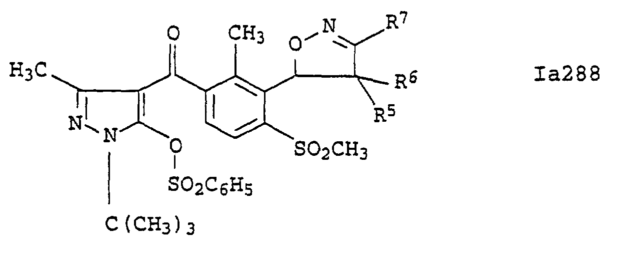

Ebenso insbesondere bevorzugt sind die Verbindungen Ia289., besonders die Verbindungen Ia289.1 - Ia289.64, die sich von den Verbindungen Ia1.1 - Ia1.64 dadurch unterscheiden, daß R² für Ethylsulfonyl und R¹² für Phenylsulfonyl stehen.

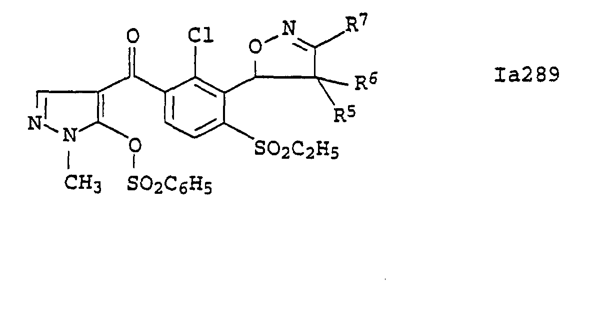

Ebenso insbesondere bevorzugt sind die Verbindungen Ia290., besonders die Verbindungen Ia290.1 - Ia290.64, die sich von den Verbindungen Ia1.1 - Ial.64 dadurch unterscheiden, daß R² für Ethylsulfonyl, R¹² für Phenylsulfonyl und R¹³ für Ethyl stehen.

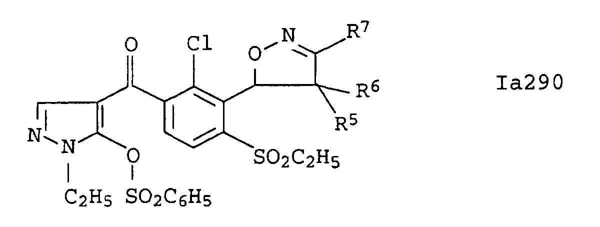

Ebenso insbesondere bevorzugt sind die Verbindungen Ia291., besonders die Verbindungen Ia291.1 - Ia291.64, die sich von den Verbindungen Ia1.1 - Ia1.64 dadurch unterscheiden, daß R² für Ethylsulfonyl, R¹² für Phenylsulfonyl und R¹³ für Isopropyl stehen.

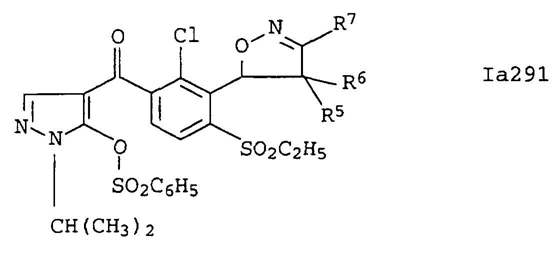

Ebenso insbesondere bevorzugt sind die Verbindungen Ia292., besonders die Verbindungen Ia292.1 - Ia292.64, die sich von den Verbindungen Ia1.1 - Ial.64 dadurch unterscheiden, daß R² für Ethylsulfonyl, R¹² für Phenylsulfonyl und R¹³ für tert.-Butyl stehen.

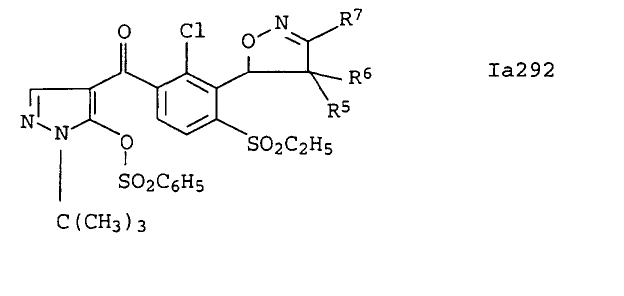

Ebenso insbesondere bevorzugt sind die Verbindungen Ia293., besonders die Verbindungen Ia293.1 - Ia293.64, die sich von den Verbindungen Ia1.1 - Ial.64 dadurch unterscheiden, daß R² für Ethylsulfonyl, R¹² für Phenylsulfonyl und R¹⁴ für Methyl stehen.

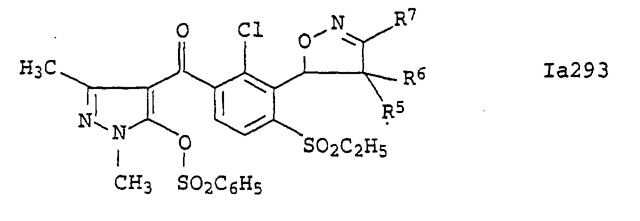

Ebenso insbesondere bevorzugt sind die Verbindungen Ia294., besonders die Verbindungen Ia294.1 - Ia294.64, die sich von den Verbindungen Ia1.1 - Ial.64 dadurch unterscheiden, daß R² für Ethylsulfonyl, R¹² für Phenylsulfonyl, R¹³ für Ethyl und R¹⁴ für Methyl stehen.

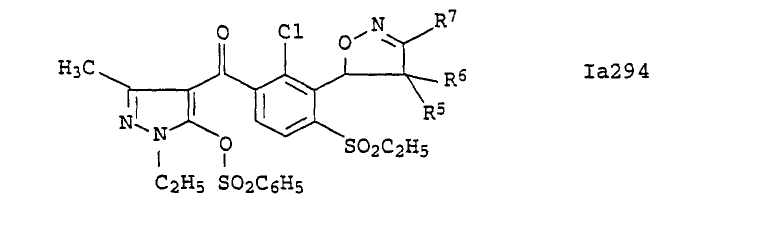

Ebenso insbesondere bevorzugt sind die Verbindungen Ia295., besonders die Verbindungen Ia295.1 - Ia295.64, die sich von den Verbindungen Ia1.1 - Ia1.64 dadurch unterscheiden, daß R² für Ethylsulfonyl, R¹² für Phenylsulfonyl, R¹³ für Isopropyl und R¹⁴ für Methyl stehen.

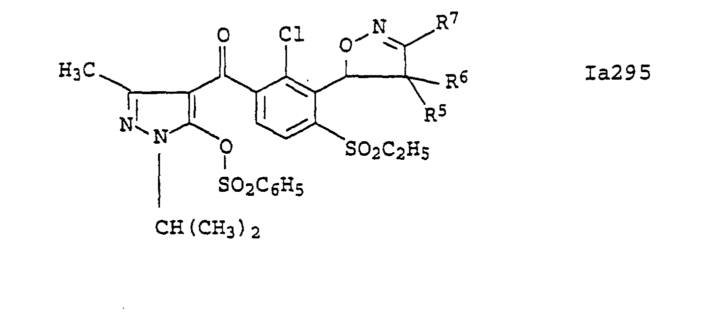

Ebenso insbesondere bevorzugt sind die Verbindungen Ia296., besonders die Verbindungen Ia296.1 - Ia296.64, die sich von den Verbindungen Ia1.1 - Ial.64 dadurch unterscheiden, daß R² für Ethylsulfonyl, R¹² für Phenylsulfonyl, R¹³ für tert.-Butyl und R¹⁴ für Methyl stehen.

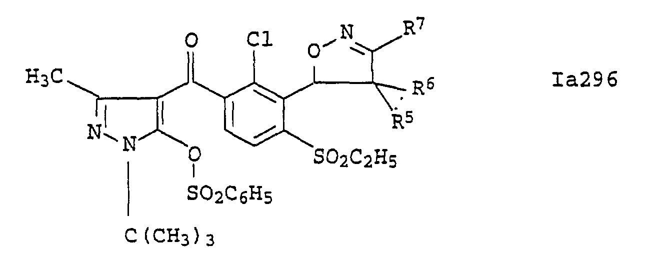

Ebenso insbesondere bevorzugt sind die Verbindungen Ia297., besonders die Verbindungen Ia297.1 - Ia297.64, die sich von den Verbindungen Ia1.1 - Ia1.64 dadurch unterscheiden, daß R¹ für Methyl, R² für Ethylsulfonyl, R¹² für Phenylsulfonyl stehen.

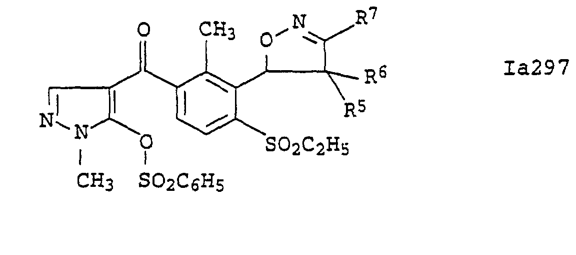

Ebenso insbesondere bevorzugt sind die Verbindungen Ia298., besonders die Verbindungen Ia298.1 - Ia298.64, die sich von den Verbindungen Ia1.1 - Ia1.64 dadurch unterscheiden, daß R¹ für Methyl, R² für Ethylsulfonyl, R¹² für Phenylsulfonyl und R¹³ für Ethyl stehen.

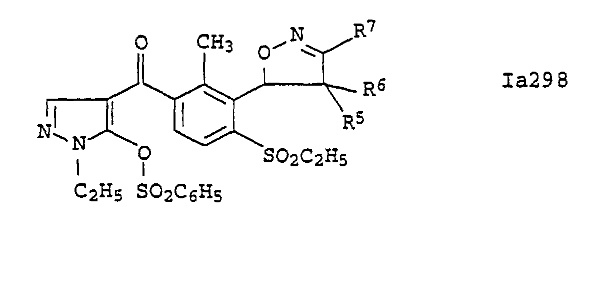

Ebenso insbesondere bevorzugt sind die Verbindungen Ia299., besonders die Verbindungen Ia299.1 - Ia299.64, die sich von den Verbindungen Ia1.1 - Ia1.64 dadurch unterscheiden, daß R¹ für Methyl, R² für Ethylsulfonyl, R¹² für Phenylsulfonyl und R¹³ für Isopropyl stehen.

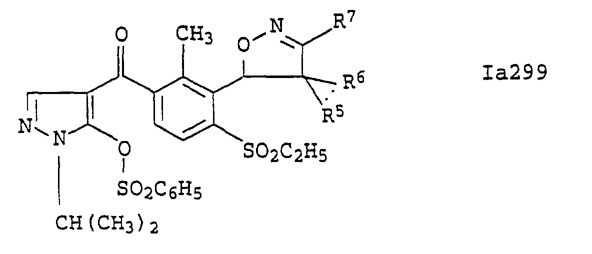

Ebenso insbesondere bevorzugt sind die Verbindungen Ia300., besonders die Verbindungen Ia300.1 - Ia300.64, die sich von den Verbindungen Ia1.1 - Ia1.64 dadurch unterscheiden, daß R¹ für Methyl, R² für Ethylsulfonyl, R¹² für Phenylsulfonyl und R¹³ für tert.-Butyl stehen.

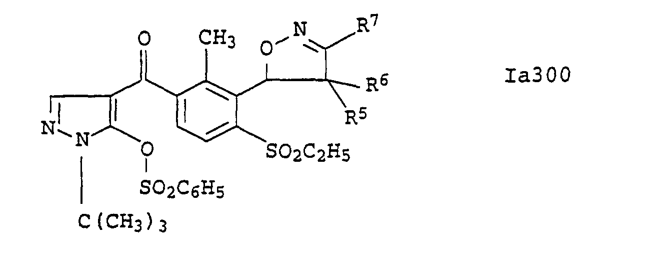

Ebenso insbesondere bevorzugt sind die Verbindungen Ia301., besonders die Verbindungen Ia301.1 - Ia301.64, die sich von den Verbindungen Ia1.1 - Ia1.64 dadurch unterscheiden, daß R¹ und R¹⁴ für Methyl, R² für Ethylsulfonyl und R¹² für Phenylsulfonyl stehen.

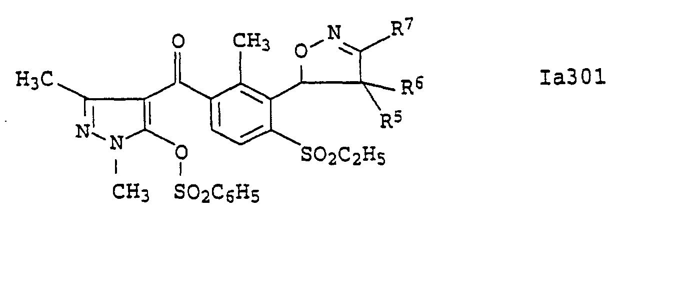

Ebenso insbesondere bevorzugt sind die Verbindungen Ia302., besonders die Verbindungen Ia302.1 - Ia302.64, die sich von den Verbindungen Ia1.1 - Ia1.64 dadurch unterscheiden, daß R¹ und R¹⁴ für Methyl, R² für Ethylsulfonyl, R¹² für Phenylsulfonyl und R¹³ für Ethyl stehen.

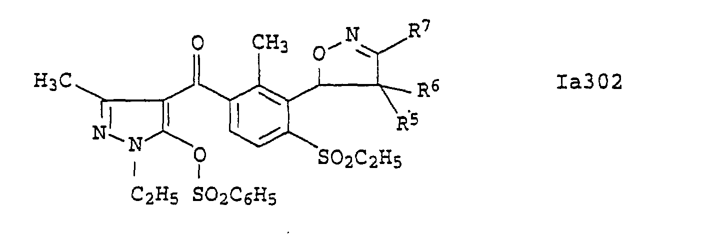

Ebenso insbesondere bevorzugt sind die Verbindungen Ia303., besonders die Verbindungen Ia303.1 - Ia303.64, die sich von den Verbindungen Ia1.1 - Ial.64 dadurch unterscheiden, daß R¹ und R¹⁴ für Methyl, R² für Ethylsulfonyl, R¹² für Phenylsulfonyl und R¹³ für Isopropyl stehen.

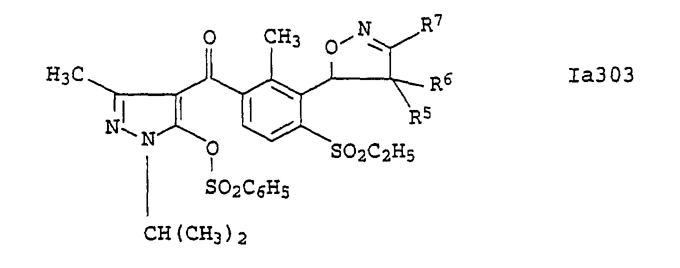

Ebenso insbesondere bevorzugt sind die Verbindungen Ia304., besonders die Verbindungen Ia304.1 - Ia304.64, die sich von den Verbindungen Ia1.1 - Ia1.64 dadurch unterscheiden, daß R¹ und R¹⁴ für Methyl, R² für Ethylsulfonyl, R¹² für Phenylsulfonyl und R¹³ für tert.-Butyl stehen.

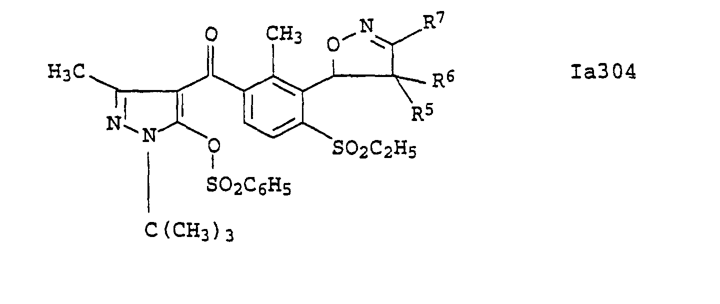

Ebenso insbesondere bevorzugt sind die Verbindungen Ia305., besonders die Verbindungen Ia305.1 - Ia305.64, die sich von den Verbindungen Ia1.1 - Ial.64 dadurch unterscheiden, daß R² für Chlor und R¹² für Phenylsulfonyl stehen.

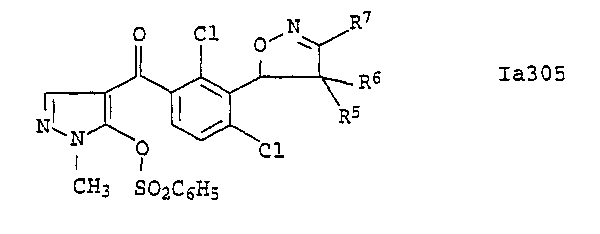

Ebenso insbesondere bevorzugt sind die Verbindungen Ia306., besonders die Verbindungen Ia306.1 - Ia306.64, die sich von den Verbindungen Ia1.1 - Ial.64 dadurch unterscheiden, daß R² für Chlor, R¹² für Phenylsulfonyl und R¹³ für Ethyl stehen.

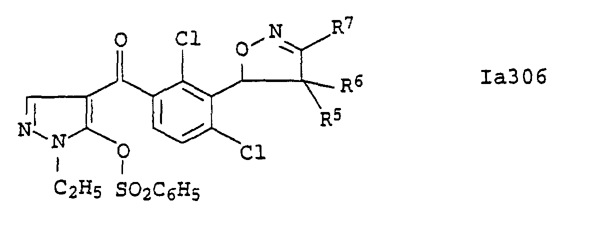

Ebenso insbesondere bevorzugt sind die Verbindungen Ia307., besonders die Verbindungen Ia307.1 - Ia307.64, die sich von den Verbindungen Ia1.1 - Ia1.64 dadurch unterscheiden, daß R² für Chlor, R¹² für Phenylsulfonyl und R¹³ für Isopropyl stehen.

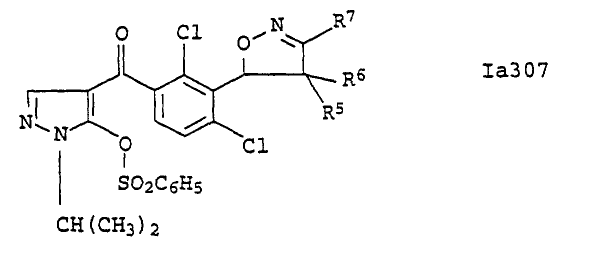

Ebenso insbesondere bevorzugt sind die Verbindungen Ia308., besonders die Verbindungen Ia308.1 - Ia308.64, die sich von den Verbindungen Ia1.1 - Ia1.64 dadurch unterscheiden, daß R² für Chlor, R¹² für Phenylsulfonyl und R¹³ für tert.-Butyl stehen.

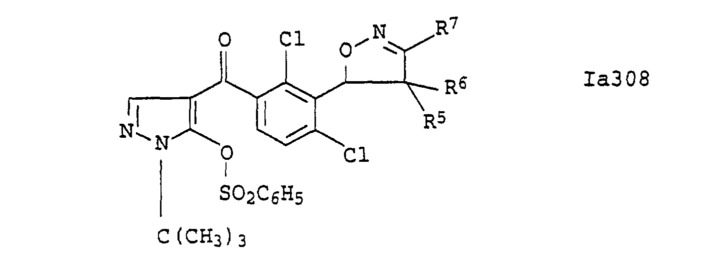

Ebenso insbesondere bevorzugt sind die Verbindungen Ia309., besonders die Verbindungen Ia309.1 - Ia309.64, die sich von den Verbindungen Ia1.1 - Ia1.64 dadurch unterscheiden, daß R² für Chlor, R¹² für Phenylsulfonyl und R¹⁴ für Methyl stehen.

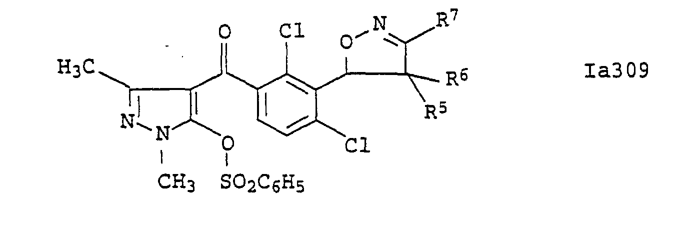

Ebenso insbesondere bevorzugt sind die Verbindungen Ia310., besonders die Verbindungen Ia310.1 - Ia310.64, die sich von den Verbindungen Ia1.1 - Ia1.64 dadurch unterscheiden, daß R² für Chlor, R¹² für Phenylsulfonyl, R¹³ für Ethyl und R¹⁴ für Methyl stehen.

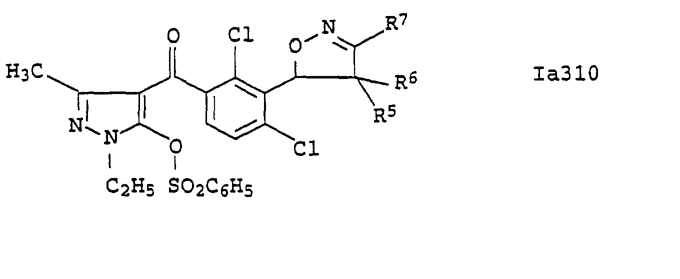

Ebenso insbesondere bevorzugt sind die Verbindungen Ia311., besonders die Verbindungen Ia311.1 - Ia311.64, die sich von den Verbindungen Ia1.1 - Ia1.64 dadurch unterscheiden, daß R² für Chlor, R¹² für Phenylsulfonyl, R¹³ für Isopropyl und R¹⁴ für Methyl stehen.

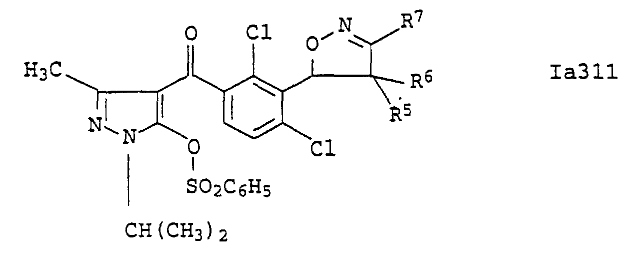

Ebenso insbesondere bevorzugt sind die Verbindungen Ia312., besonders die Verbindungen Ia312.1 - Ia312.64, die sich von den Verbindungen Ia1.1 - Ial.64 dadurch unterscheiden, daß R² für Chlor, R¹² für Phenylsulfonyl, R¹³ für tert.-Butyl und R¹⁴ für Methyl stehen.

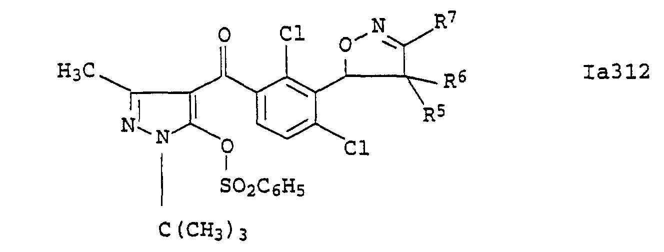

Ebenso insbesondere bevorzugt sind die Verbindungen Ia313., besonders die Verbindungen Ia313.1 - Ia313.64, die sich von den Verbindungen Ia1.1 - Ia1.64 dadurch unterscheiden, daß R¹ für Methyl, R² für Chlor und R¹² für Phenylsulfonyl stehen.

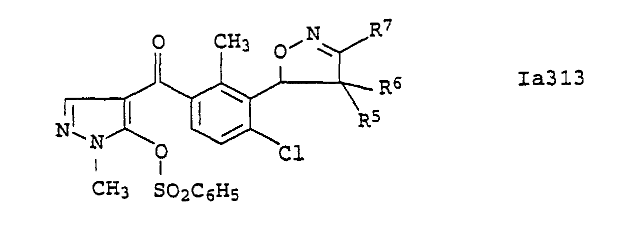

Ebenso insbesondere bevorzugt sind die Verbindungen Ia314., besonders die Verbindungen Ia314.1 - Ia314.64, die sich von den Verbindungen Ia1.1 - Ia1.64 dadurch unterscheiden, daß R¹ für Methyl, R² für Chlor, R¹² für Phenylsulfonyl und R¹³ für Ethyl stehen.

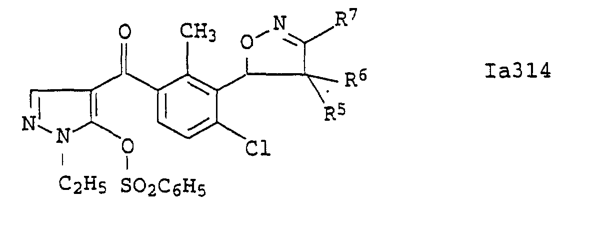

Ebenso insbesondere bevorzugt sind die Verbindungen Ia315., besonders die Verbindungen Ia315.1 - Ia315.64, die sich von den Verbindungen Ia1.1 - Ia1.64 dadurch unterscheiden, daß R¹ für Methyl, R² für Chlor, R¹² für Phenylsulfonyl und R¹³ für Isopropyl stehen.

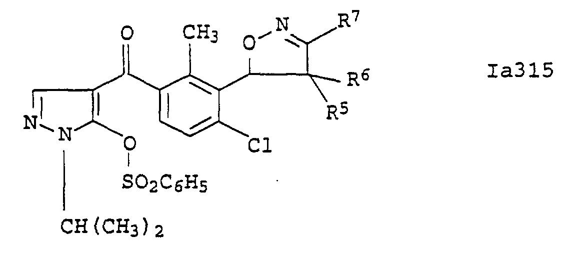

Ebenso insbesondere bevorzugt sind die Verbindungen Ia316., besonders die Verbindungen Ia316.1 - Ia316.64, die sich von den Verbindungen Ia1.1 - Ia1.64 dadurch unterscheiden, daß R¹ für Methyl, R² für Chlor, R¹² für Phenylsulfonyl und R¹³ für tert.-Butyl stehen.

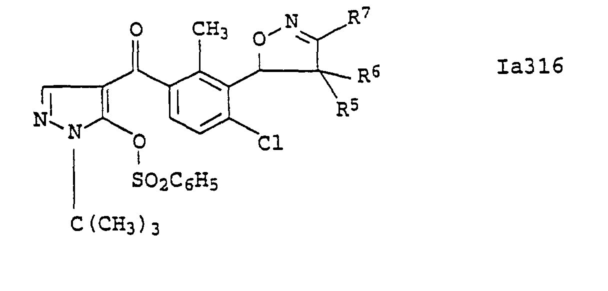

Ebenso insbesondere bevorzugt sind die Verbindungen Ia317., besonders die Verbindungen Ia317.1 - Ia317.64, die sich von den Verbindungen Ia1.1 - Ia1.64 dadurch unterscheiden, daß R¹ und R¹⁴ für Methyl, R² für Chlor und R¹² für Phenylsulfonyl stehen.

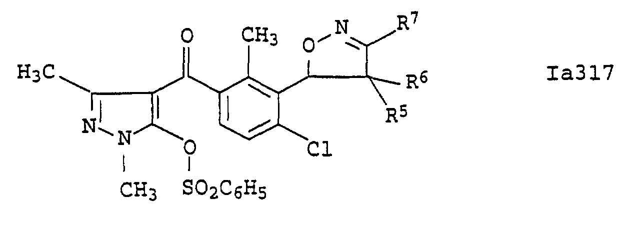

Ebenso insbesondere bevorzugt sind die Verbindungen Ia318., besonders die Verbindungen Ia318.1 - Ia318.64, die sich von den Verbindungen Ia1.1 - Ia1.64 dadurch unterscheiden, daß R¹ und R¹⁴ für Methyl, R² für Chlor, R¹² für Phenylsulfonyl und R¹³ für Ethyl stehen.

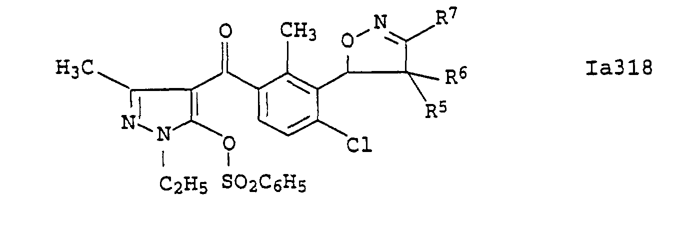

Ebenso insbesondere bevorzugt sind die Verbindungen Ia319., besonders die Verbindungen Ia319.1 - Ia319.64, die sich von den Verbindungen Ia1.1 - Ial.64 dadurch unterscheiden, daß R¹ und R¹⁴ für Methyl, R² für Chlor, R¹² für Phenylsulfonyl und R¹³ für Isopropyl stehen.

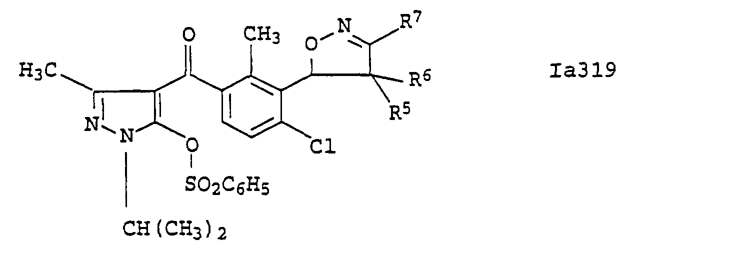

Ebenso insbesondere bevorzugt sind die Verbindungen Ia320., besonders die Verbindungen Ia320.1 - Ia320.64, die sich von den Verbindungen Ia1.1 - Ia1.64 dadurch unterscheiden, daß R¹ und R¹⁴ für Methyl, R² für Chlor, R¹² für Phenylsulfonyl und R¹³ für tert.-Butyl stehen.

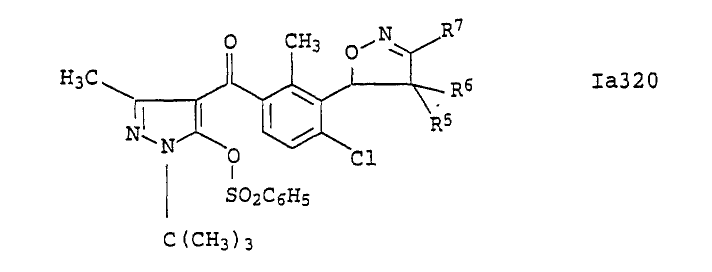

Ebenso insbesondere bevorzugt sind die Verbindungen Ia321., besonders die Verbindungen Ia321.1 - Ia321.64, die sich von den Verbindungen Ia1.1 - Ia1.64 dadurch unterscheiden, daß R¹² für 4-Methylphenylsulfonyl steht.

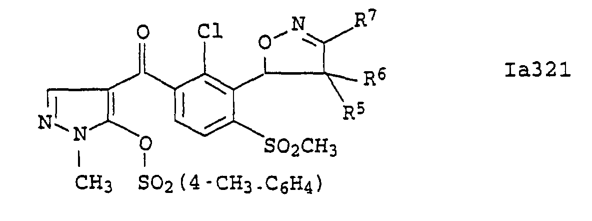

Ebenso insbesondere bevorzugt sind die Verbindungen Ia322., besonders die Verbindungen Ia322.1 - Ia322.64, die sich von den Verbindungen Ia1.1 - Ia1.64 dadurch unterscheiden, daß R¹² für 4-Methylphenylsulfonyl und R¹³ für Ethyl steht.

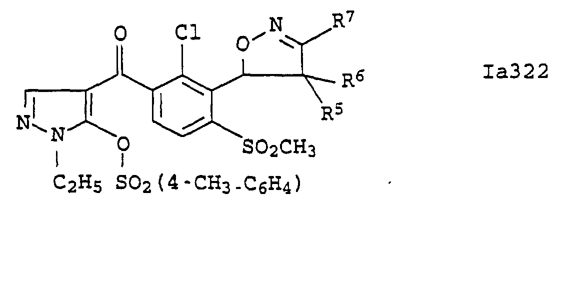

Ebenso insbesondere bevorzugt sind die Verbindungen Ia323., besonders die Verbindungen Ia323.1 - Ia323.64, die sich von den Verbindungen Ia1.1 - Ia1.64 dadurch unterscheiden, daß R¹² für 4-Methylphenylsulfonyl und R¹³ für Isopropyl stehen.

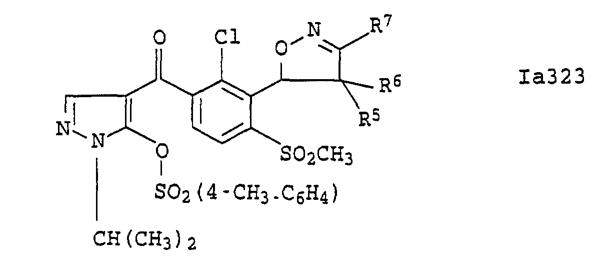

Ebenso insbesondere bevorzugt sind die Verbindungen Ia324., besonders die Verbindungen Ia324.1 - Ia324.64, die sich von den Verbindungen Ia1.1 - Ial.64 dadurch unterscheiden, daß R¹² für 4-Methylphenylsulfonyl und R¹³ für tert.-Butyl stehen.

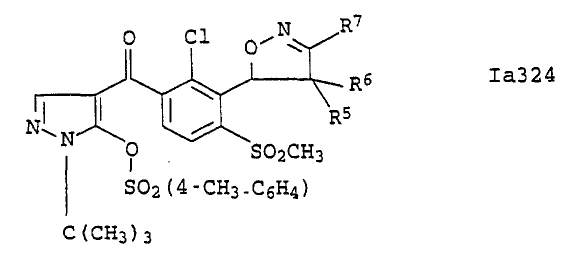

Ebenso insbesondere bevorzugt sind die Verbindungen Ia325., besonders die Verbindungen Ia325.1 - Ia325.64, die sich von den Verbindungen Ia1.1 - Ia1.64 dadurch unterscheiden, daß R¹² für 4-Methylphenylsulfonyl und R¹⁴ für Methyl stehen.

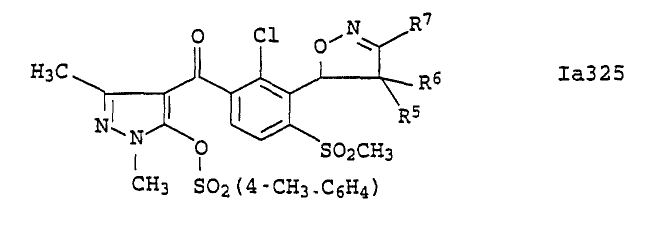

Ebenso insbesondere bevorzugt sind die Verbindungen Ia326., besonders die Verbindungen Ia326.1 - Ia326.64, die sich von den Verbindungen Ia1.1 - Ia1.64 dadurch unterscheiden, daß R¹² für 4-Methylphenylsulfonyl, R¹³ für Ethyl und R¹⁴ für Methyl stehen.

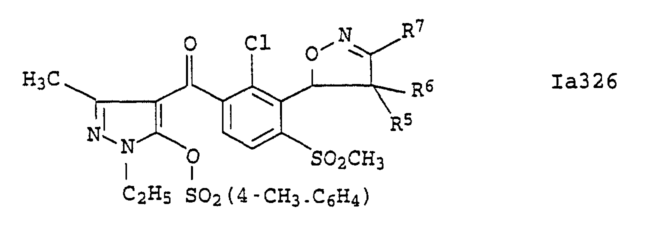

Ebenso insbesondere bevorzugt sind die Verbindungen Ia327., besonders die Verbindungen Ia327.1 - Ia327.64, die sich von den Verbindungen Ia1.1 - Ia1.64 dadurch unterscheiden, daß R¹² für 4-Methylphenylsulfonyl, R¹³ für Isopropyl und R¹⁴ für Methyl stehen.

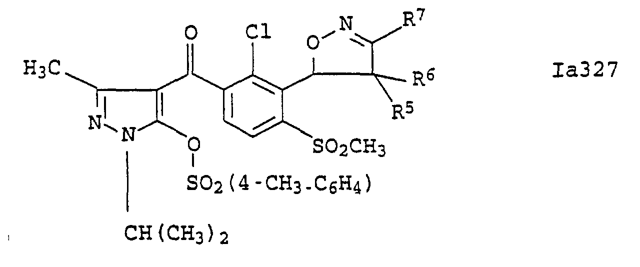

Ebenso insbesondere bevorzugt sind die Verbindungen Ia328., besonders die Verbindungen Ia328.1 - Ia328.64, die sich von den Verbindungen Ia1.1 - Ia1.64 dadurch unterscheiden, daß R¹² für 4-Methylphenylsulfonyl, R¹³ für tert.-Butyl und R¹⁴ für Methyl stehen.

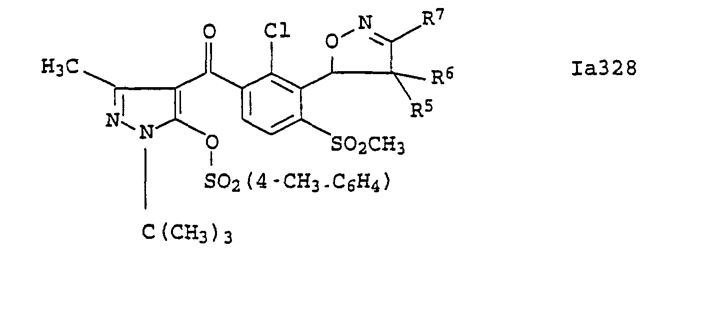

Ebenso insbesondere bevorzugt sind die Verbindungen Ia329., besonders die Verbindungen Ia329.1 - Ia329.64, die sich von den Verbindungen Ia1.1 - Ia1.64 dadurch unterscheiden, daß R¹ für Methyl und R¹² für 4-Methylphenylsulfonyl stehen.

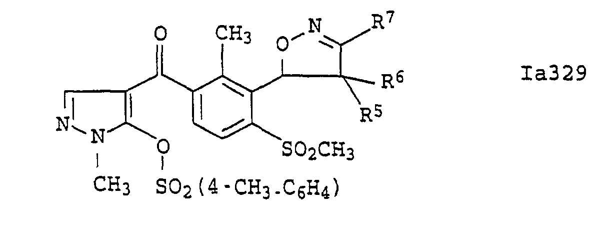

Ebenso insbesondere bevorzugt sind die Verbindungen Ia330., besonders die Verbindungen Ia330.1 - Ia330.64, die sich von den Verbindungen Ia1.1 - Ial.64 dadurch unterscheiden, daß R¹ für Methyl, R¹² für 4-Methylphenylsulfonyl und R¹³ für Ethyl stehen.

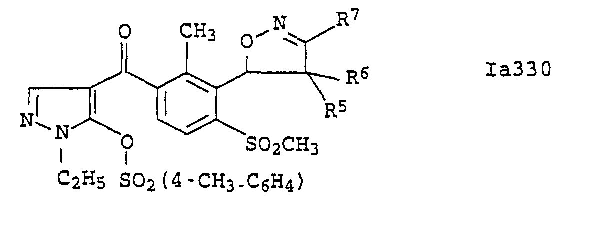

Ebenso insbesondere bevorzugt sind die Verbindungen Ia331., besonders die Verbindungen Ia331.1 - Ia331.64, die sich von den Verbindungen Ia1.1 - Ia1.64 dadurch unterscheiden, daß R¹ für Methyl, R¹² für 4-Methylphenylsulfonyl und R¹³ für Isopropyl stehen.

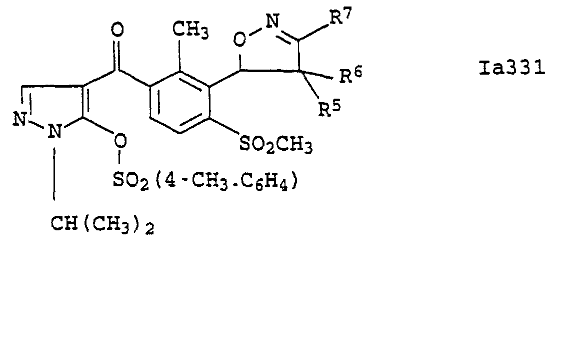

Ebenso insbesondere bevorzugt sind die Verbindungen Ia332., besonders die Verbindungen Ia332.1 - Ia332.64, die sich von den Verbindungen Ia1.1 - Ia1.64 dadurch unterscheiden, daß R¹ für Methyl, R¹² für 4-Methylphenylsulfonyl und R¹³ für tert.-Butyl stehen.

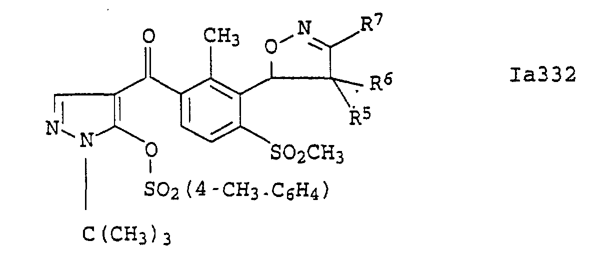

Ebenso insbesondere bevorzugt sind die Verbindungen Ia333., besonders die Verbindungen Ia333.1 - Ia333.64, die sich von den Verbindungen Ia1.2 - Ia1.64 dadurch unterscheiden, daß R¹ und R¹⁴ für Methyl und R¹² für 4-Methylphenylsulfonyl stehen.

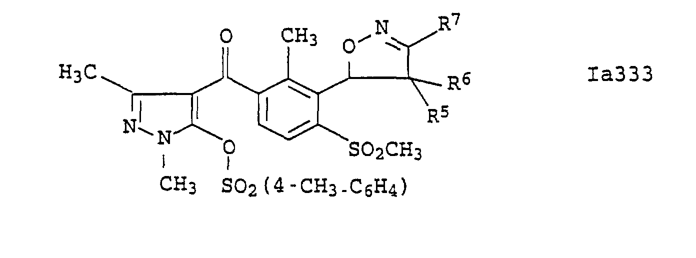

Ebenso insbesondere bevorzugt sind die Verbindungen Ia334., besonders die Verbindungen Ia334.1 - Ia334.64, die sich von den Verbindungen Ia1.1 - Ia1.64 dadurch unterscheiden, daß R¹ und R¹⁴ für Methyl, R¹² für 4-Methylphenylsulfonyl und R¹³ für Ethyl stehen.

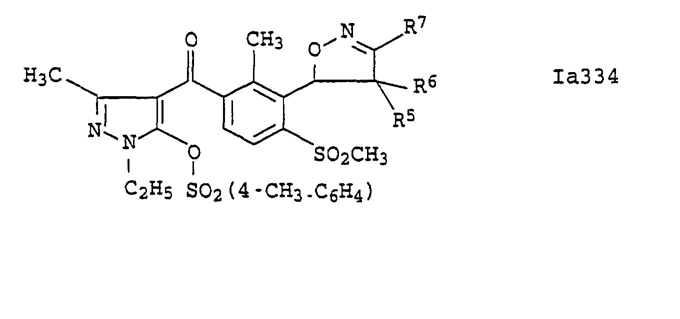

Ebenso insbesondere bevorzugt sind die Verbindungen Ia335., besonders die Verbindungen Ia335.1 - Ia334.64, die sich von den Verbindungen Ia1.1 - Ia1.64 dadurch unterscheiden, daß R¹ und R¹⁴ für Methyl, R¹² für 4-Methylphenylsulfonyl und R¹³ für Isopropyl stehen.

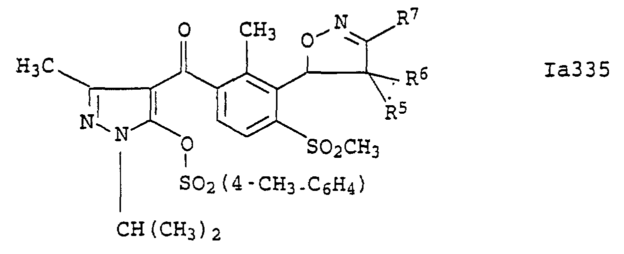

Ebenso insbesondere bevorzugt sind die Verbindungen Ia336., besonders die Verbindungen Ia336.1 - Ia336.64, die sich von den Verbindungen Ia1.1 - Ia1.64 dadurch unterscheiden, daß R¹ und R¹⁴ für Methyl, R¹² für 4-Methylphenylsulfonyl und R¹³ für tert.-Butyl stehen.

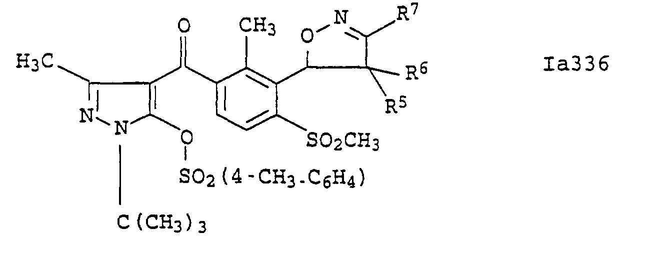

Ebenso insbesondere bevorzugt sind die Verbindungen Ia337., besonders die Verbindungen Ia337.1 - Ia337.64, die sich von den Verbindungen Ia1.1 - Ia1.64 dadurch unterscheiden, daß R² für Ethylsulfonyl und R¹² für 4-Methylphenylsulfonyl stehen.

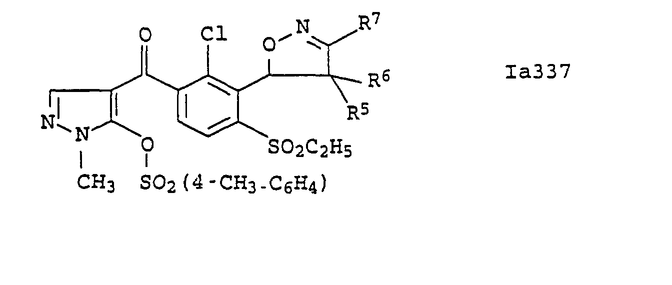

Ebenso insbesondere bevorzugt sind die Verbindungen Ia338., besonders die Verbindungen Ia338.1 - Ia338.64, die sich von den Verbindungen Ia1.1 - Ia1.64 dadurch unterscheiden, daß R² für Ethylsulfonyl, R¹² für 4-Methylphenylsulfonyl und R¹³ für Ethyl stehen.

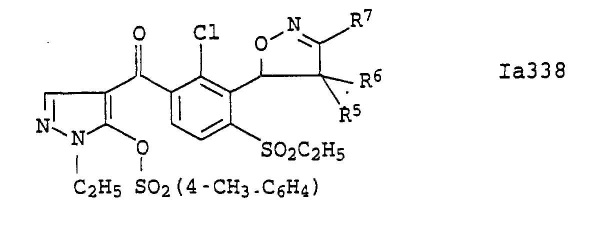

Ebenso insbesondere bevorzugt sind die Verbindungen Ia339., besonders die Verbindungen Ia339.1 - Ia339.64, die sich von den Verbindungen Ia1.1 - Ia1.64 dadurch unterscheiden, daß R² für Ethylsulfonyl, R¹² für 4-Methylphenylsulfonyl und R¹³ für Isopropyl stehen.

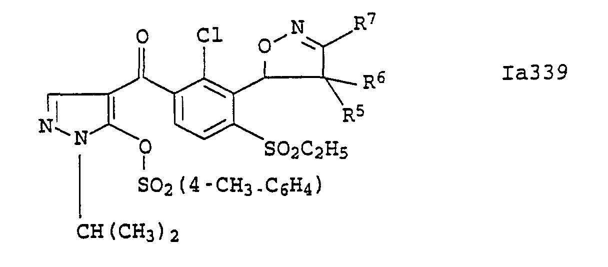

Ebenso insbesondere bevorzugt sind die Verbindungen Ia340., besonders die Verbindungen Ia340.1 - Ia340.64, die sich von den Verbindungen Ia1.1 - Ial.64 dadurch unterscheiden, daß R² für Ethylsulfonyl, R¹² für 4-Methylphenylsulfonyl und R¹³ für tert.-Butyl stehen.

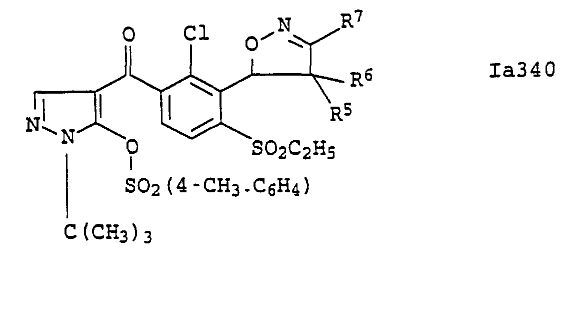

Ebenso insbesondere bevorzugt sind die Verbindungen Ia341., besonders die Verbindungen Ia341.1 - Ia341.64, die sich von den Verbindungen Ia1.1 - Ia1.64 dadurch unterscheiden, daß R² für Ethylsulfonyl, R¹² für 4-Methylphenylsulfonyl und R¹⁴ für Methyl stehen.

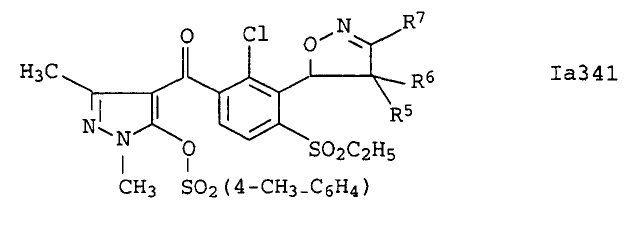

Ebenso insbesondere bevorzugt sind die Verbindungen Ia342., besonders die Verbindungen Ia342.1 - Ia342.64, die sich von den Verbindungen Ia1.1 - Ia1.64 dadurch unterscheiden, daß R² für Ethylsulfonyl, R¹² für 4-Methylphenylsulfonyl, R¹³ für Ethyl und R¹⁴ für Methyl stehen.

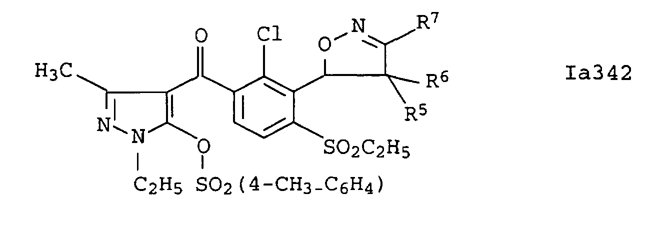

Ebenso insbesondere bevorzugt sind die Verbindungen Ia343., besonders die Verbindungen Ia343.1 - Ia343.64, die sich von den Verbindungen Ia1.1 - Ia1.64 dadurch unterscheiden, daß R² für Ethylsulfonyl, R¹² für 4-Methylphenylsulfonyl, R¹³ für Isopropyl und R¹⁴ für Methyl stehen.

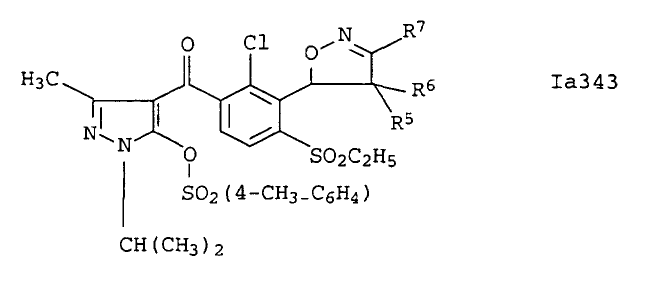

Ebenso insbesondere bevorzugt sind die Verbindungen Ia344., besonders die Verbindungen Ia344.1 - Ia344.64, die sich von den Verbindungen Ia1.1 - Ia1.64 dadurch unterscheiden, daß R² für Ethylsulfonyl, R¹² für 4-Methylphenylsulfonyl, R¹³ für tert.-Butyl und R¹⁴ für Methyl stehen.

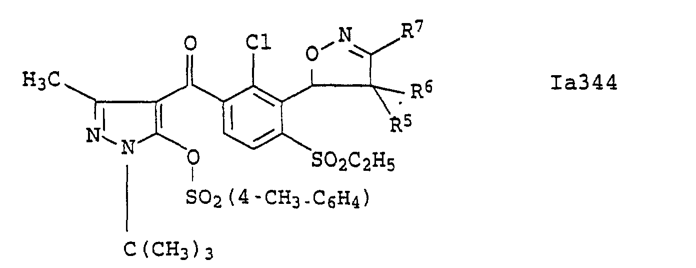

Ebenso insbesondere bevorzugt sind die Verbindungen Ia345., besonders die Verbindungen Ia345.1 - Ia345.64, die sich von den Verbindungen Ia1.1 - Ial.64 dadurch unterscheiden, daß R¹ für Methyl, R² für Ethylsulfonyl und R¹² für 4-Methylphenylsulfonyl stehen.

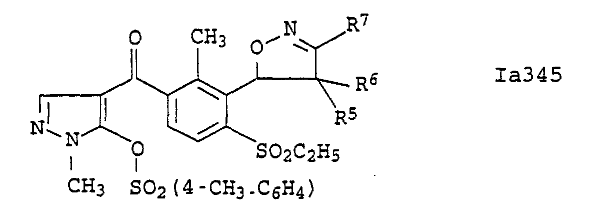

Ebenso insbesondere bevorzugt sind die Verbindungen Ia346., besonders die Verbindungen Ia346.1 - Ia346.64, die sich von den Verbindungen Ia1.1 - Ia1.64 dadurch unterscheiden, daß R¹ für Methyl, R² für Ethylsulfonyl, R¹² für 4-Methylphenylsulfonyl und R¹³ für Ethyl stehen.

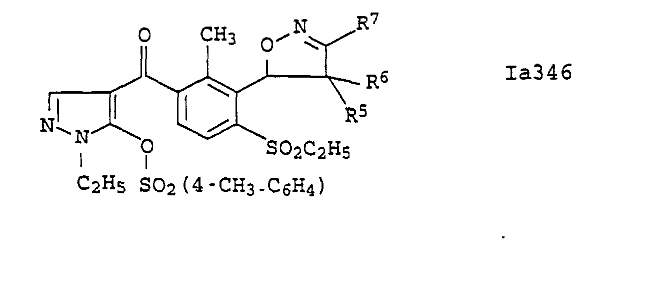

Ebenso insbesondere bevorzugt sind die Verbindungen Ia347., besonders die Verbindungen Ia347.1 - Ia347.64, die sich von den Verbindungen Ia1.1 - Ial.64 dadurch unterscheiden, daß R¹ für Methyl, R² für Ethylsulfonyl, R¹² für 4-Methylphenylsulfonyl und R¹³ für Isopropyl stehen.

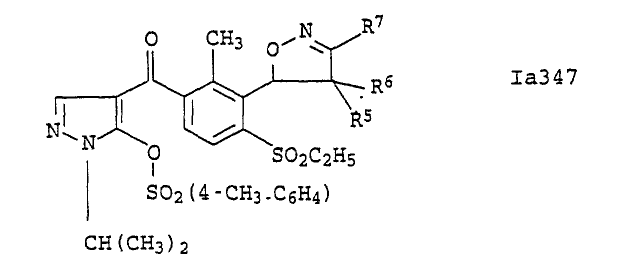

Ebenso insbesondere bevorzugt sind die Verbindungen Ia348., besonders die Verbindungen Ia348.1 - Ia348.64, die sich von den Verbindungen Ia1.1 - Ia1.64 dadurch unterscheiden, daß R¹ für Methyl, R² für Ethylsulfonyl, R¹² für 4-Methylphenylsulfonyl und R¹³ für tert.-Butyl stehen.

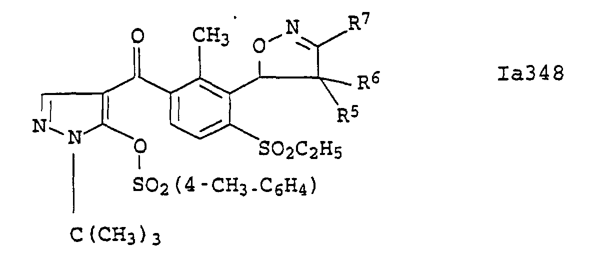

Ebenso insbesondere bevorzugt sind die Verbindungen Ia349., besonders die Verbindungen Ia349.1 - Ia348.64, die sich von den Verbindungen Ia1.1 - Ia1.64 dadurch unterscheiden, daß R¹ und R¹⁴ für Methyl, R² für Ethylsulfonyl und R¹² für 4-Methylphenylsulfonyl stehen.

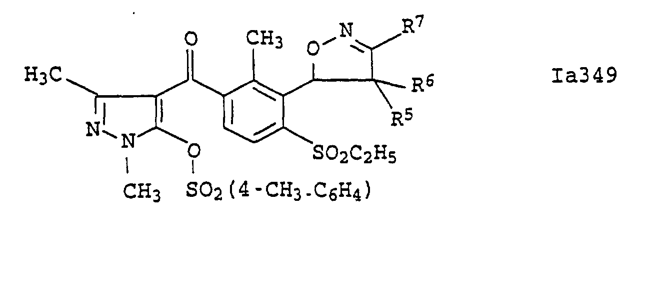

Ebenso insbesondere bevorzugt sind die Verbindungen Ia350., besonders die Verbindungen Ia350.1 - Ia350.64, die sich von den Verbindungen Ia1.1 - Ia1.64 dadurch unterscheiden, daß R¹ und R¹⁴ für Methyl, R² für Ethylsulfonyl, R¹² für 4-Methylphenylsulfonyl und R¹³ für Ethyl stehen.

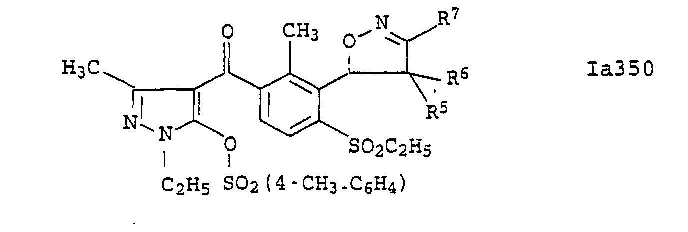

Ebenso insbesondere bevorzugt sind die Verbindungen Ia351., besonders die Verbindungen Ia351.1 - Ia351.64, die sich von den Verbindungen Ia1.1 - Ia1.64 dadurch unterscheiden, daß R¹ und R¹⁴ für Methyl, R² für Ethylsulfonyl, R¹² für 4-Methylphenylsulfonyl und R¹³ für Isopropyl stehen.

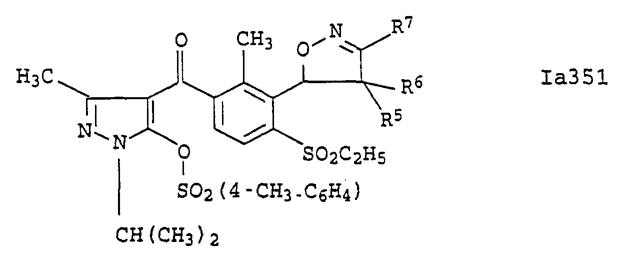

Ebenso insbesondere bevorzugt sind die Verbindungen Ia352., besonders die Verbindungen Ia352.1 - Ia352.64, die sich von den Verbindungen Ia1.1 - Ia1.64 dadurch unterscheiden, daß R¹ und R¹⁴ für Methyl, R² für Ethylsulfonyl, R¹² für 4-Methylphenylsulfonyl und R¹³ für tert.-Butyl stehen.

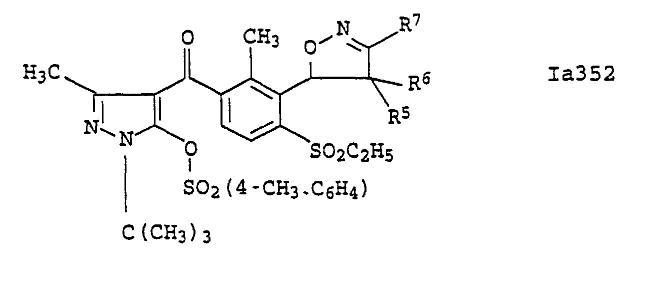

Ebenso insbesondere bevorzugt sind die Verbindungen Ia353., besonders die Verbindungen Ia353.1 - Ia353.64, die sich von den Verbindungen Ia1.1 - Ia1.64 dadurch unterscheiden, daß R² für Chlor und R¹² für 4-Methylphenylsulfonyl stehen.

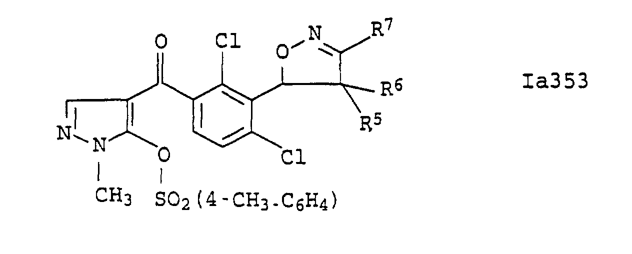

Ebenso insbesondere bevorzugt sind die Verbindungen Ia354., besonders die Verbindungen Ia354.1 - Ia354.64, die sich von den Verbindungen Ia1.1 - Ia1.64 dadurch unterscheiden, daß R² für Chlor, R¹² für 4-Methylphenylsulfonyl und R¹³ für Ethyl stehen.

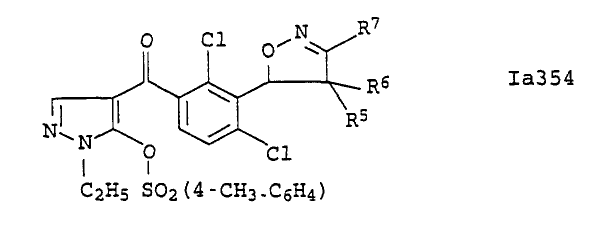

Ebenso insbesondere bevorzugt sind die Verbindungen Ia355., besonders die Verbindungen Ia355.1 - Ia355.64, die sich von den Verbindungen Ia1.1 - Ia1.64 dadurch unterscheiden, daß R² für Chlor, R¹² für 4-Methylphenylsulfonyl und R¹³ für Isopropyl stehen.

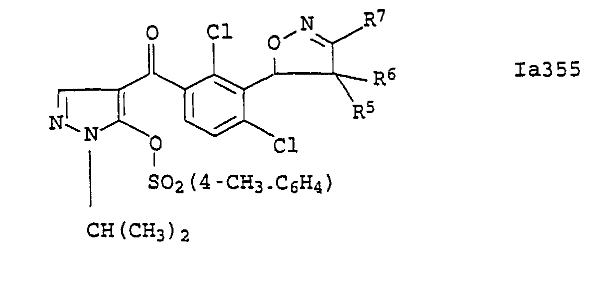

Ebenso insbesondere bevorzugt sind die Verbindungen Ia356., besonders die Verbindungen Ia356.1 - Ia356.64, die sich von den Verbindungen Ia1.1 - Ia1.64 dadurch unterscheiden, daß R² für Chlor, R¹² für 4-Methylphenylsulfonyl und R¹³ für tert.-Butyl stehen.

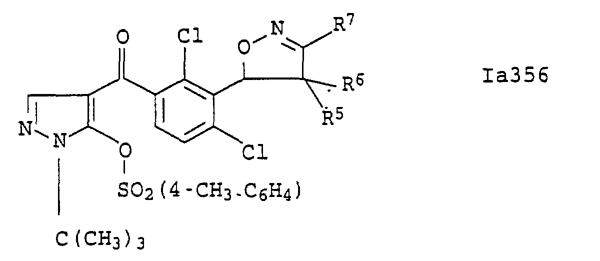

Ebenso insbesondere bevorzugt sind die Verbindungen Ia357., besonders die Verbindungen Ia357.1 - Ia357.64, die sich von den Verbindungen Ia1.1 - Ia1.64 dadurch unterscheiden, daß R² für Chlor, R¹² für 4-Methylphenylsulfonyl und R¹⁴ für Methyl stehen.

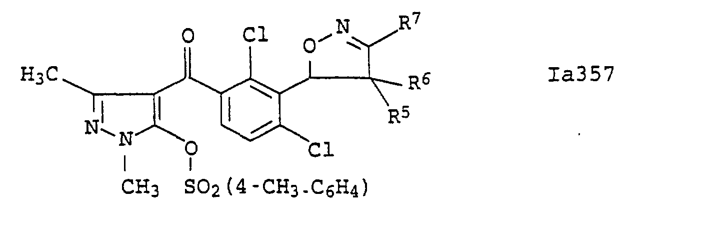

Ebenso insbesondere bevorzugt sind die Verbindungen Ia358., besonders die Verbindungen Ia358.1 - Ia358.64, die sich von den Verbindungen Ia1.1 - Ia1.64 dadurch unterscheiden, daß R² für Chlor, R¹² für 4-Methylphenylsulfonyl, R¹³ für Ethyl und R¹⁴ für Methyl stehen.

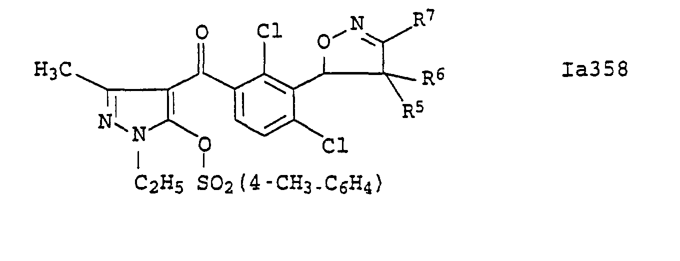

Ebenso insbesondere bevorzugt sind die Verbindungen Ia359., besonders die Verbindungen Ia359.1 - Ia359.64, die sich von den Verbindungen Ia1.1 - Ia1.64 dadurch unterscheiden, daß R² für Chlor, R¹² für 4-Methylphenylsulfonyl, R¹³ für Isopropyl und R¹⁴ für Methyl stehen.

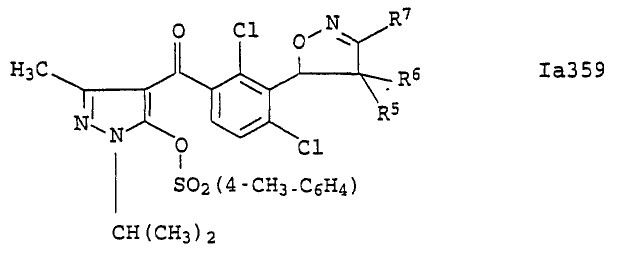

Ebenso insbesondere bevorzugt sind die Verbindungen Ia360., besonders die Verbindungen Ia360.1 - Ia360.64, die sich von den Verbindungen Ia1.1 - Ia1.64 dadurch unterscheiden, daß R² für Chlor, R¹² für 4-Methylphenylsulfonyl, R¹³ für tert.-Butyl und R¹⁴ für Methyl stehen.

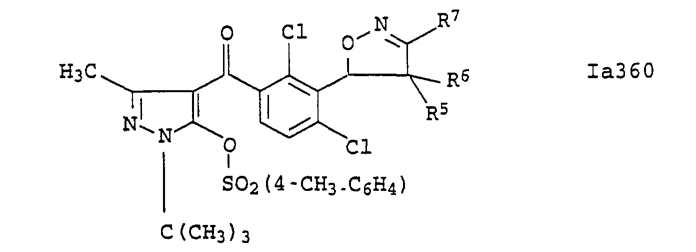

Ebenso insbesondere bevorzugt sind die Verbindungen Ia361., besonders die Verbindungen Ia361.1 - Ia361.64, die sich von den Verbindungen Ia1.1 - Ial.64 dadurch unterscheiden, daß R¹ für Methyl, R² für Chlor und R¹² für 4-Methylphenylsulfonyl stehen.

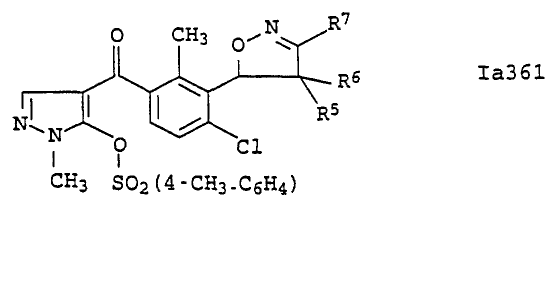

Ebenso insbesondere bevorzugt sind die Verbindungen Ia362., besonders die Verbindungen Ia362.1 - Ia362.64, die sich von den Verbindungen Ia1.1 - Ial.64 dadurch unterscheiden, daß R¹ für Methyl, R² für Chlor, R¹² für 4-Methylphenylsulfonyl und R¹³ für Ethyl stehen.

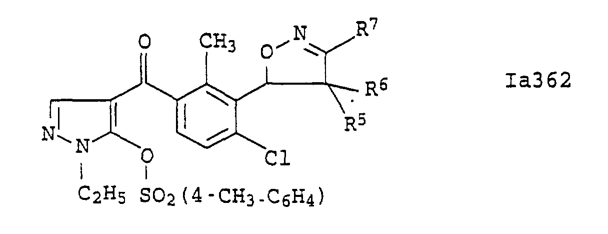

Ebenso insbesondere bevorzugt sind die Verbindungen Ia363., besonders die Verbindungen Ia363.1 - Ia363.64, die sich von den Verbindungen Ia1.1 - Ia1.64 dadurch unterscheiden, daß R¹ für Methyl, R² für Chlor, R¹² für 4-Methylphenylsulfonyl und R¹³ für Isopropyl stehen.

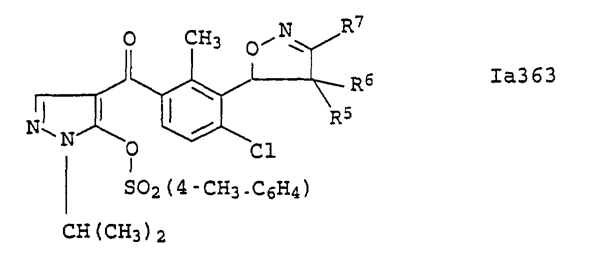

Ebenso insbesondere bevorzugt sind die Verbindungen Ia364., besonders die Verbindungen Ia364.1 - Ia364.64, die sich von den Verbindungen Ia1.1 - Ia1.64 dadurch unterscheiden, daß R¹ für Methyl, R² für Chlor, R¹² für 4-Methylphenylsulfonyl und R¹³ für tert.-Butyl stehen.

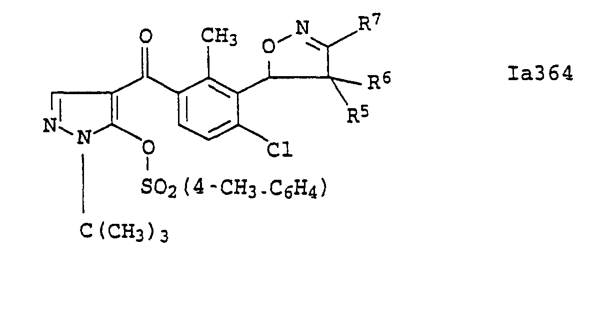

Ebenso insbesondere bevorzugt sind die Verbindungen Ia365., besonders die Verbindungen Ia365.1 - Ia365.64, die sich von den Verbindungen Ia1.1 - Ia1.64 dadurch unterscheiden, daß R¹ und R¹⁴ für Methyl, R² für Chlor und R¹² für 4-Methylphenylsulfonyl stehen.

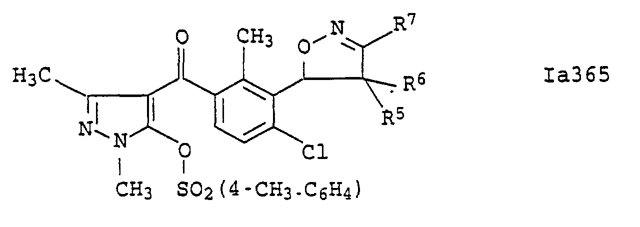

Ebenso insbesondere bevorzugt sind die Verbindungen Ia366., besonders die Verbindungen Ia366.1 - Ia366.64, die sich von den Verbindungen Ia1.1 - Ia1.64 dadurch unterscheiden, daß R¹ und R¹⁴ für Methyl, R² für Chlor, R¹² für 4-Methylphenylsulfonyl und R¹³ für Ethyl stehen.

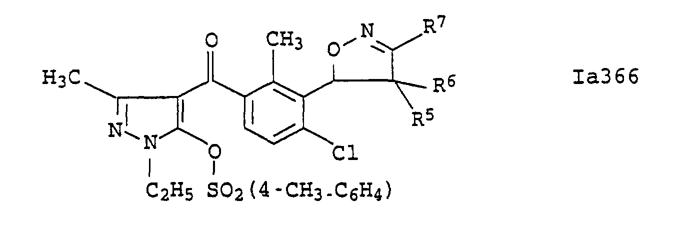

Ebenso insbesondere bevorzugt sind die Verbindungen Ia367., besonders die Verbindungen Ia367.1 - Ia367.64, die sich von den Verbindungen Ia1.1 - Ia1.64 dadurch unterscheiden, daß R¹ und R¹⁴ für Methyl, R² für Chlor, R¹² für 4-Methylphenylsulfonyl und R¹³ für Isopropyl stehen.

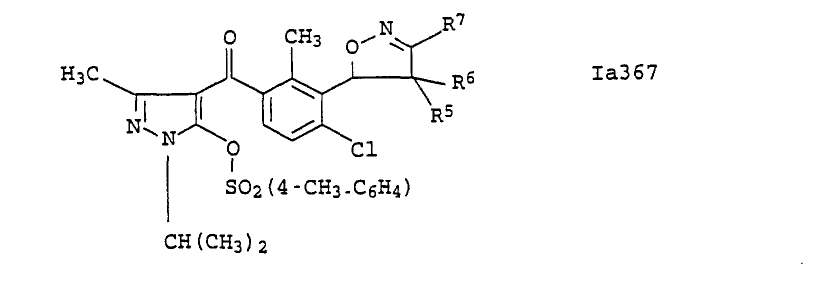

Ebenso insbesondere bevorzugt sind die Verbindungen Ia368., besonders die Verbindungen Ia368.1 - Ia368.64, die sich von den Verbindungen Ia1.1 - Ial.64 dadurch unterscheiden, daß R¹ und R¹⁴ für Methyl, R² für Chlor, R¹² für 4-Methylphenylsulfonyl und R¹³ für tert.-Butyl stehen.

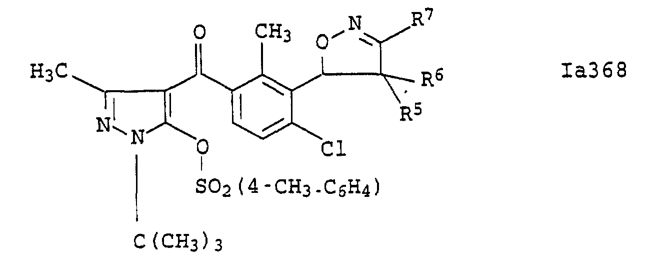

Ebenso insbesondere bevorzugt sind die Verbindungen Ia369., besonders die Verbindungen Ia369.1 bis Ia369.64, die sich von den Verbindungen Ia1.1 - Ia1.64 dadurch unterscheiden, daß R¹³ für Cyclopropyl steht.

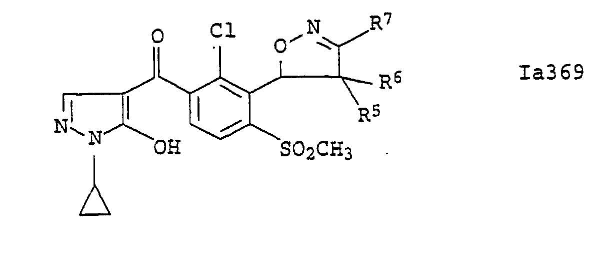

Ebenso insbesondere bevorzugt sind die Verbindungen Ia370., besonders die Verbindungen Ia370.1 bis Ia370.64, die sich von den Verbindungen Ia1.1 - Ia1.64 dadurch unterscheiden, daß R¹³ für Cyclopropyl und R¹⁴ für Methyl stehen.

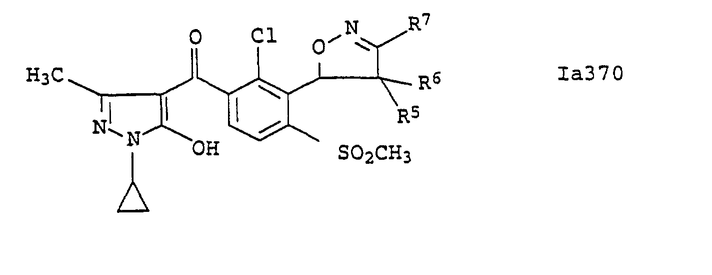

Ebenso insbesondere bevorzugt sind die Verbindungen Ia371., besonders die Verbindungen Ia371.1 bis Ia371.64, die sich von den Verbindungen Ia1.1 - Ia1.64 dadurch unterscheiden, daß R¹ für Methyl und R¹³ für Cyclopropyl stehen.

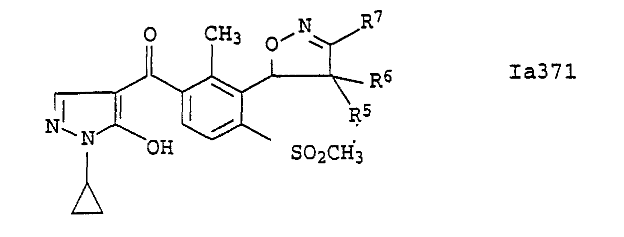

Ebenso insbesondere bevorzugt sind die Verbindungen Ia372., besonders die Verbindungen Ia372.1 bis Ia372.64, die sich von den Verbindungen Ia1.1 - Ia1.64 dadurch unterscheiden, daß R¹ für Methyl, R¹³ für Cyclopropyl und R¹⁴ für Methyl stehen.

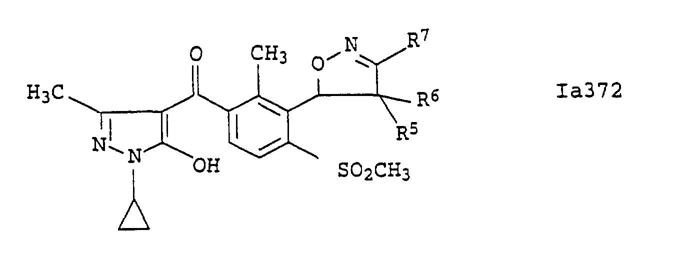

Ebenso insbesondere bevorzugt sind die Verbindungen Ia373., besonders die Verbindungen Ia373.1 bis Ia373.64, die sich von den Verbindungen Ia1.1 - Ia1.64 dadurch unterscheiden, daß R¹ für Trifluormethyl und R¹³ für Cyclopropyl stehen.

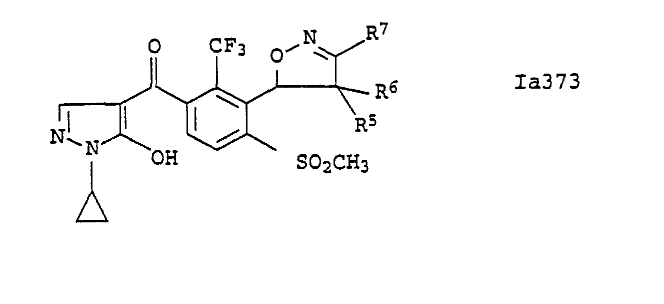

Ebenso insbesondere bevorzugt sind die Verbindungen Ia374., besonders die Verbindungen Ia374.1 bis Ia374.64, die sich von den Verbindungen Ia1.1 - Ia1.64 dadurch unterscheiden, daß R¹ für Trifluormethyl, R¹³ für Cyclopropyl und R¹⁴ für Methyl stehen.

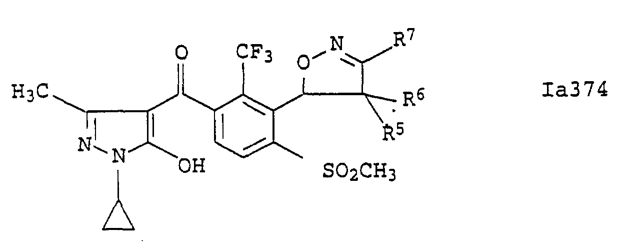

Ebenso insbesondere bevorzugt sind die Verbindungen Ia375., besonders die Verbindungen Ia375.1 bis Ia375.64, die sich von den Verbindungen Ia1.1 - Ia1.64 dadurch unterscheiden, daß R¹ für Methoxy und R¹³ für Cyclopropyl stehen.

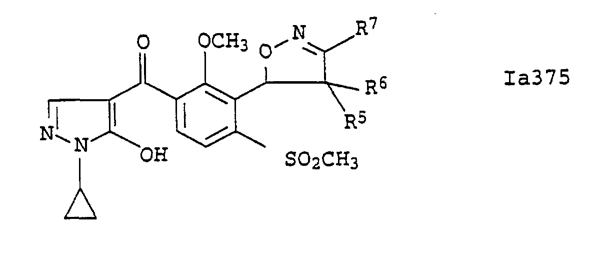

Ebenso insbesondere bevorzugt sind die Verbindungen Ia376., besonders die Verbindungen Ia376.1 bis Ia376.64, die sich von den Verbindungen Ia1.1 - Ia1.64 dadurch unterscheiden, daß R¹ für Methoxy, R¹³ für Cyclopropyl und R¹⁴ für Methyl stehen.

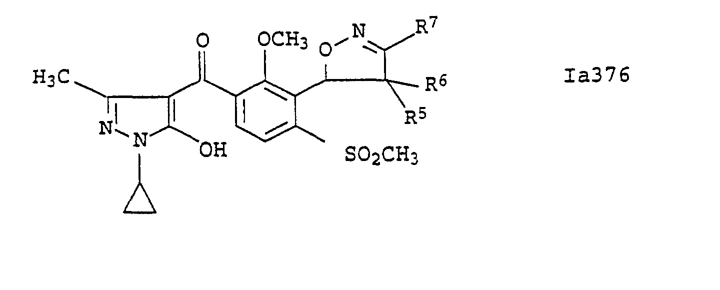

Ebenso insbesondere bevorzugt sind die Verbindungen Ia377., besonders die Verbindungen Ia377.1 bis Ia377.64, die sich von den Verbindungen Ia1.1 - Ia1.64 dadurch unterscheiden, daß R² für Ethylsulfonyl und R¹³ für Cyclopropyl stehen.

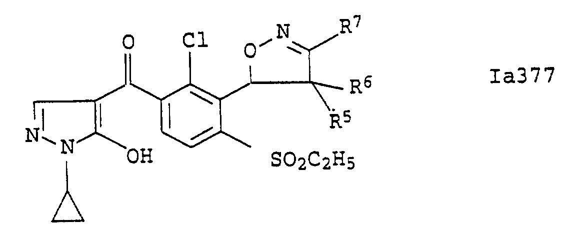

Ebenso insbesondere bevorzugt sind die Verbindungen Ia378., besonders die Verbindungen Ia378.1 bis Ia378.64, die sich von den Verbindungen Ia1.1 - Ia1.64 dadurch unterscheiden, daß R² für Ethylsulfonyl, R¹³ für Cyclopropyl und R¹⁴ für Methyl stehen.

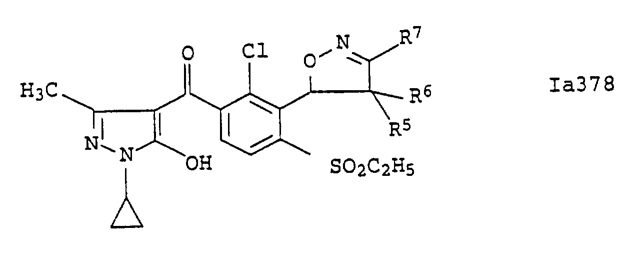

Ebenso insbesondere bevorzugt sind die Verbindungen Ia379., besonders die Verbindungen Ia379.1 bis Ia379.64, die sich von den Verbindungen Ia1.1 - Ia1.64 dadurch unterscheiden, daß R¹ für Methyl, R² für Ethylsulfonyl und R¹³ für Cyclopropyl stehen.

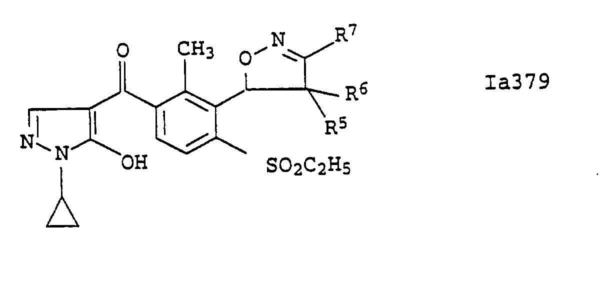

Ebenso insbesondere bevorzugt sind die Verbindungen Ia380., besonders die Verbindungen Ia380.1 bis Ia380.64, die sich von den Verbindungen Ia1.1 - Ia1.64 dadurch unterscheiden, daß R¹ für Methyl, R² für Ethylsulfonyl, R¹³ für Cyclopropyl und R¹⁴ für Methyl stehen.

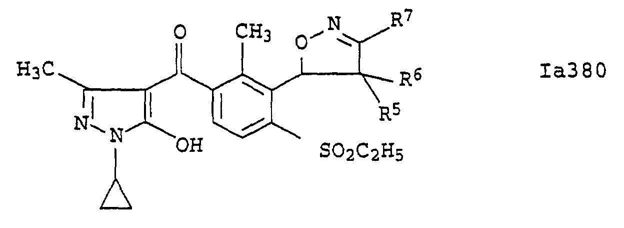

Ebenso insbesondere bevorzugt sind die Verbindungen Ia381., besonders die Verbindungen Ia381.1 bis Ia381.64, die sich von den Verbindungen Ia1.1 - Ia1.64 dadurch unterscheiden, daß R¹ für Trifluormethyl, R² für Ethylsulfonyl und R¹³ für Cyclopropyl stehen.

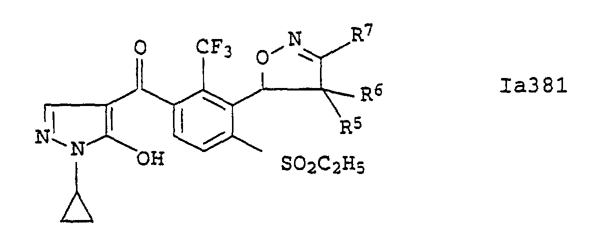

Ebenso insbesondere bevorzugt sind die Verbindungen Ia382., besonders die Verbindungen Ia382.1 bis Ia382.64, die sich von den Verbindungen Ia1.1 - Ia1.64 dadurch unterscheiden, daß R¹ für Trifluormethyl, R² für Ethylsulfonyl, R¹³ für Cyclopropyl und R¹⁴ für Methyl stehen.

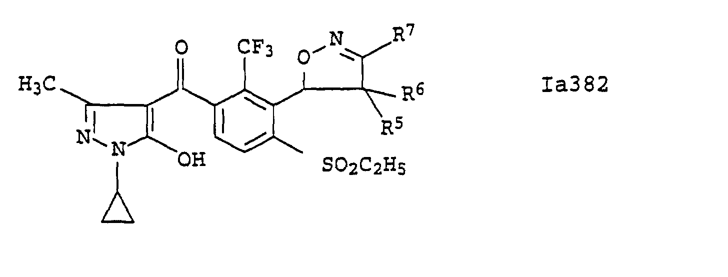

Ebenso insbesondere bevorzugt sind die Verbindungen Ia383., besonders die Verbindungen Ia383.1 bis Ia383.64, die sich von den Verbindungen Ia1.1 - Ia1.64 dadurch unterscheiden, daß R¹ für Methoxy, R² für Ethylsulfonyl und R¹³ für Cyclopropyl stehen.

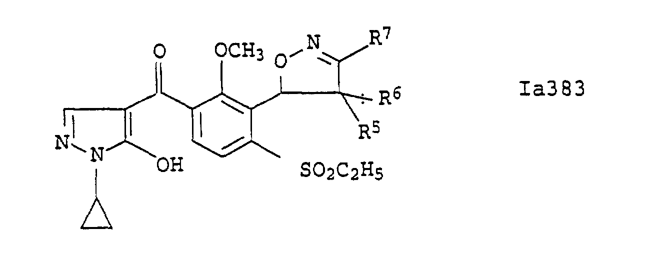

Ebenso insbesondere bevorzugt sind die Verbindungen Ia384., besonders die Verbindungen Ia384.1 bis Ia384.64, die sich von den Verbindungen Ia1.1 - Ia1.64 dadurch unterscheiden, daß R¹ für Methoxy, R² für Ethylsulfonyl, R¹³ für Cyclopropyl und R¹⁴ für Methyl stehen.

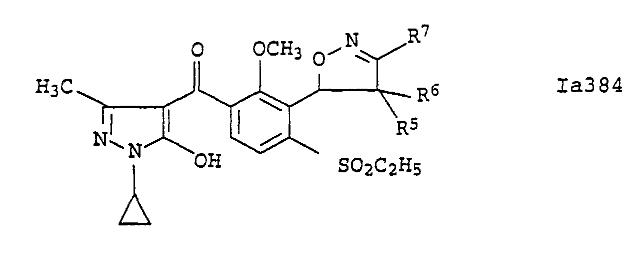

Ebenso insbesondere bevorzugt sind die Verbindungen Ia385., besonders die Verbindungen Ia385.1 bis Ia385.64, die sich von den Verbindungen Ia1.1 - Ia1.64 dadurch unterscheiden, daß R² für Chlor und R¹³ für Cyclopropyl stehen.

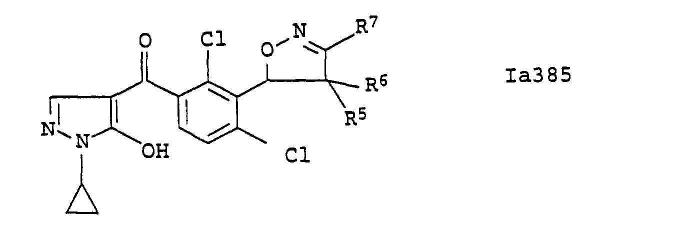

Ebenso insbesondere bevorzugt sind die Verbindungen Ia386., besonders die Verbindungen Ia386.1 bis Ia386.64, die sich von den Verbindungen Ia1.1 - Ial.64 dadurch unterscheiden, daß R² für Chlor, R¹³ für Cyclopropyl und R¹⁴ für Methyl stehen.

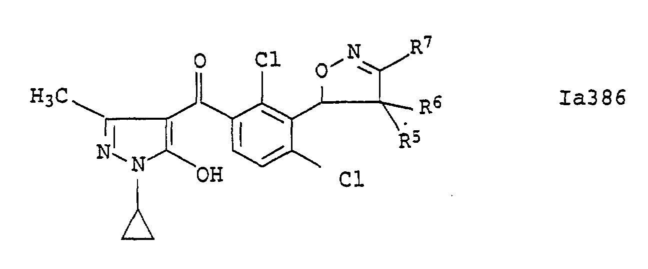

Ebenso insbesondere bevorzugt sind die Verbindungen Ia387., besonders die Verbindungen Ia387.1 bis Ia387.64, die sich von den Verbindungen Ia1.1 - Ial.64 dadurch unterscheiden, daß R¹ für Methyl, R² für Chlor und R¹³ für Cyclopropyl stehen.

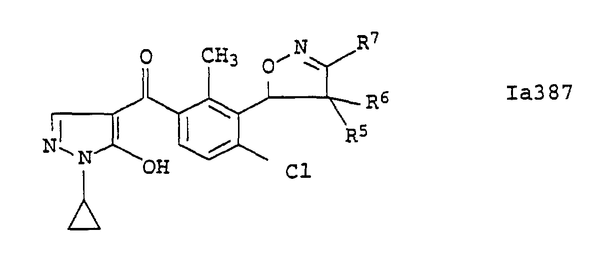

Ebenso insbesondere bevorzugt sind die Verbindungen Ia388., besonders die Verbindungen Ia388.1 bis Ia388.64, die sich von den Verbindungen Ia1.1 - Ia1.64 dadurch unterscheiden, daß R¹ für Methyl, R² für Chlor, R¹³ für Cyclopropyl und R¹⁴ für Methyl stehen.

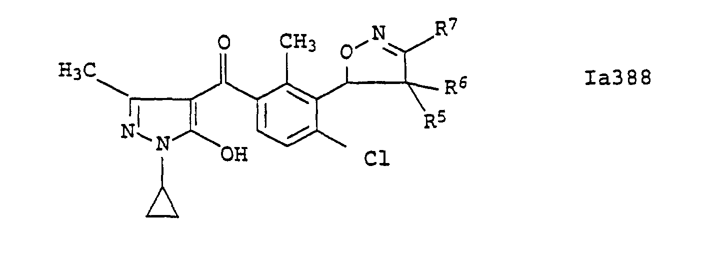

Ebenso insbesondere bevorzugt sind die Verbindungen Ia389., besonders die Verbindungen Ia389.1 bis Ia389.64, die sich von den Verbindungen Ia1.1 - Ia1.64 dadurch unterscheiden, daß R¹ für Trifluormethyl, R² für Chlor, R¹³ für Cyclopropyl stehen.

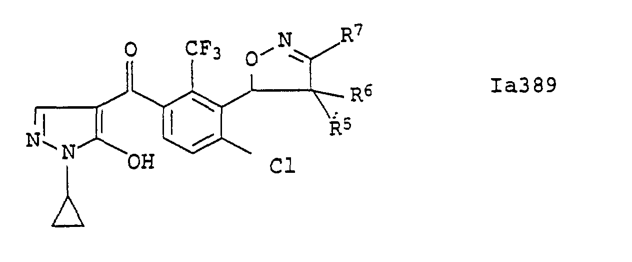

Ebenso insbesondere bevorzugt sind die Verbindungen Ia390., besonders die Verbindungen Ia390.1 bis Ia390.64, die sich von den Verbindungen Ia1.1 - Ia1.64 dadurch unterscheiden, daß R¹ für Trifluormethyl, R² für Chlor, R¹³ für Cyclopropyl und R¹⁴ für Methyl stehen.

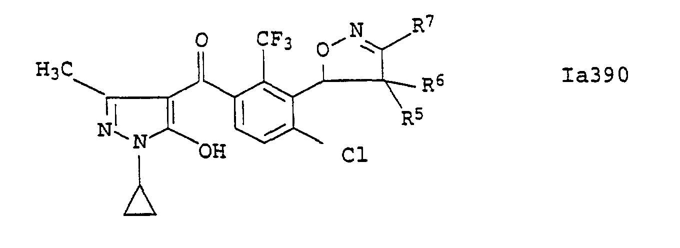

Ebenso insbesondere bevorzugt sind die Verbindungen Ia391., besonders die Verbindungen Ia391.1 bis Ia391.64, die sich von den Verbindungen Ia1.1 - Ia1.64 dadurch unterscheiden, daß R¹ für Methoxy, R² für Chlor und R¹³ für Cyclopropyl stehen.

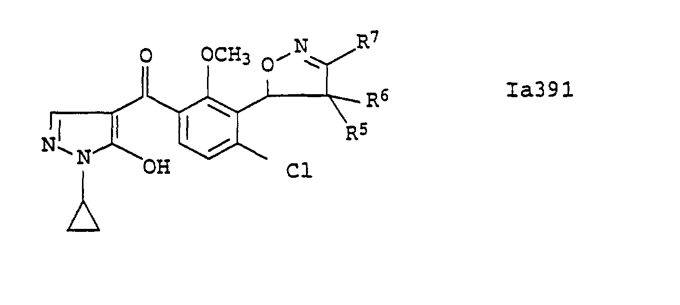

Ebenso insbesondere bevorzugt sind die Verbindungen Ia392., besonders die Verbindungen Ia392.1 bis Ia392.64, die sich von den Verbindungen Ia1.1 - Ia1.64 dadurch unterscheiden, daß R¹ für Methoxy, R² für Chlor, R¹³ für Cyclopropyl und R¹⁴ für Methyl stehen.

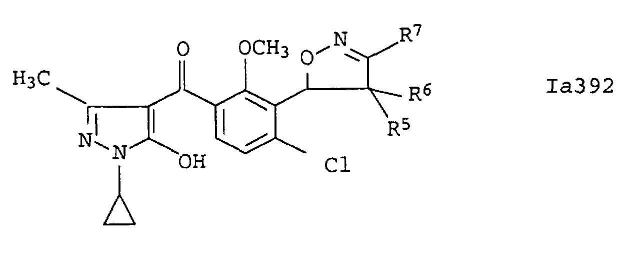

Ebenso insbesondere bevorzugt sind die Verbindungen Ia393., besonders die Verbindungen Ia393.1 bis Ia393.64, die sich von den Verbindungen Ia1.1 - Ia1.64 dadurch unterscheiden, daß R² für Trifluormethyl und R¹³ für Cyclopropyl stehen.

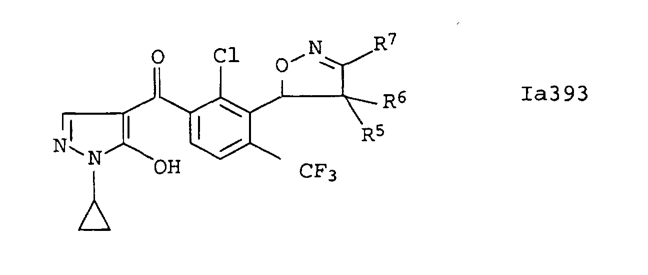

Ebenso insbesondere bevorzugt sind die Verbindungen Ia394., besonders die Verbindungen Ia394.1 bis Ia394.64, die sich von den Verbindungen Ia1.1 - Ia1.64 dadurch unterscheiden, daß R² für Trifluormethyl, R¹³ für Cyclopropyl und R¹⁴ für Methyl stehen.

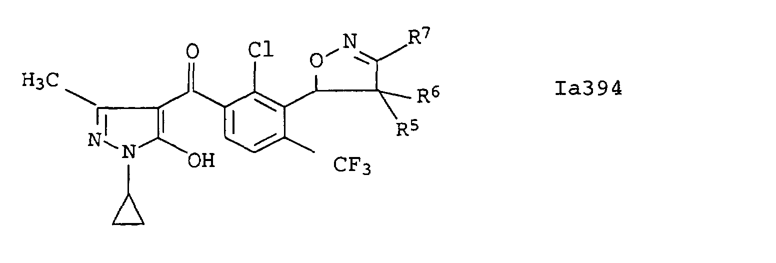

Ebenso insbesondere bevorzugt sind die Verbindungen Ia395., besonders die Verbindungen Ia395.1 bis Ia395.64, die sich von den Verbindungen Ia1.1 - Ia1.64 dadurch unterscheiden, daß R¹ Methyl, R² für Trifluormethyl und R¹³ für Cyclopropyl stehen.

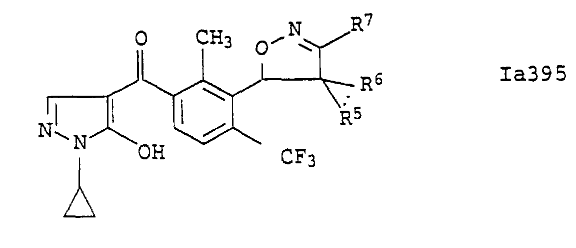

Ebenso insbesondere bevorzugt sind die Verbindungen Ia396., besonders die Verbindungen Ia396.1 bis Ia396.64, die sich von den Verbindungen Ia1.1 - Ia1.64 dadurch unterscheiden, daß R¹ für Methyl, R² für Trifluormethyl, R¹³ für Cyclopropyl und R¹⁴ für Methyl stehen.

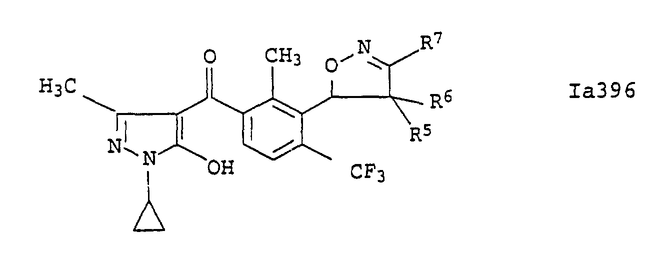

Ebenso insbesondere bevorzugt sind die Verbindungen Ia397., besonders die Verbindungen Ia397.1 bis Ia397.64, die sich von den Verbindungen Ia1.1 - Ia1.64 dadurch unterscheiden, daß R¹ und R² für Trifluormethyl und R¹³ für Cyclopropyl stehen.

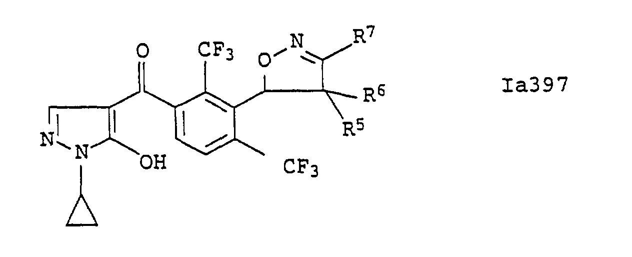

Ebenso insbesondere bevorzugt sind die Verbindungen Ia398., besonders die Verbindungen Ia398.1 bis Ia398.64, die sich von den Verbindungen Ia1.1 - Ia1.64 dadurch unterscheiden, daß R¹ und R² für Trifluormethyl, R¹³ für Cyclopropyl und R¹⁴ für Methyl stehen.

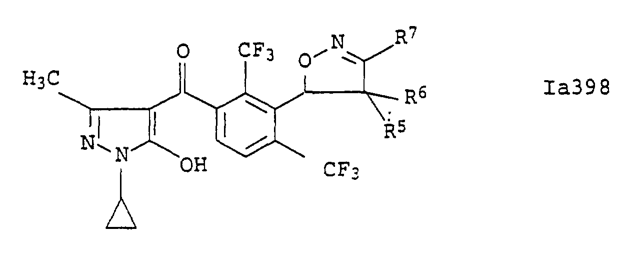

Ebenso insbesondere bevorzugt sind die Verbindungen Ia399., besonders die Verbindungen Ia399.1 bis Ia399.64, die sich von den Verbindungen Ia1.1 - Ia1.64 dadurch unterscheiden, daß R¹ für Methoxy, R² für Trifluormethyl und R¹³ für Cyclopropyl stehen.

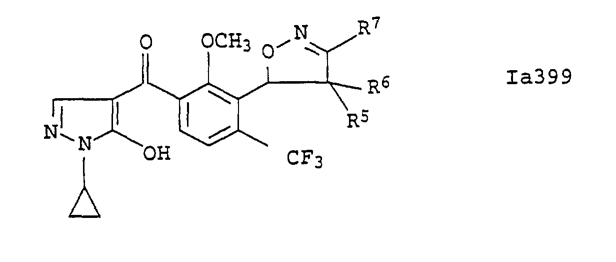

Ebenso insbesondere bevorzugt sind die Verbindungen Ia400., besonders die Verbindungen Ia400.1 bis Ia400.64, die sich von den Verbindungen Ia1.1 - Ia1.64 dadurch unterscheiden, daß R¹ für Methoxy, R² für Trifluormethyl, R¹³ für Cyclopropyl und R¹⁴ für Methyl stehen.

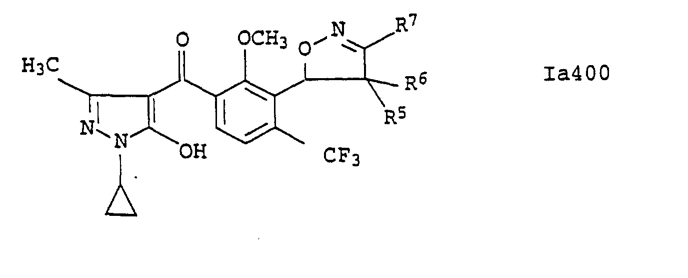

Ebenso insbesondere bevorzugt sind die Verbindungen Ia401., besonders die Verbindungen Ia401.1 bis Ia401.64, die sich von den Verbindungen Ia1.1 - Ia1.64 dadurch unterscheiden, daß R³ für Chlor und R¹³ für Cyclopropyl stehen.

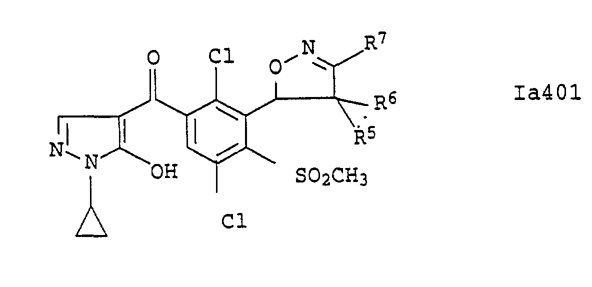

Ebenso insbesondere bevorzugt sind die Verbindungen Ia402., besonders die Verbindungen Ia402.1 bis Ia402.64, die sich von den Verbindungen Ia1.1 - Ia1.64 dadurch unterscheiden, daß R³ für Chlor, R¹³ für Cyclopropyl und R¹⁴ für Methyl stehen.

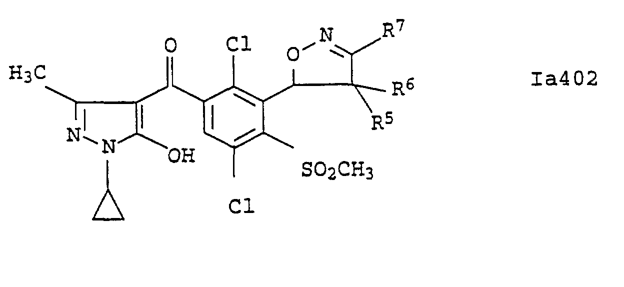

Ebenso insbesondere bevorzugt sind die Verbindungen Ia403., besonders die Verbindungen Ia403.1 bis Ia403.64, die sich von den Verbindungen Ia1.1 - Ia1.64 dadurch unterscheiden, daß R² für Ethylsulfonyl, R³ für Chlor und R¹³ für Cyclopropyl stehen.

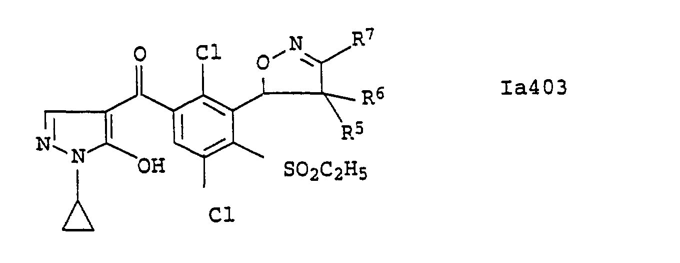

Ebenso insbesondere bevorzugt sind die Verbindungen Ia404., besonders die Verbindungen Ia404.1 bis Ia404.64, die sich von den Verbindungen Ia1.1 - Ia1.64 dadurch unterscheiden, daß R² für Ethylsulfonyl, R³ für Chlor, R¹³ für Cyclopropyl und R¹⁴ für Methyl stehen.

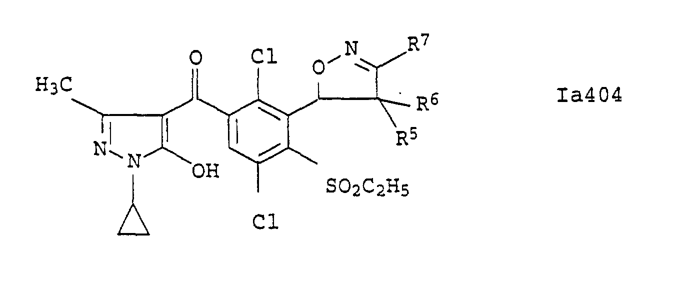

Ebenso insbesondere bevorzugt sind die Verbindungen Ia405., besonders die Verbindungen Ia405.1 bis Ia405.64, die sich von den Verbindungen Ia1.1 - Ia1.64 dadurch unterscheiden, daß R¹ und R³ für Methyl, R¹³ für Cyclopropyl stehen.

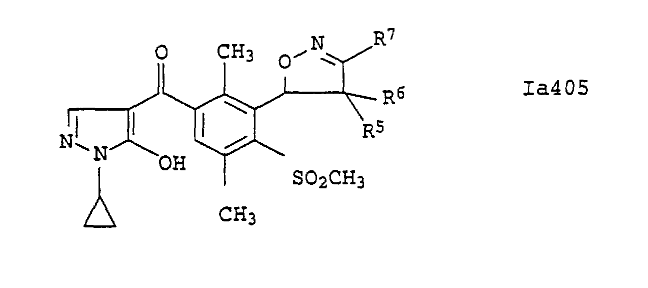

Ebenso insbesondere bevorzugt sind die Verbindungen Ia406., besonders die Verbindungen Ia406.1 bis Ia406.64, die sich von den Verbindungen Ia1.1 - Ia1.64 dadurch unterscheiden, daß R¹, R³ und R¹⁴ für Methyl und R¹³ für Cyclopropyl stehen.

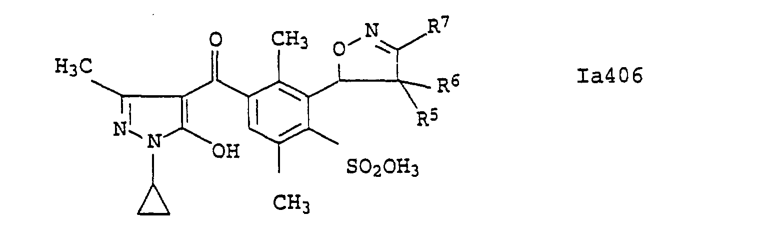

Ebenso insbesondere bevorzugt sind die Verbindungen Ia407., besonders die Verbindungen Ia407.1 bis Ia407.64, die sich von den Verbindungen Ia1.1 - Ia1.64 dadurch unterscheiden, daß R¹ und R³ für Methyl, R² für Ethylsulfonyl und R¹³ für Cyclopropyl stehen.

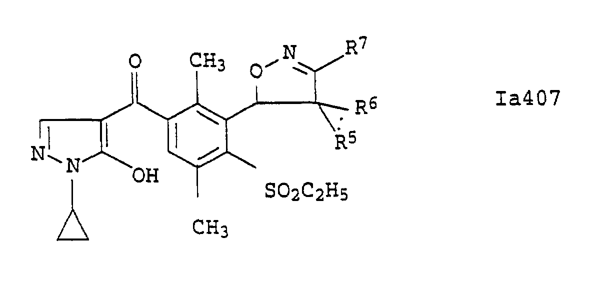

Ebenso insbesondere bevorzugt sind die Verbindungen Ia408., besonders die Verbindungen Ia408.1 bis Ia408.64, die sich von den Verbindungen Ia1.1 - Ia1.64 dadurch unterscheiden, daß R¹, R³ und R¹⁴ für Methyl, R² für Ethylsulfonyl und R¹³ für Cyclopropyl stehen.

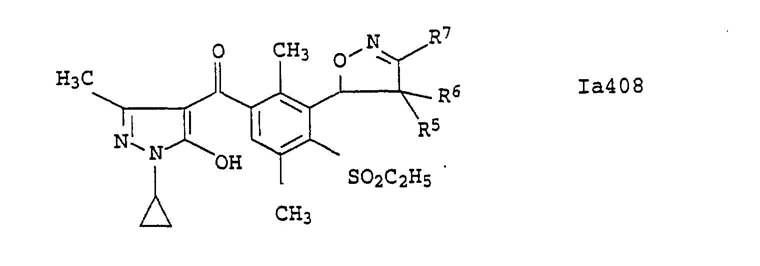

Ebenso insbesondere bevorzugt sind die Verbindungen Ia409., besonders die Verbindungen Ia409.1 bis Ia409.64, die sich von den Verbindungen Ia1.1 - Ia1.64 dadurch unterscheiden, daß R¹ und R³ für Methyl, R² für Chlor und R¹³ für Cyclopropyl stehen.

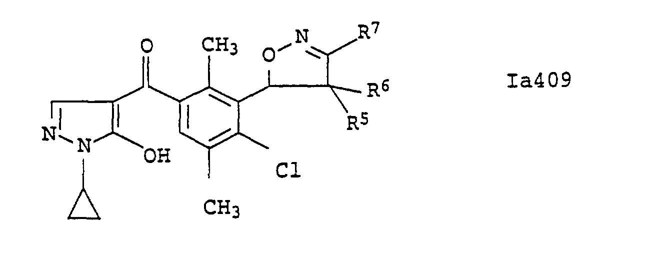

Ebenso insbesondere bevorzugt sind die Verbindungen Ia410., besonders die Verbindungen Ia410.1 bis Ia410.64, die sich von den Verbindungen Ia1.1 - Ia1.64 dadurch unterscheiden, daß R¹, R³ und R¹⁴ für Methyl, R² für Chlor und R¹³ für Cyclopropyl stehen.

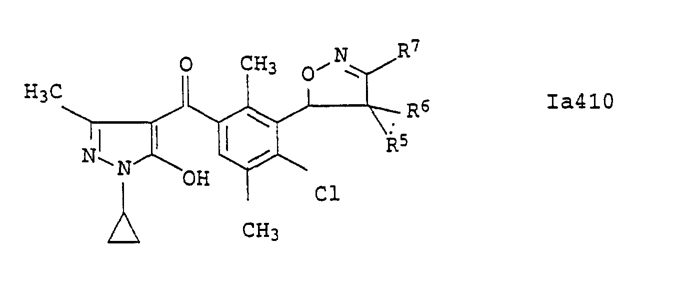

Ebenso insbesondere bevorzugt sind die Verbindungen Ia411., besonders die Verbindungen Ia411.1 bis Ia411.64, die sich von den Verbindungen Ia1.1 - Ia1.64 dadurch unterscheiden, daß für R¹² Methylsulfonyl und R¹³ für Cyclopropyl stehen.

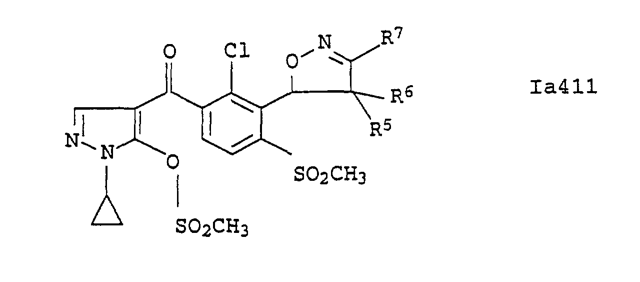

Ebenso insbesondere bevorzugt sind die Verbindungen Ia412., besonders die Verbindungen Ia412.1 bis Ia412.64, die sich von den Verbindungen Ia1.1 - Ia1.64 dadurch unterscheiden, daß für R¹² Methylsulfonyl, R¹³ für Cyclopropyl und R¹⁴ für Methyl stehen.

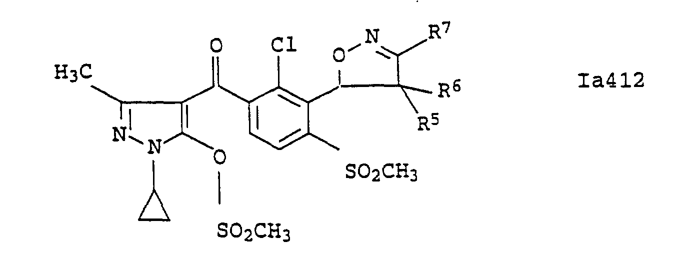

Ebenso insbesondere bevorzugt sind die Verbindungen Ia413., besonders die Verbindungen Ia413.1 bis Ia413.64, die sich von den Verbindungen Ia1.1 - Ia1.64 dadurch unterscheiden, daß R¹ für Methyl, R¹² für Methylsulfonyl und R¹³ für Cyclopropyl stehen.

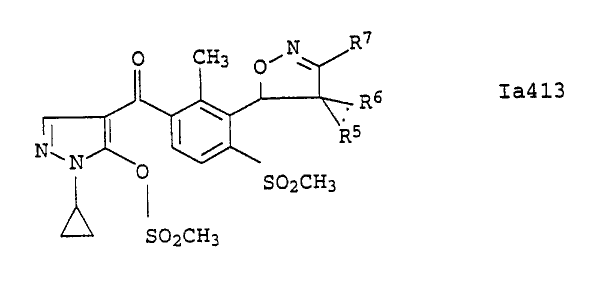

Ebenso insbesondere bevorzugt sind die Verbindungen Ia414., besonders die Verbindungen Ia414.1 bis Ia414.64, die sich von den Verbindungen Ia1.1 - Ia1.64 dadurch unterscheiden, daß R¹ und R¹⁴ für Methyl, R¹² für Methylsulfonyl und R¹³ für Cyclopropyl stehen.

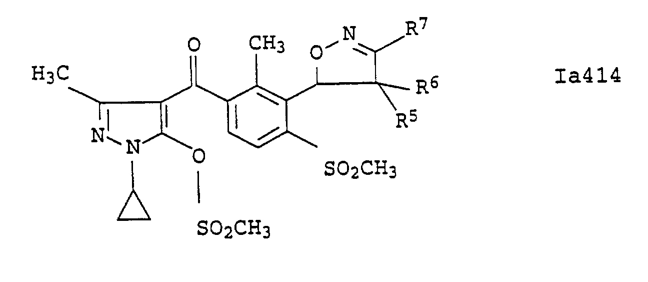

Ebenso insbesondere bevorzugt sind die Verbindungen Ia414., besonders die Verbindungen Ia414.1 bis Ia414.64, die sich von den Verbindungen Ia1.1 - Ia1.64 dadurch unterscheiden, daß R² für Ethylsulfonyl, R¹² für Methylsulfonyl und R¹³ für Cyclopropyl stehen.

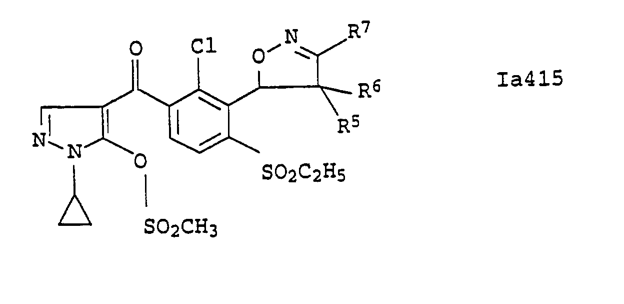

Ebenso insbesondere bevorzugt sind die Verbindungen Ia416., besonders die Verbindungen Ia416.1 bis Ia416.64, die sich von den Verbindungen Ia1.1 - Ia1.64 dadurch unterscheiden, daß R² für Ethylsulfonyl, R¹² für Methylsulfonyl, R¹³ für Cyclopropyl und R¹⁴ für Methyl stehen.

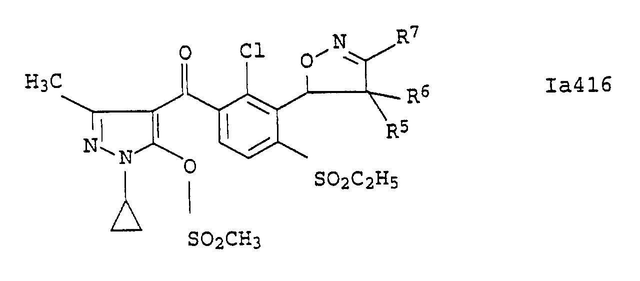

Ebenso insbesondere bevorzugt sind die Verbindungen Ia417., besonders die Verbindungen Ia417.1 bis Ia417.64, die sich von den Verbindungen Ia1.1 - Ia1.64 dadurch unterscheiden, daß R¹ für Methyl, R² für Ethylsulfonyl, R¹² für Methylsulfonyl und R¹³ für Cyclopropyl stehen.

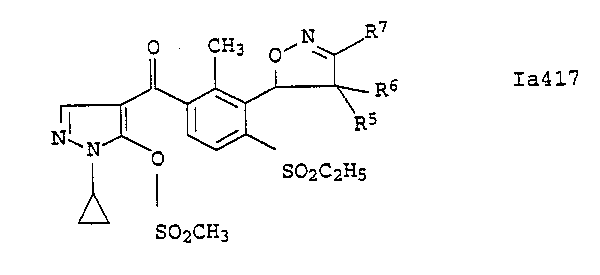

Ebenso insbesondere bevorzugt sind die Verbindungen Ia418., besonders die Verbindungen Ia418.1 bis Ia418.64, die sich von den Verbindungen Ia1.1 - Ia1.64 dadurch unterscheiden, daß R¹ und R¹⁴ für Methyl, R² für Ethylsulfonyl, R¹² für Methylsulfonyl und R¹³ für Cyclopropyl stehen.

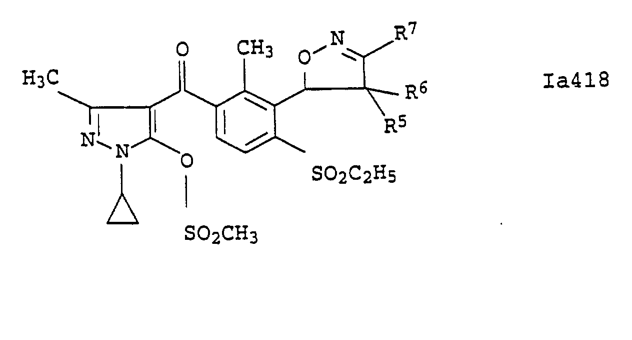

Ebenso insbesondere bevorzugt sind die Verbindungen Ia419., besonders die Verbindungen Ia419.1 bis Ia419.64, die sich von den Verbindungen Ia1.1 - Ia1.64 dadurch unterscheiden, daß R² für Chlor, R¹² für Methylsulfonyl und R¹³ für Cyclopropyl stehen.

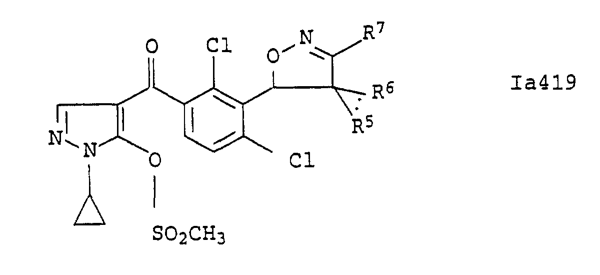

Ebenso insbesondere bevorzugt sind die Verbindungen Ia420., besonders die Verbindungen Ia420.1 bis Ia420.64, die sich von den Verbindungen Ia1.1 - Ia1.64 dadurch unterscheiden, daß R² für Chlor, R¹² für Methylsulfonyl, R¹³ für Cyclopropyl und R¹⁴ für Methyl stehen.

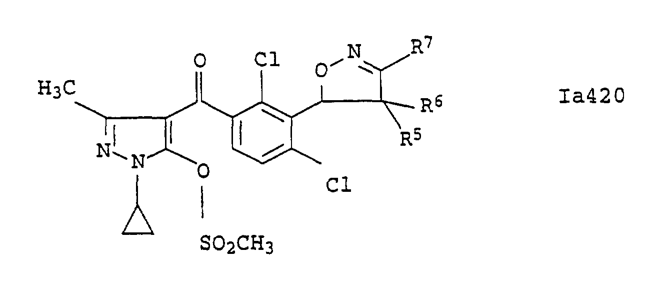

Ebenso insbesondere bevorzugt sind die Verbindungen Ia421., besonders die Verbindungen Ia421.1 bis Ia421.64, die sich von den Verbindungen Ia1.1 - Ial.64 dadurch unterscheiden, daß R¹ für Methyl, R² für Chlor, R¹² für Methylsulfonyl und R¹³ für Cyclopropyl stehen.

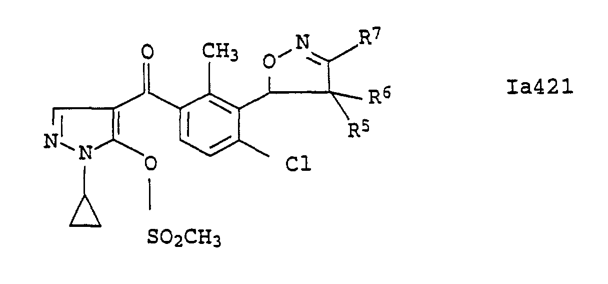

Ebenso insbesondere bevorzugt sind die Verbindungen Ia422., besonders die Verbindungen Ia422.1 bis Ia422.64, die sich von den Verbindungen Ia1.1 - Ia1.64 dadurch unterscheiden, daß R¹ und R¹⁴ für Methyl, R² für Chlor, R¹² für Methylsulfonyl und R¹³ für Cyclopropyl stehen.

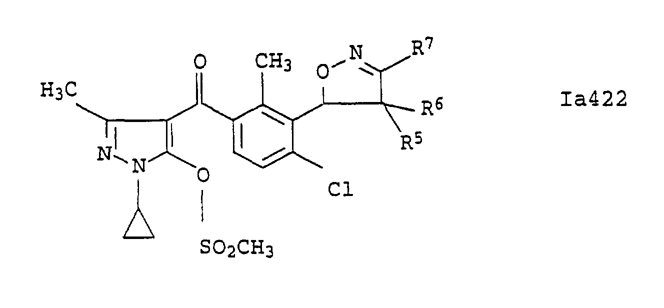

Ebenso insbesondere bevorzugt sind die Verbindungen Ia423., besonders die Verbindungen Ia423.1 bis Ia423.64, die sich von den Verbindungen Ia1.1 - Ial.64 dadurch unterscheiden, daß R¹² für 4-Methylphenylsulfonyl und R¹³ für Cyclopropyl stehen.

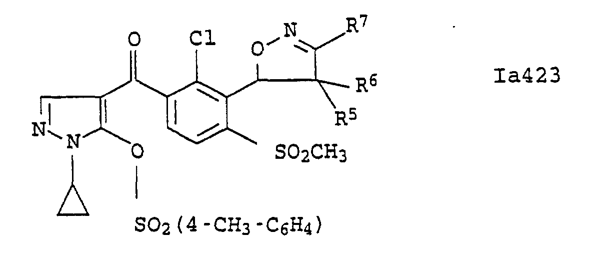

Ebenso insbesondere bevorzugt sind die Verbindungen Ia424., besonders die Verbindungen Ia424.1 bis Ia424.64, die sich von den Verbindungen Ia1.1 - Ia1.64 dadurch unterscheiden, daß R¹² für 4-Methylphenylsulfonyl, R¹³ für Cyclopropyl und R¹⁴ für Methyl stehen.

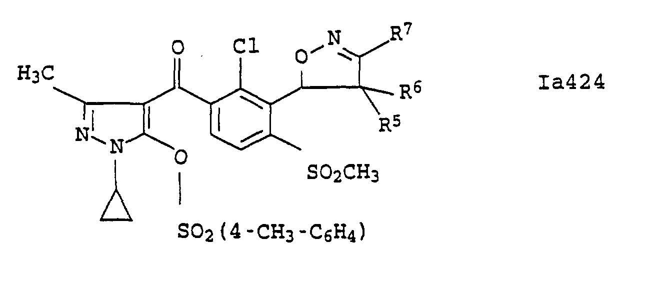

Ebenso insbesondere bevorzugt sind die Verbindungen Ia425., besonders die Verbindungen Ia425.1 bis Ia425.64, die sich von den Verbindungen Ia1.1 - Ia1.64 dadurch unterscheiden, daß R¹ für Methyl, R¹² für 4-Methylphenylsulfonyl und R¹³ für Cyclopropyl stehen.

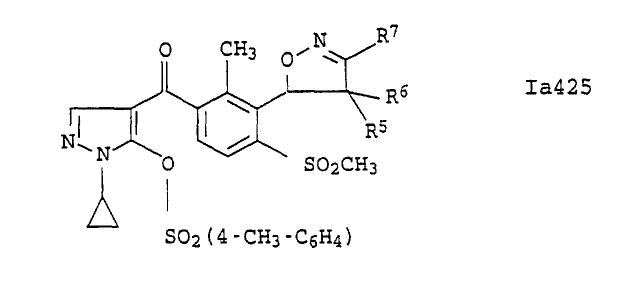

Ebenso insbesondere bevorzugt sind die Verbindungen Ia426., besonders die Verbindungen Ia426.1 bis Ia426.64, die sich von den Verbindungen Ia1.1 - Ial.64 dadurch unterscheiden, daß R¹ und R¹⁴ für Methyl, R¹² für 4-Methylphenylsulfonyl und R¹³ für Cyclopropyl stehen.

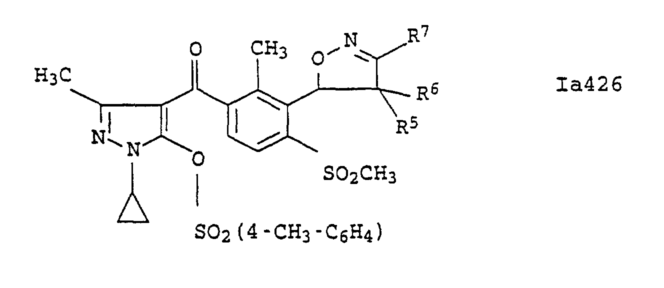

Ebenso insbesondere bevorzugt sind die Verbindungen Ia427., besonders die Verbindungen Ia427.1 bis Ia427.64, die sich von den Verbindungen Ia1.1 - Ial.64 dadurch unterscheiden, daß R² für Chlor, R¹² für 4-Methylphenylsulfonyl und R¹³ für Cyclopropyl stehen.

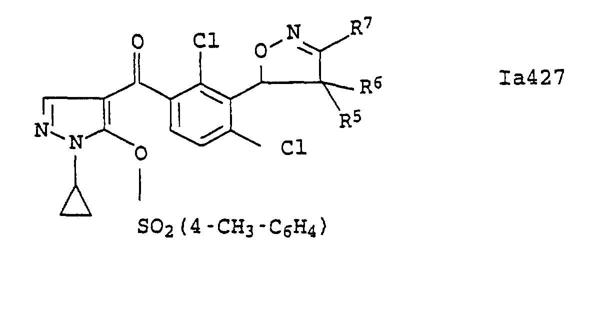

Ebenso insbesondere bevorzugt sind die Verbindungen Ia428., besonders die Verbindungen Ia428.1 bis Ia428.64, die sich von den Verbindungen Ia1.1 - Ia1.64 dadurch unterscheiden, daß R² für Chlor, R¹² für 4-Methylphenylsulfonyl, R¹³ für Cyclopropyl und R¹⁴ für Methyl stehen.

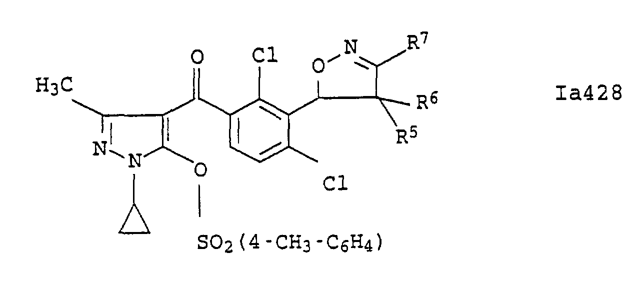

Ebenso insbesondere bevorzugt sind die Verbindungen Ia429., besonders die Verbindungen Ia429.1 bis Ia429.64, die sich von den Verbindungen Ia1.1 - Ia1.64 dadurch unterscheiden, daß R¹ für Methyl, R² für Chlor, R¹² für 4-Methylphenylsulfonyl und R¹³ für Cyclopropyl stehen.

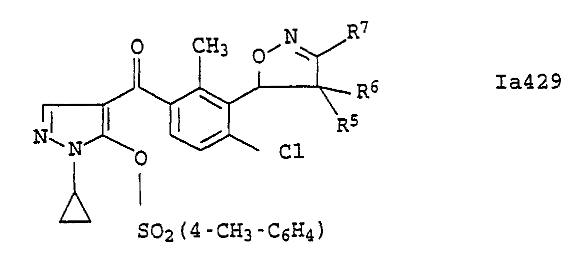

Ebenso insbesondere bevorzugt sind die Verbindungen Ia430., besonders die Verbindungen Ia430.1 bis Ia430.64, die sich von den Verbindungen Ia1.1 - Ia1.64 dadurch unterscheiden, daß R¹ und R¹⁴ für Methyl, R² für Chlor, R¹² für 4-Methylphenylsulfonyl und R¹³ für Cyclopropyl stehen.

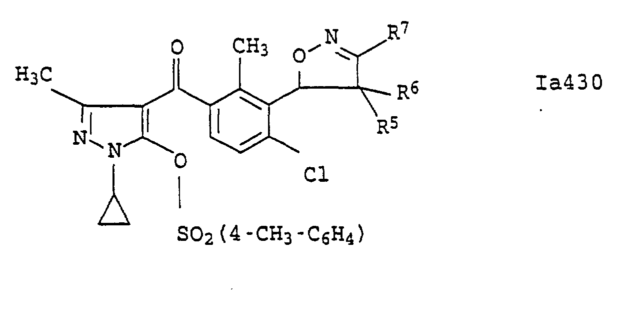

Die 3-(4,5-Dihydroisoxazol-5-yl)benzoylpyrazole der Formel I sind auf verschiedene Art und Weise erhältlich, beispielsweise nach folgendem Verfahren:

### Verfahren A:

Umsetzung von Pyrazolen der Formel II (mit Z = H) mit einer aktivierten Benzoesäure IIIα oder einer Benzoesäure IIIβ, die vorzugsweise in situ aktiviert wird, zu dem Acylierungsprodukt und anschließende Umlagerung.

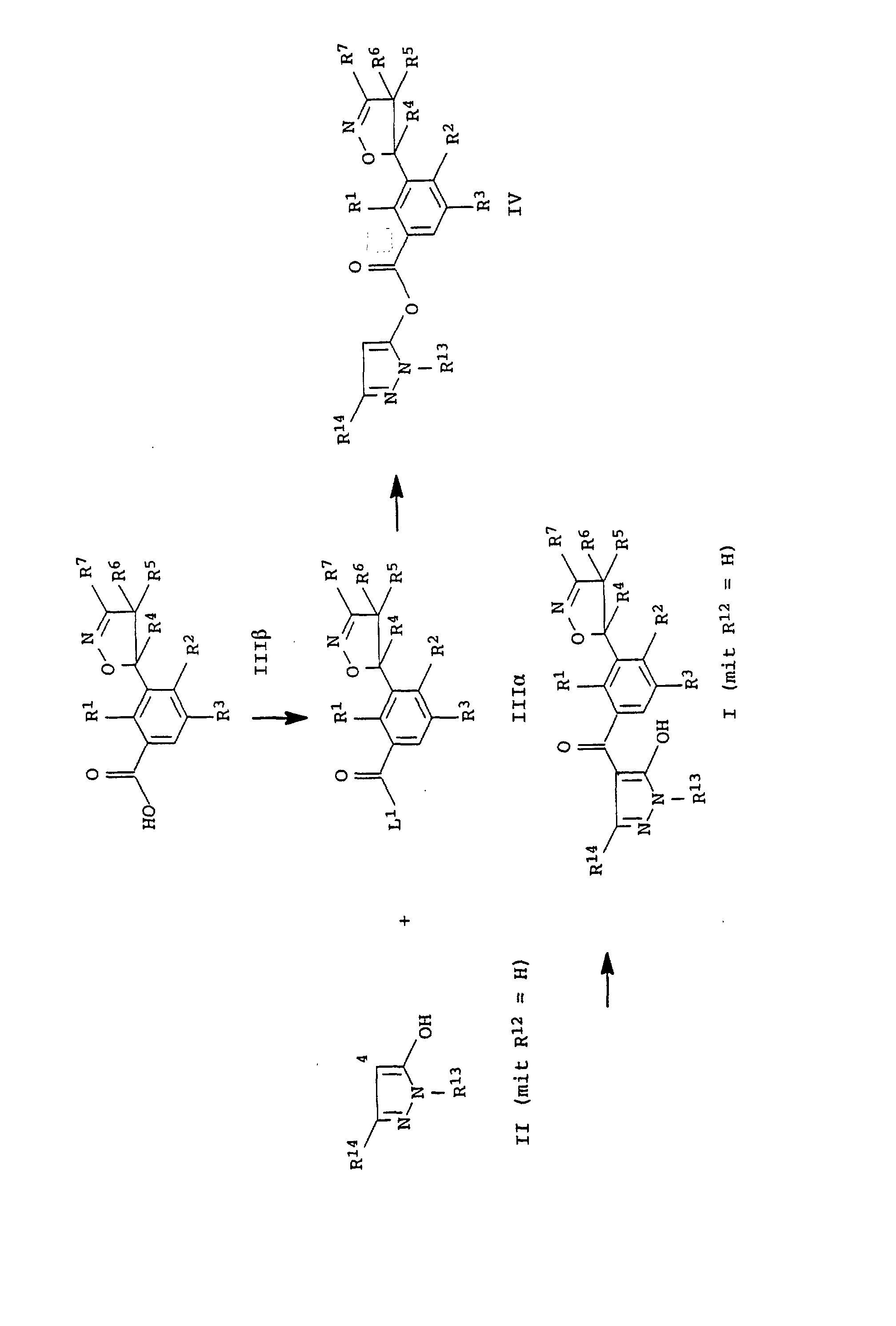

L¹ steht für eine nucleophil verdrängbare Abgangsgruppe, wie Halogen z.B Brom, Chlor, Hetaryl, z.B. Imidazolyl, Pyridyl, Carboxylat, z.B. Acetat, Trifluoracetat etc.

Die aktivierte Benzoesäure kann direkt eingesetzt werden, wie im Fall der Benzoylhalogenide oder in situ erzeugt werden, z.B. mit Dicyclohexylcarbodiimid, Triphenylphosphan/Azodicarbonsäureester, 2-Pyridindisulfid/Triphenylphosphan, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, Dimethoxyethan, polare aprotische Lösungsmittel, wie Acetonitril, Dimethylformamid, Dimethylsulfoxid oder Ester wie Essigsäureethylester oder Gemische hiervon verwendet werden.

Werden Benzoylhalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester zu den Verbindungen der Formel I erfolgt zweckmäßigerweise bei Temperaturen von 20 bis 40°C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Dimethoxyethan, Essigsäureethylester, Toluol oder Gemische hiervon verwendet werden. Bevorzugte Lösungsmittel sind Acetonitril, Dioxan und Dimethoxyethan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet, vorzugsweise in doppelt äquimolaren Verhältnis in Bezug auf den Ester.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt. (Beispiele für die Darstellung von Estern von Hydroxypyrazolen und für die Umlagerung der Ester sind z.B. in EP-A 282 944 und US 4 643 757 genannt).

Es kann aber auch zweckmäßig sein, den rohen Ester in situ zu erzeugen und die Umlagerungsreaktion ohne Isolierung, Aufreinigung des Esters durchzuführen.

### Verfahren B:

Umsetzung von 3-(4,5-Dihydroisoxazol-5-yl)benzoylpyrazole der Formel I (mit R¹² = H) mit einer Verbindung der Formel V:

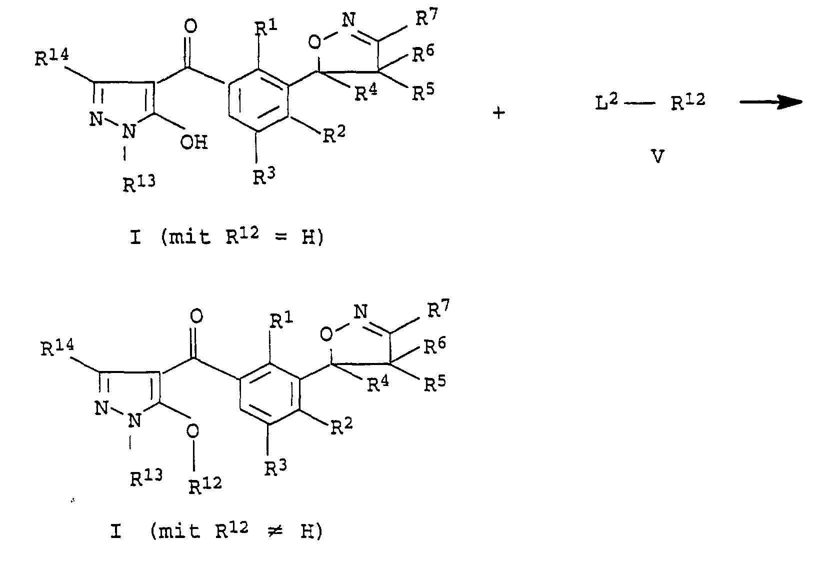

L² steht für eine nucleophil verdrängbare Abgangsgruppe, wie Halogen, z.B. Brom, Chlor, Hetaryl, z.B. Imidazolyl, Pyridyl, Sulfonat.

Die Verbindungen der Formel V können direkt eingesetzt werden, wie z.B. im Fall der Sulfonsäurehalogenide, Sulfonsäureanhydride oder in situ erzeugt werden, z.B. aktivierte Sulfonsäuren (mittels Sulfonsäure und Dicyclohexylcarbonyldiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf II, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin und Pyridin.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

Die als Ausgangsmaterialien verwendeten Pyrazole der Formel II (mit R¹² = H) sind bekannt oder können nach an sich bekannten Verfahren hergestellt werden (z.B. EP-A 240 001 und J. Prakt. Chem. 315, 383 (1973)).

3-(4,5-Dihydroisoxazol-5-yl)-Benzoesäurederivate der Formel III sind neu, 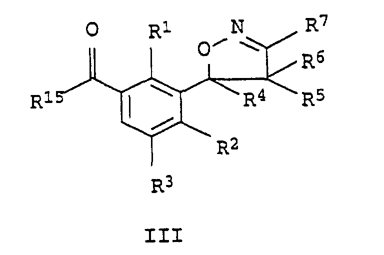 wobei die Variablen folgende Bedeutung haben.
- R¹, R²: Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
- R³: Wasserstoff, Halogen oder C₁-C₆-Alkyl;
- R⁴: Wasserstoff oder C₁-C₄-Alkyl;
- R⁵, R⁶: Wasserstoff, Halogen, Cyano, Nitro, C₁-C₄-Alkyl, C₁-C₄-Alkoxy-C₁-C₄-alkyl, Di-(C₁-C₄-alkoxy)-C₁-C₄-alkyl, Di- (C₁-C₄-alkyl)-amino-C₁-C₄-alkyl, Di-(C₁-C₄-alkyl)-amino-imino-C₁-C₄-alkyl, Hydroxy-imino-C₁-C₄-alkyl, C₁-C₆-Alkoxyimino-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl)amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy oder C1-C4-Halogenalkoxy;
oder
- R⁵ und R⁶: bilden gemeinsam eine C₂-C₆-Alkandiyl-Kette, die einbis vierfach durch C₁-C₄-Alkyl substituiert sein kann und/oder durch Sauerstoff oder einen gegebenenfalls C₁-C₄-Alkyl substituierten Stickstoff unterbrochen sein kann;
- R⁷: Halogen, Cyano, Hydroxy, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di(C₁-C₆-alkyl)amino, Di-(C₁-C₄-alkoxy)methyl, Hydroxyimino-C₁-C₄-alkyl, C₁-C₆-Alkoxyimino-C₁-C₄-alkyl oder COR⁸;
- R⁸: Wasserstoff, C₁-C₄-Alkyl, C₁-C₄-Halogenalkyl, Hydroxy, C₁-C₄-Alkoxy, C₁-C₄-Alkoxy-C₂-C₄-alkoxy, C₁-C₄-Halogenalkoxy, C₃-C₆-Alkenyloxy, C₃-C₆-Alkinyloxy oder NR⁹R¹⁰;
- R⁹: Wasserstoff oder C₁-C₄-Alkyl;
- R¹⁰: C₁-C₄-Alkyl;
- R¹⁵: Hydroxy oder abhydrolysierbarer Rest;

Beispiele für abhydrolysierbare Reste sind Alkoxy-, Phenoxy-, Alkylthio-, Phenylthio-Reste, die gegebenenfalls substituiert sein können, Halogenide, Hetarylreste, die über Stickstoff gebunden sind, Amino, Imino-Reste, die gegebenenfalls substituiert sein können etc.

Bevorzugt sind 3-(4,5-Dihydroisoxazol-5-yl)-Benzoesäurehalogenide der Formel IIIα', mit L¹' = Halogen (≙ III mit R¹⁵ = Halogen), 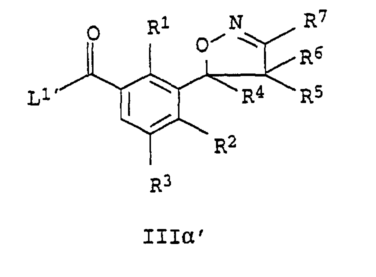 wobei die Variablen R¹ bis R⁷ die unter Formel III genannte Bedeutung haben und
- L¹': Halogen, insbesondere Chlor oder Brom bedeutet.

Ebenso bevorzugt sind 3-(4,5-Dihydroisoxazol-5-yl)-Benzoesäuren der Formel IIIβ (≙ III mit R¹⁵ = Hydroxy), 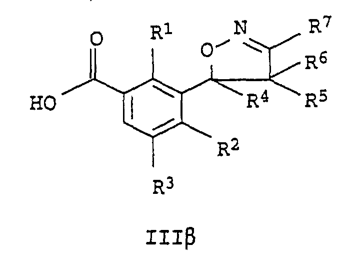 wobei die Variablen R¹ bis R⁷ die unter Formel III genannte Bedeutung haben.

Ebenso bevorzugt sind 3-(4,5-Dihydroisoxazol-5-yl)-Benzoesäureester der Formel IIIγ (≙ III mit R¹⁵ = C₁-C₆-Alkoxy), 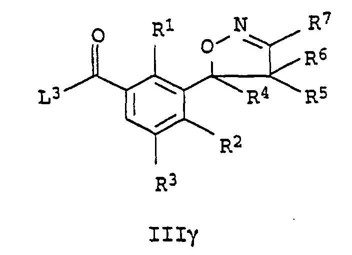 wobei die Variablen R¹ bis R⁷ die unter Formel III genannte Bedeutung haben und
- L³: C₁-C₆-Alkoxy bedeutet.

Die besonders bevorzugten Ausführungsformen der 3-(4,5-Dihydroisoxazol-5-yl)-Benzoesäurederivate der Formel III in Bezug auf die Variablen R¹ bis R⁷ entsprechen denen der 3-(4,5-Dihydroisoxazol-5-yl)benzoylpyrazole der Formel I.

Die 3-(4,5-Dihydroisoxazol-5-yl)-Benzoylhalogenide der Formel IIIα' (mit L^{1'} = Cl, Br,) können auf an sich bekannte Art und Weise durch Umsetzung der 3-(4,5-Dihydroisoxazol-5-yl)-Benzoesäuren der Formel IIIß mit Halogenierungsreagentien wie Thionylchlorid, Thionylbromid, Phosgen, Diphosgen, Triphosgen, Oxalylchlorid, Oxalylbromid hergestellt werden.

Die 3-(4,5-Dihydroisoxazol-5-yl)-Benzoesäuren der Formel IIIβ können in bekannter Weise durch saure oder basische Hydrolyse aus den entsprechenden Estern der Formel IIIγ (L³ = C₁-C₆-Alkoxy) hergestellt werden.

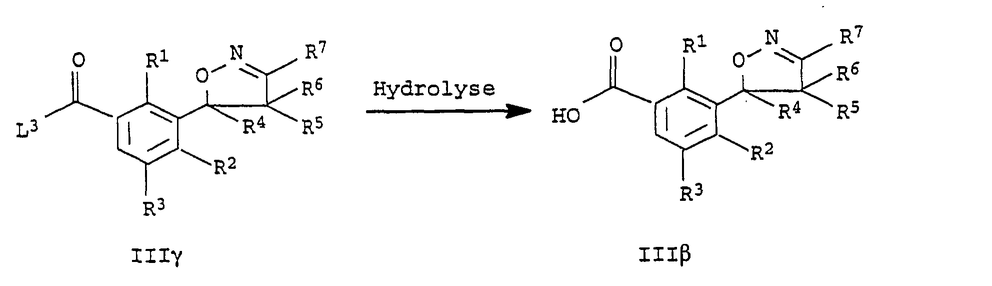

Ebenso können die 3-(4,5-Dihydroisoxazol-5-yl)-Benzoesäuren der Formel IIIβ durch Umsetzung von entsprechenden Halogen-substituierten Verbindungen der Formel VI (L⁴ = Hal), insbesondere die Iod- oder Bromverbindungen, in Gegenwart eines Palladium-, Nickel-, Cobalt- oder Rhodium-Übergangsmetallkatalysators und einer Base mit Kohlenmonoxid und Wasser unter erhöhtem Druck erhalten werden.

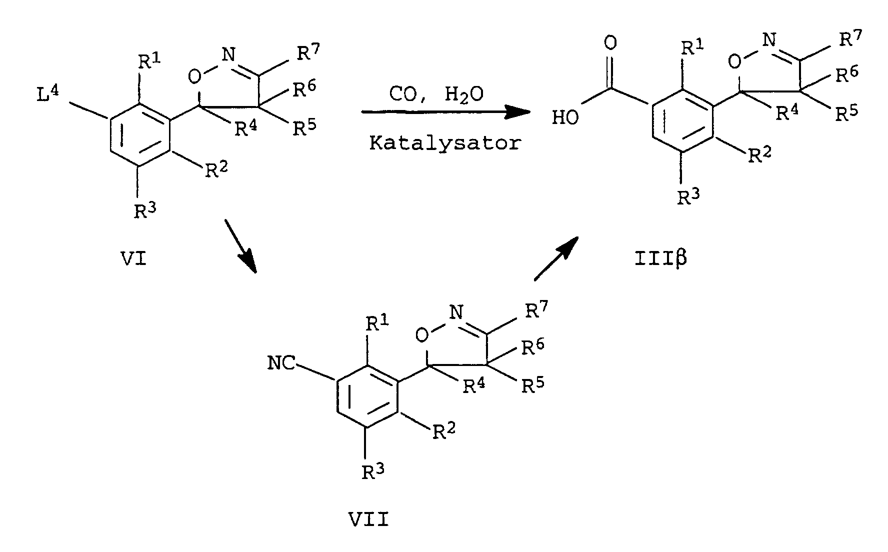

Weiterhin ist es möglich durch Rosenmund-von Braun-Reaktion Verbindungen der Formel VI in die entsprechenden Nitrile der Formel VII zu überführen (vgl. z.B. Org. Synth. Bd III, 212 (1955)) und diese durch nachfolgende Verseifung in die Verbindungen der Formel IIIβ umzuwandeln.

Die Verbindungen der Formel VI sind ebenfalls neu, 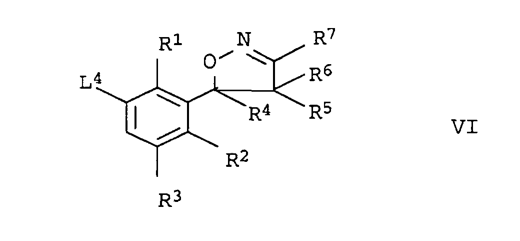 wobei die Variablen folgende Bedeutung haben:
- R¹, R²: Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
- R³: Wasserstoff, Halogen oder C₁-C₆-Alkyl;
- R⁴: Wasserstoff oder C₁-C₄-Alkyl;
- R⁵, R⁶: Wasserstoff, Halogen, Cyano, Nitro, C₁-C₄-Alkyl, C₁-C₄-Alkoxy-C₁-C₄-alkyl, Di-(C₁-C₄-alkoxy)-C₁-C₄-alkyl, Di-(C₁-C₄-alkyl)-amino-C₁-C₄-alkyl, Di-(C₁-C₄-alkyl)-amino-imino-C₁-C₄-alkyl, Hydroxy-imino-C₁-C₄-alkyl, C₁-C₆-Alkoxyimino-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl)amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy oder C1-C4-Halogenalkoxy;
oder
- R⁵ und R⁶: bilden gemeinsam eine C₂-C₆-Alkandiyl-Kette, die einbis vierfach durch C₁-C₄-Alkyl substituiert sein kann und/oder durch Sauerstoff oder einen gegebenenfalls C₁-C₄-Alkyl substituierten Stickstoff unterbrochen sein kann;
- R⁷: Halogen, Cyano, Hydroxy, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di(C₁-C₆-Alkyl)amino, Di-(C₁-C₄-alkoxy)methyl, Hydroxyimino-C₁-C₄-alkyl, C₁-C₆-Alkoxyimino-C₁-C₄-alkyl oder COR⁸;
- R⁸: Wasserstoff, C₁-C₄-Alkyl, C₁-C₄-Halogenalkyl, Hydroxy, C₁-C₄-Alkoxy, C₁-C₄-Alkoxy-C₂-C₄-alkoxy, C₁-C₄-Halogenalkoxy, C₃-C₆-Alkenyloxy, C₃-C₆-Alkinyloxy oder NR⁹R¹⁰;
- R⁹: Wasserstoff oder C₁-C₄-Alkyl;
- R¹⁰: C₁-C₄-Alkyl;
- L⁴: Halogen.

Ebenso sind die Verbindungen der Formel VII neu 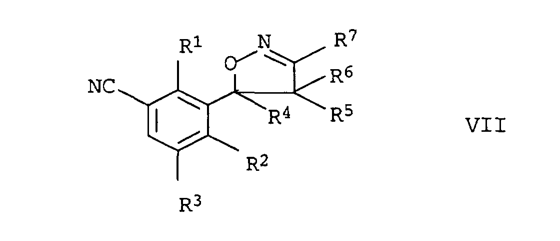 wobei die Variablen folgende Bedeutung haben:
- R¹, R²: Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
und R³ bis R⁷ die unter der Verbindung der Formel VI genannte Bedeutung haben.

Die besonderen Ausführungsformen der Verbindungen der Formel VI bzw. VII in Bezug auf die Variablen R¹ bis R⁷ entsprechen denen der 3-(4,5-Dihydroisoxazol-5-yl)benzoylpyrazole der Formel I.

Die Ester der Formel IIIγ bzw. die Halogenverbindungen der Formel VI können durch 1,3-dipolare Cycloaddition von Nitriloxiden an entsprechende Alkene der Formel VIII bzw. IX dargestellt werden.

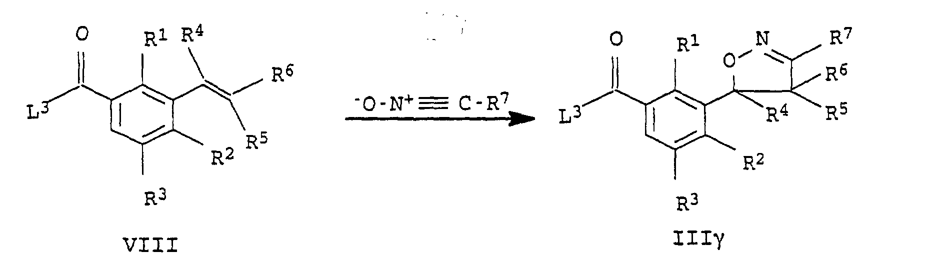

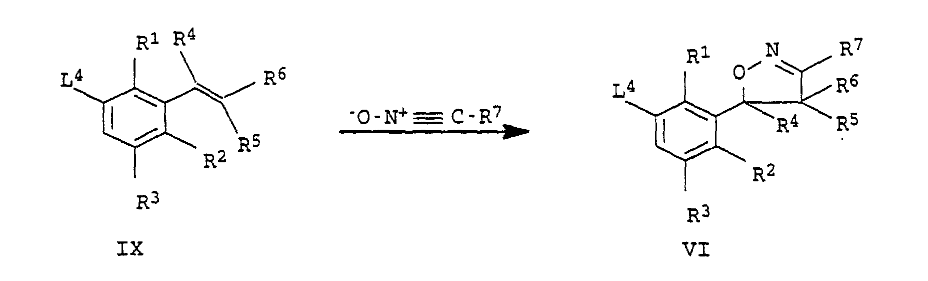

Die Nitriloxide werden in situ auf an sich bekannte Art und Weise erzeugt. Als Edukte dienen hierzu beispielsweise Aldoxime (vgl. z.B. Houben-Weyl X5, S. 858ff) oder Nitroalkane (vgl. z.B. Houben-Weyl E5/2, S. 1594ff). Die Synthese der Verbindungen der Formel VIII bzw. IX ist bekannt (vgl. z.B. WO 98/50337 bzw. WO 98/50366) oder erfolgt in Analogie zu literaturbekannten Verfahren.

### Herstellungsbeispiele:

### 4-[2-Chlor-3-(3-methyl-4,5-dihydroiaoxazol-5-yl)-4-methylsulfonyl-benzoyl]-5-hydroxy-1-methyl-1H-pyrazol (Verbindung 2.8)

Zu einer Lösung von 2,0 g (20 mmol) 5-Hydroxy-1-methyl-pyrazol und 3,3 g (24 mmol) Kaliumcarbonat in 30 ml wasserfreien Dimethoxyethan tropfte man bei 0 - 5°C eine Lösung von 6,7 g (20 mmol) 2-Chlor-3-(3-methyl-4,5-dihydroisoxazol-5-yl)-4-methylsulfonylbenzoylchlorid in 80 ml Dimethoxyethan. Nach 12 Stunden Rühren bei Raumtemperatur wurden weitere 8,2 g (60 mmol) Kaliumcarbonat zugegeben und der Ansatz 6 Stunden unter Rückfluß erhitzt. Anschließend wurde nach dem Abkühlen das Reaktionsgemisch in 1 l Wasser eingerührt und mit Methylenchlorid extrahiert. Dann wurde die wäßrige Phase mit 10 %iger Salzsäure auf einen pH von 4 gebracht und mit Methylenchlorid und pH-Nachregelung mehrmals extrahiert. Nach Trocknen der organischen Phase und entfernen des Lösungsmittels erhielt man 5,3 g (13 mmol, 67 %) der gewünschten Verbindung.
Fp: > 215°C

### 4- [2-Chlor-3-(3-methyl-4,5-dihydroisoxazol-5-yl)-4-methylsulfonyl-benzoyl]-5-hydroxy-1-cyclopropyl-1H-pyrazol (Verbindung 2.36)

Zu einer Lösung von 0,47 g (3 mmol) 5-Hydroxy-1-cyclopropylpyrazol und 0,5 g (3,6 mmol) Kaliumcarbonat in 36 ml wasserfreien Dimethoxyethan tropfte man bei 0 - 5°C eine Lösung von 1,0 g (3 mmol) 2-Chlor-3-(3-methyl-4,5-dihydroisoxazol-5-yl)-4-methylsulfonyl-benzoylchlorid in 50 ml Dimethoxyethan. Nach 12 Stunden Rühren bei Raumtemperatur werden weitere 1,0 g (7,2 mmol) Kaliumcarbonat zugegeben und der Ansatz 6 Stunden unter Rückfluß erhitzt. Anschließend wurde nach dem Abkühlen das Reaktionsgemisch in 600 ml Wasser eingerührt und mit Methylenchlorid extrahiert. Dann wurde die wäßrige Phase mit 10%iger Salzsäure auf pH 4 gestellt und mit Methylenchlorid extrahiert. Nach Trocknen der organischen Phase und Entfernen des Lösungsmittels erhielt man 0,8 g (1,8 mmol, 63 %) der gewünschten Verbindung.
Fp: > 220°C

In Tabelle 2 sind neben der voranstehenden Verbindung noch weitere Derivate der Formel I aufgeführt, die in analoger Weise hergestellt wurden oder herstellbar sind.

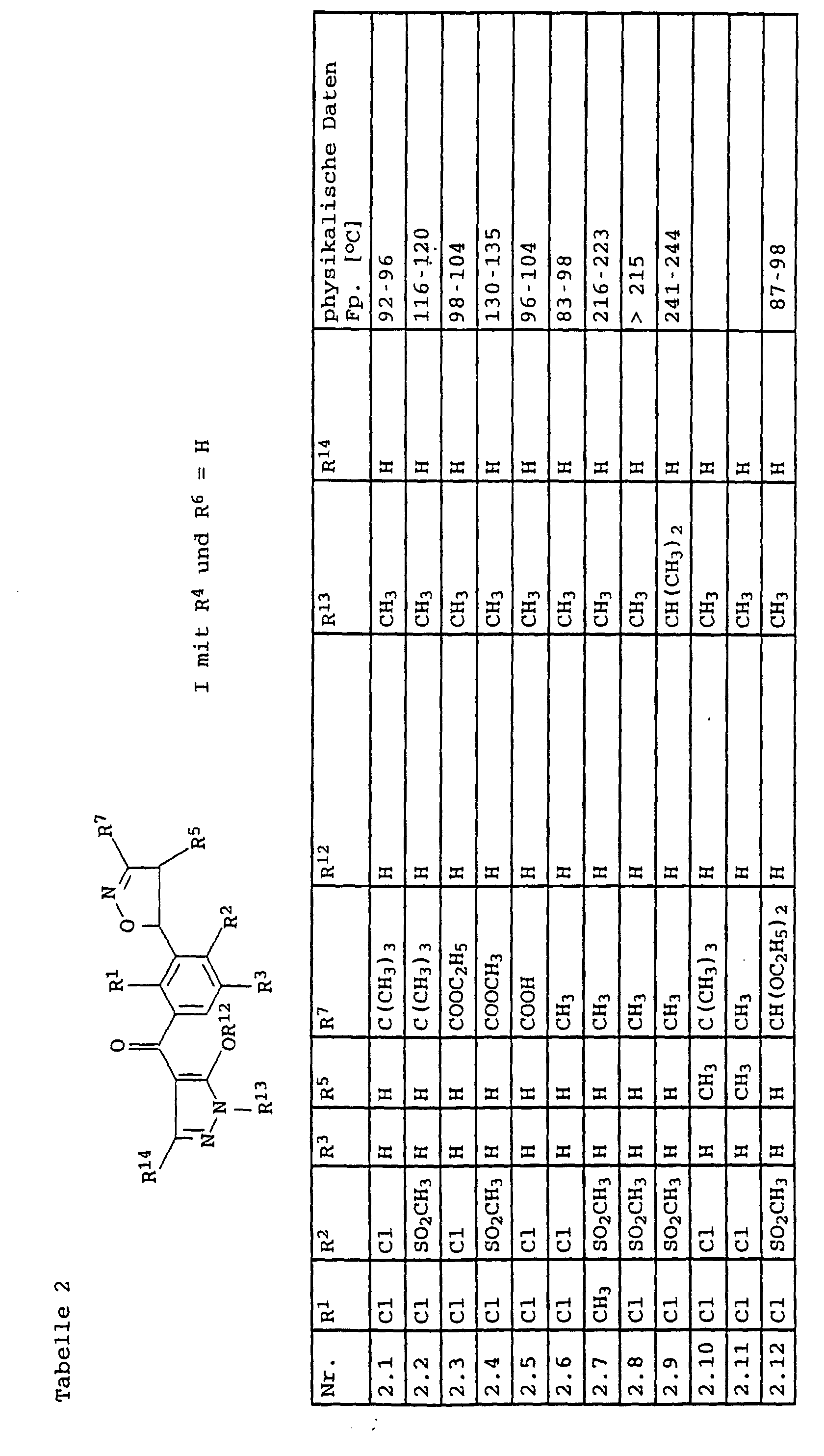

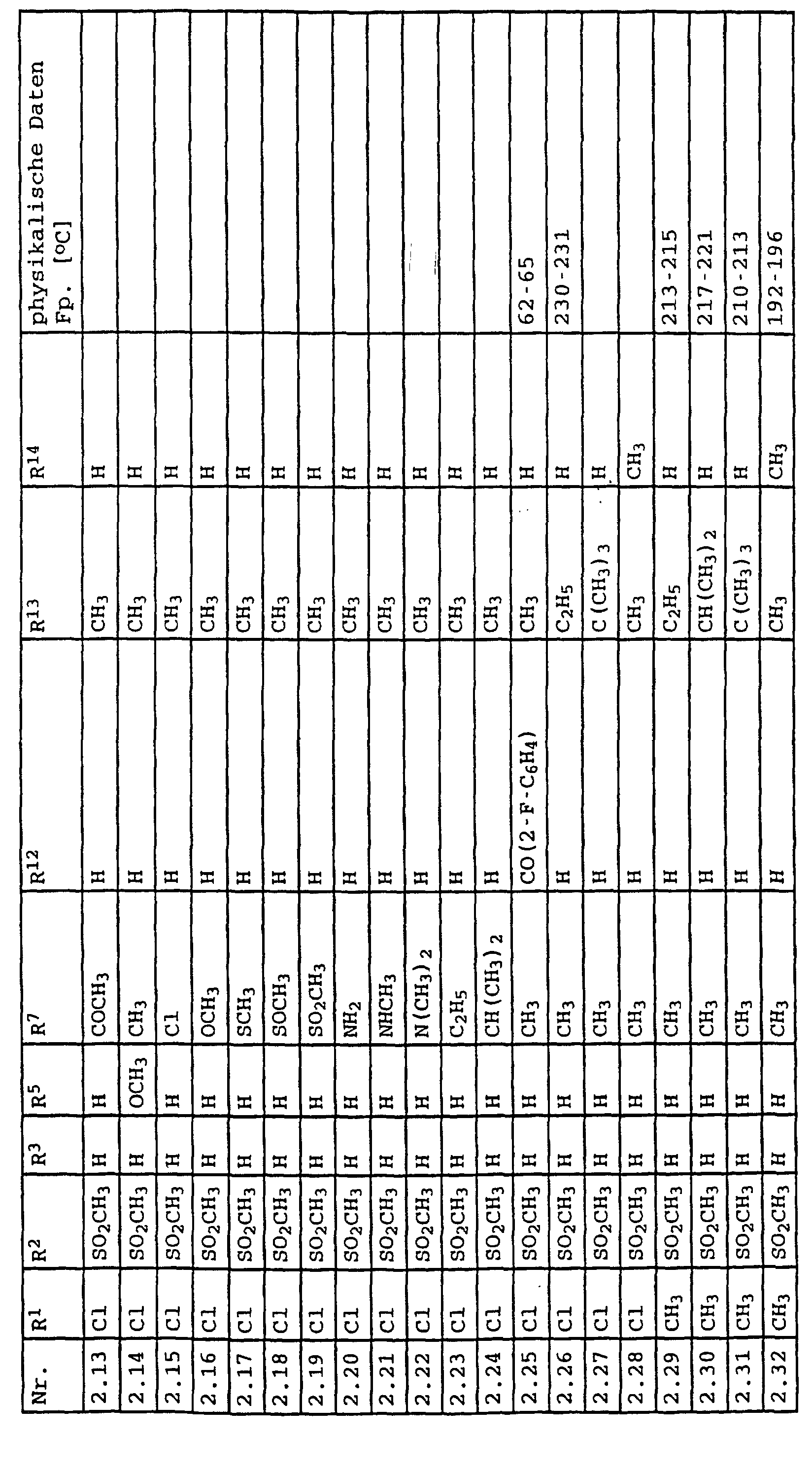

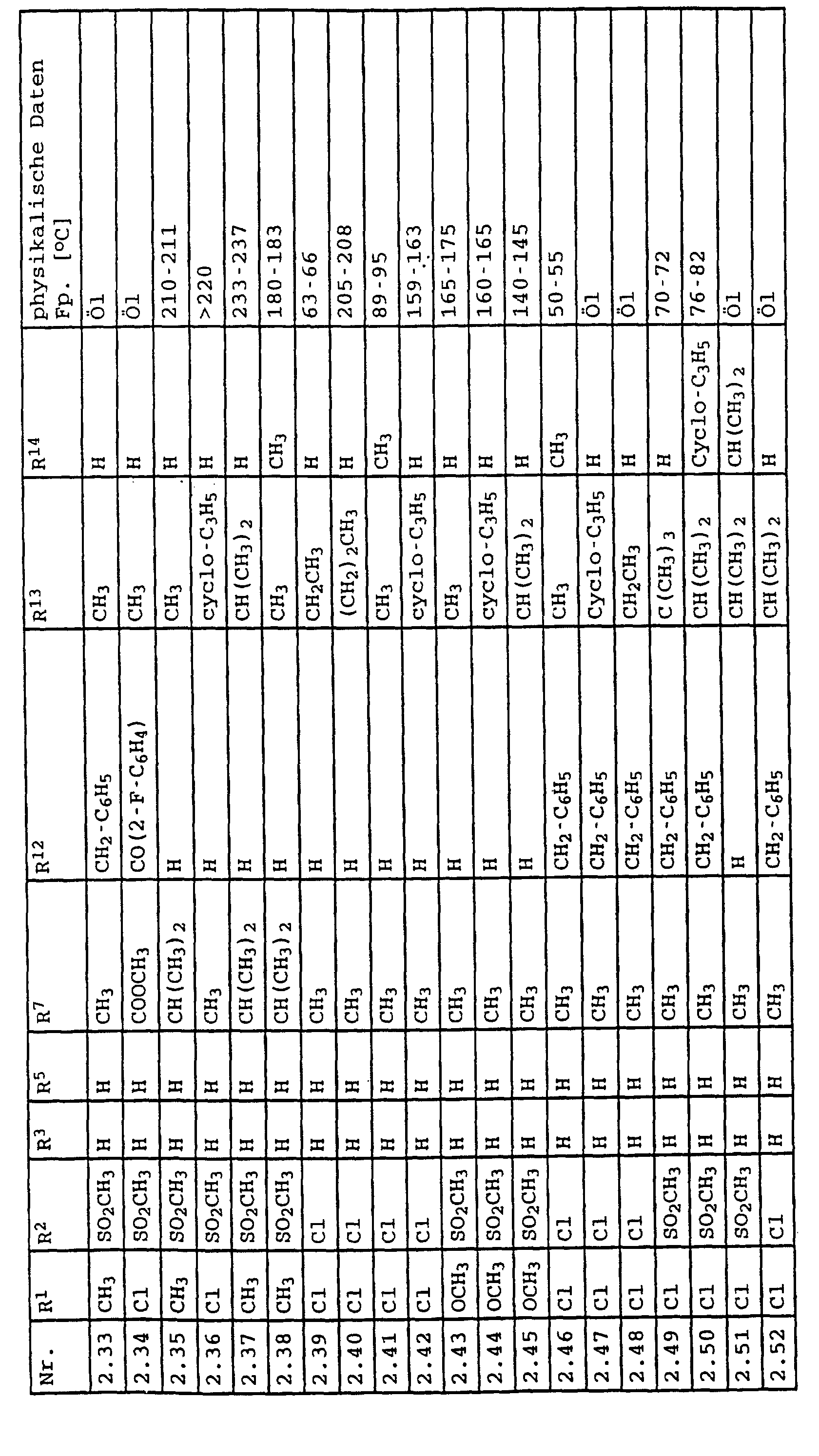

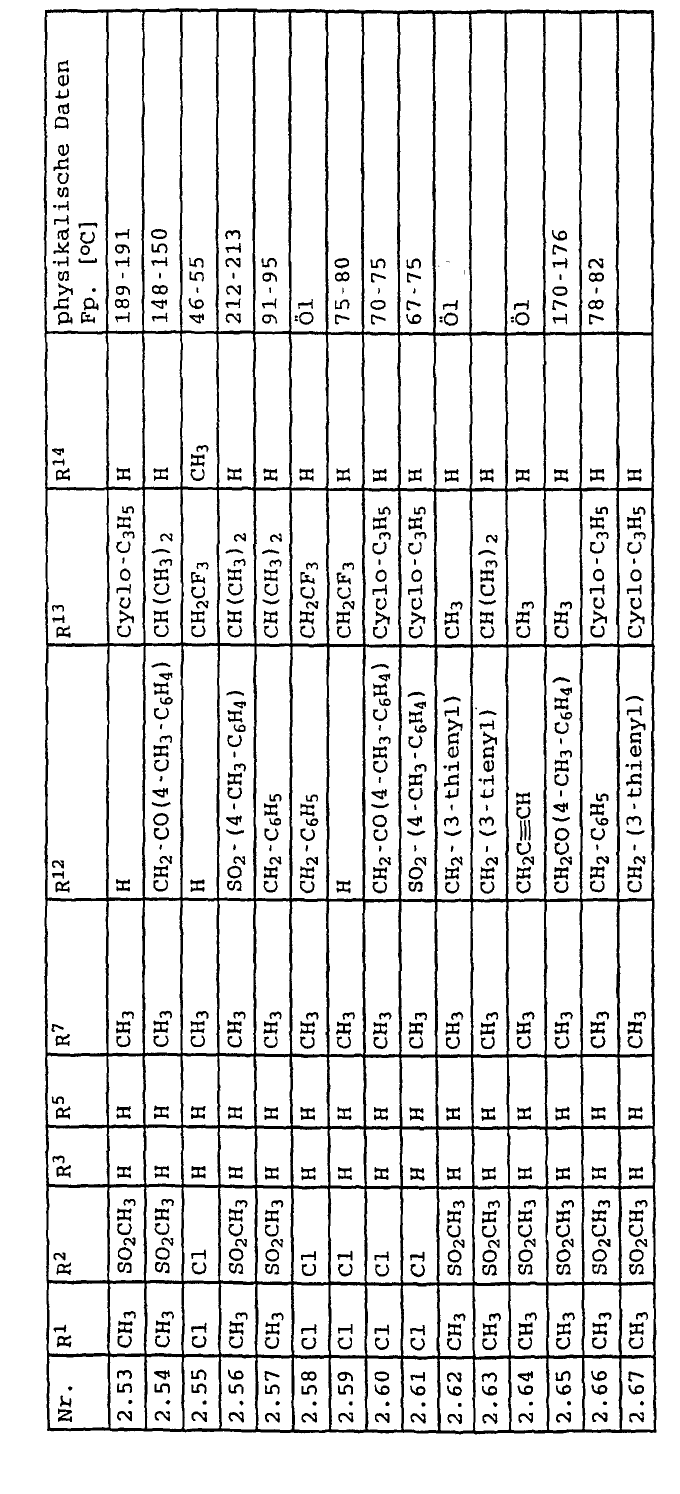

Nachfolgend sind die Synthesen einiger Ausgangsstoffe aufgeführt:

### 2,4-Dichlor-3-(3-t-butyl-4,5-dihydroisoxazol-5-yl)-benzoesäuremethylester

Zu einer Lösung von 2,6 g (10 mmol) 2,4-Dichlor-3-ethenylbenzoesäuremethylester und 1 g (10 mmol) 2,2-Dimethylpropionaldoxim in 100 ml Dichlormethan wurden bei Raumtemperatur 17 ml einer 12 %igen wäßrigen Natriumhypochloritlösung unter heftigen Rühren getropft. Nach jeweils 2 Stunden Rühren wurden zweimal weitere 0,5 g (5 mmol) des Oxims und 9 ml der Hypochloritlösung zugegeben. Es wurde weiter 12 Stunden bei Raumtemperatur gerührt und dann das Reaktionsgemisch in 350 ml Wasser eingerührt. Nach Extraktion mit Dichlormethan, Trocknen und Entfernen des Lösungsmittels wurde der Rückstand chromatographiert.
Ausbeute: 1,5 g (45 % d. Th.) gelbes Harz

### 2-Chlor-3-(3-ethoxycarbonyl-4,5-dihydroisoxazol-5-yl)-4-methylsulfonylbenzoesäuremethylester.

Zu einer Lösung von 8,5 g (31 mmol) 2-Chlor-3-ethenyl-4-methylsulfonylbezoesäuremethylester und 7,2 g (46 mmol) 2-Chlor-2-hydroxyiminoessigsäureethylester in 200 ml Dichlormethan wurden bei Raumtempertur langsam 6,4 ml (46 mmol) Triethylamin getropft. Nach jeweils 2 Stunden Rühren wurden 3 weitere Portionen von jeweils 4,8 g (31 mmol) 2-Chlor-2-hydroxyiminoessigsäureethylester und danach 4,3 ml (31 mmol) Triethylamin zugegeben. Nach weiteren 12 Stunden Rühren bei Raumtemperatur wurde das Reaktionsgemisch in 600 ml Wasser eingerührt. Nach Extraktion mit Dichlormethan, Trocknen und Entfernen des Lösungsmittels wurde der Rückstand chromatographiert.
Ausbeute: 5,7 g (47 % d. Th.) klares Harz.

### 2-Chlor-3-(3-methyl-4,5-dihydroisoxazol-5-yl)-4-methylsulfonylbenzoesäure

### a) 2-Chlor-3 (3-methyl-4,5-dihydroisoxazol-5-yl)-4-methylsulfonylbenzoesäuremethylester.

Zu einer Lösung von 40 g (145 mmol) 2-Chlor-3-ethenyl-4-methylsulfonylbenzoesäuremethylester und 50 g (220 mmol) Di-t-butyldicarbonat in 300 ml Acetonitril gab man eine Spatelspitze 4-Dimethylaminopyridin und tropfte dann langsam 50 g (640 mmol) Nitroethan zu. Nach 12 Stunden Rühren bei Raumtemperatur wurden weitere 32,8 g (145 mmol) Di-t-butyldicarbonat und 22,9 g (290 mmol) Nitroethan zugegeben. Nach weitere 12 Sunden wurde das Lösungsmittel abdestilliert und der Rückstand mit Essigsäureethylester digeriert. Nach Absaugen verblieben 32,1 g der gewünschten Verbindung. Aus der Mutterlauge erhielt man nach Entfernen des Lösungsmittel und anschließender Chromatographie weitere 6,3 g.
Ausbeute: 39,4 g (82 % d. Th.)
Fp.: 175°C

### b) 2-Chlor-3-(3-methyl-4,5-dihydroisoxazol-5-yl)-4-methylsulfonylbenzoesäure

Zu einer Lösung von 38,4 g (116 mmol) 2-Chlor-3-(3-methyl-45-dihydroisoxazol-5-yl)-4-methylsulfonylbenzoesäuremethylester in 400 ml Methanol und 400 ml Tetrahydrofuran gab man 69,4 g (174 mmol) 10 %ig Natronlauge. Man rührt 12 Stunden bei Raumtemperatur, reduzierte das Lösungsmittelvolumen auf die Hälfte und goß in 1 l Wasser. Mit 10 %ige Salzsäure wurde der pH-Wert von 1 eingestellt und der sich bildende Niederschlag abgesaugt.
Ausbeute: 35,2 g (96 % d. Th.)
Fp.: >220°C

### 2-Methyl-3-(3-methyl-4,5-dihydroisoxazol-5-yl)-4-methylsulfonylbenzoesäure.

### a) 2-Methyl-3- (3-methyl-4,5-dihydroisoxazol-5-yl)-4-methylsulfonylbrombenzol

Zu einer Lösung von 13,6 g (50 mmol) 2-Methyl-3-ethenyl-4-methylsulfonyl-brombenzol und 16,7 g (74 mmol) Di-t-butyldicarbonat in 100 ml Acetonitril gab man eine Spatelspitze 4-Dimethylaminopyridin und tropfte dann langsam 17,9 g (229 mmol) Nitroethan zu. Nach 6 Stunden Rühren bei Raumtemperatur wurden weitere 11,1 g (50 mmol) Di-t-butyldicarbonat und 7,8 g (100 mmol) Nitroethan zugegeben und 12 Stunden bei Raumtemperatur gerührt. Nach Entfernen des Lösungsmittels wurde der Rückstand chromatographiert.
Ausbeute: 6,4 g (38 % d. Th.)

### b) 2-Methyl-3-(3-methyl-4,5-dihydroisoxazol-5-yl)-4-methylsulfonylbenzoesäure

6,4 g (19 mmol) 2-Methyl-3-(3-methyl-4,5-dihydroisoxazol-5 yl)-4-methylsulfonyl-brombenzol wurden in 65 ml Toluol und 30 ml Wasser gelöst und mit 240 mg (1 mmol) Palladiumacetat, 1,1 g (4 mmol) Tricyclohexylphosphan, 810 mg (19 mmol) Lithiumchlorid und 5,4 ml (38 mmol) Triethylamin versetzt. Die entstandene Lösung wurde dann in einem Autoklaven im für 36 Stunden bei 140°C unter einen Kohlenmonoxiddruck von 20 bar gerührt. Nach Abkühlen und Entspannen wurden unlösliche Bestandteile ahfiltriert und die Phasen getrennt. Die organische Phase wurde anschließend zweimal mit Wasser das etwas Triethylamin enthielt, extrahiert. Dann wurden die vereinigten wäßrigen Phasen mit 10 % Salzsäure auf pH 1 gebracht und der entstandene Niederschlag abfiltriert.
Ausbeute: 2,5 g (44 % d. Th.)
Fp.: 199-205°C

In der nachfolgende Tabelle 3 sind neben den voranstehend beschriebenen Verbindungen weitere 3-(4,5-Dihydroisoxazol-5-yl)-Benzoesäurederivate der Formel III aufgeführt, die in analoger Weise hergestellt werden oder herstellbar sind.

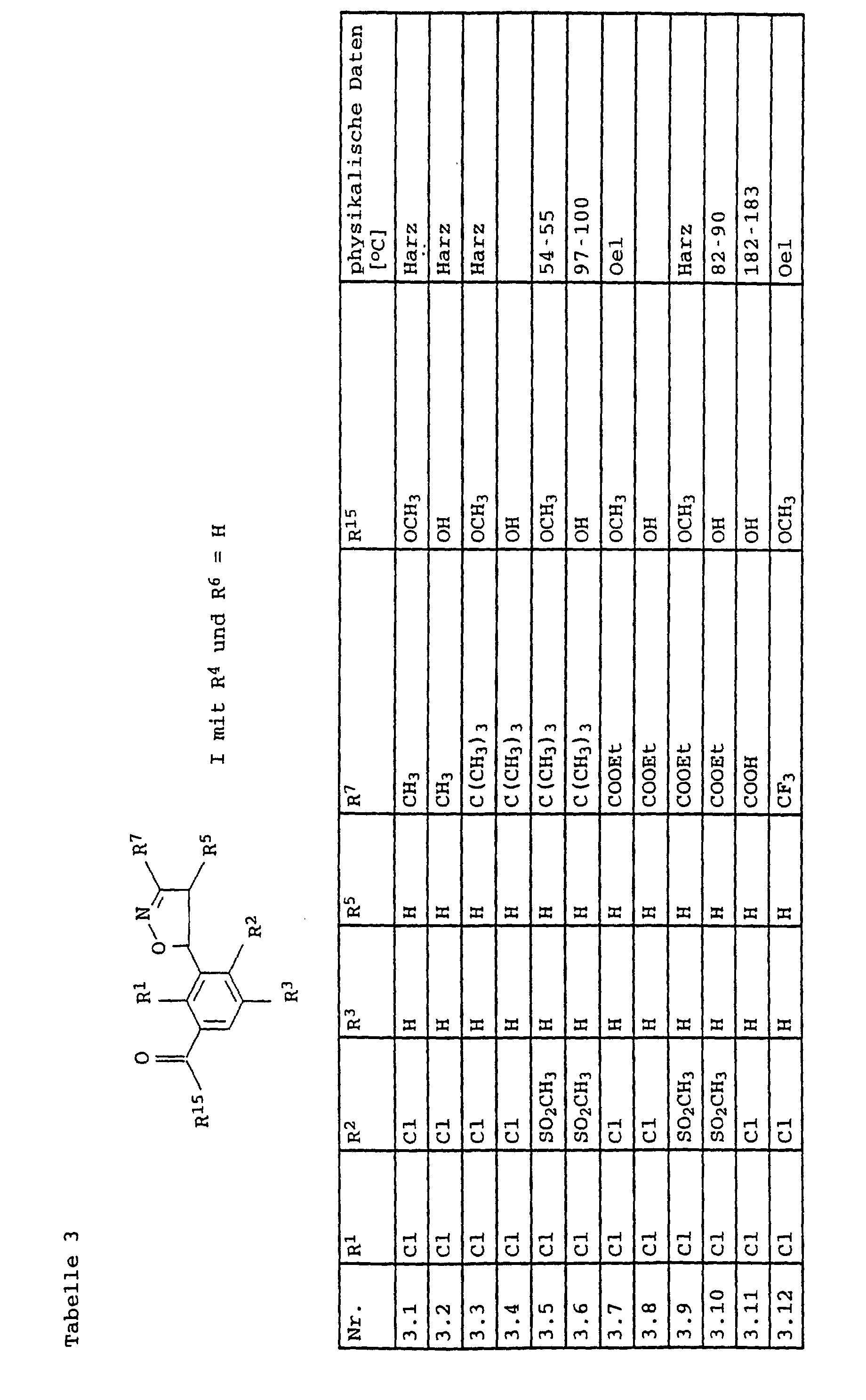

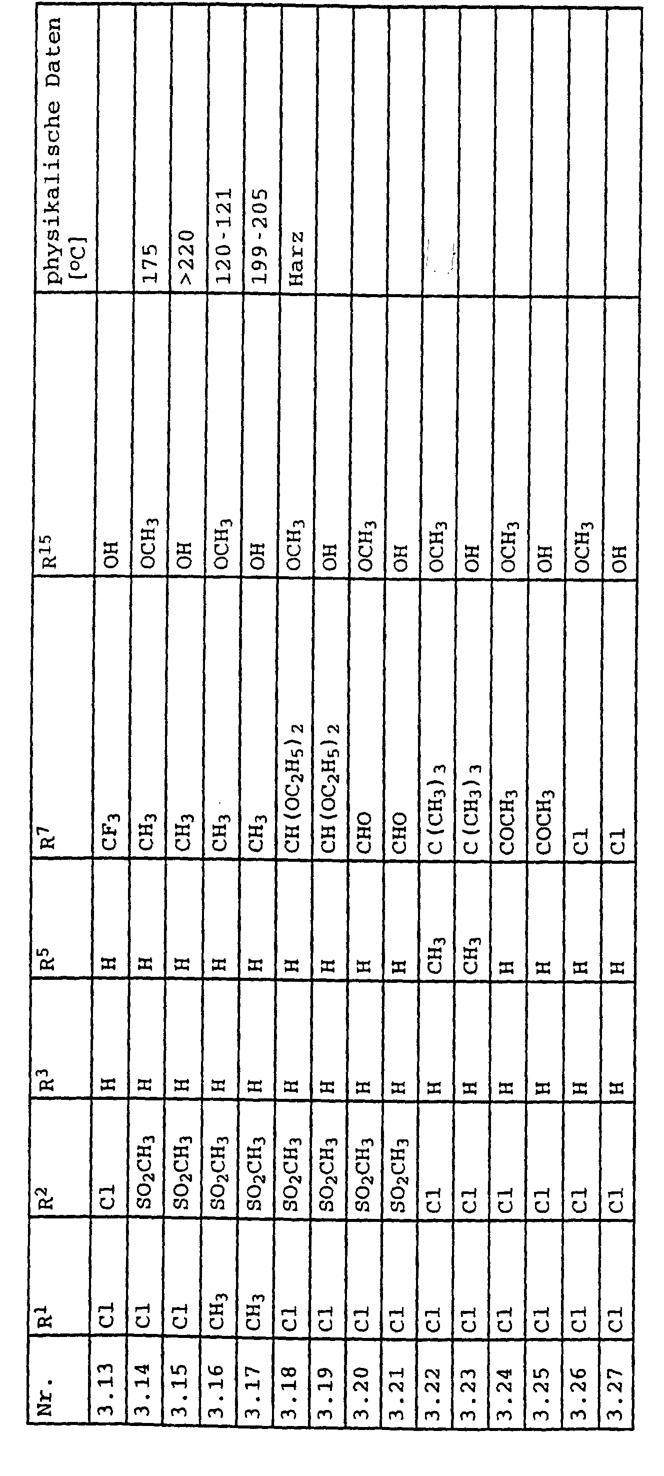

Die 3-(4,5-Dihydroisoxazol-5-yl)benzoylpyrazole der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die 3-(4,5-Dihydroisoxazol-5-yl)benzoylpyrazole als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. 2.7 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. 2.7 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. 2.7 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. 2.7 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile des Wirkstoffs Nr. 2.7 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.7 werden mit 2 Gewichtsteilen.Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. 2.7 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. 2.7 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die 3-(4,5-Dihydroisoxazol-5-yl)benzoylpyrazole der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der 3-(4,5-Dihydroisoxazol-5-yl)benzoylpyrazole der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 125 bzw. 62,5 g/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Avena fatua | Flughafer | wild oat |
| Bromus inermis | unbegrannte Trepse | brome |
| Matricaria chamomilla | Echte Kamille | wild chamomile |
| Matricaria inodora | geruchlose Kamille | false chamomile |
| tritricum aestivum | Sommerweizen | spring wheat |

Bei einer Aufwandmenge von 125 bzw. 62,5 g/ha a.S. zeigte die Verbindung 2.7 im Nachauflauf eine sehr gute Wirkung gegen die oben genannten Schadplanzen und Verträglichkeit in Sommerweizen.

Die aus WO 97/41118 bekannte Vergleichsverbindung A (Verbindung 20 aus Tabelle 1) führt dagegen bei gleichen Aufwandmengen zu Schäden in der Kulturpflanze Sommerweizen, sowie zu einer schwächeren herbiziden Aktivität gegenüber der genannten Schadpflanzen.

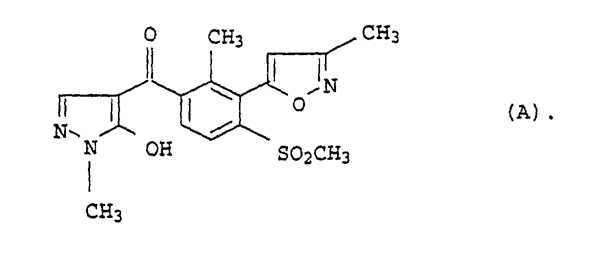

## Patentansprüche

1. 3 - (4,5-Dihydroisoxazol-5-yl)benzoylpyrazole der Formel I 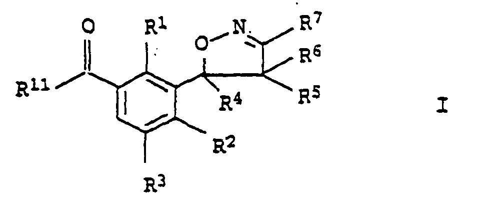 in der die Variablen folgende Bedeutungen haben:
R¹, R² Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
R³ Wasserstoff, Halogen oder C₁-C₆-Alkyl;
R⁴ Wasserstoff oder C₁-C₄-Alkyl;
R⁵, R⁶ Wasserstoff, Halogen, Cyano, Nitro, C₁-C₄-Alkyl, C₁-C₄-Alkoxy-C₁-C₄-alkyl, Di-(C₁-C₄-alkoxy)-C₁-C₄-alkyl, Di-(C₁-C₄-alkyl)-amino-C₁-C₄-alkyl, Di-(C₁-C₄-alkyl)-amino-imino-C₁-C₄-alkyl, Hydroxyimino-C₁-C₄-alkyl, C₁-C₆-Alkoxyimiao-C₁-C₄-alkyl, C₁-C₄-Alkoxycarbonyl-C₁-C₄-alkyl, C₁-C₄-Alkylthio-C₁-C₄-alkyl, C₁-C₄-Halogeaalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl)-amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy oder C₁-C₄-Halogenalkoxy;
oder
R⁵ und R⁶ bilden gemeinsam eine C₂-C₆-Alkandiyl-Kette, die ein- bis vierfach durch C₁-C₄-Alkyl substituiert sein kann und/oder durch Sauerstoff oder einen gegebenenfalls C₁-C₄-Alkyl substituierten Stickstoff unterbrochen sein kann;
R⁷ Halogen, Cyano, Hydroxy, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfoayl, C₁-C₆-Halogenalkylsulfonyl, Amino, C₁-C₆-Alkylamino, Di-(C₁-C₆-alkyl)amino, Di-(C₁-C₄-alkoxy)methyl, Hydroxyimino-C₁-C₄-alkyl, C₁-C₆-Alkoxyimino-C₁-C₄-alkyl oder COR⁸;
R⁸ Wasserstoff, C₁-C₄-Alkyl, C₁-C₄-Halogenalkyl, Hydroxy, C₁-C₄-Alkoxy, C₁-C₄-Alkoxy-C₂-C₄-alkoxy, C₁-C₄-Halogenalkoxy, C₃-C₆-Alkenyloxy, C₃-C₆-Alkinyloxy oder NR⁹R¹⁰;
R⁹ Wasserstoff oder C₁-C₄-Alkyl;
R¹⁰ C₁-C₄-Alkyl;
R¹¹ ein in 4-Stellung verknüpftes Pyrazol der Formel II 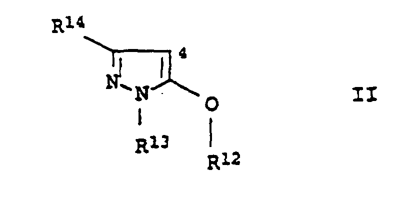 wobei
R¹² Wasserstoff, C₁-C₄-Alkyl, C₁-C₄-Halogenalkyl, C₃-C₄-Alkenyl, C₃-C₄-Alkinyl, C₁-C₄-Alkylcarbonyl, C₁-C₄-Halogenalkylcarbonyl, C₁-C₄-Alkylsulfonyl, C₁-C₄-Halogenalkylsulfonyl, Thienylmethyl, Phenyl, Benzyl, Phenylcarbonyl, Phenylsulfonyl oder Phenylcarbonylmethyl, wobei der Phenylrest der fünf letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;
R¹³ C₁-C₆-Alkyl, C₁-C₆-Halogenalkyl oder C₃-C₆-Cycloalkyl;
R¹⁴ Wasserstoff, C₁-C₆-Alkyl oder C₃-C₆-Cycloalkyl;
bedeuten;
sowie deren landwirtschaftlich brauchbaren Salze.

2. 3-(4,5-Dihydroisoxazol-5-yl)-benzoylpyrazole der Formel I nach Anspruch 1, wobei R³ Wasserstoff bedeutet.

3. 3-(4,5-Dihydroisoxazol-5-yl)-benzoylpyrazole der Formel I nach den Ansprüchen 1 oder 2, wobei
R¹,R² Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alyklsulfinyl, C₁-C₆-Halogenalkylsulfinyl, C₁-C₆-Alkylsulfonyl oder C₁-C₆-Halogenalkylsulfonyl;
bedeuten.

4. 3-(4,5-Dihydroisoxazol-5-yl)-benzoylpyrazole der Formel I nach den Ansprüchen 1 bis 3, wobei
R¹² für C₁-C₄-Alkylsulfonyl, C₁-C₄-Halogenalkylsulfonyl oder Phenylsulfonyl, wobei der Phenylrest partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;
steht.

5. 3-(4,5-Dihydroisoxazol-5-yl)-benzoylpyrazole der Formel I nach den Ansprüchen 1 bis 3, wobei
R¹² für Benzyl, wobei der Phenylrest partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;

6. 3-(9,5-Dihydroisoxazol-5-yl)-benzoylpyrazole der Formel I nach den Ansprüchen 1 bis 3, wobei R¹² für Wasserstoff steht.

7. 3-(4,5-Dihydroisoxazol-5-yl)-benzoylpyrazole der Formel I nach den Ansprüchen 1 bis 6, wobei
R⁵ Wasserstoff, Halogen, Cyano, Nitro, C₁-C₄-Alkyl, C₁-C₄-Alkoxy-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl)-amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy oder C₁-C₄-Halogenalkoxy;
R⁶ Wasserstoff oder C₁-C₄-Alkyl;

8. 3-(4,5-Dihydroisoxazol-5-yl)-benzoylpyrazole der Formel I nach den Ansprüchen 1 bis 7, wobei R⁵ und R⁶ für Wasserstoff stehen.

9. Verfahren zur Herstellung von 3-(4,5-Dihydroisoxazol-5-yl)-beazoylpyrazole der Formel I gemäß Anspruch 1, **dadurch gekennzeichnet, daß** man das Pyrazol der Formel II mit R¹² = H, wobei die Variablen R¹³ und R¹⁴ die unter Anspruch 1 genannten Bedeutungen haben, 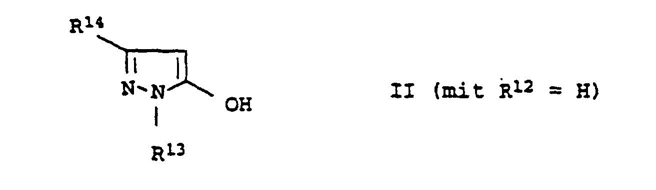 mit einer aktivierten Carbonsäure IIIα oder mit einer Carbonsäure IIIβ, 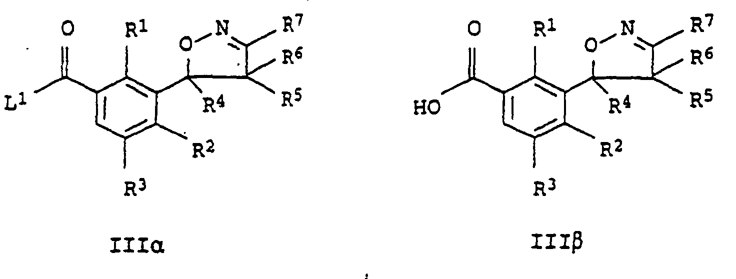 wobei die Variablen R¹ bis R⁷, die unter Anspruch 1 genannten Bedeutungen haben und L¹ für eine nucleophil verdrängbare Abgangsgruppe steht, acyliert und das Acylierungsprodukt, gegebenenfalls in Gegenwart eines Katalysators, zu den Verbindungen I (mit R¹² = H) umlagert und gewünschtenfalls zur Herstellung von 3-(4,5-Dihydroisoxazol-5-yl)-benzoylpyrazolen der Formel I mit R¹² ≠ Wasserstoff mit einer Verbindung der Formel V,
L² ― R¹² V
wobei R¹² die unter Anspruch 1 genannte Bedeutung hat mit Ausnahme von Wasserstoff und L² für eine nucleophil verdrängbare Abgangsgruppe steht, umsetzt.

10. 3-(4,5-Dihydroisoxazol-5-yl)-benzoylpyrazole der Formel III, 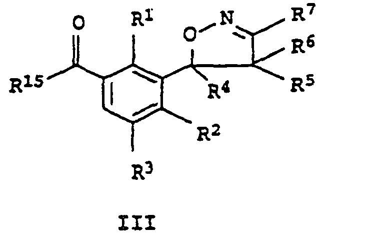 wobei R¹⁵ für Hydroxy oder einen abhydrolysierbaren Rest steht und die Variablen R¹ bis R⁷, die unter den Ansprüchen 1 bis 8 genannte Bedeutung haben.

11. 3-(4,5-Dihydroisoxazol-5-yl)-benzoylpyrazole der Formel III nach Anspruch 10, wobei .
R¹⁵ Halogen, Hydroxy oder C₁-C₆-Alkoxy;
bedeutet.

12. Verbindungen der Formel VI 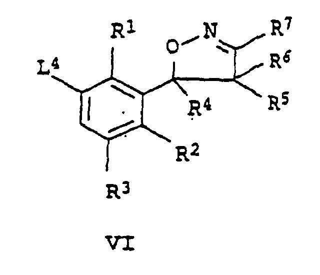 wobei
R¹, R² Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alyklsulfinyl, C₁-C₆-Halogeaalkylsulfinyl, C₁-C₆-Alkylsulfonyl oder C₁-C₆-Halogenalkylsulfonyl;
bedeuten;
und L⁴ für Halogen und die Variablen R³ bis R⁷ die unter den Ansprüchen 1 bis 8 genannte Bedeutung haben.

13. Verbindungen der Formel VII 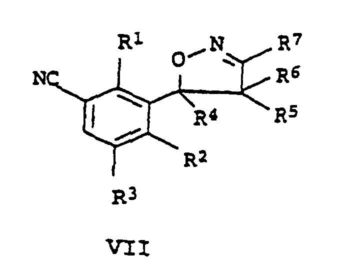 wobei die Variablen R¹ bis R⁷, die unter den Ansprüchen 1 bis 8 ;genannte Bedeutung haben.

14. Mittel, enthaltend eine herbizid wirksame Menge mindestens eines 3-(4,-5-Dihydroisoxazol-5-yl)-beazoylpyrazols der Formel I oder eines landwirtschaftlich brauchbaren Salzes von I gemäß den Ansprüchen 1 bis 8, und für die Formulierung von Pflanzenschutzmitteln übliche Hilfsmittel.

15. Verfahren zur Herstellung von Mitteln gemäß Anspruch 14, **dadurch gekennzeichnet, daß** man eine herbizid wirksame Menge mindestens eines 3-(4,5-Dihydroisoxazol-5-yl)-benzoylpyrazols der Formel I oder eines landwirtschaftlich brauchbaren Salzes von I gemäß den Ansprüchen 1 bis 8 und für die Formulierung von Pflanzenschutzmitteln übliche Hilfsmittel mischt.

16. Verfahren zur Bekämpfung von unerwünschtem Pflanzenwuchs, **dadurch gekennzeichnet, daß** man eine herbizid wirksame Menge mindestens eines 3-(4,5-Dihydroisoxazol-5-yl)-benzoylpyrazols der Formel I oder eines landwirtschaftlich brauchbaren Salzes von I gemäß den Ansprüchen 1 bis 8, auf Pflanzen, deren Lebensraum und/oder auf Samen einwirken läßt.

17. Verwendung der 3-(4,5-Dihydroisoxazol-5-yl)-benzoylpyrazole der Formel I und deren landwirtschaftlich brauchbaren Salze gemäß den Ansprüchen 1 bis 8 als Herbizide.

## Claims

1. A 3-(4,5-dihydroisoxazol-5-yl)benzoylpyrazole of the formula I 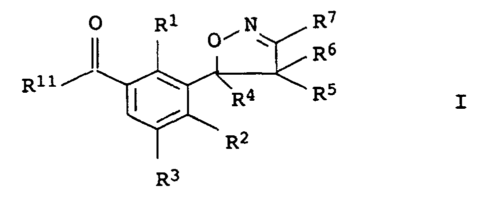 where:
R¹, R² are hydrogen, nitro, halogen, cyano, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl or C₁-C₆-haloalkylsulfonyl;
R³ is hydrogen, halogen or C₁-C₆-alkyl;
R⁴ is hydrogen or C₁-C₄-alkyl;
R⁵, R⁶ are hydrogen, halogen, cyano, nitro, C₁-C₄-alkyl, C₁-C₄-alkoxy-C₁-C₄-alkyl, di(C₁-C₄-alkoxy)-C₁-C₄-alkyl, di(C₁-C₄-alkyl)amino-C₁-C₄-alkyl, di(C₁-C₄-alkyl)aminoimino-C₁-C₄-alkyl, hydroxyimino-C₁-C₄-alkyl, C₁-C₆-alkoxyimino-C₁-C₄-alkyl, C₁-C₄-alkoxycarbonyl-C₁-C₄-alkyl, C₁-C₄-alkylthio-C₁-C₄-alkyl, C₁-C₄-haloalkyl, C₁-C₄-cyanoalkyl, C₃-C₈-cycloalkyl, C₁-C₄-alkoxy, C₁-C₄-alkoxy-C₂-C₄-alkoxy, C₁-C₄-haloalkoxy, C₁-C₄-alkylthio, C₁-C₄-haloalkylthio, di(C₁-C₄-alkyl)amino, COR⁸, phenyl or benzyl, where the two last-mentioned substituents may be partially or fully halogenated and/or may carry one to three of the following groups:
nitro, cyano, C₁-C₄-alkyl, C₁-C₄-haloalkyl, C₁-C₄-alkoxy or C₁-C₄-haloalkoxy;
or
R⁵ and R⁶ together form a C₂-C₆-alkanediyl chain which may be mono- to tetrasubstituted by C₁-C₄-alkyl and/or may be interrupted by oxygen or an unsubstituted or C₁-C₄-alkyl-substituted nitrogen;
R⁷ is halogen, cyano, hydroxyl, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, amino, C₁-C₆-alkylamino, di(C₁-C₆-alkyl)amino, di(C₁-C₄-alkoxy)methyl, hydroxyimino-C₁-C₄-alkyl, C₁-C₆-alkoxyimino-C₁-C₄-alkyl or COR⁸;
R⁸ is hydrogen, C₁-C₄-alkyl, C₁-C₄-haloalkyl, hydroxyl, C₁-C₄-alkoxy, C₁-C₄-alkoxy-C₂-C₄-alkoxy, C₁-C₄-haloalkoxy, C₃-C₆-alkenyloxy, C₃-C₆-alkynyloxy or NR⁹R¹⁰;
R⁹ is hydrogen or C₁-C₄-alkyl;
R¹⁰ is C₁-C₄-alkyl;
R¹¹ is a pyrazole, attached in the 4-position, of the formula II 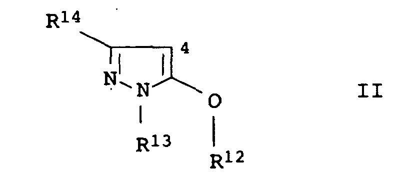 where
R¹² is hydrogen, C₁-C₄-alkyl, C₁-C₄-haloalkyl, C₃-C₄-alkenyl, C₃-C₄-alkynyl, C₁-C₄-alkylcarbonyl, C₁-C₄-haloalkylcarbonyl, C₁-C₄-alkylsulfonyl, C₁-C₄-haloalkylsulfonyl, thienylmethyl, phenyl, benzyl, phenylcarbonyl, phenylsulfonyl or phenylcarbonylmethyl, where the phenyl radical of the five last-mentioned substituents may be partially or fully halogenated and/or may carry one to three of the following groups:
nitro, cyano, C₁-C₄-alkyl, C₁-C₄-haloalkyl, C₁-C₄-alkoxy or C₁-C₄-haloalkoxy;
R¹³ is C₁-C₆-alkyl, C₁-C₆-haloalkyl or C₃-C₆-cycloalkyl;
R¹⁴ is hydrogen, C₁-C₆-alkyl or C₃-C₆-cycloalkyl;
and its agriculturally useful salts.

2. A 3-(4,5-dihydroisoxazol-5-yl)benzoylpyrazole of the formula I as claimed in claim 1 where R³ is hydrogen.

3. A 3-(4,5-dihydroisoxazol-5-yl)benzoylpyrazole of the formula I as claimed in claim 1 or 2 where
R¹, R² are nitro, halogen, cyano, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl or C₁-C₆-haloalkylsulfonyl.

4. A 3-(4,5-dihydroisoxazol-5-yl)benzoylpyrazole of the formula I as claimed in any of claims 1 to 3 where
R¹² is C₁-C₄-alkylsulfonyl, C₁-C₄-halosulfonyl or phenylsulfonyl, where the phenyl radical may be partially or fully halogenated and/or may carry one to three of the following groups:
nitro, cyano, C₁-C₄-alkyl, C₁-C₄-haloalkyl, C₁-C₄-alkoxy or C₁-C₄-haloalkoxy.

5. A 3-(4,5-dihydroisoxazol-5-yl)benzoylpyrazole of the formula I as claimed in any of claims 1 to 3 where
R¹² is benzyl, where the phenyl radical may be partially or fully halogenated and/or may carry one to three of the following groups:
nitro, cyano, C₁-C₄-alkyl, C₁-C₄-haloalkyl, C₁-C₄-alkoxy or C₁-C₄-haloalkoxy.

6. A 3-(4,5-dihydroisoxazol-5-yl)benzoylpyrazole of the formula I as claimed in any of claims 1 to 3 where R¹² is hydrogen.

7. A 3-(4,5-dihydroisoxazol-5-yl)benzoylpyrazole of the formula I as claimed in any of claims 1 to 6 where
R⁵ is hydrogen, halogen, cyano, nitro, C₁-C₄-alkyl, C₁-C₄-alkoxy-C₁-C₄-alkyl, C₁-C₄-alkoxycarbonyl-C₁-C₄-alkyl, C₁-C₄-alkylthio-C₁-C₄-alkyl, C₁-C₄-haloalkyl, C₁-C₄-cyanoalkyl, C₃-C₈-cycloalkyl, C₁-C₄-alkoxy, C₁-C₄-alkoxy-C₂-C₄-alkoxy, C₁-C₄-haloalkoxy, C₁-C₄-alkylthio, C₁-C₄-haloalkylthio, di(C₁-C₄-alkyl)amino, COR⁸, phenyl or benzyl, where the two last mentioned substituents may be partially or fully halogenated and/or may carry one to three of the following groups:
nitro, cyano, C₁-C₄-alkyl, C₁-C₄-haloalkyl, C₁-C₄-alkoxy or C₁-C₄-haloalkoxy;
R⁶ is hydrogen or C₁-C₄-alkyl.

8. A 3-(4,5-dihydroisoxazol-5-yl)benzoylpyrazole of the formula I as claimed in any of claims 1 to 7 where R⁵ and R⁶ are hydrogen.

9. A process for preparing 3-(4,5-dihydroisoxazol-5-yl)benzoylpyrazoles of the formula I as claimed in claim 1, which comprises acylating the pyrazole of the formula II where R¹² = H and where the variables R¹³ and R¹⁴ are as defined under claim 1 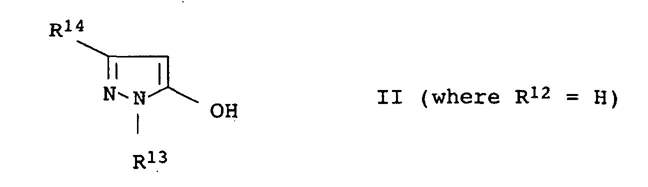 with an activated carboxylic acid IIIα or with a carboxylic acid IIIβ, 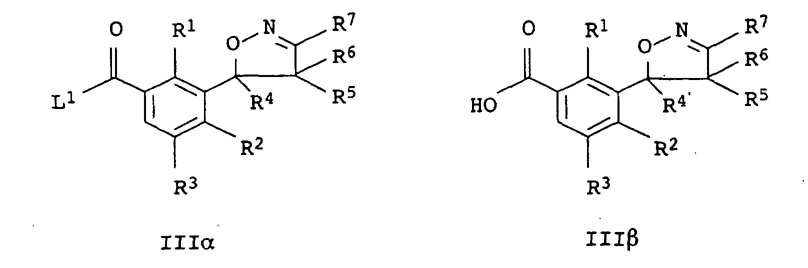 where the variables R¹ to R⁷ are as defined under claim 1 and L¹ is a nucleophilically displaceable leaving group and rearranging the acylation product, if appropriate in the presence of a catalyst, to the compounds I (where R¹² = H), followed, if desired, by reaction with a compound of the formula V
L²―R¹² V
where R¹² is as defined under claim 1 except for hydrogen and L² is a nucleophilically displaceable leaving group, to prepare 3-(4,5-dihydroisoxazol-5-yl)benzoylpyrazoles of the formula I where R¹² ≠ hydrogen.

10. A 3-(4,5-dihydroisoxazol-5-yl)benzoylpyrazole of the formula III, 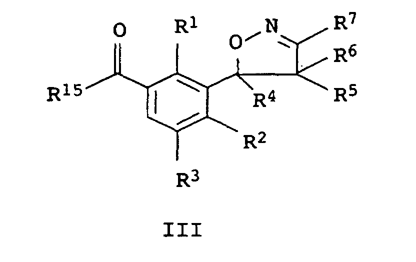 where R¹⁵ is hydroxyl or a radical that can be removed by hydrolysis and the variables R¹ to R⁷ are as defined under claims 1 to 8.

11. A 3-(4,5-dihydroisoxazol-5-yl)benzoylpyrazole of the formula III as claimed in claim 10 where
R¹⁵ is halogen, hydroxyl or C₁-C₆-alkoxy.

12. A compound of the formula VI 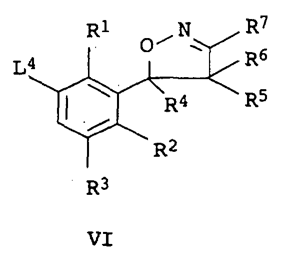 where
R¹, R² are nitro, halogen, cyano, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl or C₁-C₆-haloalkylsulfonyl;
and L⁴ is halogen and the variables R³ to R⁷ are as defined under claims 1 to 8.

13. A compound of the formula VII 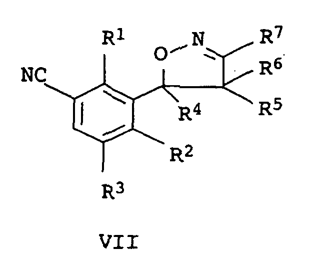 where the variables R¹ to R⁷ are as defined under claims 1 to 8.

14. A composition, comprising a herbicidally effective amount of at least one 3-(4,5-dihydroisoxazol-5-yl)benzoylpyrazole of the formula I or an agriculturally useful salt of I as claimed in any of claims 1 to 8 and auxiliaries which are customary for formulating crop protection agents.

15. A process for preparing compositions as claimed in claim 13, which comprises mixing a herbicidally effective amount of at least one 3-(4,5-dihydroisoxazol-5-yl)benzoylpyrazole of the formula I or an agriculturally useful salt of I as claimed in any of claims 1 to 8 and auxiliaries which are customary for formulating crop protection agents.

16. A method for controlling undesirable vegetation, wherein a herbicidally effective amount of at least one 3-(4,5-dihydroisoxazol-5-yl)benzoylpyrazole of the formula I or an agriculturally useful salt of I as claimed in any of claims 1 to 8 is allowed to act on plants, their habitat and/or seeds.

17. The use of the 3-(4,5-dihydroisoxazol-5-yl)benzoylpyrazoles of the formula I and their agriculturally useful salts as claimed in any of claims 1 to 8 as herbicides.

## Revendications

1. 3-(4,5-dihydroisoxazol-5-yl)benzoylpyrazoles répondant à la formule 1 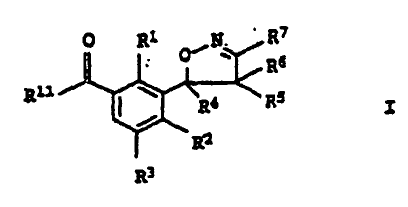 dans laquelle les variables possèdent les significations ci-après :
R¹, R² représentent un atome d'hydrogène, un groupe nitro, un atome d'halogène, un groupe cyano, un groupe alkyle en C₁-C₆, un groupe halogénalkyle en C₁-C₆, un groupe alcoxy en C₁-C₆, un groupe halogénalcoxy en C₁-C₆, un groupe (alkyle en C₁-C₆)thio, un groupe (halogénalkyle en C₁-C₆)thio, un groupe (alkyle en C₁-C₆)sulfinyle, un groupe (halogénalkyle en C₁-C₆)sulfinyle, un groupe (alkyle en C₁-C₆)sulfonyle ou un groupe (halogénalkyle en C₁-C₆)sulfonyle ;
R³ représente un atome d'hydrogène, un atome d'halogène ou un groupe alkyle en C₁-C₆ ;
R⁴ représente un atome d'hydrogène ou un groupe alkyle en C₁-C₄ ;
R⁵, R⁶ représentent un atome d'hydrogène, un atome d'halogène, un groupe cyano, un groupe nitro, un groupe alkyle en C₁-C₄, un groupe (alcoxy en C₁-C₄)(alkyle en C₁-C₄), un groupe di(alcoxy en C₁-C₄)(alkyle en C₁-C₄), un groupe di(alkyle en C₁-C₄)amino(alkyle en C₁-C₄), un groupe di(alkyle en C₁-C₄)aminoimino(alkyle en C₁-C₄), un groupe hydroxyimino(alkyle en C₁-C₄), un groupe (alcoxy en C₁-C₆)imino(alkyle en C₁-C₄), un groupe (alcoxy en C₁-C₄)carbonyl(alkyle en C₁-C₄), un groupe (alkyle en C₁-C₄)thio(alkyle en C₁-C₄), un groupe halogénalkyle en C₁-C₄, un groupe cyanoalkyle en C₁-C₄, un groupe cycloalkyle en C₃-C₈, un groupe alcoxy en C₁-C₄, un groupe (alcoxy en C₁-C₄)alcoxy en C₂-C₄, un groupe halogénalcoxy en C₁-C₄, un groupe (alkyle en C₁-C₄)thio, un groupe (halogénalkyle en C₁-C₄)thio, un groupe di(alkyle en C₁-C₄)amino, un groupe COR⁸, un groupe phényle ou un groupe benzyle, les deux substituants mentionnés en dernier lieu pouvant être partiellement ou complètement halogénés et/ou pouvant porter de 1 à 3 groupes parmi ceux repris ci-après : un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄ ;
ou
R⁵ et R⁶ forment ensemble une chaîne alcane(en C₂-C₆)diyle qui peut porter, à titre de substituants, de 1 à 4 groupes alkyle en C₁-C₄ et/ou qui peut être interrompu par un atome d'oxygène ou par un atome d'azote le cas échéant substitué par un groupe alkyle en C₁-C₄ ;
R⁷ représente un atome d'halogène, un groupe cyano, un groupe hydroxyle, un groupe alkyle en C₁-C₆, un groupe halogénalkyle en C₁-C₆, un groupe alcoxy en C₁-C₆, un groupe halogénalcoxy en C₁-C₆, un groupe (alkyle en C₁-C₆)thio, un groupe (halogénalkyle en C₁-C₆)thio, un groupe (alkyle en C₁-C₆)sulfinyle, un groupe (halogénalkyle en C₁-C₆)sulfinyle, un groupe (alkyle en C₁-C₆)sulfonyle, un groupe (halogénalkyle en C₁-C₆)sulfonyle, un groupe amino, un groupe (alkyle en C₁-C₆)amino, un groupe di(alkyle en C₁-C₆)amino, un groupe di(alcoxy en C₁-C₄)méthyle, un groupe hydroxyimino(alkyle en C₁-C₄), un groupe (alcoxy en C₁-C₆)imino(alkyle en C₁-C₄) ou un groupe COR⁸ ;
R⁸ représente un atome d'hydrogène, un groupe alkyle en C₁-C₄, un groupe halogénalkyle en C₁-C₄, un groupe hydroxyle, un groupe alcoxy en C₁-C₄, un groupe (alcoxy en C₁-C₄)(alcoxy en C₂-C₄), un groupe halogénalcoxy en C₁-C₄, un groupe (alcényle en C₃-C₆)oxy, un groupe (alcynyle en C₃-C₆)oxy ou un groupe NR⁹R¹⁰ ;
R⁹ représente un atome d'hydrogène ou un groupe alkyle en C₁-C₄ ;
R¹⁰ représente un groupe alkyle en C₁-C₄ ;
R¹¹ représente un noyau pyrazole lié en position 4, répondant à la formule II 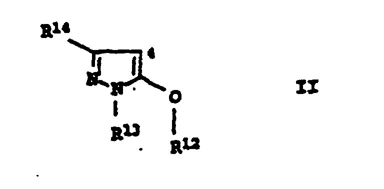 dans laquelle
R¹² représente un atome d'hydrogène, un groupe alkyle en C₁-C₄, un groupe halogénalkyle en C₁-C₄, un groupe alcényle en C₃-C₄, un groupe alcynyle en C₃-C₄, un groupe (alkyle en C₁-C₄)carbonyle, un groupe (halogénalkyle en C₁-C₄)carbonyle, un groupe (alkyle en C₁-C₄)sulfonyle, un groupe (halogénalkyle en C₁-C₄)sulfonyle, un groupe thiénylméthyle, un groupe phényle, un groupe benzyle, un groupe phénylcarbonyle, un groupe phénylsulfonyle ou un groupe phénylcarbonylméthyle, le radical phényle des cinq substituants mentionnés en dernier lieu pouvant être partiellement ou complètement halogénés et/ou pouvant porter de 1 à 3 groupes parmi ceux repris ci-après : un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄ ;
R¹³ représente un groupe alkyle en C₁-C₆, un groupe halogénalkyle en C₁-C₆ ou un groupe cycloalkyle en C₃-C₆ ;
R¹⁴ représente un atome d'hydrogène, un groupe alkyle en C₁-C₆ ou un groupe cycloalkyle en C₃-C₆ ;
ainsi que leurs sels utilisables en agriculture.

2. 3-(4,5-dihydroisoxazol-5-yl)benzoylpyrazoles répondant à la formule I selon la revendication 1, dans lesquels R³ représente un atome d'hydrogène.

3. 3-(4,5-dihydroisoxazol-5-yl)benzoylpyrazoles répondant à la formule I selon la revendication 1 ou 2, dans lesquels
R¹, R² représentent un groupe nitro, un atome d'halogène, un groupe cyano, un groupe alkyle en C₁-C₆, un groupe halogénalkyle en C₁-C₆, un groupe alcoxy en C₁-C₆, un groupe halogénalcoxy en C₁-C₆, un groupe (alkyle en C₁-C₆)thio, un groupe (halogénalkyle en C₁-C₆)thio, un groupe (alkyle en C₁-C₆)sulfinyle, un groupe (halogénalkyle en C₁-C₆)sulfinyle, un groupe (alkyle en C₁-C₆)sulfonyle ou un groupe (halogénalkyle en C₁-C₆)sulfonyle.

4. 3-(4,5-dihydroisoxazol-5-yl)benzoylpyrazoles répondant à la formule I selon les revendications 1 à 3, dans lesquels
R¹² représente un groupe (alkyle en C₁-C₄)sulfonyle, un groupe (halogénalkyle en C₁-C₄)sulfonyle ou un groupe phénylsulfonyle, le radical phényle pouvant être partiellement ou complètement halogénés et/ou pouvant porter de 1 à 3 groupes parmi ceux repris ci-après : un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄.

5. 3-(4,5-dihydroisoxazol-5-yl)benzoylpyrazoles répondant à la formule I selon les revendications 1 à 3, dans lesquels
R¹² représente un groupe benzyle, le radical phényle pouvant être partiellement ou complètement halogénés et/ou pouvant porter de 1 à 3 groupes parmi ceux repris ci-après : un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄.

6. 3-(4,5-dihydroisoxazol-5-yl)benzoylpyrazoles répondant à la formule I selon les revendications 1 à 3, dans lesquels R¹² représente un atome d'hydrogène.

7. 3-(4,5-dihydroisoxazol-5-yl)benzoylpyrazoles répondant à la formule I selon les revendications 1 à 6, dans lesquels
R⁵ représente un atome d'hydrogène, un atome d'halogène, un groupe cyano, un groupe nitro, un groupe alkyle en C₁-C₄, un groupe (alcoxy en C₁-C₄)(alkyle en C₁-C₄), un groupe (alcoxy en C₁-C₄)carbonyl(alkyle en C₁-C₄), un groupe (alkyle en C₁-C₄)thio(alkyle en C₁-C₄), un groupe halogénalkyle en C₁-C₄, un groupe cyanoalkyle en C₁-C₄, un groupe cycloalkyle en C₃-C₈, un groupe alcoxy en C₁-C₄, un groupe (alcoxy en C₁-C₄)(alcoxy en C₂-C₄), un groupe halogénalcoxy en C₁-C₄, un groupe (alkyle en C₁-C₄)thio, un groupe (halogénalkyle en C₁-C₄)thio, un groupe di(alkyle en C₁-C₄)amino, un groupe COR⁸, un groupe phényle ou un groupe benzyle, les deux substituants mentionnés en dernier lieu pouvant être partiellement ou complètement halogénés et/ou pouvant porter de 1 à 3 groupes parmi ceux repris ci-après : un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄ ;
R⁶ représente un atome d'hydrogène ou un groupe alkyle en C₁-C₄.

8. 3-(4,5-dihydroisoxazol-5-yl)benzoylpyrazoles répondant à la formule I selon les revendications 1 à 7, dans lesquels R⁵ et R⁶ représentent un atome d'hydrogène.

9. Procédé pour la préparation de 3-(4,5-dihydroisoxazol-5-yl)-benzoylpyrazoles répondant à la formule I selon la revendication 1, **caractérisé en ce qu'**on soumet à une acylation le pyrazole répondant à la formule II dans laquelle R¹² représente un atome d'hydrogène, les variables R¹³ et R¹⁴ possédant les significations mentionnées à la revendication 1, 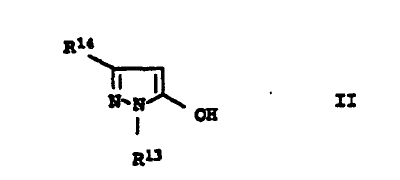 (dans laquelle R¹² représente un atome d'hydrogène)
avec un acide carboxylique activé IIIα ou avec un acide carboxylique IIIβ, 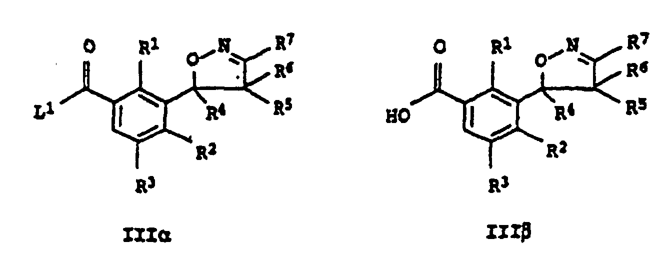 dans lesquelles les variables R¹ à R⁷ ont les significations mentionnées à la revendication 1, et L¹ représente un groupe sortant apte à un déplacement nucléophile, et on soumet le produit d'acylation à une transposition, le cas échéant en présence d'un catalyseur, pour obtenir les composés I (dans lesquels R¹² représente un atome d'hydrogène) et, si on le souhaite, pour la préparation des 3-(4,5-dihydroisoxazol-5-yl)-benzoylpyrazoles répondant à la formule I dans lesquels R¹² ne représente pas un atome d'hydrogène, on le fait réagir avec un composé répondant à la formule V
L²―R¹² V
dans laquelle R¹² a la signification indiquée à la revendication 1, avec cette exception qu'il ne représente pas un atome d'hydrogène, et L² représente un groupe sortant apte à un déplacement nucléophile.

10. 3-(4,5-dihydroisoxazol-5-yl)benzoylpyrazoles répondant à la formule III, 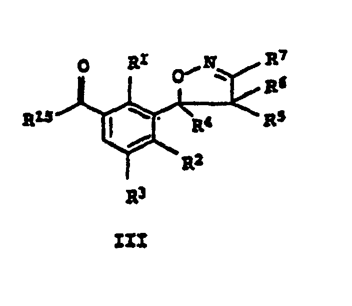 dans laquelle R¹⁵ représente un groupe hydroxyle ou un radical apte à être éliminé par hydrolyse et les variables R¹ à R⁷ possèdent la signification mentionnée aux revendications 1 à 8.

11. 3-(4,5-dihydroisoxazol-5-yl)benzoylpyrazoles répondant à la formule III, selon la revendication 10, dans lesquels
R¹⁵ représente un atome d'halogène, un groupe hydroxyle ou un groupe alcoxy en C₁-C₆.

12. Composés répondant à la formule VI 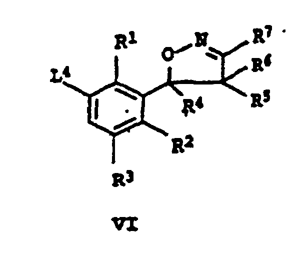 dans laquelle
R¹, R² représentent un groupe nitro, un atome d'halogène, un groupe cyano, un groupe alkyle en C₁-C₆, un groupe halogénalkyle en C₁-C₆, un groupe alcoxy en C₁-C₆, un groupe halogénalcoxy en C₁-C₆, un groupe (alkyle en C₁-C₆)thio, un groupe (halogénalkyle en C₁-C₆)thio, un groupe (alkyle en C₁-C₆)sulfinyle, un groupe (halogénalkyle en C₁-C₆)sulfinyle, un groupe (alkyle en C₁-C₆)sulfonyle ou un groupe (halogénalkyle en C₁-C₆)sulfonyle ;
et L⁴ représente un atome d'halogène et les variables R³ à R⁷ possèdent la signification mentionnée aux revendications 1 à 8.

13. Composés répondant à la formule VII 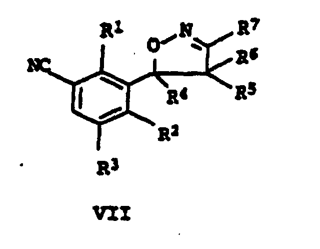 dans laquelle les variables R¹ à R⁷ possèdent la signification mentionnée aux revendications 1 à 8.

14. Agent contenant une quantité efficace comme herbicide d'au moins un 3-(4,5-dihydroisoxazol-5-yl)benzoylpyrazole répondant à la formule I ou d'un sel de I utilisable en agriculture selon les revendications 1 à 8 et des adjuvants habituels pour la formulation d'agents de protection des plantes.

15. Procédé pour la préparation d'agents selon la revendication 13, **caractérisé en ce qu'**on mélange une quantité efficace comme herbicide d'au moins un 3-(4,5-dihydroisoxazol-5-yl)benzoylpyrazole répondant à la formule I ou d'un sel de I utilisable en agriculture selon les revendications 1 à 8 et des adjuvants habituels pour la formulation d'agents de protection des plantes.

16. Procédé pour lutter pour la croissance non désirée de plantes, **caractérisé en ce qu'**on laisse agir une quantité efficace comme herbicide d'au moins un 3-(4,5-dihydroisoxazol-5-yl)benzoylpyrazole répondant à la formule I ou d'un sel de I utilisable en agriculture selon les revendications 1 à 8, sur des plantes, sur leur habitat et/ou sur des semences.

17. Utilisation des 3-(4,5-dihydroisoxazol-5-yl)benzoylpyrazoles répondant à la formule I ou de leurs sels utilisables en agriculture selon les revendications 1 à 8, à titre d'herbicides.
